# EUROPEAN PATENT APPLICATION

(11) **EP 4 821 916 A1**
(43) Date of publication of application: **14.10.2026**
(21) Application number: 25169217.4
(22) Date of filing: 08.04.2025
(51) Int. Cl.: C07C 233/47, C07C 233/63, C07C 271/38, C07C 275/30, C07C 275/62

(54) **SUBSTITUTED FORMYL TERTIARY ALCOHOL DERIVATIVES AND THEIR USE AS HERBICIDALLY ACTIVE SUBSTANCES**

(71) Applicant: Bayer Aktiengesellschaft, 51373 Leverkusen (DE)
(72) Inventor: The designation of the inventor has not yet been filed
(74) Representative: BIP Patents

(57) **Abstract**

The present invention relates to substituted formyl tertiary alcohol derivatives of the general formula (I), salts or N-oxides thereof, 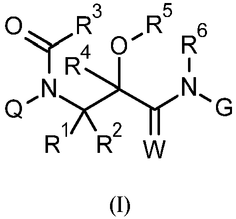 where the radicals in the general formula (I) correspond to the definitions given in the description, and to their use as herbicides, in particular for controlling broad-leaved weeds and/or weed grasses in crops of useful plants and/or as plant growth regulators for influencing the growth of crops of useful plants.

## Description

The invention relates to the technical field of herbicides and/or plant growth regulators. Specifically, the invention primarily relates to substituted formyl tertiary alcohol derivatives, salts or N-oxides thereof, and compositions comprising said substituted formyl tertiary alcohol derivatives. In addition, the present invention relates to processes for the preparation of said substituted formyl tertiary alcohol derivatives and their use as herbicides and/or plant growth regulators.

The prior art discloses several substituted formyl tertiary alcohol derivatives that display beneficial properties and uses. The patent WO 2011/35332 describes the use of these types of compounds as chemerin-R modulators.

WO 2021/173721 illustrates that substituted formyl tertiary alcohol derivatives are useful as androgen receptor degrader ligands. In addition, WO 2022/206350 discloses that substituted formyl tertiary alcohol derivatives inhibit the pharmaceutical relevant target MetAP2.

Several documents such as WO 2018/228985, WO 2018/228986, WO 2021/170464, WO 2022/268563 and WO 2024/197109 show that compounds that are related to the described substituted formyl tertiary alcohol derivatives bearing similar amido side chains are very effective at controlling unwanted vegetation in crops of useful plants.

However, substituted formyl tertiary alcohol derivatives, salts or N-oxides thereof bearing substituted amido side chains have not been previously reported. Surprisingly, it has now been found that substituted formyl tertiary alcohol derivatives, salts or N-oxides thereof bearing substituted amido side chains are particularly suitable as herbicides.

In their application, herbicides that are known to date for controlling harmful plants in crops of useful plants or herbicides for controlling unwanted vegetation sometimes have disadvantages, be it (a) that they have no or insufficient herbicidal activity against particularly harmful plants, (b) that the spectrum of harmful plants which can be controlled with an active compound is not broad enough, and/or (c) that the selectivity of the herbicides in and their compatibility with crop plants is too low, thereby causing unwanted damage and/or unwanted reduced harvest yields of the crops and/or (d) that they have a toxicologically unfavourable profile.

Furthermore, some active compounds which can be used as plant growth regulators for a number of useful plants cause an unwanted reduction of harvest yields in other useful plants or are not compatible with the crop plant, or only within a narrow application rate range. Additionally, some of the known active compounds cannot be produced economically on an industrial scale owing to precursors and reagents which are difficult to obtain, or that they have only insufficient chemical stabilities.

Thus, there is still a need for alternative herbicides, in particular highly active herbicides that are useful at low application rates and/or having good compatibility with crop plants, for the selective application in plant crops or use on non-crop land. It is also desirable to provide alternative chemical active compounds which may be used in an advantageous manner as herbicides or plant growth regulators.

It is therefore an objective of the present invention to provide compounds having herbicidal activity which are highly effective against economically important harmful plants even at relatively low application rates and that can be used selectively in one or more crop plants.

It has now been found that the compounds of the following formula (I), salts or N-oxides thereof meet said objective(s).

The present invention accordingly provides substituted formyl tertiary alcohol derivatives of the general formula (I), salts or N-oxides thereof, 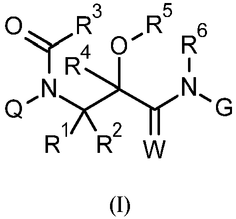 in which
- Q: represents an aryl or heteroaryl residue, wherein each of the previously mentioned two residues is unsubstituted or is independently substituted by one or more residues selected from the group R⁷,
- R¹ and R²: represent independently of one another hydrogen, halogen, cyano, (C₁-C₈)-alkyl, (C₁-C₈)-alkoxy or, together with the carbon atom to which they are attached form a (C₃-C₈)-cycloalkyl or (C₃-C₈)-cycloalkoxy ring system,
- R³: represents hydrogen, amino, (C₁-C₈)-alkyl, (C₁-C₈)-haloalkyl, (C₂-C₈)-alkenyl, (C₂-C₈)-haloalkenyl, (C₂-C₈)-alkynyl, (C₂-C₈)-haloalkynyl, (C₁-C₈)-alkoxy, (C₁-C₈)-haloalkoxy, (C₁-C₈)-alkoxy-(C₁-C₈)-alkyl, (C₁-C₈)-haloalkoxy-(C₁-C₈)-alkyl, (C₁-C₈)-alkoxy-(C₃-C₈)-cycloalkyl, (C₁-C₈)-haloalkoxy-(C₃-C₈)-cycloalkyl, (C₃-C₈)-cycloalkyl, (C₃-C₈)-halocycloalkyl, (C₃-C₈)- cycloalkoxy, (C₃-C₈)-halocycloalkoxy, (C₁-C₈)-alkylamino, (C₂-C₁₂)-dialkylamino, amino-C(O)NR⁹R¹⁰, (C₁-C₈)-alkylamino-C(O)NR⁹R¹⁰, or represents phenoxy, unsubstituted or independently substituted by one to five residues selected from halogen, (C₁-C₈)-alkyl, (C₁-C₈)-haloalkyl, (C₁-C₈)-alkoxy, (C₁-C₈)-haloalkoxy or (C₃-C₈)-cycloalkyl,
- R⁴: represents hydrogen, halogen, cyano, (C₁-C₈)-alkyl, (C₁-C₈)-haloalkyl, (C₂-C₈)-alkenyl, (C₂-C₈)-haloalkenyl, (C₂-C₈)-alkynyl, (C₂-C₈)-haloalkynyl, (C₁-C₈)-alkoxy, (C₁-C₈)-haloalkoxy, (C₃-C₈)-cycloalkyl or (C₃-C₈)-halocycloalkyl,
- R⁵: represents hydrogen, (C₁-C₈)-alkyl, (C₁-C₈)-haloalkyl, (C₂-C₈)-alkenyl, (C₂-C₈)-haloalkenyl, (C₂-C₈)-alkynyl, (C₂-C₈)-haloalkynyl, (C₃-C₈)-cycloalkyl, (C₃-C₈)-halocycloalkyl, -C(O)R⁸, - C(O)OR⁸, -S(O)OR⁸, -CONR⁹R¹⁰ or -S(O)₂NR⁹R¹⁰, or represents phenyl-(C₁-C₈)-carbonyl, unsubstituted or independently substituted by one to five residues selected from halogen, cyano, (C₁-C₈)-alkyl, (C₁-C₈)-haloalkyl, (C₁-C₈)-alkoxy, (C₁-C₈)-haloalkoxy or (C₃-C₈)-cycloalkyl,
- R⁶: represents hydrogen, (C₁-C₈)-alkyl, (C₁-C₈)-haloalkyl, (C₁-C₈)-hydroxyalkyl, (C₁-C₈)-hydroxyhaloalkyl, (C₃-C₈)-cycloalkyl, (C₃-C₈)-halocycloalkyl, (C₁-C₈)-cyanoalkyl, (C₃-C₈)-cycloalkyl-(C₁-C₈)-alkyl, (C₂-C₈)-alkenyl, (C₂-C₈)-haloalkenyl, (C₂-C₈)-alkynyl, (C₂-C₈)-halo alkynyl, (C₁-C₈)-alkoxy, (C₁-C₈)-haloalkoxy, (C₁-C₈)-alkylcarbonyl, arylcarbonyl, (C₁-C₈)-alkyloxycarbonyl or aryloxycarbonyl,
- R⁷: represents halogen, nitro, cyano, (C₁-C₈)-alkyl, (C₁-C₈)-haloalkyl, (C₂-C₈)-alkenyl, (C₂-C₈)-haloalkenyl, (C₂-C₈)-alkynyl, (C₂-C₈)-haloalkynyl, (C₁-C₈)-alkoxy, (C₁-C₈)-haloalkoxy, (C₁-C₈)-alkylthio, (C₁-C₈)-haloalkylthio, (C₁-C₈)-alkylsulfinyl, (C₁-C₈)-haloalkylsulfinyl, (C₁-C₈)-alkylsulfonyl, (C₁-C₈)-haloalkylsulfonyl, (C₃-C₈)-cycloalkyl, (C₃-C₈)-halocycloalkyl, (C₃-C₈)-cycloalkoxy, (C₃-C₈)-halocycloalkoxy, (C₃-C₈)-cycloalkylthio, (C₃-C₈)-halocycloalkylthio, (C₃-C₈)-cycloalkylsulfinyl, (C₃-C₈)-halocycloalkylsulfinyl, (C₃-C₈)-cycloalkylsulfonyl, (C₃-C₈)-halocycloalkylsulfonyl, (C₃-C₈)-cycloalkyl-(C₁-C₈)-alkyl or (C₁-C₈)-alkyl-(C₃-C₈)-cycloalkyl
- W: represents oxygen or sulfur,
- G: represents a residue selected from G1-G25 shown in the table below, wherein the selected residue is substituted by a residue selected from the group Y,

| | | | | |
|---|---|---|---|---|
| 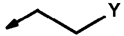 | 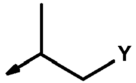 | 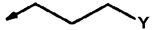 | 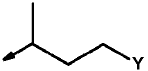 | 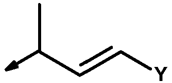 |
| G-1 | G-2 | G-3 | G-4 | G-5 |
| 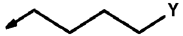 | 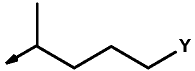 | 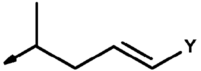 | 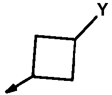 | 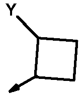 |
| G-6 | G-7 | G-8 | G-9 | G-10 |
| 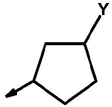 | 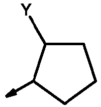 | 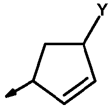 | 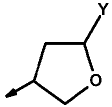 | 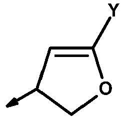 |
| G-11 | G-12 | G-13 | G-14 | G-15 |
| 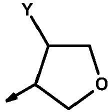 | 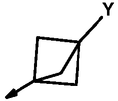 | 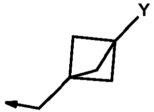 | 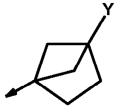 | 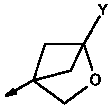 |
| G-16 | G-17 | G-18 | G-19 | G-20 |
| 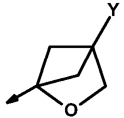 | 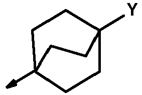 | 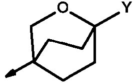 | 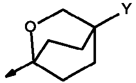 | 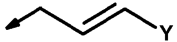 |
| G-21 | G-22 | G-23 | G-24 | G-25 |

where the arrow represents a bond to the nitrogen atom of formula (I)
- Y: represents -C(O)OR⁸, -C(O)NR⁹R¹⁰, -C(S)NR⁹R¹⁰, -C(O)N(R⁹)S(O)₂R¹¹ or - C(O)N(R⁹)S(O)₂NR⁹R¹⁰,
- R⁸, R⁹, R¹⁰ and R¹¹: represent the same or different substituents selected independently from eachother from hydrogen, (C₁-C₈)-alkyl, (C₂-C₈)-alkenyl, (C₂-C₈)-alkynyl, (C₁-C₈)-cyanoalkyl, (C₁-C₈)-haloalkyl, 0(C₂-C₈)-haloalkenyl, (C₂-C₈)-haloalkynyl, (C₃-C₈)-cycloalkyl, (C₃-C₈)-halocycloalkyl, (C₄-C₈)-cycloalkenyl, (C₄-C₈)-halocycloalkenyl, (C₁-C₈)-alkoxy, (C₁-C₈)-haloalkoxy, (C₁-C₈)-alkoxy-(C₁-C₈)-alkyl, (C₁-C₈)-haloalkoxy-(C₁-C₈)-alkyl, (C₁-C₈)-alkylthio-(C₁-C₈)-alkyl, (C₁-C₈)-alkylS(O)ₓ-(C₁-C₈)-alkyl, (C₁-C₈)-haloalkylthio-(C₁-C₈)-alkyl, (C₁-C₈)-alkoxy-(C₁-C₈)-haloalkyl, aryl, aryl-(C₁-C₈)-alkyl, heteroaryl, heteroaryl-(C₁-C₈)-alkyl, (C₃-C₈)-cycloalkyl-(C₁-C₄)-alkyl, (C₄-C₈)-cycloalkenyl-(C₁-C₈)-alkyl, heterocyclyl, bis-[(C₁-C₈)-alkyl]aminocarbonyl-(C₁-C₈)-alkyl, (C₁-C₈)-alkyl-amino-carbonyl-(C₁-C₈)-alkyl, aryl-(C₁-C₈)-alkyl-aminocarbonyl-(C₁-C₈)-alkyl or heterocyclyl-(C₁-C₈)-alkyl,
- R⁹ and R¹⁰: can represent, with the nitrogen atom to which they are attached, a fully saturated or partially saturated, optionally interrupted by heteroatoms and optionally further substituted 3- to 10-membered monocyclic or bicyclic ring,
and
- x: represents 0, 1 or 2.

The compounds of the general formula (I) can form salts by addition of a suitable inorganic or organic acid, for example mineral acids, for example HCl, HBr, H₂SO₄, H₃PO₄ or HNO₃, or organic acids, for example carboxylic acids such as formic acid, acetic acid, propionic acid, oxalic acid, lactic acid or salicylic acid or sulfonic acids, for example p-toluenesulfonic acid, onto a basic group, for example amino, alkylamino, dialkylamino, piperidino, morpholino or pyridino. In such a case, these salts will comprise the conjugated base of the acid as the anion. Suitable substituents in deprotonated form, for example sulfonic acids, particular sulfonamides or carboxylic acids, are capable of forming internal salts with groups, such as amino groups, which are themselves protonatable. Salts may also be formed by action of a base on compounds of the general formula (I). Suitable bases are, for example, organic amines such as trialkylamines, morpholine, piperidine and pyridine, and the hydroxides, carbonates and bicarbonates of ammonium, alkali metals or alkaline earth metals, especially sodium hydroxide, potassium hydroxide, sodium carbonate, potassium carbonate, sodium bicarbonate and potassium bicarbonate. These salts are compounds in which the acidic hydrogen is replaced by an agriculturally suitable cation, for example metal salts, especially alkali metal salts or alkaline earth metal salts, in particular sodium and potassium salts, or else ammonium salts, salts with organic amines or quaternary ammonium salts, for example with cations of the formula [NR^{a}R^{b}R^{c}R^{d}]⁺ in which R^{a} to R^{d} are each independently an organic radical, especially alkyl, aryl, arylalkyl or alkylaryl. Also suitable are alkylsulfonium and alkylsulfoxonium salts, such as (C₁-C₄)-trialkylsulfonium and (C₁-C₄)-trialkylsulfoxonium salts.

The substituted formyl tertiary alcohol derivatives of the general formula (I) according to the invention can, depending on external conditions such as pH, solvent and temperature, be present in various tautomeric structures, all of which are embraced by the general formula (I).

The compounds of the formula (I) used in accordance with the invention and salts or N-oxides thereof are also referred to hereinafter as "compounds of the general formula (I)".

The invention preferably provides compounds of the general formula (I), salts or N-oxides thereof in which
- Q: represents an aryl or heteroaryl residue, wherein each of the previously mentioned two residues is unsubstituted or is independently substituted by one or more residues selected from the group R⁷,
- R¹ and R²: represent independently of one another hydrogen, halogen, cyano, (C₁-C₄)-alkyl, (C₁-C₄)-alkoxy or, together with the carbon atom to which they are attached form a (C₃-C₆)-cycloalkyl or (C₃-C₆)-cycloalkoxy ring system,
- R³: represents hydrogen, amino, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₁-C₄)-alkoxy, (C₁-C₄)-haloalkoxy, (C₁-C₄)-alkoxy-(C₁-C₄)-alkyl, (C₁-C₄)-haloalkoxy-(C₁-C₄)-alkyl, (C₁-C₄)-alkoxy-(C₃-C₆)-cycloalkyl, (C₁-C₄)-haloalkoxy-(C₃-C₆)-cycloalkyl, (C₃-C₆)-cycloalkyl, (C₃-C₆)-halocycloalkyl, (C₃-C₆)-cycloalkoxy, (C₃-C₆)-halocycloalkoxy, (C₁-C₄)-alkylamino, (C₂-C₈)-dialkylamino, amino-C(O)NR⁹R10, (C₁-C₄)-alkylamino-C(O)NR⁹R¹⁰, or represents phenoxy, unsubstituted or independently substituted by one to five residues selected from halogen, (C₁-C₄)-alkyl, (C₁-C₄)-haloalkyl, (C₁-C₄)-alkoxy, (C₁-C₄)-haloalkoxy or (C₃-C₆)-cycloalkyl,
- R⁴: represents hydrogen, halogen, cyano, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₁-C₄)-alkoxy, (C₁-C₄)-haloalkoxy, (C₃-C₆)-cycloalkyl or (C₃-C₆)-halocycloalkyl,
- R⁵: represents hydrogen, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₃-C₆)-cycloalkyl, (C₃-C₆)-halocycloalkyl, -C(O)R⁸, - C(O)OR⁸, -S(O)OR⁸, -CONR⁹R¹⁰ or -S(O)₂NR⁹R¹⁰, or represents phenyl-(C₁-C₄)-carbonyl, unsubstituted or independently substituted by one to five residues selected from halogen, cyano, (C₁-C₄)-alkyl, (C₁-C₄)-haloalkyl, (C₁-C₄)-alkoxy, (C₁-C₄)-haloalkoxy or (C₃-C₆)-cycloalkyl,
- R⁶: represents hydrogen, (C₁-C₄)-alkyl, (C₁-C₄)-haloalkyl, (C₁-C₄)-hydroxyalkyl, (C₁-C₄)-hydroxyhaloalkyl, (C₃-C₆)-cycloalkyl, (C₃-C₆)-halocycloalkyl, (C₁-C₄)-cyanoalkyl, (C₃-C₆)-cycloalkyl-(C₁-C₄)-alkyl, (C₂-C₄)-alkenyl, (C₂-C₄)-haloalkenyl, (C₂-C₄)-alkynyl, (C₂-C₄)-halo alkynyl, (C₁-C₄)-alkoxy, (C₁-C₄)-haloalkoxy, (C₁-C₄)-alkylcarbonyl, arylcarbonyl, (C₁-C₄)-alkyloxycarbonyl or aryloxycarbonyl,
- R⁷: represents halogen, nitro, cyano, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₁-C₄)-alkoxy, (C₁-C₄)-haloalkoxy, (C₁-C₄)-alkylthio, (C₁-C₄)-haloalkylthio, (C₁-C₄)-alkylsulfinyl, (C₁-C₄)-haloalkylsulfinyl, (C₁-C₄)-alkylsulfonyl, (C₁-C₄)-haloalkylsulfonyl, (C₃-C₆)-cycloalkyl, (C₃-C₆)-halocycloalkyl, (C₃-C₆)-cycloalkoxy, (C₃-C₆)-halocycloalkoxy, (C₃-C₆)-cycloalkylthio, (C₃-C₆)-halocycloalkylthio, (C₃- C₆)-cycloalkylsulfinyl, (C₃-C₆)-halocycloalkylsulfinyl, (C₃-C₆)-cycloalkylsulfonyl, (C₃-C₆)-halocycloalkylsulfonyl, (C₃-C₆)-cycloalkyl-(C₁-C₄)-alkyl or (C₁-C₄)-alkyl-(C₃-C₆)-cycloalkyl,
- W: represents oxygen or sulfur,
- G: represents a residue selected from G1-G25 shown in the table below, wherein the selected residue is substituted by a residue selected from the group Y,

| | | | | |
|---|---|---|---|---|
| 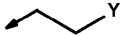 | 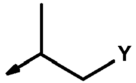 | 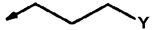 | 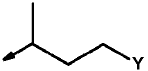 | 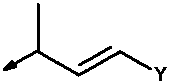 |
| G-1 | G-2 | G-3 | G-4 | G-5 |
| 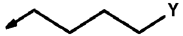 | 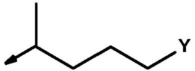 | 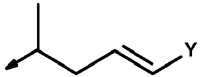 | 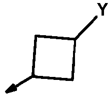 | 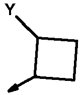 |
| G-6 | G-7 | G-8 | G-9 | G-10 |
| 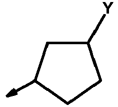 | 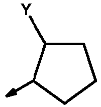 | 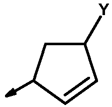 | 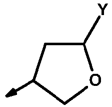 | 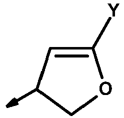 |
| G-11 | G-12 | G-13 | G-14 | G-15 |
| 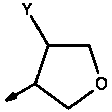 | 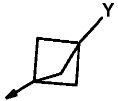 | 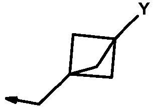 | 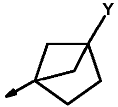 | 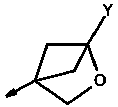 |
| G-16 | G-17 | G-18 | G-19 | G-20 |
| 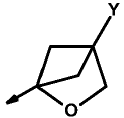 | 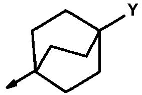 | 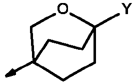 | 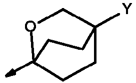 | 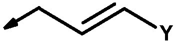 |
| G-21 | G-22 | G-23 | G-24 | G-25 |

where the arrow represents a bond to the nitrogen atom of formula (I)
- Y: represents -C(O)OR⁸, -C(O)NR⁹R¹⁰, -C(S)NR⁹R¹⁰, -C(O)N(R⁹)S(O)₂R¹¹ or - C(O)N(R⁹)S(O)₂NR⁹R¹⁰,
- R⁸, R⁹, R¹⁰ and R¹¹: represent the same or different substituents selected independently from eachother from hydrogen, (C₁-C₄)-alkyl, (C₂-C₄)-alkenyl, (C₂-C₄)-alkynyl, (C₁-C₄)-cyanoalkyl, (C₁-C₄)-haloalkyl, (C₂-C₄)-haloalkenyl, (C₂-C₄)-haloalkynyl, (C₃-C₆)-cycloalkyl, (C₃-C₆)-halocycloalkyl, (C₄-C₆)-cycloalkenyl, (C₄-C₆)-halocycloalkenyl, (C₁-C₄)-alkoxy, (C₁-C₄)-haloalkoxy, (C₁-C₄)-alkoxy-(C₁-C₄)-alkyl, (C₁-C₄)-haloalkoxy-(C₁-C₄)-alkyl, (C₁-C₄)-alkylthio-(C₁-C₄)-alkyl, (C₁-C₄)-alkylS(O)ₓ-(C₁-C₄)-alkyl, (C₁-C₄)-haloalkylthio-(C₁-C₄)-alkyl, (C₁-C₄)-alkoxy-(C₁-C₄)-haloalkyl, aryl, aryl-(C₁-C₄)-alkyl, heteroaryl, heteroaryl-(C₁-C₄)-alkyl, (C₃-C₆)-cycloalkyl-(C₁-C₄)-alkyl, (C₄-C₆)-cycloalkenyl-(C₁-C₄)-alkyl, heterocyclyl, bis-[(C₁-C₄)-alkyl]aminocarbonyl-(C₁-C₄)-alkyl, (C₁-C₄)-alkyl-amino-carbonyl-(C₁-C₄)-alkyl, aryl-(C₁-C₄)-alkyl-aminocarbonyl-(C₁-C₄)-alkyl or heterocyclyl-(C₁-C₄)-alkyl,
- R⁹ and R¹⁰: can represent, with the nitrogen atom to which they are attached, a fully saturated or partially saturated, optionally interrupted by heteroatoms and optionally further substituted 3- to 10-membered monocyclic or bicyclic ring,
and
- x: represents 0, 1 or 2.

The invention more preferably provides compounds of the general formula (I), salts or N-oxides thereof in which
- Q: represents a phenyl or heteroaryl residue, wherein each of the previously mentioned two residues is unsubstituted or is independently substituted by one or more residues selected from the group R⁷,
- R¹ and R²: represent independently of one another hydrogen, halogen, cyano, (C₁-C₄)-alkyl, (C₁-C₄)-alkoxy or, together with the carbon atom to which they are attached form a (C₃-C₆)-cycloalkyl or (C₃-C₆)-cycloalkoxy ring system,
- R³: represents hydrogen, amino, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₁-C₄)-alkoxy, (C₁-C₄)-haloalkoxy, (C₁-C₄)-alkoxy-(C₁-C₄)-alkyl, (C₁-C₄)-haloalkoxy-(C₁-C₄)-alkyl, (C₁-C₄)-alkoxy-(C₃-C₆)-cycloalkyl, (C₃-C₆)-cycloalkyl, (C₃-C₆)-halocycloalkyl, (C₃-C₆)-cycloalkoxy, (C₃-C₆)-halocycloalkoxy, (C₁-C₄)-alkylamino, (C₂-C₈)-dialkylamino, amino-C(O)NR⁹R¹⁰, (C₁-C₄)-alkylamino-C(O)NR⁹R¹⁰,
- R⁴: represents hydrogen, halogen, cyano, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₁-C₄)-alkoxy, (C₁-C₄)-haloalkoxy, (C₃-C₆)-cycloalkyl or (C₃-C₆)-halocycloalkyl,
- R⁵: represents hydrogen, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₃-C₆)-cycloalkyl, (C₃-C₆)-halocycloalkyl,
- R⁶: represents hydrogen, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₃-C₆)-cycloalkyl, (C₃-C₆)-halocycloalkyl,
- R⁷: represents nitro, cyano, (C₁-C₄)-alkyl, (C₁-C₄)-haloalkyl,
- W: represents oxygen or sulfur,
- G: represents a residue selected from G1-G25 shown in the table below, wherein the selected residue is substituted by a residue selected from the group Y,

| | | | | |
|---|---|---|---|---|
| 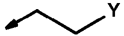 | 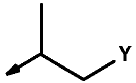 | 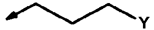 | 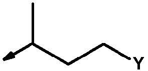 | 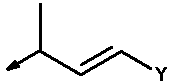 |
| G-1 | G-2 | G-3 | G-4 | G-5 |
| 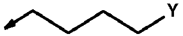 | 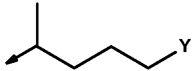 | 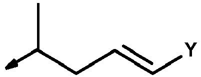 | 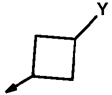 | 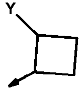 |
| G-6 | G-7 | G-8 | G-9 | G-10 |
| 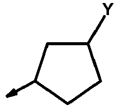 | 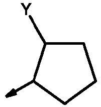 | 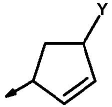 | 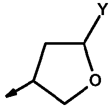 | 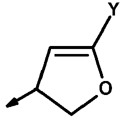 |
| G-11 | G-12 | G-13 | G-14 | G-15 |
| 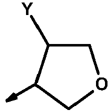 | 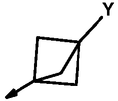 | 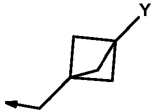 | 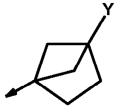 | 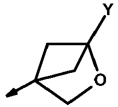 |
| G-16 | G-17 | G-18 | G-19 | G-20 |
| 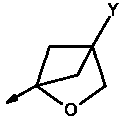 | 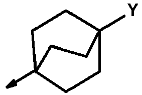 | 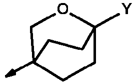 | 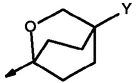 | 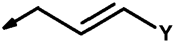 |
| G-21 | G-22 | G-23 | G-24 | G-25 |

where the arrow represents a bond to the nitrogen atom of formula (I)
- Y: represents -C(O)OR⁸, -C(O)NR⁹R¹⁰, -C(S)NR⁹R¹⁰,
- R⁸, R⁹and R¹⁰: represent the same or different substituents selected independently from each other from hydrogen, (C₁-C₄)-alkyl, (C₂-C₄)-alkenyl, (C₂-C₄)-alkynyl, (C₃-C₆)-cycloalkyl,
and
- R⁹ and R¹⁰: can represent, with the nitrogen atom to which they are attached, a fully saturated or partially saturated, optionally interrupted by heteroatoms and optionally further substituted 3- to 6-membered monocyclic or bicycle ring.

The invention particularly preferably provides compounds of the general formula (I), salts or N-oxides thereof in which
- Q: represents phenyl which is unsubstituted or independently substituted by one or more residues selected from the group R⁷,
- R¹ and R²: represent independently of one another hydrogen, fluoro, chloro, cyano, methyl, ethyl, methoxy, ethoxy,
- R³: represents hydrogen, amino, methyl, ethyl, propyl, chloromethyl, fluoromethyl, difluoromethyl, trifluoromethyl, cyclopropyl, methoxy, ethoxy, propoxy, methoxymethyl, ethoxymethyl, methoxyethyl, ethoxyethyl, methoxypropyl, methylamino, ethylamino, propylamino, dimethylamino, methylethylamino, vinyl, 1-propenyl, 2-propenyl, ethinyl or ureido,
- R⁴: represents hydrogen, halogen fluoro, chloro, cyano, methyl, ethyl, propyl,, chloromethyl, fluoromethyl, difluoromethyl, trifluoromethyl, vinyl, 1-propenyl, 2-propenyl, ethinyl, cyclopropyl, methoxy, ethoxy, propoxy,
- R⁵: represents hydrogen, methyl, ethyl, cyclopropyl, vinyl, 1-propenyl, 2-propenyl, ethinyl,
- R⁶: represents hydrogen, methyl, ethyl, cyclopropyl, vinyl, 1-propenyl, 2-propenyl, ethinyl,
- R⁷: represents fluoro, chloro, nitro, cyano, methyl, ethyl,
- W: represents oxygen or sulfur,
- G: represents a residue selected from G1-G25 shown in the table below, wherein the selected residue is substituted by a residue selected from the group Y,

| | | | | |
|---|---|---|---|---|
| 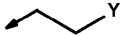 | 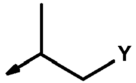 | 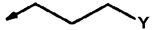 | 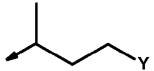 | 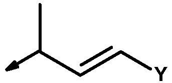 |
| G-1 | G-2 | G-3 | G-4 | G-5 |
| 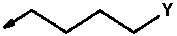 | 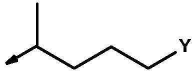 | 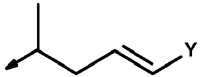 | | |
| G-6 | G-7 | G-8 | | |
| 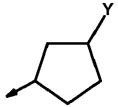 | 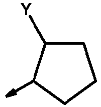 | 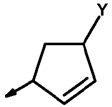 | 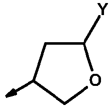 | 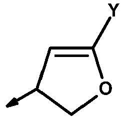 |
| G-11 | G-12 | G-13 | G-14 | G-15 |
| 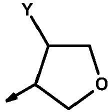 | | | | |
| G-16 | | | | |
| | | | | 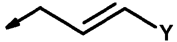 |
| | | | | G-25 |

where the arrow represents a bond to the nitrogen atom of formula (I)
- Y: represents -C(O)OR⁸, -C(O)NR⁹R¹⁰, -C(S)NR⁹R¹⁰, -C(O)N(R⁹)S(O)₂R¹¹ or - C(O)N(R⁹)S(O)₂NR⁹R¹⁰,
- R⁸, R⁹, R¹⁰ and R¹¹: represent the same or different substituents selected independently from each other from hydrogen, methyl, ethyl, cyclopropyl, vinyl, 1-propenyl, 2-propenyl, ethinyl.

The invention most preferably provides compounds of the general formula (I), salts or N-oxides thereof in which
- Q: represents 3,5-difluorophenyl, 3,5-dichlorophenyl or 3-fluoro-5-methyl-phenyl,
- R¹: represents hydrogen,
- R²: represents hydrogen,
- R³: represents hydrogen, methyl, ethyl, cyclopropyl, chloromethyl, methoxy, ethoxy, methoxymethyl, amino, methylamino, dimethylamino or ureido,
- R⁴: represents methyl, ethyl, fluoromethyl, cyclopropyl or vinyl,
- R⁵: represents hydrogen,
- R⁶: represents hydrogen,
- W: represents oxygen,
- G: rac-(3S,5R)-5-methoxycarbonyltetrahydrofuran-3-yl, (1S,4S)-4-methoxycarbonylcyclopent-2-en-1-yl, (E,1R)-4-ethoxy-1-methyl-4-oxo-but-2-enyl, (1R)-3-ethoxy-1-methyl-3-oxo-propyl, (1R)-4-methoxy-1-methyl-4-oxo-butyl, (1R,3S)-3-methoxycarbonylcyclopentyl, (E,1R)-4-methoxy-1-methyl-4-oxo-but-2-enyl or (1S,4S)-4-carboxycyclopent-2-en-1-yl.

According to another embodiment of the present invention,
- G: represents G-13, G-2, G-5 or G-14.

According to another embodiment of the present invention,
R¹ and R² represent hydrogen.

According to another embodiment of the present invention,
Q represents halogen or (C₁-C₄)-alkyl substituted phenyl.

According to another embodiment of the present invention,
- R⁶: represents hydrogen.

According to another embodiment of the present invention,
- R⁵: represents hydrogen.

The definitions of radicals listed above in general terms or within areas of preference apply both to the end products of the general formula (I) and correspondingly to the starting materials or intermediates required for preparation in each case. These radical definitions can be combined with one another as desired, i.e. including combinations between the given preferred ranges.

Primarily for reasons of higher herbicidal activity, better selectivity and/or better producibility, compounds of the abovementioned general formula (I) according to the invention and/or their salts and N-oxides or their use according to the invention are of particular interest in which individual radicals have one of the preferred meanings already specified or specified below, or in particular those in which one or more of the preferred meanings already specified or specified below occur in combination.

With regard to the compounds of the general formula (I) according to the invention, the terms used above and further below will be elucidated. These are familiar to the person skilled in the art and especially have the definitions elucidated hereinafter:

Unless defined differently, names of chemical groups are generally to be understood such that attachment to the skeleton or the remainder of the molecule is via the structural element of the relevant chemical group mentioned last, i.e. for example in the case of (C₂-C₈)-alkenyloxy via the oxygen atom and in the case of heterocyclyl-(C₁-C₈)-alkyl or R¹⁷O(O)C-(C₁-C₈)-alkyl in each case via the carbon atom of the alkyl group.

According to the invention, "ureido" refers to NH₂CONH-.

According to the invention, "alkylsulfonyl" - alone or as part of a chemical group - refers to straight-chain or branched alkylsulfonyl, preferably having 1 to 8 or 1 to 6 carbon atoms, for example (but not limited to) (C₁-C₆)-alkylsulfonyl such as methylsulfonyl, ethylsulfonyl, propylsulfonyl, 1-methylethylsulfonyl, butylsulfonyl, 1-methylpropylsulfonyl, 2-methylpropylsulfonyl, 1,1-dimethylethylsulfonyl, pentylsulfonyl, 1-methylbutylsulfonyl, 2-methylbutylsulfonyl, 3-methylbutylsulfonyl, 1,1-dimethylpropylsulfonyl, 1,2-dimethylpropylsulfonyl, 2,2-dimethylpropylsulfonyl, 1-ethylpropylsulfonyl, hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethylpropylsulfonyl, 1,2,2-trimethylpropylsulfonyl, 1-ethyl-1-methylpropylsulfonyl and 1-ethyl-2-methylpropylsulfonyl.

According to the invention, "heteroarylsulfonyl" refers to optionally substituted pyridylsulfonyl, pyrimidinylsulfonyl, pyrazinylsulfonyl or optionally substituted polycyclic heteroarylsulfonyl, here in particular optionally substituted quinolinylsulfonyl, for example substituted by fluorine, chlorine, bromine, iodine, cyano, nitro, alkyl, haloalkyl, haloalkoxy, amino, alkylamino, alkylcarbonylamino, dialkylamino or alkoxy groups.

According to the invention, "alkylthio" - alone or as part of a chemical group - refers to straight-chain or branched S-alkyl, preferably having 1 to 8 or 1 to 6 carbon atoms, such as (C₁-C₁₀)-, (C₁-C₆)- or (C₁-C₄)-alkylthio, for example (but not limited to) (C₁-C₆)-alkylthio such as methylthio, ethylthio, propylthio, 1-methylethylthio, butylthio, 1-methylpropylthio, 2-methylpropylthio, 1,1-dimethylethylthio, pentylthio, 1-methylbutylthio, 2-methylbutylthio, 3-methylbutylthio, 1,1-dimethylpropylthio, 1,2-dimethylpropylthio, 2,2-dimethylpropylthio, 1-ethylpropylthio, hexylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio and 1-ethyl-2-methylpropylthio.

According to the invention, "alkenylthio" denotes an alkenyl radical bonded via a sulfur atom, alkynylthio denotes an alkynyl radical bonded via a sulfur atom, cycloalkylthio denotes a cycloalkyl radical bonded via a sulfur atom, and cycloalkenylthio denotes a cycloalkenyl radical bonded via a sulfur atom.

According to the invention, "alkylsulfinyl (alkyl-S(=O)-)", unless defined differently elsewhere, denotes alkyl radicals which are bonded to the skeleton via -S(=O)-, such as (C₁-C₁₀)-, (C₁-C₆)- or (C₁-C₄)-alkylsulfinyl, for example (but not limited to) (C₁-C₆)-alkylsulfinyl such as methylsulfinyl, ethylsulfinyl, propylsulfinyl, 1-methylethylsulfinyl, butylsulfinyl, 1-methylpropylsulfinyl, 2-methylpropylsulfinyl, 1,1-dimethylethylsulfinyl, pentylsulfinyl, 1-methylbutylsulfinyl, 2-methylbutylsulfinyl, 3-methylbutylsulfinyl, 1,1-dimethylpropylsulfinyl, 1,2-dimethylpropylsulfinyl, 2,2-dimethylpropylsulfinyl, 1-ethylpropylsulfinyl, hexylsulfinyl, 1-methylpentylsulfinyl, 2-methylpentylsulfinyl, 3-methylpentylsulfinyl, 4-methylpentylsulfinyl, 1,1-dimethylbutylsulfinyl, 1,2-dimethylbutylsulfinyl, 1,3-dimethylbutylsulfinyl, 2,2-dimethylbutylsulfinyl, 2,3-dimethylbutylsulfinyl, 3,3-dimethylbutylsulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropylsulfinyl and 1-ethyl-2-methylpropylsulfinyl.

Analogously, "alkenylsulfinyl" and "alkynylsulfinyl" are defined in accordance with the invention respectively as alkenyl and alkynyl radicals bonded to the skeleton via -S(=O)-, such as (C₂-C₁₀)-, (C₂-C₆)- or (C₂-C₄)-alkenylsulfinyl or (C₃-C₁₀)-, (C₃-C₆)- or (C₃-C₄)-alkynylsulfinyl.

Analogously, "alkenylsulfonyl" and "alkynylsulfonyl" are defined in accordance with the invention respectively as alkenyl and alkynyl radicals bonded to the skeleton via -S(=O)₂-, such as (C₂-C₁₀)-, (C₂-C₆)- or (C₂-C₄)-alkenylsulfonyl or (C₃-C₁₀)-, (C₃-C₆)- or (C₃-C₄)-alkynylsulfonyl.

"Alkoxy" denotes an alkyl radical attached via an oxygen atom, for example (but not limited to) (C₁-C₆)-alkoxy such as methoxy, ethoxy, propoxy, 1-methylethoxy, butoxy, 1-methylpropoxy, 2-methylpropoxy, 1,1-dimethylethoxy, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy. Alkenyloxy denotes an alkenyl radical attached via an oxygen atom, and alkynyloxy denotes an alkynyl radical attached via an oxygen atom, such as (C₂-C₁₀)-, (C₂-C₆)- or (C₂-C₄)-alkenoxy and (C₃-C₁₀)-, (C₃-C₆)- or (C₃-C₄)-alkynoxy.

"Cycloalkyloxy" denotes a cycloalkyl radical bonded via an oxygen atom and cycloalkenyloxy denotes a cycloalkenyl radical bonded via an oxygen atom.

According to the invention, "alkylcarbonyl" (alkyl-C(=O)-), unless defined differently elsewhere, represents alkyl radicals bonded to the skeleton via -C(=O)-, such as (C₁-C₁₀)-, (C₁-C₆)- or (C₁-C₄)-alkylcarbonyl. Here, the number of the carbon atoms refers to the alkyl radical in the alkylcarbonyl group.

Analogously, "alkenylcarbonyl" and "alkynylcarbonyl", unless defined differently elsewhere, in accordance with the invention, respectively represent alkenyl and alkynyl radicals bonded to the skeleton via -C(=O)-, such as (C₂-C₁₀)-, (C₂-C₆)- or (C₂-C₄)-alkenylcarbonyl and (C₂-C₁₀)-, (C₂-C₆)- or (C₂-C₄)-alkynylcarbonyl. Here, the number of the carbon atoms refers to the alkenyl or alkynyl radical in the alkenylcarbonyl or alkynylcarbonyl group.

"Alkoxycarbonyl (alkyl-O-C(=O)-)", unless defined differently elsewhere: alkyl radicals bonded to the skeleton via -O-C(=O)-, such as (C₁-C₁₀)-, (C₁-C₆)- or (C₁-C₄)-alkoxycarbonyl. Here, the number of the carbon atoms refers to the alkyl radical in the alkoxycarbonyl group. Analogously, "alkenyloxycarbonyl" and "alkynyloxycarbonyl", unless defined differently elsewhere, in accordance with the invention, respectively represent alkenyl and alkynyl radicals bonded to the skeleton via -O-C(=O)-, such as (C₂-C₁₀)-, (C₂-C₆)- or (C₂-C₄)-alkenyloxycarbonyl and (C₃-C₁₀)-, (C₃-C₆)- or (C₃-C₄)-alkynyloxycarbonyl. Here, the number of the carbon atoms refers to the alkenyl or alkynyl radical in the alkenyloxycarbonyl or alkynyloxycarbonyl group.

According to the invention, the term "alkylcarbonyloxy" (alkyl-C(=O)-O-), unless defined differently elsewhere, represents alkyl radicals bonded to the skeleton via the oxygen of a carbonyloxy group (-C(=O)-O-), such as (C₁-C₁₀)-, (C₁-C₆)- or (C₁-C₄)-alkylcarbonyloxy. Here, the number of the carbon atoms refers to the alkyl radical in the alkylcarbonyloxy group.

Analogously, "alkenylcarbonyloxy" and "alkynylcarbonyloxy" are defined in accordance with the invention respectively as alkenyl and alkynyl radicals bonded to the skeleton via the oxygen of (-C(=O)-O-), such as (C₂-C₁₀)-, (C₂-C₆)- or (C₂-C₄)-alkenylcarbonyloxy or (C₂-C₁₀)-, (C₂-C₆)- or (C₂-C₄)-alkynylcarbonyloxy. Here, the number of the carbon atoms refers to the alkenyl or alkynyl radical in the alkenyl- or alkynylcarbonyloxy group respectively.

In short forms such as C(O)R¹³, C(O)OR¹³, OC(O)NR¹¹R¹² or C(O)NR¹¹R¹², the short form O shown in brackets represents an oxygen atom attached to the adjacent carbon atom via a double bond.

In short forms such as OC(S)OR¹³, OC(S)SR¹⁴, OC(S)NR¹¹R¹², the short form S shown in brackets represents a sulfur atom attached to the adjacent carbon atom via a double bond.

The term "aryl" denotes an optionally substituted mono-, bi- or polycyclic aromatic system having preferably 6 to 14, especially 6 to 10, ring carbon atoms, for example phenyl, naphthyl, anthryl, phenanthrenyl and the like, preferably phenyl.

The term "optionally substituted aryl" also includes polycyclic systems, such as tetrahydronaphthyl, indenyl, indanyl, fluorenyl, biphenylyl, where the bonding site is on the aromatic system. In systematic terms, "aryl" is generally also encompassed by the term "optionally substituted phenyl". Preferred aryl substituents here are, for example, hydrogen, halogen, alkyl, cycloalkyl, cycloalkylalkyl, cycloalkenyl, halocycloalkyl, alkenyl, alkynyl, aryl, arylalkyl, arylalkenyl, heteroaryl, heteroarylalkyl, heterocyclyl, heterocyclylalkyl, alkoxyalkyl, alkylthio, haloalkylthio, haloalkyl, alkoxy, haloalkoxy, cycloalkoxy, cycloalkylalkoxy, aryloxy, heteroraryloxy, alkoxyalkoxy, alkynylalkoxy, alkenyloxy, bisalkylaminoalkoxy, tris[alkyl]silyl, bis[alkyl]arylsilyl, bis[alkyl]alkylsilyl, tris[alkyl]silylalkynyl, arylalkynyl, heteroarylalkynyl, alkylalkynyl, cycloalkylalkynyl, haloalkylalkynyl, heterocyclyl-N-alkoxy, nitro, cyano, amino, alkylamino, bis-alkylamino, alkylcarbonylamino, cycloalkylcarbonylamino, arylcarbonylamino, alkoxycarbonylamino, alkoxycarbonylalkylamino, arylalkoxycarbonylalkylamino, hydroxycarbonyl, alkoxycarbonyl, aminocarbonyl, alkylaminocarbonyl, cycloalkylaminocarbonyl, bisalkylaminocarbonyl, heteroarylalkoxy, arylalkoxy.

A heterocyclic radical (heterocyclyl) contains at least one heterocyclic ring (=carbocyclic ring in which at least one carbon atom has been replaced by a heteroatom, preferably by a heteroatom from the group of N, O, S, P) which is saturated, unsaturated, partially saturated or heteroaromatic and may be unsubstituted or substituted, in which case the bonding site is localized on a ring atom. If the heterocyclyl radical or the heterocyclic ring is optionally substituted, it may be fused to other carbocyclic or heterocyclic rings. In the case of optionally substituted heterocyclyl, polycyclic systems are also included, for example 8-azabicyclo[3.2.1]octanyl, 8-azabicyclo[2.2.2]octanyl or 1-azabicyclo[2.2.1]heptyl. Optionally substituted heterocyclyl also includes spirocyclic systems, such as, for example, 1-oxa-5-azaspiro[2.3]hexyl. Unless defined differently, the heterocyclic ring preferably contains 3 to 9 ring atoms, especially 3 to 6 ring atoms, and one or more, preferably 1 to 4, especially 1, 2 or 3, heteroatoms in the heterocyclic ring, preferably from the group of N, O and S, but no two oxygen atoms should be directly adjacent, for example with one heteroatom from the group of N, O and S: 1- or 2- or 3-pyrrolidinyl, 3,4-dihydro-2H-pyrrol-2- or -3-yl, 2,3-dihydro-1H-pyrrol-1- or -2- or -3- or -4- or - 5-yl; 2,5-dihydro-1H-pyrrol-1- or -2- or -3-yl, 1- or 2- or 3- or 4-piperidinyl; 2,3,4,5-tetrahydropyridin-2- or -3- or -4- or -5-yl or -6-yl; 1,2,3,6-tetrahydropyridin-1- or -2- or -3- or -4- or -5- or -6-yl; 1,2,3,4-tetrahydropyridin-1- or -2- or -3- or -4- or -5- or -6-yl; 1,4-dihydropyridin-1- or -2- or -3- or -4-yl; 2,3-dihydropyridin-2- or -3- or -4- or -5- or -6-yl; 2,5-dihydropyridin-2- or -3- or -4- or -5- or -6-yl, 1- or 2- or 3- or 4-azepanyl; 2,3,4,5-tetrahydro-1H-azepin-1- or -2- or -3- or -4- or -5- or -6- or -7-yl; 2,3,4,7-tetrahydro-1H-azepin-1- or -2- or -3- or -4- or -5- or -6- or -7-yl; 2,3,6,7-tetrahydro-1H-azepin-1- or -2- or -3- or -4-yl; 3,4,5,6-tetrahydro-2H-azepin-2- or -3- or -4- or -5- or -6- or -7-yl; 4,5-dihydro-1H-azepin-1- or -2- or -3- or -4-yl; 2,5-dihydro-1H-azepin-1- or -2- or -3- or -4- or -5- or -6- or -7-yl; 2,7-dihydro-1H-azepin-1- or -2- or -3- or -4-yl; 2,3-dihydro-1H-azepin-1- or -2- or -3- or -4- or -5- or -6- or -7-yl; 3,4-dihydro-2H-azepin-2- or -3- or -4- or -5- or -6- or -7-yl; 3,6-dihydro-2H-azepin-2- or -3- or -4- or -5- or -6- or -7-yl; 5,6-dihydro-2H-azepin-2- or -3- or -4- or -5- or -6- or -7-yl; 4,5-dihydro-3H-azepin-2- or -3- or -4- or -5- or -6- or -7-yl; 1H-azepin-1- or -2- or -3- or -4- or -5- or -6- or -7-yl; 2H-azepin-2- or -3- or -4- or -5- or -6- or -7-yl; 3H-azepin-2- or -3- or -4- or -5- or -6- or -7-yl; 4H-azepin-2- or -3- or -4- or -5- or -6- or -7-yl, 2- or 3-oxolanyl (= 2- or 3-tetrahydrofuranyl); 2,3-dihydrofuran-2- or -3- or -4- or -5-yl; 2,5-dihydrofuran-2- or -3-yl, 2- or 3- or 4-oxanyl (= 2- or 3- or 4-tetrahydropyranyl); 3,4-dihydro-2H-pyran-2- or -3- or -4- or -5- or -6-yl; 3,6-dihydro-2H-pyran-2- or -3- or -4- or -5- or -6-yl; 2H-pyran-2- or -3- or -4- or -5- or -6-yl; 4H-pyran-2- or -3- or -4-yl, 2- or 3- or 4-oxepanyl; 2,3,4,5-tetrahydrooxepin-2- or -3- or -4- or -5- or -6- or -7-yl; 2,3,4,7-tetrahydrooxepin-2- or - 3- or -4- or -5- or -6- or -7-yl; 2,3,6,7-tetrahydrooxepin-2- or -3- or -4-yl; 2,3-dihydrooxepin-2- or -3- or -4- or -5- or -6- or -7-yl; 4,5-dihydrooxepin-2- or -3- or -4-yl; 2,5-dihydrooxepin-2- or -3- or -4- or -5- or -6- or -7-yl; oxepin-2- or -3- or -4- or -5- or -6- or -7-yl; 2- or 3-tetrahydrothiophenyl; 2,3-dihydrothiophen-2- or -3- or -4- or -5-yl; 2,5-dihydrothiophen-2- or -3-yl; tetrahydro-2H-thiopyran-2- or -3- or -4-yl; 3,4-dihydro-2H-thiopyran-2- or -3- or -4- or -5- or -6-yl; 3,6-dihydro-2H-thiopyran-2- or -3- or -4- or -5- or -6-yl; 2H-thiopyran-2- or -3- or -4- or -5- or -6-yl; 4H-thiopyran-2- or -3- or -4-yl. Preferred 3-membered and 4-membered heterocycles are, for example, 1- or 2-aziridinyl, oxiranyl, thiiranyl, 1- or 2- or 3-azetidinyl, 2- or 3-oxetanyl, 2- or 3-thietanyl, 1,3-dioxetan-2-yl. Further examples of "heterocyclyl" are a partially or fully hydrogenated heterocyclic radical having two heteroatoms from the group consisting of N, O and S, such as, for example, 1- or 2- or 3- or 4-pyrazolidinyl; 4,5-dihydro-3H-pyrazol-3- or -4- or -5-yl; 4,5-dihydro-1H-pyrazol-1- or -3- or -4- or -5-yl; 2,3-dihydro-1H-pyrazol-1- or -2- or -3- or -4- or -5-yl; 1- or 2- or 3- or 4-imidazolidinyl; 2,3-dihydro-1H-imidazol-1- or -2- or - 3- or -4-yl; 2,5-dihydro-1H-imidazol-1- or -2- or -4- or -5-yl; 4,5-dihydro-1H-imidazol-1- or -2- or -4- or -5-yl; hexahydropyridazin-1- or -2- or -3- or -4-yl; 1,2,3,4-tetrahydropyridazin-1- or -2- or -3- or -4- or -5- or -6-yl; 1,2,3,6-tetrahydropyridazin-1- or -2- or -3- or -4- or -5- or -6-yl; 1,4,5,6-tetrahydropyridazin-1- or -3- or -4- or -5- or -6-yl; 3,4,5,6-tetrahydropyridazin-3- or -4- or -5-yl; 4,5-dihydropyridazin-3- or -4-yl; 3,4-dihydropyridazin-3- or -4- or -5- or -6-yl; 3,6-dihydropyridazin-3- or - 4-yl; 1,6-dihydropyriazin-1- or -3- or -4- or -5- or -6-yl; hexahydropyrimidin-1- or -2- or -3- or -4-yl; 1,4,5,6-tetrahydropyrimidin-1- or -2- or -4- or -5- or -6-yl; 1,2,5,6-tetrahydropyrimidin-1- or -2- or -4- or -5- or -6-yl; 1,2,3,4-tetrahydropyrimidin-1- or -2- or -3- or -4- or -5- or -6-yl; 1,6-dihydropyrimidin-1- or -2- or -4- or -5- or -6-yl; 1,2-dihydropyrimidin-1- or -2- or -4- or -5- or -6-yl; 2,5-dihydropyrimidin-2- or -4- or -5-yl; 4,5-dihydropyrimidin-4- or -5- or -6-yl; 1,4-dihydropyrimidin-1- or -2- or -4- or -5- or -6-yl; 1- or 2- or 3-piperazinyl; 1,2,3,6-tetrahydropyrazin-1- or -2- or -3- or -5- or -6-yl; 1,2,3,4-tetrahydropyrazin-1- or -2- or -3- or -4- or -5- or -6-yl; 1,2-dihydropyrazin-1- or -2- or -3- or -5- or -6-yl; 1,4-dihydropyrazin-1- or -2- or -3-yl; 2,3-dihydropyrazin-2- or -3- or -5- or -6-yl; 2,5-dihydropyrazin-2- or -3-yl; 1,3-dioxolan-2- or -4- or -5-yl; 1,3-dioxol-2- or -4-yl; 1,3-dioxan-2- or -4- or -5-yl; 4H-1,3-dioxin-2- or -4- or -5- or -6-yl; 1,4-dioxan-2- or -3- or -5- or -6-yl; 2,3-dihydro-1,4-dioxin-2- or -3- or - 5- or -6-yl; 1,4-dioxin-2- or -3-yl; 1,2-dithiolan-3- or -4-yl; 3H-1,2-dithiol-3- or -4- or -5-yl; 1,3-dithiolan-2- or -4-yl; 1,3-dithiol-2- or -4-yl; 1,2-dithian-3- or -4-yl; 3,4-dihydro-1,2-dithiin-3- or -4- or - 5- or -6-yl; 3,6-dihydro-1,2-dithiin-3- or -4-yl; 1,2-dithiin-3- or -4-yl; 1,3-dithian-2- or -4- or -5-yl; 4H-1,3-dithiin-2- or -4- or -5- or -6-yl; isoxazolidin-2- or -3- or -4- or -5-yl; 2,3-dihydroisoxazol-2- or -3- or -4- or -5-yl; 2,5-dihydroisoxazol-2- or -3- or -4- or -5-yl; 4,5-dihydroisoxazol-3- or -4- or -5-yl; 1,3-oxazolidin-2- or -3- or -4- or -5-yl; 2,3-dihydro-1,3-oxazol-2- or -3- or -4- or -5-yl; 2,5-dihydro-1,3-oxazol-2- or -4- or -5-yl; 4,5-dihydro-1,3-oxazol-2- or -4- or -5-yl; 1,2-oxazinan-2- or -3- or -4- or -5- or -6-yl; 3,4-dihydro-2H-1,2-oxazin-2- or -3- or -4- or -5- or -6-yl; 3,6-dihydro-2H-1,2-oxazin-2- or -3- or - 4- or -5- or -6-yl; 5,6-dihydro-2H-1,2-oxazin-2- or -3- or -4- or -5- or -6-yl; 5,6-dihydro-4H-1,2-oxazin-3- or -4- or -5- or -6-yl; 2H-1,2-oxazin-2- or -3- or -4- or -5- or -6-yl; 6H-1,2-oxazin-3- or -4- or -5- or - 6-yl; 4H-1,2-oxazin-3- or -4- or -5- or -6-yl; 1,3-oxazinan-2- or -3- or -4- or -5- or -6-yl; 3,4-dihydro-2H-1,3-oxazin-2- or -3- or -4- or -5- or -6-yl; 3,6-dihydro-2H-1,3-oxazin-2- or -3- or -4- or -5- or -6-yl; 5,6-dihydro-2H-1,3-oxazin-2- or -4- or -5- or -6-yl; 5,6-dihydro-4H-1,3-oxazin-2- or -4- or -5- or -6-yl; 2H-1,3-oxazin-2- or -4- or -5- or -6-yl; 6H-1,3-oxazin-2- or -4- or -5- or -6-yl; 4H-1,3-oxazin-2- or -4- or -5- or -6-yl; morpholin-2- or -3- or -4-yl; 3,4-dihydro-2H-1,4-oxazin-2- or -3- or -4- or -5- or -6-yl; 3,6-dihydro-2H-1,4-oxazin-2- or -3- or -5- or -6-yl; 2H-1,4-oxazin-2- or -3- or -5- or -6-yl; 4H-1,4-oxazin-2- or -3-yl; 1,2-oxazepan-2- or -3- or -4- or -5- or -6- or -7-yl; 2,3,4,5-tetrahydro-1,2-oxazepin-2- or -3- or -4- or -5- or -6- or -7-yl; 2,3,4,7-tetrahydro-1,2-oxazepin-2- or -3- or -4- or -5- or -6- or -7-yl; 2,3,6,7-tetrahydro-1,2-oxazepin-2- or -3- or -4- or -5- or -6- or -7-yl; 2,5,6,7-tetrahydro-1,2-oxazepin-2- or -3- or -4- or -5- or -6- or -7-yl; 4,5,6,7-tetrahydro-1,2-oxazepin-3- or -4- or -5- or -6- or -7-yl; 2,3-dihydro-1,2-oxazepin-2- or -3- or -4- or -5- or -6- or -7-yl; 2,5-dihydro-1,2-oxazepin-2- or -3- or -4- or - 5- or -6- or -7-yl; 2,7-dihydro-1,2-oxazepin-2- or -3- or -4- or -5- or -6- or -7-yl; 4,5-dihydro-1,2-oxazepin-3- or -4- or -5- or -6- or -7-yl; 4,7-dihydro-1,2-oxazepin-3- or -4- or -5- or -6- or -7-yl; 6,7-dihydro-1,2-oxazepin-3- or -4- or -5- or -6- or -7-yl; 1,2-oxazepin-3- or -4- or -5- or -6- or -7-yl; 1,3-oxazepan-2- or -3- or -4- or -5- or -6- or -7-yl; 2,3,4,5-tetrahydro-1,3-oxazepin-2- or -3- or -4- or -5- or - 6- or -7-yl; 2,3,4,7-tetrahydro-1,3-oxazepin-2- or -3- or -4- or -5- or -6- or -7-yl; 2,3,6,7-tetrahydro-1,3-oxazepin-2- or -3- or -4- or -5- or -6- or -7-yl; 2,5,6,7-tetrahydro-1,3-oxazepin-2- or -4- or -5- or -6- or - 7-yl; 4,5,6,7-tetrahydro-1,3-oxazepin-2- or -4- or -5- or -6- or -7-yl; 2,3-dihydro-1,3-oxazepin-2- or -3- or -4- or -5- or -6- or -7-yl; 2,5-dihydro-1,3-oxazepin-2- or -4- or -5- or -6- or -7-yl; 2,7-dihydro-1,3-oxazepin-2- or -4- or -5- or -6- or -7-yl; 4,5-dihydro-1,3-oxazepin-2- or -4- or -5- or -6- or -7-yl; 4,7-dihydro-1,3-oxazepin-2- or -4- or -5- or -6- or -7-yl; 6,7-dihydro-1,3-oxazepin-2- or -4- or -5- or -6- or - 7-yl; 1,3-oxazepin-2- or -4- or -5- or -6- or -7-yl; 1,4-oxazepan-2- or -3- or -5- or -6- or -7-yl; 2,3,4,5-tetrahydro-1,4-oxazepin-2- or -3- or -4- or -5- or -6- or -7-yl; 2,3,4,7-tetrahydro-1,4-oxazepin-2- or -3- or -4- or -5- or -6- or -7-yl; 2,3,6,7-tetrahydro-1,4-oxazepin-2- or -3- or -5- or -6- or -7-yl; 2,5,6,7-tetrahydro-1,4-oxazepin-2- or -3- or -5- or -6- or -7-yl; 4,5,6,7-tetrahydro-1,4-oxazepin-2- or -3- or -4- or -5- or -6- or -7-yl; 2,3-dihydro-1,4-oxazepin-2- or -3- or -5- or -6- or -7-yl; 2,5-dihydro-1,4-oxazepin-2- or -3- or -5- or -6- or -7-yl; 2,7-dihydro-1,4-oxazepin-2- or -3- or -5- or -6- or -7-yl; 4,5-dihydro-1,4-oxazepin-2- or -3- or -4- or -5- or -6- or -7-yl; 4,7-dihydro-1,4-oxazepin-2- or -3- or -4- or -5- or -6- or - 7-yl; 6,7-dihydro-1,4-oxazepin-2- or -3- or -5- or -6- or -7-yl; 1,4-oxazepin-2- or -3- or -5- or -6- or -7-yl; isothiazolidin-2- or -3- or -4- or -5-yl; 2,3-dihydroisothiazol-2- or -3- or -4- or -5-yl; 2,5-dihydroisothiazol-2- or -3- or -4- or -5-yl; 4,5-dihydroisothiazol-3- or -4- or -5-yl; 1,3-thiazolidin-2- or - 3- or -4- or -5-yl; 2,3-dihydro-1,3-thiazol-2- or -3- or -4- or -5-yl; 2,5-dihydro-1,3-thiazol-2- or -4- or -5-yl; 4,5-dihydro-1,3-thiazol-2- or -4- or -5-yl; 1,3-thiazinan-2- or -3- or -4- or -5- or -6-yl; 3,4-dihydro-2H-1,3-thiazin-2- or -3- or -4- or -5- or -6-yl; 3,6-dihydro-2H-1,3-thiazin-2- or -3- or -4- or -5- or -6-yl; 5,6-dihydro-2H-1,3-thiazin-2- or -4- or -5- or -6-yl; 5,6-dihydro-4H-1,3-thiazin-2- or -4- or -5- or -6-yl; 2H-1,3-thiazin-2- or -4- or -5- or -6-yl; 6H-1,3-thiazin-2- or -4- or -5- or -6-yl; 4H-1,3-thiazin-2- or -4- or -5- or -6-yl. Further examples of "heterocyclyl" are a partially or fully hydrogenated heterocyclic radical having 3 heteroatoms from the group of N, O and S, for example 1,4,2-dioxazolidin-2- or -3- or - 5-yl; 1,4,2-dioxazol-3- or -5-yl; 1,4,2-dioxazinan-2- or -3- or -5- or -6-yl; 5,6-dihydro-1,4,2-dioxazin-3- or -5- or -6-yl; 1,4,2-dioxazin-3- or -5- or -6-yl; 1,4,2-dioxazepan-2- or -3- or -5- or -6- or -7-yl; 6,7-dihydro-5H-1,4,2-dioxazepin-3- or -5- or -6- or -7-yl; 2,3-dihydro-7H-1,4,2-dioxazepin-2- or -3- or -5- or -6- or -7-yl; 2,3-dihydro-5H-1,4,2-dioxazepin-2- or -3- or -5- or -6- or -7-yl; 5H-1,4,2-dioxazepin-3- or -5- or -6- or -7-yl; 7H-1,4,2-dioxazepin-3- or -5- or -6- or -7-yl. Structural examples of heterocycles which are optionally substituted further are also listed below:

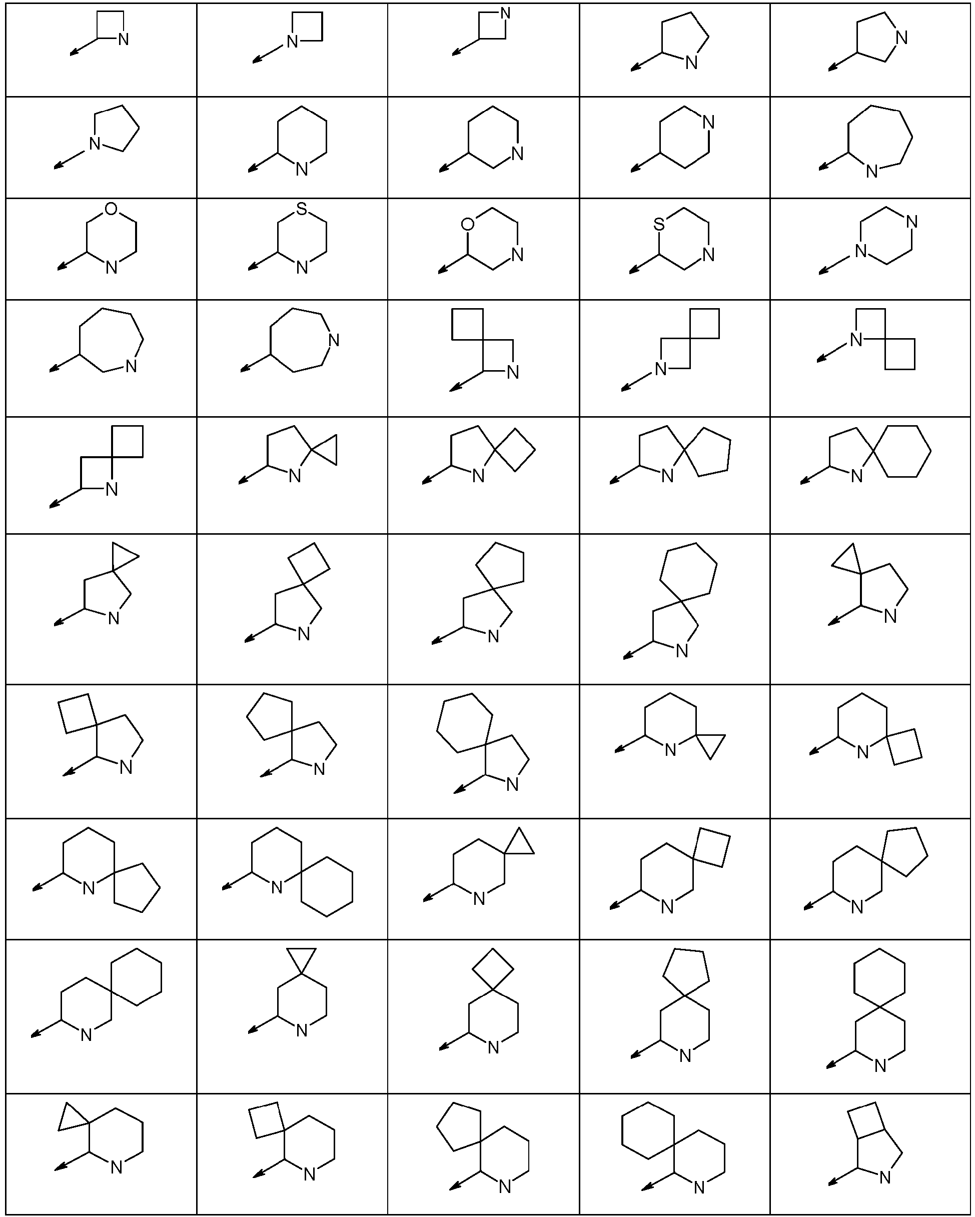

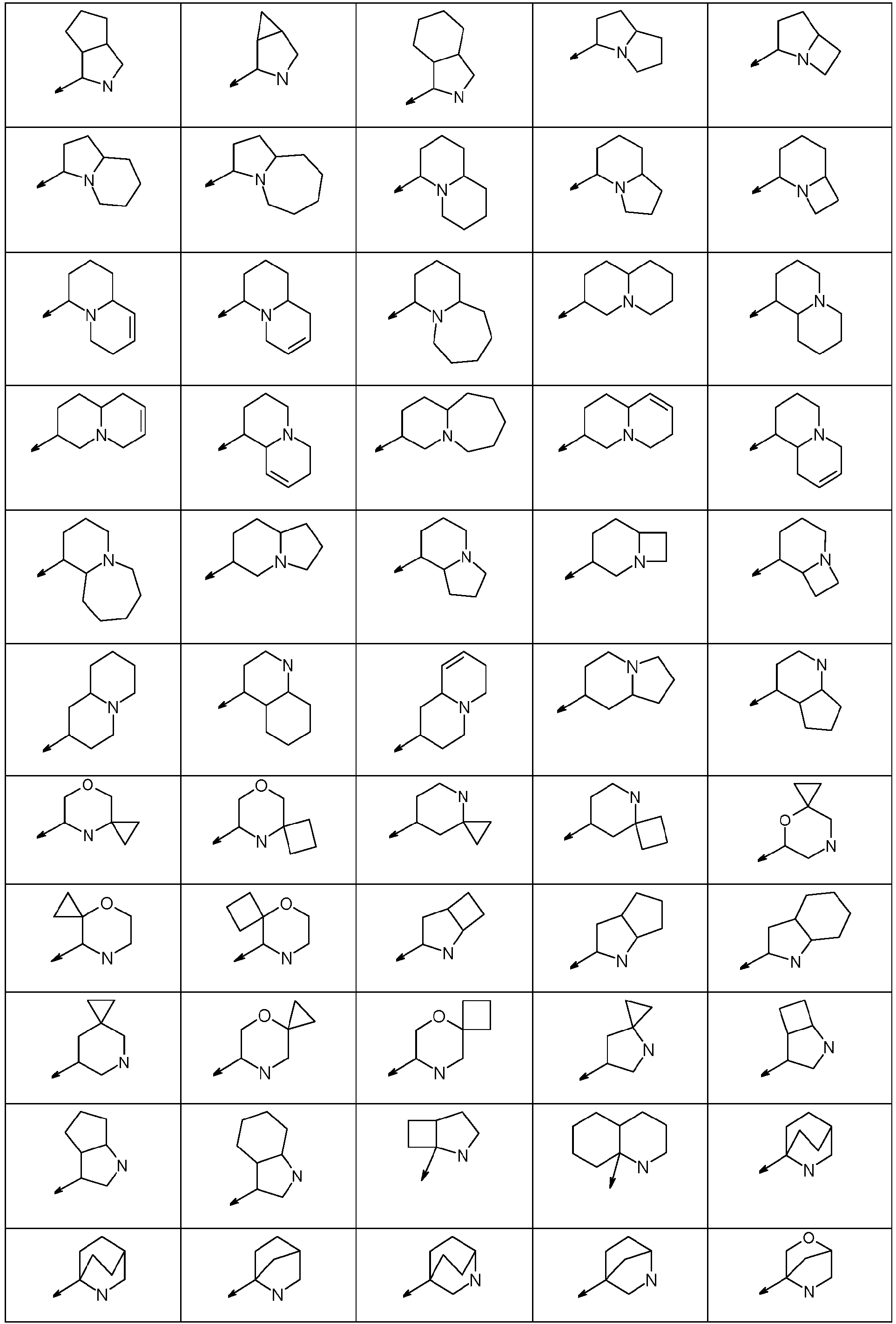

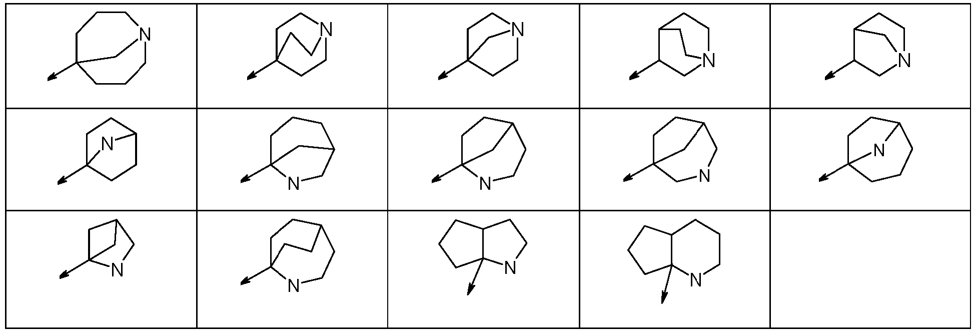

The heterocycles listed above are preferably substituted, for example, by hydrogen, halogen, alkyl, haloalkyl, hydroxyl, alkoxy, cycloalkoxy, aryloxy, alkoxyalkyl, alkoxyalkoxy, cycloalkyl, halocycloalkyl, aryl, arylalkyl, heteroaryl, heterocyclyl, alkenyl, alkylcarbonyl, cycloalkylcarbonyl, arylcarbonyl, heteroarylcarbonyl, alkoxycarbonyl, hydroxycarbonyl, cycloalkoxycarbonyl, cycloalkylalkoxycarbonyl, alkoxycarbonylalkyl, arylalkoxycarbonyl, arylalkoxycarbonylalkyl, alkynyl, alkynylalkyl, alkylalkynyl, trisalkylsilylalkynyl, nitro, amino, cyano, haloalkoxy, haloalkylthio, alkylthio, hydrothio, hydroxyalkyl, oxo, heteroarylalkoxy, arylalkoxy, heterocyclylalkoxy, heterocyclylalkylthio, heterocyclyloxy, heterocyclylthio, heteroaryloxy, bisalkylamino, alkylamino, cycloalkylamino, hydroxycarbonylalkylamino, alkoxycarbonylalkylamino, arylalkoxycarbonylalkylamino, alkoxycarbonylalkyl(alkyl)amino, aminocarbonyl, alkylaminocarbonyl, bisalkylaminocarbonyl, cycloalkylaminocarbonyl, hydroxycarbonylalkylaminocarbonyl, alkoxycarbonylalkylaminocarbonyl, arylalkoxycarbonylalkylaminocarbonyl.

When a base structure is substituted "by one or more radicals" from a list of radicals (= group) or a generically defined group of radicals, this in each case includes simultaneous substitution by a plurality of identical and/or structurally different radicals.

In the case of a partially or fully saturated nitrogen heterocycle, this may be joined to the remainder of the molecule either via carbon or via the nitrogen.

Suitable substituents for a substituted heterocyclic radical are the substituents specified further down, and additionally also oxo and thioxo. The oxo group as a substituent on a ring carbon atom is then, for example, a carbonyl group in the heterocyclic ring. As a result, lactones and lactams are preferably also included. The oxo group may also occur on the ring heteroatoms, which may exist in different oxidation states, for example in the case of N and S, and in that case form, for example, the divalent -N(O)-, - S(O)- (also SO for short) and -S(O)₂- (also SO₂ for short) groups in the heterocyclic ring. In the case of - N(O)- and -S(O)- groups, both enantiomers in each case are included.

According to the invention, the expression "heteroaryl" refers to heteroaromatic compounds, i.e. fully unsaturated aromatic heterocyclic compounds, preferably 5- to 7-membered rings having 1 to 4, preferably 1 or 2, identical or different heteroatoms, preferably O, S or N. Inventive heteroaryls are, for example, 1H-pyrrol-1-yl; 1H-pyrrol-2-yl; 1H-pyrrol-3-yl; furan-2-yl; furan-3-yl; thien-2-yl; thien-3-yl, 1H-imidazol-1-yl; 1H-imidazol-2-yl; 1H-imidazol-4-yl; 1H-imidazol-5-yl; 1H-pyrazol-1-yl; 1H-pyrazol-3-yl; 1H-pyrazol-4-yl; 1H-pyrazol-5-yl, 1H-1,2,3-triazol-1-yl, 1H-1,2,3-triazol-4-yl, 1H-1,2,3-triazol-5-yl, 2H-1,2,3-triazol-2-yl, 2H-1,2,3-triazol-4-yl, 1H-1,2,4-triazol-1-yl, 1H-1,2,4-triazol-3-yl, 4H-1,2,4-triazol-4-yl, 1,2,4-oxadiazol-3-yl, 1,2,4-oxadiazol-5-yl, 1,3,4-oxadiazol-2-yl, 1,2,3-oxadiazol-4-yl, 1,2,3-oxadiazol-5-yl, 1,2,5-oxadiazol-3-yl, azepinyl, pyridin-2-yl, pyridin-3-yl, pyridin-4-yl, pyrazin-2-yl, pyrazin-3-yl, pyrimidin-2-yl, pyrimidin-4-yl, pyrimidin-5-yl, pyridazin-3-yl, pyridazin-4-yl, 1,3,5-triazin-2-yl, 1,2,4-triazin-3-yl, 1,2,4-triazin-5-yl, 1,2,4-triazin-6-yl, 1,2,3-triazin-4-yl, 1,2,3-triazin-5-yl, 1,2,4-, 1,3,2-, 1,3,6- and 1,2,6-oxazinyl, isoxazol-3-yl, isoxazol-4-yl, isoxazol-5-yl, 1,3-oxazol-2-yl, 1,3-oxazol-4-yl, 1,3-oxazol-5-yl, isothiazol-3-yl, isothiazol-4-yl, isothiazol-5-yl, 1,3-thiazol-2-yl, 1,3-thiazol-4-yl, 1,3-thiazol-5-yl, oxepinyl, thiepinyl, 1,2,4-triazolonyl and 1,2,4-diazepinyl, 2H-1,2,3,4-tetrazol-5-yl, 1H-1,2,3,4-tetrazol-5-yl, 1,2,3,4-oxatriazol-5-yl, 1,2,3,4-thiatriazol-5-yl, 1,2,3,5-oxatriazol-4-yl, 1,2,3,5-thiatriazol-4-yl. The heteroaryl groups of the invention may also be substituted by one or more identical or different radicals. If two adjacent carbon atoms are part of a further aromatic ring, the systems are fused heteroaromatic systems, such as benzofused or polyannelated heteroaromatics. Preferred examples are quinolines (e.g. quinolin-2-yl, quinolin-3-yl, quinolin-4-yl, quinolin-5-yl, quinolin-6-yl, quinolin-7-yl, quinolin-8-yl); isoquinolines (e.g. isoquinolin-1-yl, isoquinolin-3-yl, isoquinolin-4-yl, isoquinolin-5-yl, isoquinolin-6-yl, isoquinolin-7-yl, isoquinolin-8-yl); quinoxaline; quinazoline; cinnoline; 1,5-naphthyridine; 1,6-naphthyridine; 1,7-naphthyridine; 1,8-naphthyridine; 2,6-naphthyridine; 2,7-naphthyridine; phthalazine; pyridopyrazines; pyridopyrimidines; pyridopyridazines; pteridines; pyrimidopyrimidines. Examples of heteroaryl are also 5- or 6-membered benzofused rings from the group of 1H-indol-1-yl, 1H-indol-2-yl, 1H-indol-3-yl, 1H-indol-4-yl, 1H-indol-5-yl, 1H-indol-6-yl, 1H-indol-7-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, 1H-indazol-1-yl, 1H-indazol-3-yl, 1H-indazol-4-yl, 1H-indazol-5-yl, 1H-indazol-6-yl, 1H-indazol-7-yl, 2H-indazol-2-yl, 2H-indazol-3-yl, 2H-indazol-4-yl, 2H-indazol-5-yl, 2H-indazol-6-yl, 2H-indazol-7-yl, 2H-isoindol-2-yl, 2H-isoindol-1-yl, 2H-isoindol-3-yl, 2H-isoindol-4-yl, 2H-isoindol-5-yl, 2H-isoindol-6-yl; 2H-isoindol-7-yl, 1H-benzimidazol-1-yl, 1H-benzimidazol-2-yl, 1H-benzimidazol-4-yl, 1H-benzimidazol-5-yl, 1H-benzimidazol-6-yl, 1H-benzimidazol-7-yl, 1,3-benzoxazol-2-yl, 1,3-benzoxazol-4-yl, 1,3-benzoxazol-5-yl, 1,3-benzoxazol-6-yl, 1,3-benzoxazol-7-yl, 1,3-benzothiazol-2-yl, 1,3-benzothiazol-4-yl, 1,3-benzothiazol-5-yl, 1,3-benzothiazol-6-yl, 1,3-benzothiazol-7-yl, 1,2-benzisoxazol-3-yl, 1,2-benzisoxazol-4-yl, 1,2-benzisoxazol-5-yl, 1,2-benzisoxazol-6-yl, 1,2-benzisoxazol-7-yl, 1,2-benzisothiazol-3-yl, 1,2-benzisothiazol-4-yl, 1,2-benzisothiazol-5-yl, 1,2-benzisothiazol-6-yl, 1,2-benzisothiazol-7-yl.

The term "halogen" denotes, for example, fluorine, chlorine, bromine or iodine. If the term is used for a radical, "halogen" denotes, for example, a fluorine, chlorine, bromine or iodine atom.

According to the invention, "alkyl" means a straight-chain or branched open-chain, saturated hydrocarbon radical which is optionally mono- or polysubstituted, and in the latter case is referred to as "substituted alkyl". Preferred substituents are halogen atoms, alkoxy, haloalkoxy, cyano, alkylthio, haloalkylthio, amino or nitro groups, particular preference being given to methoxy, methyl, fluoroalkyl, cyano, nitro, fluorine, chlorine, bromine or iodine. The prefix "bis" also includes the combination of different alkyl radicals, e.g. methyl(ethyl) or ethyl(methyl).

"Haloalkyl", "-alkenyl" and "-alkynyl" respectively denote alkyl, alkenyl and alkynyl partially or fully substituted by identical or different halogen atoms, for example monohaloalkyl such as CH₂CH₂Cl, CH₂CH₂Br, CHClCH₃, CH₂Cl, CH₂F; perhaloalkyl such as CCl₃, CClF₂, CFCl₂, CF₂CClF₂, CF₂CClFCF₃; polyhaloalkyl such as CH₂CHFCl, CF₂CClFH, CF₂CBrFH, CH₂CF₃; the term perhaloalkyl also encompasses the term perfluoroalkyl.

"Partially fluorinated alkyl" denotes a straight-chain or branched, saturated hydrocarbon which is mono- or polysubstituted by fluorine, where the fluorine atoms in question may be present as substituents on one or more different carbon atoms of the straight-chain or branched hydrocarbon chain, for example CHFCH₃, CH₂CH₂F, CH₂CH₂CF₃, CHF₂, CH₂F, CHFCF₂CF₃.

"Partially fluorinated haloalkyl" denotes a straight-chain or branched, saturated hydrocarbon which is substituted by different halogen atoms with at least one fluorine atom, where any other halogen atoms optionally present are selected from the group consisting of fluorine, chlorine or bromine, iodine. The corresponding halogen atoms may be present as substituents on one or more different carbon atoms of the straight-chain or branched hydrocarbon chain. Partially fluorinated haloalkyl also includes full substitution of the straight or branched chain by halogen including at least one fluorine atom.

"Haloalkoxy" is, for example, OCF₃, OCHF₂, OCH₂F, OCF₂CF₃, OCH₂CF₃ and OCH₂CH₂Cl; this applies correspondingly to haloalkenyl and other halogen-substituted radicals.

The expression "(C₁-C₄)-alkyl" mentioned here by way of example is a brief notation for straight-chain or branched alkyl having one to 4 carbon atoms according to the range stated for carbon atoms, i.e. encompasses the methyl, ethyl, 1-propyl, 2-propyl, 1-butyl, 2-butyl, 2-methylpropyl or tert-butyl radicals. General alkyl radicals with a larger specified range of carbon atoms, e.g. "(C₁-C₆)-alkyl", correspondingly also encompass straight-chain or branched alkyl radicals with a greater number of carbon atoms, i.e. according to the example also the alkyl radicals having 5 and 6 carbon atoms.

Unless stated specifically, preference is given to the lower carbon skeletons, for example having from 1 to 6 carbon atoms, or having from 2 to 6 carbon atoms in the case of unsaturated groups, in the case of the hydrocarbon radicals such as alkyl, alkenyl and alkynyl radicals, including in composite radicals. Alkyl radicals, including in composite radicals such as alkoxy, haloalkyl, etc., are, for example, methyl, ethyl, n-propyl or i-propyl, n-, i-, t- or 2-butyl, pentyls, hexyls such as n-hexyl, i-hexyl and 1,3-dimethylbutyl, heptyls such as n-heptyl, 1-methylhexyl and 1,4-dimethylpentyl; alkenyl and alkynyl radicals are defined as the possible unsaturated radicals corresponding to the alkyl radicals, where at least one double bond or triple bond is present. Preference is given to radicals having one double bond or triple bond.

The term "alkenyl" also includes, in particular, straight-chain or branched open-chain hydrocarbon radicals having more than one double bond, such as 1,3-butadienyl and 1,4-pentadienyl, but also allenyl or cumulenyl radicals having one or more cumulated double bonds, for example allenyl (1,2-propadienyl), 1,2-butadienyl and 1,2,3-pentatrienyl. Alkenyl denotes, for example, vinyl which may optionally be substituted by further alkyl radicals, for example (but not limited thereto) (C₂-C₆)-alkenyl such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

The term "alkynyl" also includes, in particular, straight-chain or branched open-chain hydrocarbon radicals having more than one triple bond, or else having one or more triple bonds and one or more double bonds, for example 1,3-butatrienyl or 3-penten-1-yn-1-yl. (C₂-C₆)-Alkynyl denotes, for example, ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl and 1-ethyl-1-methyl-2-propynyl.

The term "cycloalkyl" refers to a carbocyclic saturated ring system having preferably 3-8 ring carbon atoms, for example cyclopropyl, cyclobutyl, cyclopentyl or cyclohexyl, which optionally has further substitution, preferably by hydrogen, alkyl, alkoxy, cyano, nitro, alkylthio, haloalkylthio, halogen, alkenyl, alkynyl, haloalkyl, amino, alkylamino, bisalkylamino, alkoxycarbonyl, hydroxycarbonyl, arylalkoxycarbonyl, aminocarbonyl, alkylaminocarbonyl, cycloalkylaminocarbonyl. In the case of optionally substituted cycloalkyl, cyclic systems with substituents are included, also including substituents with a double bond on the cycloalkyl radical, for example an alkylidene group such as methylidene. In the case of optionally substituted cycloalkyl, polycyclic aliphatic systems are also included, for example bicyclo[1.1.0]butan-1-yl, bicyclo[1.1.0]butan-2-yl, bicyclo[2.1.0]pentan-1-yl, bicyclo[1.1.1]pentan-1-yl, bicyclo[2.1.0]pentan-2-yl, bicyclo[2.1.0]pentan-5-yl, bicyclo[2.1.1]hexyl, bicyclo[2.2.1]hept-2-yl, bicyclo[2.2.2]octan-2-yl, bicyclo[3.2.1]octan-2-yl, bicyclo[3.2.2]nonan-2-yl, adamantan-1-yl and adamantan-2-yl, but also systems such as 1,1'-bi(cyclopropyl)-1-yl, 1,1'-bi(cyclopropyl)-2-yl, for example. The term "(C₃-C₇)-cycloalkyl" is a brief notation for cycloalkyl having three to 7 carbon atoms, corresponding to the range specified for carbon atoms.

In the case of substituted cycloalkyl, spirocyclic aliphatic systems are also included, for example spiro[2.2]pent-1-yl, spiro[2.3]hex-1-yl, spiro[2.3]hex-4-yl, 3-spiro[2.3]hex-5-yl, spiro[3.3]hept-1-yl, spiro[3.3]hept-2-yl.

"Cycloalkenyl" denotes a carbocyclic, nonaromatic, partially unsaturated ring system having preferably 4-8 carbon atoms, e.g. 1-cyclobutenyl, 2-cyclobutenyl, 1-cyclopentenyl, 2-cyclopentenyl, 3-cyclopentenyl, or 1-cyclohexenyl, 2-cyclohexenyl, 3-cyclohexenyl, 1,3-cyclohexadienyl or 1,4-cyclohexadienyl, also including substituents with a double bond on the cycloalkenyl radical, for example an alkylidene group such as methylidene. In the case of optionally substituted cycloalkenyl, the elucidations for substituted cycloalkyl apply correspondingly.

The term "alkylidene", also, for example, in the form (C₁-C₁₀)-alkylidene, means the radical of a straight-chain or branched open-chain hydrocarbon radical which is bonded via a double bond. Possible bonding sites for alkylidene are naturally only positions on the base structure where two hydrogen atoms can be replaced by the double bond; radicals are, for example, =CH₂, =CH-CH₃, =C(CH₃)-CH₃, =C(CH₃)-C₂H₅ or =C(C₂H₅)-C₂H₅. Cycloalkylidene denotes a carbocyclic radical bonded via a double bond.

The term "alkylene", also, for example, in the form (C₁-C₈)-alkylene, denotes the radical of a straight-chain or branched open-chain hydrocarbon radical which is attached at two positions to further groups.

"Cycloalkylalkyloxy" denotes a cycloalkylalkyl radical bonded via an oxygen atom and "arylalkyloxy" denotes an arylalkyl radical bonded via an oxygen atom.

"Alkoxyalkyl" represents an alkoxy radical bonded via an alkyl group and "alkoxyalkoxy" denotes an alkoxyalkyl radical bonded via an oxygen atom, for example (but not limited to) methoxymethoxy, methoxyethoxy, ethoxyethoxy, methoxy-n-propyloxy.

"Alkylthioalkyl" represents an alkylthio radical bonded via an alkyl group and "alkylthioalkylthio" denotes an alkylthioalkyl radical bonded via an oxygen atom.

"Arylalkoxyalkyl" represents an aryloxy radical bonded via an alkyl group and "heteroaryloxyalkyl" denotes a heteroaryloxy radical bonded via an alkyl group.

"Haloalkoxyalkyl" represents a bonded haloalkoxy radical and "haloalkylthioalkyl" denotes a haloalkylthio radical, bonded via an alkyl group.

"Arylalkyl" represents an aryl radical bonded via an alkyl group, "heteroarylalkyl" denotes a heteroaryl radical bonded via an alkyl group, and "heterocyclylalkyl" denotes a heterocyclyl radical bonded via an alkyl group.

"Cycloalkylalkyl" represents a cycloalkyl radical bonded via an alkyl group, for example (but not limited thereto) cyclopropylmethyl, cyclobutylmethyl, cyclopentylmethyl, cyclohexylmethyl, 1-cyclopropyleth-1-yl, 2-cyclopropyleth-1-yl, 1-cyclopropylprop-1-yl, 3-cyclopropylprop-1-yl.

"Arylalkenyl" represents an aryl radical bonded via an alkenyl group, "heteroarylalkenyl" denotes a heteroaryl radical bonded via an alkenyl group, and "heterocyclylalkenyl" denotes a heterocyclyl radical bonded via an alkenyl group.

"Arylalkynyl" represents an aryl radical bonded via an alkynyl group, "heteroarylalkynyl" denotes a heteroaryl radical bonded via an alkynyl group, and "heterocyclylalkynyl" denotes a heterocyclyl radical bonded via an alkynyl group.

According to the invention, "haloalkylthio" - on its own or as constituent part of a chemical group - represents straight-chain or branched S-haloalkyl, preferably having 1 to 8, or having 1 to 6 carbon atoms, such as (C₁-C₈)-, (C₁-C₆)- or (C₁-C₄)-haloalkylthio, for example (but not limited thereto) trifluoromethylthio, pentafluoroethylthio, difluoromethyl, 2,2-difluoroeth-1-ylthio, 2,2,2-difluoroeth-1-ylthio, 3,3,3-prop-1-ylthio.

"Halocycloalkyl" and "halocycloalkenyl" denote cycloalkyl and cycloalkenyl, respectively, which are partially or fully substituted by identical or different halogen atoms, such as B, F, Cl and Br, or by haloalkyl, such as trifluoromethyl or difluoromethyl, for example 1-fluorocycloprop-1-yl, 2-fluorocycloprop-1-yl, 2,2-difluorocycloprop-1-yl, 1-fluorocyclobut-1-yl, 1-trifluoromethylcycloprop-1-yl, 2-trifluoromethylcycloprop-1-yl, 1-chlorocycloprop-1-yl, 2-chlorocycloprop-1-yl, 2,2-dichlorocycloprop-1-yl, 3,3-difluorocyclobutyl.

According to the invention, "trialkylsilyl" - on its own or as constituent part of a chemical group - represents straight-chain or branched Si-alkyl, preferably having 1 to 8, or having 1 to 6 carbon atoms, such as tri[(C₁-C₈)-, (C₁-C₆)- or (C₁-C₄)-alkyl]silyl, for example (but not limited thereto) trimethylsilyl, triethylsilyl, tri(n-propyl)silyl, tri(isopropyl)silyl, tri(n-butyl)silyl, tri(1-methylprop-1-yl)silyl, tri(2-methylprop-1-yl)silyl, tri(1,1-dimethyleth-1-yl)silyl, tri(2,2-dimethyleth-1-yl)silyl.

"Trialkylsilylalkynyl" represents a trialkylsilyl radical bonded via an alkynyl group.

Synthesis of substituted N-phenyl-N-aminouracils of the general formula (I).

If the compounds can form, through a hydrogen shift, tautomers whose structure is not formally covered by the general formula (I), these tautomers are nevertheless covered by the definition of the inventive compounds of the general formula (I), unless a particular tautomer is under consideration. For example, many carbonyl compounds may be present both in the keto form and in the enol form, both forms being encompassed by the definition of the compound of the general formula (I).

Depending on the nature of the substituents and the manner in which they are attached, the compounds of the general formula (I) may be present as stereoisomers. The general formula (I) embraces all possible stereoisomers defined by the specific three-dimensional form thereof, such as enantiomers, diastereomers, Z and E isomers. If, for example, one or more alkenyl groups are present, diastereomers (Z and E isomers) may occur. If, for example, one or more asymmetric carbon atoms are present, enantiomers and diastereomers may occur. Stereoisomers can be obtained from the mixtures obtained in the preparation by customary separation methods. The chromatographic separation can be affected either on the analytical scale to find the enantiomeric excess or the diastereomeric excess, or else on the preparative scale to produce test specimens for biological testing. It is likewise possible to selectively prepare stereoisomers by using stereoselective reactions with use of optically active starting materials and/or auxiliaries. The invention thus also relates to all stereoisomers which are embraced by the general formula (I) but are not shown in their specific stereomeric form, and to mixtures thereof.

If the compounds are obtained as solids, the purification can also be carried out by recrystallization or digestion. If individual compounds of general formula (I) cannot be obtained in a satisfactory manner by the routes described below, they can be prepared by derivatization of other compounds of general formula (I).

Suitable isolation methods, purification methods and methods for separating stereoisomers of compounds of the general formula (I) are methods generally known to the person skilled in the art from analogous cases, for example by physical processes such as crystallization, chromatographic methods, in particular column chromatography and HPLC (high pressure liquid chromatography), distillation, optionally under reduced pressure, extraction and other methods, any mixtures that remain can generally be separated by chromatographic separation, for example on chiral solid phases. Suitable for preparative amounts or on an industrial scale are processes such as crystallization, for example of diastereomeric salts which can be obtained from the diastereomer mixtures using optically active acids and, if appropriate, provided that acidic groups are present, using optically active bases.

### Synthesis of substituted formyl tertiary alcohol derivatives of the general formula (I):

The substituted formyl tertiary alcohol derivatives of the general formula (I) according to the invention can be prepared using known processes. The synthesis route used and examined proceed from commercially available or easily synthesised substituted amino aromatic (III) and substituted epoxy ester (IV) building blocks (Scheme 1). In the following schemes, the groups Q, G, R¹, R², R³, R⁴, R⁵ and R⁶ of the general formula (I) have the meanings defined above unless exemplary, but not limiting definitions are given and W = oxygen. The synthesis route for the substituted formyl tertiary alcohol derivatives of the general formula (I) proceeds from a substituted amino aromatic compound (III). The substituted amino aromatic compound (III) is reacted with a substituted epoxy ester (IV) in the presence of a suitable Lewis acid (e.g. LiOTf) to afford an α-hydroxyester (V) (cf. Synlett, 2023, 34, 2471-2475). Hydrolysis of the α-hydroxyester (V) under basic conditions (e.g. aq. NaOH in MeOH) affords α-hydroxyacid (VI), which is then coupled with a suitably substituted amine (VII) under appropriate conditions (including but not limited to EDC, HOBt, NEt₃ in CH₂Cl₂) to afford amino tertiary alcohols (VIII) (cf. Chem. Soc. Rev., 2009, 38, 606-631). In the following synthetic procedure amino tertiary alcohols (VIII) are treated with an appropriate electrophile in the presence of a suitable base (e.g. pyridine) and solvent (e.g. CH₂Cl₂) to furnish formyl tertiary alcohols (IX) (cf. Angew. Chem. Int. Ed., 2023, 62, e202218935). To complete the synthesis of formyl tertiary alcohol derivatives of the general formula (II), formyl tertiary alcohols (IX) are treated with an appropriate electrophile in the presence of a suitable base (e.g. K₂CO₃) and a compatible solvent (e.g. MeCN) with an optional additive (e.g. Ag₂O) (cf. J. Am. Chem. Soc., 1995, 117, 3389-3404).

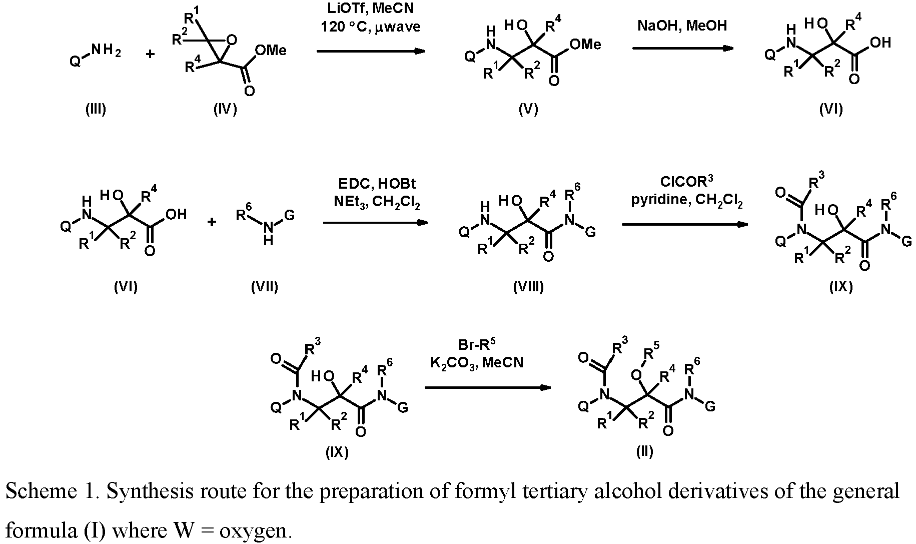

Selected detailed synthesis examples for the compounds of the general formula (I) according to the invention are given below. The example numbers mentioned correspond to the numbering scheme in Scheme 1 and Table 1. The ¹H NMR spectroscopy data reported for the chemical examples described in the sections that follow were obtained on Bruker instruments at 600 MHz, 400 MHz or 300 MHz using CDCl₃ or DMSO-*d*₆ as the solvent with tetramethylsilane (δ = 0.00 ppm) as the internal standard. The signals listed have the meanings given below: br = broad; s = singlet, d = doublet, t = triplet, dd = doublet of doublets, ddd = doublet of a doublet of doublets, m = multiplet, q = quartet, qu = quintet, sext = sextet, sept = septet, dq = doublet of quartets, dt = doublet of triplets.

### Synthesis examples:

### No. I-002: (1S,4R)-4-[[3-(3,5-difluoro-N-methoxycarbonyl-anilino)-2-hydroxy-2-methyl-propanoyl] amino]cyclopent-2-ene-1-carboxylate

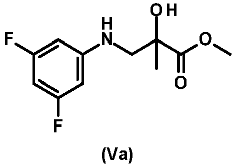

To a stirred mixture of 3,5-difluoroaniline (1.50 g, 11.6 mmol) and methyl 2-methyloxirane-2-carboxylate (1.75g, 15.1 mmol) in MeCN (15 mL) at room temperature was added lithium trifluoromethanesulfonate (2.72 g, 17.4 mmol) and the resulting mixture heated to 110 °C in the microwave for 8 h. The reaction mixture was cooled to room temperature and the solvent removed *in vacuo.* The resulting residue was purified by silica gel column chromatography, eluting with heptane/EtOAc (7:3) to afford the desired product Va (2.60 g, 87%). ¹H NMR (400 MHz, CDCl₃): δ_{H} 6.20-6.11 (m, 3 H), 4.26 (br s, 1 H), 3.79 (s, 3 H), 3.56-3.46 (m, 2 H), 3.22 (dd, *J =* 12.7, 3.9 Hz, 1 H), 1.48 (s, 3 H).

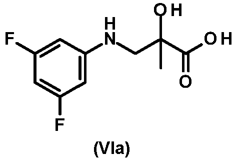

To a solution of compound Va (2.40 g, 9.79 mmol) in methanol (40 mL) was added 2 N aq. NaOH solution (5.87 mL, 11.7 mmol) and the resulting mixture stirred at room temperature for 48 h. The solvent was then removed *in vacuo* and the residue re-dissolved in water. This solution was acidified with 2 N aq. HCl solution, extracted with EtOAc (75 mL), and the layers separated. The organic layer was dried over anhydrous sodium sulfate, filtered, and the solvent removed *in vacuo* to afford the desired product VIa (2.30 g, 97%). ¹H NMR (400 MHz, DMSO-*d*₆): δ_{H} 12.64 (s, 1 H), 6.33 (br d, *J* = 2.5 Hz, 1 H), 6.32-6.29 (m, 1 H), 6.18 (tt, *J =* 9.5, 2.3 Hz, 1 H), 3.35 (d, *J =* 13.0 Hz, 1 H), 3.09 (d, *J =* 13.4 Hz, 1 H), 1.30 (s, 3 H).

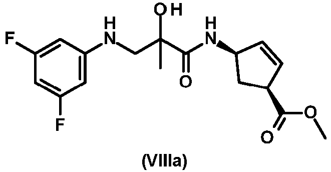

To a solution of compound VIa (1.30 g, 5.62 mmol) in CH₂Cl₂ (30 mL) was added HOBt (0.760 g, 5.62 mmol) and the resulting mixture stirred for 30 min. (1S,4R)-4-aminocyclopent-2-ene-1-carboxylate (1.50 g, 8.43 mmol) was then added, followed by triethylamine (1.57 mL, 11.25 mmol) and EDCI (1.40 g, 7.31 mmol) and the resulting mixture stirred for 48 h at room temperature. Water was then added, the organic layer separated using a phase separation column and the solvent removed *in vacuo.* The crude residue was purified by silica gel column chromatography, eluting with heptane/EtOAc (7:3) to afford the desired product VIIIa (1.90 g, 95%). ¹H NMR (400 MHz, CDCl₃): δ_{H} 7.21-7.10 (m, 1 H), 6.19-6.10 (m, 3 H), 5.93-5.82 (m, 2 H), 5.04-4.94 (m, 1 H), 4.38-4.24 (m, 1 H), 3.72 (d, *J=* 1.0 Hz, 3 H), 3.57-3.49 (m, 2 H), 3.21-3.08 (m, 2 H), 2.40 (ddt, *J =* 14.0, 11.5, 8.4, 8.4 Hz, 1 H), 1.87 (tt, *J =* 13.6, 2.7 Hz, 1 H), 1.46 (d, *J=* 3.5 Hz, 3 H).

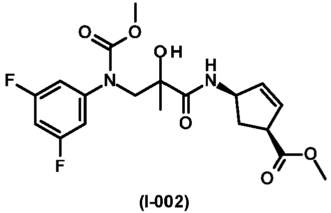

To a solution of compound VIIIa (50 mg, 0.14 mmol) in CH₂Cl₂ (5 mL) under argon at room temperature was added pyridine (0.04 mL, 0.45 mmol) and the resulting solution stirred for 5 min before methyl chloroformate (0.03 mL, 0.42 mmol) was added. The reaction mixture was stirred under continued conditions for a further 30 min, at which time it was diluted with CH₂Cl₂ and washed with 2 N aq. HCl solution followed by satd. aq. NaCl solution. The layers were separated using a phase separation column and the organic phase concentrated *in vacuo* to afford the desired product I-002 (45 mg, 73%).

### No. I-054: (E,4S)-4-[[2-[(3,5-difluoro-N-methoxycarbonyl-anilino)methyl]-2-hydroxy-but-3-enoyl] amino]pent-2-enoate

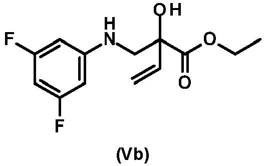

To a stirred mixture of 3,5-difluoroaniline (2.00 g, 15.5 mmol) and ethyl 2-vinyloxirane-2-carboxylate (2.86 g, 20.1 mmol) in MeCN (15 mL) at room temperature was added lithium trifluoromethanesulfonate (3.63 g, 23.2 mmol) and the resulting mixture heated to 110 °C in the microwave for 8 h. The reaction mixture was cooled to room temperature and the solvent removed *in vacuo.* The resulting residue was purified by silica gel column chromatography, eluting with heptane/EtOAc (7:3) to afford the desired product Vb (3.20 g, 72%). ¹H NMR (400 MHz, CDCl₃): δ_{H} 6.17-6.10 (m, 3 H), 5.99 (dd, *J =* 17.1, 10.5 Hz, 1 H), 5.62 (d, *J =* 17.0 Hz, 1 H), 5.32 (d, *J =* 10.5 Hz, 1 H), 4.28-4.15 (m, 3 H), 3.65-3.55 (m, 2 H), 3.18 (dd, *J =* 12.7, 3.3 Hz, 1 H), 1.26 (t, *J =* 7.2 Hz, 3 H).

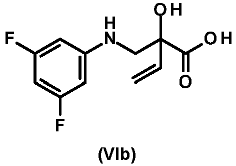

To a solution of compound Vb (1.20 g, 4.42 mmol) in ethanol (40 mL) was added 2 N aq. NaOH (4.42 mL, 8.85 mmol) solution and the resulting mixture stirred at room temperature for 18 h. The solvent was then removed *in vacuo* and the residue re-dissolved in water. This solution was acidified with 2 N aq. HCl solution, extracted with EtOAc (75 mL), and the layers separated. The organic layer was dried over anhydrous sodium sulfate, filtered, and the solvent removed *in vacuo* to afford the desired product VIb (1.00 g, 88%). ¹H NMR (400 MHz, CDCl₃): δ_{H} 6.20-6.14 (m, 3 H), 6.04 (dd, *J =* 17.1, 10.6 Hz, 1 H), 5.66 (dd, *J =* 17.1, 0.9 Hz, 1 H), 5.39 (dd, *J =* 10.6, 0.9 Hz, 1 H), 3.66 (d, *J =* 12.9 Hz, 1 H), 3.26 (d, *J =* 12.8 Hz, 1 H).

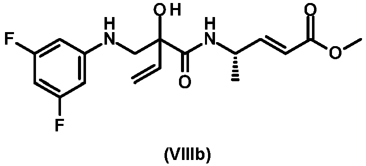

To a solution of compound VIb (0.15 g, 0.62 mmol) in CH₂Cl₂ (80 mL) was added HOBt (0.83 g, 0.62 mmol) and the resulting mixture stirred for 30 min. Simultaneously, methyl (2*R*,4*S*)-4-((*tert-*butoxycarbonyl)amino)pent-2-enoate (0.21 g, 0.93 mmol) was dissolved in CH₂Cl₂ (1 mL), to which was added TFA (2 mL) and the resulting mixture stirred for 15 min. The mixture was then diluted with CH₂Cl₂ (10 mL) and washed with satd. aq. NaHCO₃ solution. The organic layer was separated using a phase separator and the solvent removed *in vacuo* to afford the free amine. The free amine was directly added to the reaction mixture containing Compound VIb, followed by triethylamine (0.17 mL, 1.23 mmol) and EDCI (0.15 g, 0.80 mmol) and the resulting mixture stirred for 18 h at room temperature. Water was then added, the organic layer separated using a phase separation column and the solvent removed *in vacuo.* The crude residue was purified by silica gel column chromatography, eluting with heptane/EtOAc (7:3) to afford the desired product VIIIb (180 mg, 82%). ¹H NMR (400 MHz, CDCl₃): δ_{H} 6.85 (ddd, *J* = 15.7, 9.5, 4.9 Hz, 1 H), 6.73 (br t, *J =* 8.1 Hz, 1 H), 6.22-6.08 (m, 4 H), 5.85 (ddd, *J =* 15.8, 14.2, 1.8 Hz, 1 H), 5.53 (dd, *J =* 17.3, 3.3 Hz, 1 H), 5.36 (dd, *J =* 10.8, 5.7 Hz, 1 H), 4.67 (ddddd, *J =* 8.5, 6.8, 5.2, 3.3, 1.6 Hz, 1 H), 3.75-3.64 (m, 4 H), 3.28 (dd, *J =* 13.2, 5.2 Hz, 1 H), 3.03 (br s, 1 H), 1.33-1.22 (m, 4 H).

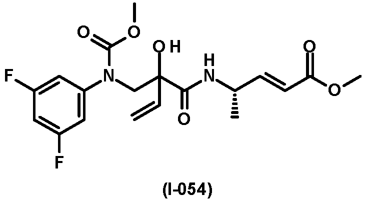

To a solution of compound VIIIb (0.15 g, 0.42 mmol) in CH₂Cl₂ (12 mL) under argon at room temperature was added pyridine (0.11 mL, 1.35 mmol) and the resulting solution stirred for 5 min before methyl chloroformate (0.10 mL, 1.27 mmol) was added. The reaction mixture was stirred under continued conditions for a further 30 min, at which time it was diluted with CH₂Cl₂ and washed with 2 N aq. HCl solution followed by satd. aq. NaCl solution. The layers were separated using a phase separation column and the organic phase concentrated *in vacuo* to afford the desired product I-054 (0.18 g, 98%).

### No. I-047: methyl (1R,3S)-3-[[2-[(3,5-difluoro-N-methoxycarbonyl-anilino)methyl]-3-fluoro-2-hydroxy-propanoyl]amino]cyclopentanecarboxylate

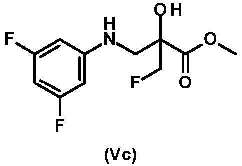

To a stirred mixture of 3,5-difluoroaniline (1.00 g, 7.75 mmol) and methyl 2-(fluoromethyl)oxirane-2-carboxylate (1.35 g, 10.07 mmol) in MeCN (20 mL) at room temperature was added lithium trifluoromethanesulfonate (1.81 g, 11.6 mmol) and the resulting mixture heated to 120 °C in the microwave for 4 h. The reaction mixture was cooled to room temperature and the solvent removed *in vacuo.* The resulting residue was dissolved in EtOAc (60 mL), washed twice with water before being washed with brine. The organic phase was collected, dried over anhydrous sodium sulfate, filtered and concentrated *in vacuo* to afford the crude product. The crude product was purified by silica gel column chromatography, eluting with heptane/EtOAc (7:3) to afford the desired product Vc (1.60 g, 76%). ¹H NMR (400 MHz, CDCl₃): δ_{H} 6.21-6.11 (m, 3 H), 4.68 (d, *J =* 9.4 Hz, 0.5 H), 4.56 (dd, *J* = 9.4, 1.7 Hz, 1 H), 4.44 (d, *J =* 9.4 Hz, 0.5 H), 4.22 (br s, 1 H), 3.83 (s, 3 H), 3.68 (s, 1 H), 3.51 (d, *J* = 12.8 Hz, 1 H), 3.29 (d, *J =* 12.8 Hz, 1 H).

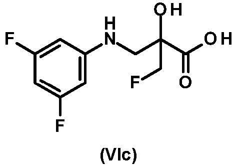

To a solution of compound Vc (1.60 g, 6.08 mmol) in methanol (85 mL) was added 2 N aq. NaOH (6.08 mL, 12.2 mmol) solution and the resulting mixture stirred at room temperature for 18 h. The solvent was then removed *in vacuo* and the residue redissolved in water. This solution was acidified with 2 N aq. HCl solution, extracted with EtOAc (50 mL), and the layers separated. The organic layer was dried over anhydrous sodium sulfate, filtered, and the solvent removed *in vacuo* to afford the desired product VIc (1.60 g, quant.). ¹H NMR (400 MHz, DMSO-*d*₆): δ_{H} 13.41-12.91 (m, 1 H), 6.37-6.30 (m, 2 H), 6.30-6.14 (m, 2 H), 5.83-5.65 (m, 1 H), 4.67-4.62 (m, 0.5 H), 4.53 (d, *J =* 8.9 Hz, 1 H), 4.41 (d, *J =* 9.3 Hz, 0.5 H), 3.47-3.29 (m, 1 H), 3.28-3.14 (m, 1 H).

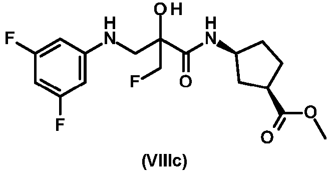

To a solution of compound VIc (200 mg, 0.80 mmol) in CH₂Cl₂ (20 mL) was added HOBt (108 mg, 0.80 mmol) and the resulting mixture stirred for 30 min. Methyl (1R,3S)-3-aminocyclopentanecarboxylate (216 mg, 1.20 mmol) was then added, followed by triethylamine (0.22 mL, 1.61 mmol) and EDCI (200 mg, 1.04 mmol) and the resulting mixture stirred for 18 h at room temperature. Water was then added, the organic layer separated using a phase separation column and the solvent removed *in vacuo.* The crude residue was purified by silica gel column chromatography, eluting with heptane/EtOAc (7:3) to afford the desired product VIIIc (36 mg, 11%). ¹H NMR (400 MHz, CDCl₃): δ_{H} 7.53-7.44 (m, 1 H), 6.20-6.12 (m, 3 H), 4.71-4.65 (m, 0.5 H), 4.60-4.52 (m, 1 H), 4.45-4.29 (m, 2.5 H), 3.72 (d, *J =* 1.3 Hz, 3 H), 3.70-3.68 (m, 0.5 H), 3.63 (s, 0.5 H), 3.57-3.47 (m, 1 H), 3.35 (br d, *J* = 13.0 Hz, 1 H), 2.98-2.91 (m, 1 H), 2.14-2.00 (m, 2 H), 1.93-1.67 (m, 4 H).

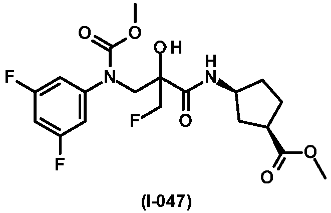

To a solution of compound VIIIc (88 mg, 0.24 mmol) in CH₂Cl₂ (10 mL) under argon at room temperature was added pyridine (0.06 mL, 0.75 mmol) and the resulting solution stirred for 5 min before methyl chloroformate (0.05 mL, 0.71 mmol) was added. The reaction mixture was stirred under continued conditions for a further 30 min, at which time it was diluted with CH₂Cl₂ and washed with 2 N aq. HCl solution followed by satd. aq. NaCl solution. The layers were separated using a phase separation column and the organic phase concentrated *in vacuo* to afford the desired product I-047 (19 mg, 18%).

### No. I-036: (E,4S)-4-[[2-[(3,5-dichloro-N-methoxycarbonyl-anilino)methyl]-2-hydroxy-butanoyl]amino] pent-2-enoate

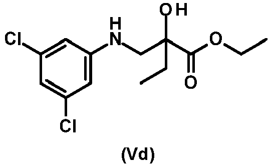

To a stirred mixture of 3,5-dichloroaniline (1.30 g, 8.02 mmol) and ethyl 2-ethyloxirane-2-carboxylate (1.27 g, 8.83 mmol) in MeCN (20 mL) at room temperature was added lithium trifluoromethanesulfonate (1.88 g, 12.0 mmol) and the resulting mixture heated to 120 °C in the microwave for 3 h. The reaction mixture was cooled to room temperature and the solvent removed *in vacuo.* The resulting residue was purified by silica gel column chromatography, eluting with heptane/EtOAc (7:3) to afford the desired product Vd (0.90 g, 37%). ¹H NMR (400 MHz, CDCl₃): δ_{H} 6.66 (t*, J =* 1.7 Hz, 1 H), 6.49 (d, *J =* 1.9 Hz, 2 H), 4.28-4.12 (m, 2 H), 3.53 (br d, *J* = 12.7 Hz, 3 H), 3.18 (d, *J* = 12.7 Hz, 1 H), 1.85-1.68 (m, 2 H), 1.26 (t, *J* = 7.2 Hz, 3 H), 0.90 (t, *J =* 7.5 Hz, 3 H).

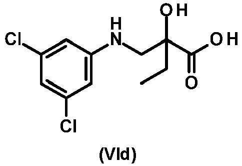

To a solution of compound Vd (1.30 g, 4.25 mmol) in ethanol (22 mL) was added 2 N aq. NaOH (8.49 mL, 16.98 mmol) solution and the resulting mixture stirred at room temperature for 18 h. The solvent was then removed *in vacuo* and the residue redissolved in water. This solution was acidified with 2 N aq. HCl solution, extracted with CH₂Cl₂/MeOH 9:1, and the layers separated. The organic layer was dried over anhydrous sodium sulfate, filtered, and the solvent removed *in vacuo* to afford the desired product VId (1.14 g, 94%). ¹H NMR (400 MHz, DMSO-*d*₆): δ_{H} 12.91-12.57 (m, 1 H), 6.68 (d, *J =* 1.9 Hz, 2 H), 6.57 (t, *J =* 1.7 Hz, 1 H), 6.09 (br s, 1 H), 3.40 (br d, *J =* 13.4 Hz, 2 H), 3.08 (d, *J =* 13.0 Hz, 1 H), 1.70 (dd, *J* = 13.7, 7.3 Hz, 1 H), 1.64-1.54 (m, 1 H), 0.83 (t, *J =* 7.5 Hz, 3 H).

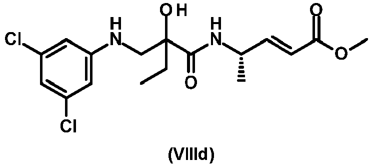

To a solution of compound VId (0.10 g, 0.36 mmol) in CH₂Cl₂ (5 mL) was added HOBt (0.05 g, 0.36 mmol) and the resulting mixture stirred for 30 min. Simultaneously, methyl (2*R*,4*S*)-4-((*tert-*butoxycarbonyl)amino)pent-2-enoate (0.12 g, 0.54 mmol) was dissolved in CH₂Cl₂ (1 mL), to which was added TFA (2 mL) and the resulting mixture stirred for 15 min. The mixture was then diluted with CH₂Cl₂ (10 mL) and washed with satd. aq. NaHCO₃ solution. The organic layer was separated using a phase separator and the solvent removed *in vacuo* to afford the free amine. The free amine was directly added to the reaction mixture containing Compound VId, followed by triethylamine (0.20 mL, 1.44 mmol) and EDCI (0.09 g, 0.47 mmol) and the resulting mixture stirred for 18 h at room temperature. Water was then added, the organic layer separated using a phase separation column and the solvent removed *in vacuo.* The crude residue was purified by silica gel column chromatography, eluting with heptane/EtOAc (7:3) to afford the desired product VIIId (0.11 g, 76%). ¹H NMR (400 MHz, CDCl₃): δ_{H} 6.87 (ddd, *J* = 15.7, 6.1, 4.9 Hz, 1 H), 6.82-6.72 (m, 1 H), 6.72-6.70 (m, 1 H), 6.57-6.53 (m, 2 H), 5.88 (ddd, *J* = 15.7, 9.9, 1.7 Hz, 1 H), 4.77-4.67 (m, 1 H), 4.25-4.00 (m, 1 H), 3.74 (d, *J* = 4.8 Hz, 3 H), 3.60 (dd, *J =* 15.9, 13.0 Hz, 1 H), 3.19-3.14 (m, 1 H), 2.75-2.63 (m, 1 H), 1.99-1.89 (m, 1 H), 1.67 (dqd, *J* = 14.4, 7.3, 7.3, 7.3, 4.3 Hz, 1 H), 1.29 (dd, *J* = 10.2, 7.0 Hz, 3 H), 0.95 (t, *J* = 7.6 Hz, 3 H).

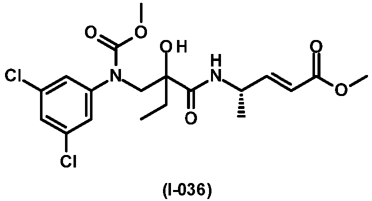

To a solution of compound VIIId (0.11 g, 0.28 mmol) in CH₂Cl₂ (5.6 mL) under argon at room temperature was added pyridine (0.07 mL) and the resulting solution stirred for 5 min before methyl chloroformate (0.07 mL, 0.85 mmol) was added. The reaction mixture was stirred under continued conditions for a further 2 h, at which time it was diluted with CH₂Cl₂ and washed with 2 N aq. HCl solution followed by satd. aq. NaCl solution. The layers were separated using a phase separation column and the organic phase concentrated *in vacuo* to afford the desired product I-036 (0.05 g, 35%).

### No. I-035: (E,4S)-4-[[2-cyclopropyl-3-(3,5-dichloro-N-methoxycarbonyl-anilino)-2-hydroxy-propanoyl] amino]pent-2-enoate

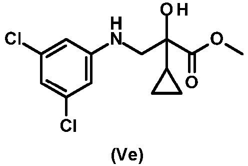

To a stirred mixture of 3,5-dichloroaniline (1.90 g, 11.7 mmol) and methyl 2-cyclopropyloxirane-2-carboxylate (2.17g, 15.3 mmol) in MeCN (10 mL) at room temperature was added lithium trifluoromethanesulfonate (2.74 g, 17.6 mmol) and the resulting mixture heated to 120 °C in the microwave for 3 h. The reaction mixture was cooled to room temperature and the solvent removed *in vacuo.* The resulting residue was purified by silica gel column chromatography, eluting with heptane/EtOAc (7:3) to afford the desired product Ve (1.58 g, 43%). ¹H NMR (400 MHz, CDCl₃): δ_{H} 6.73-6.66 (m, 1 H), 6.54 (d, *J =* 1.7 Hz, 1 H), 6.51 (d, *J =* 1.7 Hz, 2 H), 3.78 (s, 3 H), 3.67 (d, *J* = 12.7 Hz, 1 H), 3.25 (d, *J =* 12.7 Hz, 1 H), 3.12 (br s, 1 H), 1.15 (tt, *J =* 8.3, 5.2 Hz, 1 H), 0.61-0.54 (m, 1 H), 0.49-0.32 (m, 3 H).

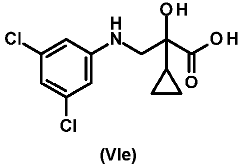

To a solution of compound Ve (1.80 g, 5.92 mmol) in methanol (50 mL) was added 2 N aq. NaOH (7.17 mL, 14.33 mmol) solution and the resulting mixture stirred at room temperature for 18 h. The solvent was then removed *in vacuo* and the residue redissolved in water. This solution was acidified with 6 N aq. HCl solution, extracted with EtOAc, and the layers separated. The organic layer was dried over anhydrous sodium sulfate, filtered, and the solvent removed *in vacuo* to afford the desired product VIe (1.22 g, 68%). ¹H NMR (400 MHz, CDCl₃): δ_{H} 6.73-6.69 (m, 1 H), 6.55 (d*, J =* 1.7 Hz, 2 H), 3.71 (d*, J =* 12.9 Hz, 1 H), 3.31 (d*, J =* 12.7 Hz, 1 H), 1.26-1.18 (m, 1 H), 0.65-0.57 (m, 1 H), 0.55-0.39 (m, 3 H).

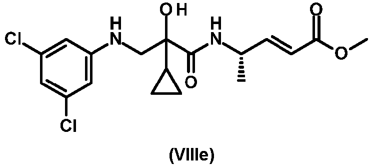

To a solution of compound VIe (0.20 g, 0.69 mmol) in CH₂Cl₂ (20 mL) was added HOBt (0.09 g, 0.69 mmol) and the resulting mixture stirred for 30 min. Simultaneously, methyl (2*R*,4*S*)-4-((*tert-*butoxycarbonyl)amino)pent-2-enoate (0.24 g, 1.03 mmol) was dissolved in CH₂Cl₂ (1 mL), to which was added TFA (2 mL) and the resulting mixture stirred for 15 min. The mixture was then diluted with CH₂Cl₂ (10 mL) and washed with satd. aq. NaHCO₃ solution. The organic layer was separated using a phase separator, and the solvent removed *in vacuo* to afford the free amine. The free amine was directly added to the reaction mixture containing Compound VIe, followed by triethylamine (0.19 mL, 1.38 mmol) and EDCI (0.20 g, 0.90 mmol) and the resulting mixture stirred for 18 h at room temperature. Water was then added, the organic layer separated using a phase separation column and the solvent removed *in vacuo.* The crude residue was purified by silica gel column chromatography, eluting with heptane/EtOAc (7:3) to afford the desired product VIIIe (0.113 g, 41%). ¹H NMR (400 MHz, CDCl₃): δ_{H} 6.88 (dt, *J =* 15.7, 4.9 Hz, 1 H), 6.75 (dt, *J =* 5.7, 1.7 Hz, 1 H), 6.60 (d, *J =* 1.6 Hz, 3 H), 5.88 (ddd, *J =* 15.7, 10.6, 1.8 Hz, 1 H), 4.75-4.67 (m, 1 H), 3.76-3.65 (m, 4 H), 3.30 (dd, *J =* 13.1, 3.6 Hz, 1 H), 2.84-2.72 (m, 1 H), 1.34-1.13 (m, 5 H), 0.60-0.45 (m, 4 H).

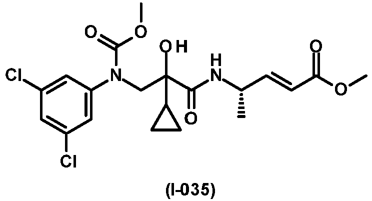

To a solution of compound VIIIe (0.11 g, 0.27 mmol) in CH₂Cl₂ (10 mL) under argon at room temperature was added pyridine (0.07 mL, 0.88 mmol) and the resulting solution stirred for 5 min before methyl chloroformate (0.06 mL, 0.82 mmol) was added. The reaction mixture was stirred under continued conditions for a further 4 h, at which time it was diluted with CH₂Cl₂ and washed with 2 N aq. HCl solution followed by satd. aq. NaCl solution. The layers were separated using a phase separation column and the organic phase concentrated *in vacuo* to afford the desired product I-035 (12 mg, 9%).

In analogy to the preparation examples cited above and recited at the appropriate point and taking account of the general details relating to the preparation of substituted formyl tertiary alcohol derivatives, the compounds cited below are obtained.

**Table 1: Examples of preferred compounds of the general formula (II)**

| 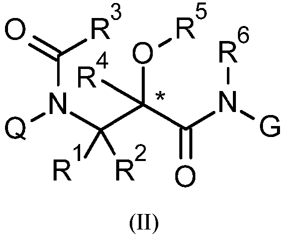 | | | | | | | | |
|---|---|---|---|---|---|---|---|---|
| **Ex. No.** | **R¹** | **R²** | **R³** | **R^{4*}** | **R⁵** | **R⁶** | **Q** | **G** |
| I-001 | H | H | methoxy | methyl | H | H | 3,5-difluorophenyl | rac-(3S,5R)-5-methoxycarbonyltetrahydrofuran-3-yl |
| I-002 | H | H | methoxy | methyl | H | H | 3,5-difluorophenyl | (1S,4S)-4-methoxycarbonylcyclopent-2-en-1-yl |
| I-003 | H | H | methoxy | methyl | H | H | 3,5-difluorophenyl | (E,1R)-4-ethoxy-1-methyl-4-oxo-but-2-enyl |
| I-004 | H | H | methoxy | methyl | H | H | 3,5-difluorophenyl | (1R)-3-ethoxy-1-methyl-3-oxopropyl |
| I-005 | H | H | methoxy | methyl | H | H | 3,5-dichlorophenyl | (1S,4S)-4-methoxycarbonylcyclopent-2-en-1-yl |
| I-006 | H | H | methoxy | methyl | H | H | 3,5-dichlorophenyl | (1R)-3-ethoxy-1-methyl-3-oxo-propyl |
| I-007 | H | H | methoxy | methyl | H | H | 3,5-dichlorophenyl | rac-(3S,5R)-5-methoxycarbonyltetrahydrofuran-3-yl |
| I-008 | H | H | methoxy | methyl | H | H | 3,5-dichlorophenyl | (E,1R)-4-ethoxy-1-methyl-4-oxo-but-2-enyl |
| I-009 | H | H | methyl | methyl | H | H | 3,5-dichlorophenyl | rac-(3S,5R)-5-methoxycarbonyltetrahydrofuran-3-yl |
| I-010 | H | H | methyl | methyl | H | H | 3,5-dichlorophenyl | (E,1R)-4-ethoxy-1-methyl-4-oxo-but-2-enyl |
| I-011 | H | H | methyl | methyl | H | H | 3,5-dichlorophenyl | (1S,4S)-4-methoxycarbonylcyclopent-2-en-1-yl |
| I-012 | H | H | methyl | methyl | H | H | 3,5-dichlorophenyl | (1R)-3-ethoxy-1-methyl-3-oxo-propyl |
| I-013 | H | H | cyclopropy l | methyl | H | H | 3,5-dichlorophenyl | (1S,4S)-4-methoxycarbonylcyclopent-2-en-1-yl |
| I-014 | H | H | cyclopropy l | methyl | H | H | 3,5-dichlorophenyl | (E,1R)-4-ethoxy-1-methyl-4-oxo-but-2-enyl |
| I-015 | H | H | methyl | methyl | H | H | 3,5-difluorophenyl | (1S,4S)-4-methoxycarbonylcyclopent-2-en-1-yl |
| I-016 | H | H | chlorometh yl | methyl | H | H | 3,5-difluorophenyl | (1S,4S)-4-methoxycarbonylcyclopent-2-en-1-yl |
| I-017 | H | H | cyclopropy l | methyl | H | H | 3,5-difluorophenyl | (1S,4S)-4-methoxycarbonylcyclopent-2-en-1-yl |
| I-018 | H | H | methoxyme thyl | methyl | H | H | 3,5-difluorophenyl | (1S,4S)-4-methoxycarbonylcyclopent-2-en-1-yl |
| I-019 | H | H | ethyl | methyl | H | H | 3,5-difluorophenyl | (1S,4S)-4-methoxycarbonylcyclopent-2-en-1-yl |
| I-020 | H | H | Ethoxy | methyl | H | H | 3,5-difluorophenyl | (1S,4S)-4-methoxycarbonylcyclopent-2-en-1-yl |
| I-021 | H | H | methoxy | (S)-methyl | H | H | 3,5-difluorophenyl | (1S,4S)-4-methoxycarbonylcyclopent-2-en-1-yl |
| I-022 | H | H | methoxy | (R)-methyl | H | H | 3,5-difluorophenyl | (1S,4S)-4-methoxycarbonylcyclopent-2-en-1-yl |
| I-023 | H | H | methoxy | vinyl | H | H | 3,5-difluorophenyl | (1S,4S)-4-methoxycarbonylcyclopent-2-en-1-yl |
| I-023 | H | H | methoxy | vinyl | H | H | 3,5-difluorophenyl | (1S,4S)-4-methoxycarbonylcyclopent-2-en-1-yl |
| I-024 | H | H | methoxy | cyclopro pyl | H | H | 3,5-difluorophenyl | (1R)-3-ethoxy-1-methyl-3-oxo-propyl |
| I-025 | H | H | methoxy | cyclopro pyl | H | H | 3,5-difluorophenyl | (1R)-4-methoxy-1-methyl-4-oxo-butyl |
| I-026 | H | H | methoxy | cyclopro pyl | H | H | 3,5-difluorophenyl | (E,1R)-4-ethoxy-1-methyl-4-oxo-but-2-enyl |
| I-027 | H | H | methoxy | cyclopro pyl | H | H | 3,5-difluorophenyl | rac-(3S,5R)-5-methoxycarbonyltetrahydrofuran-3-yl |
| I-028 | H | H | methoxy | cyclopro pyl | H | H | 3,5-difluorophenyl | (1S,4S)-4-methoxycarbonylcyclopent-2-en-1-yl |
| I-029 | H | H | methoxy | cyclopro pyl | H | H | 3,5-difluorophenyl | (1R,3S)-3-methoxycarbonylcyclopentyl |
| I-030 | H | H | methoxy | cyclopro pyl | H | H | 3,5-dichlorophenyl | (1R,3S)-3-methoxvcarbonylcyclopentyl |
| I-031 | H | H | methoxy | cyclopro pyl | H | H | 3,5-dichlorophenyl | (1S,4S)-4-methoxycarbonylcyclopent-2-en-1-yl |
| I-032 | H | H | methoxy | cyclopro pyl | H | H | 3,5-dichlorophenyl | rac-(3S,5R)-5-methoxycarbonyltetrahydrofuran-3-yl |
| I-033 | H | H | methoxy | cyclopro pyl | H | H | 3,5-dichlorophenyl | (1R)-3-ethoxy-1-methyl-3-oxo-propyl |
| I-034 | H | H | methoxy | cyclopro pyl | H | H | 3,5-dichlorophenyl | (1R)-4-methoxy-1-methyl-4-oxo-butyl |
| I-035 | H | H | methoxy | cyclopro pyl | H | H | 3,5-dichlorophenyl | (E,1R)-4-methoxy-1-methyl-4-oxo-but-2-enyl |
| I-036 | H | H | methoxy | ethyl | H | H | 3,5-dichlorophenyl | (E,1R)-4-methoxy-1-methyl-4-oxo-but-2-enyl |
| I-037 | H | H | methoxy | ethyl | H | H | 3,5-difluorophenyl | (1R,3S)-3-methoxycarbonylcyclopentyl |
| I-038 | H | H | methoxy | ethyl | H | H | 3,5-difluorophenyl | (1S,4S)-4-methoxycarbonylcyclopent-2-en-1-yl |
| I-039 | H | H | methoxy | ethyl | H | H | 3,5-difluorophenyl | rac-(3S,5R)-5-methoxycarbonyltetrahydrofuran-3-yl |
| I-040 | H | H | methoxy | ethyl | H | H | 3,5-difluorophenyl | (1R)-3-ethoxy-1-methyl-3-oxo-propyl |
| I-041 | H | H | methoxy | fluorome thyl | H | H | 3,5-difluorophenyl | (1R)-3-ethoxy-1-methyl-3-oxo-propyl |
| I-042 | H | H | methoxy | ethyl | H | H | 3,5-difluorophenyl | (1R)-4-methoxy-1-methyl-4-oxo-butyl |
| I-042 | H | H | methoxy | ethyl | H | H | 3,5-difluorophenyl | (1R)-4-methoxy-1-methyl-4-oxo-butyl |
| I-043 | H | H | amino | methyl | H | H | 3,5-difluorophenyl | (E,1R)-4-methoxy-1-methyl-4-oxo-but-2-enyl |
| I-044 | H | H | methoxy | ethyl | H | H | 3,5-dichlorophenyl | rac-(3S,5R)-5-methoxycarbonyltetrahydrofuran-3-yl |
| I-045 | H | H | methoxy | ethyl | H | H | 3,5-dichlorophenyl | (1R)-4-methoxy-1-methyl-4-oxo-butyl |
| I-046 | H | H | methoxy | ethyl | H | H | 3,5-dichlorophenyl | (1R)-3-ethoxy-1-methyl-3-oxo-propyl |
| I-047 | H | H | methoxy | fluorome thyl | H | H | 3,5-difluorophenyl | (1R,3S)-3-methoxvcarbonylcyclopentyl |
| I-047 | H | H | methoxy | fluorome thyl | H | H | 3,5-difluorophenyl | (1R,3S)-3-methoxycarbonylcyclopentyl |
| I-048 | H | H | amino | methyl | H | H | 3,5-difluorophenyl | (1S,4S)-4-methoxycarbonylcyclopent-2-en-1-yl |
| I-049 | H | H | methoxy | ethyl | H | H | 3,5-dichlorophenyl | (1R,3S)-3-methoxycarbonylcyclopentyl |
| I-050 | H | H | methoxy | ethyl | H | H | 3,5-difluorophenyl | (E,1R)-4-methoxy-1-methyl-4-oxo-but-2-enyl |
| I-051 | H | H | methoxy | ethyl | H | H | 3,5-dichlorophenyl | (1S,4S)-4-methoxycarbonylcyclopent-2-en-1-yl |
| I-052 | H | H | methoxy | vinyl | H | H | 3,5-difluorophenyl | (1R,3S)-3-methoxycarbonylcyclopentyl |
| I-053 | H | H | methoxy | methyl | H | H | 3,5-dichlorophenyl | (1R)-4-methoxy-1-methyl-4-oxo-butyl |
| I-054 | H | H | methoxy | vinyl | H | H | 3,5-difluorophenyl | (E,1R)-4-methoxy-1-methyl-4-oxo-but-2-enyl |
| I-055 | H | H | methoxy | methyl | H | H | 3,5-dichlorophenyl | (1R,3S)-3-methoxvcarbonvlcyclopentyl |
| I-056 | H | H | methoxy | methyl | H | H | 3,5-difluorophenyl | (1R,3S)-3-methoxycarbonylcyclopentyl |
| I-057 | H | H | methoxy | (S)-methyl | H | H | 3-fluoro-5-methylphenyl | (E,1R)-4-methoxy-1-methyl-4-oxo-but-2-enyl |
| I-058 | H | H | methoxy | (R)-methyl | H | H | 3-fluoro-5-methylphenyl | (E,1R)-4-methoxy-1-methyl-4-oxo-but-2-enyl |
| I-059 | H | H | amino | methyl | H | H | 3,5-dichlorophenyl | (E,1R)-4-methoxy-1-methyl-4-oxo-but-2-enyl |
| I-060 | H | H | amino | methyl | H | H | 3,5-dichlorophenyl | (1S,4S)-4-methoxycarbonylcyclopent-2-en-1-yl |
| I-061 | H | H | methoxy | methyl | H | H | 3,5-difluorophenyl | (1R)-4-methoxy-1-methyl-4-oxo-butyl |
| I-062 | H | H | methoxy | methyl | H | H | 3-fluoro-5-methylphenyl | (1R,3S)-3-methoxvcarbonylcyclopentyl |
| I-063 | H | H | methoxy | methyl | H | H | 3-fluoro-5-methylphenyl | (1S,4S)-4-methoxycarbonylcyclopent-2-en-1-vl |
| I-064 | H | H | amino | (S)-methyl | H | H | 3,5-difluorophenyl | (1S,4S)-4-methoxycarbonylcyclopent-2-en-1-yl |
| I-065 | H | H | amino | methyl | H | H | 3,5-dichlorophenyl | (1R)-3-ethoxy-1-methyl-3-oxo-propyl |
| I-066 | H | H | amino | (S)-methyl | H | H | 3,5-difluorophenyl | (E,1R)-4-methoxy-1-methyl-4-oxo-but-2-enyl |
| I-067 | H | H | methoxy | (S)-Vinyl | H | H | 3,5-difluorophenyl | (1R)-3-ethoxy-1-methyl-3-oxo-propyl |
| I-068 | H | H | methoxy | (R)-vinyl | H | H | 3,5-difluorophenyl | (1R)-3-ethoxy-1-methyl-3-oxo-propyl |
| I-069 | H | H | methoxy | (S)-methyl | H | H | 3,5-difluorophenyl | (E,1R)-4-methoxy-1-methyl-4-oxo-but-2-enyl |
| I-070 | H | H | methoxy | (S)-vinyl | H | H | 3,5-difluorophenyl | (1S,4S)-4-methoxycarbonylcyclopent-2-en-1-yl |
| I-071 | H | H | methoxy | methyl | H | H | 3-fluoro-5-methylphenyl | (E,1R)-4-methoxy-1-methyl-4-oxo-but-2-enyl |
| I-072 | H | H | methoxy | vinyl | H | H | 3,5-difluorophenyl | (1R)-4-methoxy-1-methyl-4-oxo-butyl |
| I-073 | H | H | amino | (R)-methyl | H | H | 3,5-difluorophenyl | (E,1R)-4-methoxy-1-methyl-4-oxo-but-2-enyl |
| I-074 | H | H | ureido | (S)-methyl | H | H | 3,5-difluorophenyl | (E,1R)-4-methoxy-1-methyl-4-oxo-but-2-enyl |
| I-075 | H | H | methoxy | (R)-methyl | H | H | 3,5-difluorophenyl | (E,1R)-4-methoxy-1-methyl-4-oxo-but-2-enyl |
| I-076 | H | H | amino | (S)-methyl | H | H | 3,5-difluorophenyl | (1R)-3-ethoxy-1-methyl-3-oxo-propyl |
| I-077 | H | H | amino | (R)-methyl | H | H | 3,5-difluorophenyl | (1R)-3-ethoxy-1-methyl-3-oxo-propyl |
| I-078 | H | H | amino | (R)-methyl | H | H | 3,5-difluorophenyl | (1S,4S)-4-methoxycarbonylcyclopent-2-en-1-yl |
| I-079 | H | H | ureido | (S)-methyl | H | H | 3,5-difluorophenyl | (1S,4S)-4-methoxycarbonylcyclopent-2-en-1-yl |
| I-080 | H | H | ureido | (R)-methyl | H | H | 3,5-difluorophenyl | (E,1R)-4-methoxy-1-methyl-4-oxo-but-2-enyl |
| I-081 | H | H | ureido | (R)-methyl | H | H | 3,5-difluorophenyl | (1S,4S)-4-methoxycarbonylcyclopent-2-en-1-yl |
| I-082 | H | H | methoxy | cyclopro pvl | H | H | 3-fluoro-5-methylphenyl | (1R,3S)-3-methoxycarbonylcyclopentyl |
| I-083 | H | H | methoxy | vinyl | H | H | 3,5-dichlorophenyl | (1R,3S)-3-methoxycarbonylcyclopentyl |
| I-084 | H | H | methoxy | vinyl | H | H | 3,5-dichlorophenyl | (1S,4S)-4-methoxycarbonylcyclopent-2-en-1-yl |
| I-085 | H | H | methoxy | vinyl | H | H | 3,5-dichlorophenyl | (E,1R)-4-methoxy-1-methyl-4-oxo-but-2-enyl |
| I-086 | H | H | methoxy | vinyl | H | H | 3-fluoro-5-methylphenyl | (E,1R)-4-methoxy-1-methyl-4-oxo-but-2-enyl |
| I-087 | H | H | methoxy | vinyl | H | H | 3-fluoro-5-methylphenyl | (1R)-4-methoxy-1-methyl-4-oxo-butyl |
| I-088 | H | H | methoxy | vinyl | H | H | 3-fluoro-5-methylphenyl | (1S,4S)-4-methoxycarbonylcyclopent-2-en-1-yl |
| I-089 | H | H | methoxy | vinyl | H | H | 3-fluoro-5-methylphenyl | (1R,3S)-3-methoxycarbonylcyclopentyl |
| I-090 | H | H | methoxy | methyl | H | H | 3-fluoro-5-methylphenyl | (1R)-4-methoxy-1-methyl-4-oxo-butyl |
| I-091 | H | H | methoxy | cyclopro pyl | H | H | 3-fluoro-5-methylphenyl | (E,1R)-4-methoxy-1-methyl-4-oxo-but-2-enyl |
| I-092 | H | H | methoxy | cyclopro pyl | H | H | 3-fluoro-5-methylphenyl | (1R)-4-methoxy-1-methyl-4-oxo-butyl |
| I-093 | H | H | methoxy | vinyl | H | H | 3,5-dichlorophenyl | (1R)-4-methoxy-1-methyl-4-oxo-butyl |
| I-094 | H | H | methoxy | (S)-cyclopro pyl | H | H | 3-fluoro-5-methylphenyl | (1S,4S)-4-methoxycarbonylcyclopent-2-en-1-yl |
| I-095 | H | H | methylami no | methyl | H | H | 3,5-difluorophenyl | (1S,4S)-4-methoxycarbonylcyclopent-2-en-1-yl |
| I-096 | H | H | methoxy | (R)-cyclopro pyl | H | H | 3-fluoro-5-methylphenyl | (1S,4S)-4-methoxycarbonylcyclopent-2-en-1-yl |
| I-097 | H | H | methoxy | (R)-vinyl | H | H | 3,5-difluorophenyl | (1S,4S)-4-methoxycarbonylcyclopent-2-en-1-yl |
| I-098 | H | H | methylami no | methyl | H | H | 3,5-dichlorophenyl | (1S,4S)-4-methoxycarbonylcyclopent-2-en-1-yl |
| I-099 | H | H | methoxy | (S)-fluorome thyl | H | H | 3,5-difluorophenyl | (1S,4S)-4-methoxycarbonylcyclopent-2-en-1-yl |
| I-100 | H | H | methoxy | (R)-fluorome thyl | H | H | 3,5-difluorophenyl | (1S,4S)-4-methoxycarbonylcyclopent-2-en-1-yl |
| I-101 | H | H | methoxy | fluorome thyl | H | H | 3-fluoro-5-methylphenyl | (1R,3S)-3-methoxycarbonylcyclopentyl |
| I-102 | H | H | methoxy | fluorome thyl | H | H | 3-fluoro-5-methylphenyl | (1S,4S)-4-methoxycarbonylcyclopent-2-en-1-yl |
| I-103 | H | H | methoxy | fluorome thyl | H | H | 3-fluoro-5-methylphenyl | (1R)-4-methoxy-1-methyl-4-oxo-butyl |
| I-104 | H | H | methoxy | fluorome thyl | H | H | 3-fluoro-5-methylphenyl | (E,1R)-4-methoxy-1-methyl-4-oxo-but-2-enyl |
| I-105 | H | H | methoxy | fluorome thyl | H | H | 3,5-difluorophenyl | (1R)-4-methoxy-1-methyl-4-oxo-butyl |
| I-106 | H | H | methoxy | fluorome thyl | H | H | 3,5-difluorophenyl | (E,1R)-4-methoxy-1-methyl-4-oxo-but-2-enyl |
| I-107 | H | H | methylami no | methyl | H | H | 3,5-difluorophenyl | (E,1R)-4-methoxy-1-methyl-4-oxo-but-2-enyl |
| I-108 | H | H | dimethyla mino | methyl | H | H | 3,5-difluorophenyl | (1S,4S)-4-methoxycarbonylcyclopent-2-en-1-yl |
| I-109 | H | H | methoxy | fluorome thyl | H | H | 3,5-dichlorophenyl | (E,1R)-4-methoxy-1-methyl-4-oxo-but-2-enyl |
| I-110 | H | H | methoxy | fluorome thyl | H | H | 3,5-dichlorophenyl | (1R)-4-methoxy-1-methyl-4-oxo-butyl |
| I-111 | H | H | methoxy | fluorome thyl | H | H | 3,5-dichlorophenyl | (1S,4S)-4-methoxycarbonylcyclopent-2-en-1-yl |
| I-112 | H | H | methoxy | fluorome thyl | H | H | 3,5-dichlorophenyl | (1R,3S)-3-methoxvcarbonylcyclopentyl |
| I-113 | H | H | methoxy | (S)-vinyl | H | H | 3,5-difluorophenyl | (1R)-4-methoxy-1-methyl-4-oxo-butyl |
| I-114 | H | H | methoxy | (S)-vinyl | H | H | 3,5-difluorophenyl | (E,1R)-4-methoxy-1-methyl-4-oxo-but-2-enyl |
| I-115 | H | H | methoxy | (R)-vinyl | H | H | 3,5-difluorophenyl | (1R)-4-methoxy-1-methyl-4-oxo-butyl |
| I-116 | H | H | methoxy | (R)-vinyl | H | H | 3,5-difluorophenyl | (E,1R)-4-methoxy-1-methyl-4-oxo-but-2-enyl |
| I-117 | H | H | methylami no | methyl | H | H | 3,5-dichlorophenyl | (E,1R)-4-methoxy-1-methyl-4-oxo-but-2-enyl |
| I-118 | H | H | dimethyla mino | methyl | H | H | 3,5-difluorophenyl | (E,1R)-4-methoxy-1-methyl-4-oxo-but-2-enyl |
| I-119 | H | H | dimethyla mino | methyl | H | H | 3,5-dichlorophenyl | (1S,4S)-4-methoxycarbonylcyclopent-2-en-1-yl |
| I-120 | H | H | methoxy | (S)-vinyl | H | H | 3,5-dichlorophenyl | (E,1R)-4-methoxy-1-methyl-4-oxo-but-2-enyl |
| I-121 | H | H | methoxy | (S)-vinyl | H | H | 3,5-dichlorophenyl | (1R)-4-methoxy-1-methyl-4-oxo-butyl |
| I-122 | H | H | methoxy | (S)-vinyl | H | H | 3,5-dichlorophenyl | (1S,4S)-4-methoxycarbonylcyclopent-2-en-1-yl |
| I-123 | H | H | methoxy | (R)-vinyl | H | H | 3,5-dichlorophenyl | (E,1R)-4-methoxy-1-methyl-4-oxo-but-2-enyl |
| I-124 | H | H | methoxy | (R)-vinyl | H | H | 3,5-dichlorophenyl | (1R)-4-methoxy-1-methyl-4-oxo-butyl |
| I-125 | H | H | methoxy | (R)-vinyl | H | H | 3,5-dichlorophenyl | (1S,4S)-4-methoxycarbonylcyclopent-2-en-1-yl |
| I-126 | H | H | methoxy | (S)-vinyl | H | H | 3,5-dichlorophenyl | (1S,4S)-4-carboxycyclopent-2-en-1-yl |
| I-127 | H | H | methoxy | (R)-vinyl | H | H | 3,5-dichlorophenyl | (1S,4S)-4-carboxycyclopent-2-en-1-yl |
| I-128 | H | H | methoxy | (S)-vinyl | H | H | 3-fluoro-5-methylphenyl | (E,1R)-4-methoxy-1-methyl-4-oxo-but-2-enyl |
| I-129 | H | H | methoxy | (S)-vinyl | H | H | 3-fluoro-5-methylphenyl | (1R)-4-methoxy-1-methyl-4-oxo-butyl |
| I-130 | H | H | methoxy | (S)-vinyl | H | H | 3-fluoro-5-methylphenyl | (1S,4S)-4-methoxycarbonylcyclopent-2-en-1-yl |
| I-131 | H | H | methoxy | (R)-vinyl | H | H | 3-fluoro-5-methylphenyl | (E,1R)-4-methoxy-1-methyl-4-oxo-but-2-enyl |
| I-132 | H | H | methoxy | (R)-vinyl | H | H | 3-fluoro-5-methylphenyl | (1R)-4-methoxy-1-methyl-4-oxo-butyl |
| I-133 | H | H | methoxy | (R)-vinyl | H | H | 3-fluoro-5-methylphenyl | (1S,4S)-4-methoxycarbonylcyclopent-2-en-1-yl |
| I-134 | H | H | methoxy | (S)-vinyl | H | H | 3-fluoro-5-methylphenyl | (1S,4S)-4-carboxycyclopent-2-en-1-yl |
| I-135 | H | H | methoxy | (R)-vinyl | H | H | 3-fluoro-5-methylphenyl | (1S,4S)-4-carboxycyclopent-2-en-1-yl |
| I-136 | H | H | H | methyl | H | H | 3,5-difluorophenyl | (1S,4S)-4-methoxycarbonylcyclopent-2-en-1-yl |
| I-137 | H | H | H | methyl | H | H | 3,5-difluorophenyl | rac-(3S,5R)-5-methoxycarbonyltetrahydrofuran-3-yl |
| I-138 | H | H | H | methyl | H | H | 3,5-difluorophenyl | (E,1R)-4-ethoxy-1-methyl-4-oxo-but-2-enyl |
| I-139 | H | H | H | methyl | H | H | 3,5-dichlorophenyl | (1S,4S)-4-methoxycarbonylcyclopent-2-en-1-yl |
| I-140 | H | H | H | methyl | H | H | 3,5-dichlorophenyl | (1R)-3-ethoxy-1-methyl-3-oxo-propyl |
| I-141 | H | H | H | methyl | H | H | 3,5-dichlorophenyl | rac-(3S,5R)-5-methoxycarbonyltetrahydrofuran-3-yl |
| I-142 | H | H | H | methyl | H | H | 3,5-dichlorophenyl | (E,1R)-4-ethoxy-1-methyl-4-oxo-but-2-enyl |

| | | | | | | | | |
|---|---|---|---|---|---|---|---|---|
| * For the R4 substituent the stereochemistry is arbitrarily assigned as either (S) or (R) to show that the compound is a single enantiomer at this position, but that the exact identity of the enantiomer is not known. | | | | | | | | |

Spectroscopic data of selected table examples:
¹H-NMR data of selected examples are written in the form of ¹H-NMR-peak lists. To each signal peak are listed the δ-value in ppm and the signal intensity in round brackets. Between the δ-value - signal intensity pairs are semicolons as delimiters.

The peak list of an example has therefore the form:
δ ₁ (intensity₁); δ₂ (intensity₂);........; δᵢ (intensityᵢ);......; δₙ (intensityₙ)

Intensity of sharp signals correlates with the height of the signals in a printed example of a NMR spectrum in cm and shows the real relations of signal intensities. From broad signals several peaks or the middle of the signal and their relative intensity in comparison to the most intensive signal in the spectrum can be shown.

For calibrating chemical shift for ¹H spectra, we use tetramethylsilane and/or the chemical shift of the solvent used, especially in the case of spectra measured in DMSO. Therefore in NMR peak lists, tetramethylsilane peak can occur but not necessarily.

The ¹H-NMR peak lists are similar to classical ¹H-NMR prints and contains therefore usually all peaks, which are listed at classical NMR-interpretation.

Additionally, they can show like classical ¹H-NMR prints signals of solvents, stereoisomers of the target compounds, which are also object of the invention, and/or peaks of impurities.

To show compound signals in the delta-range of solvents and/or water the usual peaks of solvents, for example peaks of DMSO in DMSO-d₆ and the peak of water are shown in our ¹H-NMR peak lists and have usually on average a high intensity.

The peaks of stereoisomers of the target compounds and/or peaks of impurities have usually on average a lower intensity than the peaks of target compounds (for example with a purity >90%).

Such stereoisomers and/or impurities can be typical for the specific preparation process. Therefore, their peaks can help to recognize the reproduction of our preparation process via "side-products-fingerprints".

An expert, who calculates the peaks of the target compounds with known methods (MestreC, ACD-simulation, but also with empirically evaluated expectation values) can isolate the peaks of the target compounds as needed optionally using additional intensity filters. This isolation would be similar to relevant peak picking at classical ¹H-NMR interpretation.

Further details of NMR-data description with peak lists you find in the publication "Citation of NMR Peaklist Data within Patent Applications" of the Research Disclosure Database Number 564025.

| |
|---|
| |
| I-001: ¹H-NMR(400.6 MHz, CDCl3): |
| δ= 7.6557 (0.6); 7.6456 (0.6); 7.2631 (14.4); 6.7492 (0.5); 6.7440 (0.9); 6.7396 (1.0); 6.7353 (2.2); 6.7300 (2.6); 6.7271 (2.6); 6.7247 (2.8); 6.7219 (2.3); 6.7178 (2.0); 6.7137 (1.9); 6.7112 (2.2); 6.7069 (3.2); 6.7000 (1.0); 5.3009 (10.8); 4.5826 (0.8); 4.5725 (1.1); 4.5604 (1.4); 4.5496 (1.0); 4.5470 (1.1); 4.5376 (0.9); 4.4660 (0.6); 4.4591 (0.6); 4.4525 (0.6); 4.4457 (0.6); 4.0418 (0.8); 4.0303 (1.0); 4.0285 (1.0); 4.0179 (2.2); 4.0070 (1.6); 4.0051 (1.4); 3.9941 (1.2); 3.9808 (2.7); 3.9738 (3.0); 3.9664 (5.0); 3.9297 (0.8); 3.9062 (1.2); 3.9030 (1.2); 3.8992 (1.2); 3.8828 (0.9); 3.8796 (0.9); 3.8759 (0.8); 3.8219 (14.9); 3.8020 (15.3); 3.7915 (0.6); 3.7341 (16.0); 3.7315 (15.6); 2.5631 (0.7); 2.5472 (0.6); 2.5451 (0.6); 2.5404 (0.7); 2.5285 (0.8); 2.5243 (0.6); 2.5217 (0.6); 2.5106 (0.6); 2.5063 (0.7); 2.0641 (0.6); 2.0378 (0.8); 2.0299 (0.8); 2.0037 (0.5); 1.5915 (6.9); 1.3339 (9.7); 1.3241 (10.0); -0.0002 (16.2); - 0.0084 (0.7) |
| I-002: ¹H-NMR(400.6 MHz, CDCl3): |
| δ= 7.2609 (46.0); 6.7452 (3.5); 6.7402 (1.5); 6.7280 (3.5); 6.7229 (4.1); 6.7115 (1.3); 6.7043 (2.8); 5.9543 (0.5); 5.9504 (0.6); 5.9441 (0.6); 5.9404 (0.7); 5.9359 (1.2); 5.9304 (1.2); 5.9254 (0.7); 5.9218 (0.8); 5.9176 (0.9); 5.9116 (0.7); 5.8328 (0.7); 5.8272 (1.2); 5.8215 (0.8); 5.8137 (1.4); 5.8088 (1.4); 5.8035 (0.8); 5.7953 (1.0); 5.7896 (0.6); 5.3003 (3.1); 4.9192 (0.6); 4.8978 (0.6); 4.0389 (1.1); 4.0023 (4.3); 3.9823 (2.9); 3.9793 (2.8); 3.9426 (0.7); 3.7438 (15.2); 3.7387 (16.0); 3.7336 (14.9); 3.7181 (13.6); 3.5448 (0.6); 3.5411 (0.7); 3.5349 (0.8); 3.5237 (0.8); 2.5359 (0.9); 2.5225 (0.6); 2.5179 (0.9); 2.5015 (1.0); 2.4970 (0.6); 2.4831 (1.0); 1.9098 (0.6); 1.9050 (0.9); 1.8987 (1.0); 1.8878 (0.5); 1.8804 (0.5); 1.8753 (0.6); 1.8703 (0.8); 1.8645 (0.9); 1.8601 (0.6); 1.5526 (10.0); 1.3592 (9.8); 1.3507 (10.2); 1.2550 (2.4); 0.0080 (1.4); -0.0002 (50.9); -0.0084 (2.4) |
| I-003: ¹H-NMR(400.6 MHz, CDCl3): |
| δ= 7.2614 (29.0); 7.1936 (0.5); 7.1783 (0.6); 7.1573 (0.5); 6.9198 (1.1); 6.9064 (1.2); 6.8799 (1.8); 6.8670 (2.3); 6.8400 (1.3); 6.8275 (1.3); 6.7366 (1.0); 6.7306 (1.5); 6.7240 (1.9); 6.7202 (1.6); 6.7147 (1.7); 6.7062 (2.0); 6.7036 (2.4); 6.6963 (2.1); 6.6908 (1.7); 6.6765 (1.8); 6.6715 (1.0); 5.8985 (1.3); 5.8943 (1.3); 5.8793 (1.3); 5.8749 (1.4); 5.8593 (1.2); 5.8551 (1.2); 5.8400 (1.2); 5.8356 (1.3); 5.3003 (16.0); 4.2345 (1.1); 4.2167 (3.5); 4.1988 (3.9); 4.1956 (2.9); 4.1809 (1.7); 4.1776 (2.8); 4.1597 (1.0); 4.0149 (0.8); 3.9991 (3.1); 3.9934 (3.0); 3.9783 (2.4); 3.9435 (2.1); 3.9070 (0.8); 3.7438 (14.0); 3.7319 (14.4); 1.5575 (9.7); 1.4936 (0.5); 1.4836 (1.0); 1.3730 (10.2); 1.3611 (9.4); 1.3154 (7.4); 1.3047 (5.0); 1.2972 (11.1); 1.2869 (8.5); 1.2794 (8.4); 1.2691 (4.2); 1.2615 (4.4); 0.0080 (0.9); -0.0002 (32.7); - 0.0085 (1.6) |
| I-004: ¹H-NMR(400.6 MHz, CDCl3): |
| δ= 7.2603 (43.0); 6.7317 (0.6); 6.7197 (0.5); 4.1552 (0.9); 4.1372 (0.8); 3.9870 (0.6); 3.9773 (0.6); 3.9707 (1.2); 3.7236 (5.5); 2.4931 (0.6); 1.5382 (16.0); 1.3388 (2.2); 1.3342 (2.3); 1.2993 (0.8); 1.2815 (2.2); 1.2637 (2.4); 1.2459 (0.8); 1.2356 (1.2); 1.2319 (1.3); 1.2188 (1.2); 1.2150 (1.2); 0.0080 (1.4); - 0.0002 (48.7); -0.0084 (2.1) |
| I-005: ¹H-NMR(400.6 MHz, CDCl3): |
| δ= 7.4366 (0.6); 7.2619 (17.8); 7.2568 (4.1); 7.2523 (2.2); 7.0784 (4.8); 7.0738 (4.6); 7.0613 (4.8); 7.0567 (4.6); 5.9563 (0.6); 5.9524 (0.6); 5.9501 (0.6); 5.9462 (0.6); 5.9300 (0.5); 5.9203 (0.6); 5.9162 (0.7); 5.9142 (0.6); 5.9101 (0.6); 5.8630 (0.6); 5.8574 (1.1); 5.8518 (0.6); 5.8493 (0.5); 5.8436 (0.9); 5.8379 (0.5); 5.8357 (0.7); 5.8300 (1.2); 5.8243 (0.6); 5.8219 (0.5); 5.8162 (0.9); 4.0353 (1.5); 3.9987 (4.4); 3.9707 (2.0); 3.9631 (1.9); 3.9341 (0.6); 3.9265 (0.7); 3.7446 (15.8); 3.7382 (16.0); 3.7289 (13.6); |
| 3.7139 (13.0); 3.5417 (0.6); 3.5381 (0.5); 3.5360 (0.6); 3.5325 (0.6); 3.5302 (0.6); 3.5268 (0.6); 2.5678 (0.7); 2.5334 (0.8); 2.5225 (0.7); 2.5088 (0.5); 2.4877 (0.8); 1.9243 (0.7); 1.9003 (0.9); 1.8897 (1.0); 1.8650 (0.6); 1.5773 (2.2); 1.3577 (9.1); 1.3490 (9.0); 0.0080 (0.7); -0.0002 (24.2); -0.0085 (0.7) |
| I-006: ¹H-NMR(400.6 MHz, CDCl3): |
| δ= 7.2616 (11.4); 7.2578 (1.2); 7.2562 (1.0); 7.2531 (2.0); 7.2516 (1.8); 7.2486 (1.1); 7.2471 (1.0); 7.0852 (3.1); 7.0806 (3.0); 7.0620 (3.1); 7.0574 (3.0); 5.3004 (1.1); 4.1978 (0.6); 4.1868 (0.5); 4.1797 (1.0); 4.1729 (0.7); 4.1690 (1.0); 4.1618 (0.9); 4.1551 (2.0); 4.1513 (1.0); 4.1372 (2.0); 4.1194 (0.6); 3.9820 (1.5); 3.9636 (2.3); 3.9610 (1.9); 3.7188 (16.0); 2.5137 (1.1); 2.5115 (1.1); 2.5059 (1.0); 2.4972 (1.8); 2.4921 (1.0); 2.4827 (0.9); 1.5710 (2.3); 1.3381 (5.9); 1.3332 (6.1); 1.3014 (2.2); 1.2836 (5.2); 1.2641 (5.2); 1.2519 (3.2); 1.2463 (2.5); 1.2354 (5.5); 1.2189 (3.0); -0.0002 (17.3) |
| I-007: ¹H-NMR(400.6 MHz, CDCl3): |
| δ= 7.6595 (0.6); 7.6393 (0.6); 7.2647 (1.7); 7.2618 (22.0); 7.2556 (1.9); 7.2541 (3.0); 7.2495 (1.5); 7.0593 (8.5); 7.0547 (8.3); 5.3005 (7.4); 4.5866 (0.7); 4.5766 (0.9); 4.5734 (0.8); 4.5638 (1.6); 4.5536 (0.8); 4.5499 (0.9); 4.5407 (0.8); 4.4450 (0.5); 4.0616 (0.8); 4.0484 (0.8); 4.0383 (1.4); 4.0253 (1.3); 4.0164 (1.5); 4.0080 (0.7); 4.0033 (1.1); 3.9788 (2.3); 3.9713 (2.3); 3.9486 (3.0); 3.9425 (0.8); 3.9118 (0.8); 3.8969 (0.6); 3.8909 (0.6); 3.8209 (15.3); 3.8088 (0.7); 3.8004 (16.0); 3.7915 (1.0); 3.7877 (0.8); 3.7295 (15.0); 3.7269 (14.4); 2.5629 (0.6); 2.5278 (0.6); 2.0672 (0.8); 2.0603 (0.8); 2.0331 (0.7); 2.0260 (0.7); 1.5679 (4.3); 1.3339 (9.0); 1.3210 (9.4); 1.2548 (0.6); 0.0080 (0.8); -0.0002 (33.4); -0.0085 (1.0) |
| I-008: ¹H-NMR(400.6 MHz, CDCl3): |
| δ= 7.2692 (1.6); 7.2646 (3.8); 7.2625 (15.8); 7.2602 (2.2); 7.2554 (1.7); 7.2508 (3.1); 7.2462 (1.7); 7.0541 (6.2); 7.0495 (6.2); 7.0330 (6.1); 7.0284 (6.1); 6.9449 (1.3); 6.9317 (1.3); 6.9057 (1.4); 6.8925 (1.4); 6.8783 (1.4); 6.8658 (1.4); 6.8390 (1.5); 6.8265 (1.5); 5.9169 (1.5); 5.9127 (1.5); 5.8774 (1.7); 5.8765 (1.8); 5.8733 (1.8); 5.8721 (1.8); 5.8371 (1.4); 5.8327 (1.4); 5.3005 (4.9); 4.2348 (1.2); 4.2170 (3.9); 4.2129 (0.8); 4.2114 (0.8); 4.1992 (4.0); 4.1950 (2.4); 4.1936 (2.4); 4.1813 (1.3); 4.1770 (2.5); 4.1758 (2.5); 4.1591 (0.8); 4.0317 (0.5); 4.0049 (0.9); 3.9950 (2.6); 3.9800 (2.3); 3.9683 (2.2); 3.9255 (1.9); 3.8889 (0.8); 3.7914 (0.6); 3.7410 (16.0); 3.7274 (15.9); 1.5769 (2.2); 1.3728 (10.1); 1.3588 (8.8); 1.3339 (5.2); 1.3159 (8.7); 1.3047 (4.6); 1.2968 (6.6); 1.2869 (9.2); 1.2788 (9.4); 1.2691 (4.3); 1.2609 (4.6); 0.0080 (0.7); -0.0002 (23.5); -0.0085 (0.7) |
| I-009: ¹H-NMR(400.6 MHz, CDCl3): |
| δ= 7.6792 (0.6); 7.6620 (0.6); 7.3663 (1.3); 7.3616 (2.8); 7.3558 (2.8); 7.3512 (1.4); 7.2622 (29.6); 7.1014 (5.6); 7.0967 (5.9); 7.0948 (6.1); 7.0902 (5.7); 6.0379 (1.1); 5.9669 (1.3); 5.3008 (16.0); 4.6043 (0.7); 4.5947 (0.8); 4.5853 (0.8); 4.5815 (0.9); 4.5760 (0.9); 4.5719 (0.8); 4.5621 (0.8); 4.5528 (0.8); 4.1307 (1.4); 4.1102 (1.1); 4.0947 (1.7); 4.0791 (0.9); 4.0746 (1.5); 4.0692 (1.3); 4.0627 (0.8); 4.0559 (1.1); 4.0490 (0.8); 4.0392 (1.0); 4.0256 (0.9); 3.9842 (0.7); 3.9768 (0.6); 3.9031 (2.0); 3.9005 (1.9); 3.8681 (1.2); 3.8643 (1.2); 3.8283 (15.0); 3.8023 (15.6); 2.5849 (0.5); 2.5783 (0.5); 2.5620 (0.5); 2.5445 (0.5); 2.5418 (0.6); 2.1358 (1.5); 2.1235 (0.5); 2.0895 (0.9); 2.0801 (0.7); 2.0457 (2.5); 1.9438 (15.4); 1.5704 (13.7); 1.4378 (0.7); 1.4236 (0.7); 1.3325 (8.8); 1.3214 (9.2); 1.2776 (0.6); 1.2597 (1.4); 1.2420 (0.7); 0.0080 (0.9); -0.0002 (31.6); -0.0086 (0.9) |
| I-010: ¹H-NMR(400.6 MHz, CDCl3): |
| δ= 7.3708 (1.8); 7.3662 (3.5); 7.3617 (1.8); 7.3494 (1.8); 7.3449 (3.5); 7.3403 (1.8); 7.2891 (0.6); 7.2617 (48.1); 7.2351 (0.6); 7.0831 (7.5); 7.0785 (7.3); 7.0667 (7.4); 7.0621 (7.1); 6.9495 (1.5); 6.9362 (1.6); 6.9104 (1.6); 6.8970 (1.7); 6.8803 (1.6); 6.8677 (1.6); 6.8410 (1.7); 6.8284 (1.7); 6.6975 (2.4); 6.0691 (2.0); 5.9381 (1.7); 5.9339 (1.7); 5.8989 (1.6); 5.8948 (1.6); 5.8796 (1.8); 5.8752 (1.7); 5.8403 (1.6); 5.8360 (1.6); 5.3006 (4.8); 4.6751 (0.6); 4.6712 (0.6); 4.6578 (0.5); 4.6538 (0.5); 4.5920 (0.5); 4.5708 (0.5); 4.2227 (1.4); 4.2109 (1.0); 4.2049 (4.3); 4.1945 (2.8); 4.1933 (2.8); 4.1871 (4.5); 4.1766 (2.9); 4.1694 (1.5); 4.1587 (1.0); 4.1265 (1.5); 4.1116 (1.6); 4.0908 (2.1); 4.0759 (2.0); 3.9356 (1.9); 3.9001 (1.4); 3.8432 (1.8); 3.8078 (1.4); 2.1362 (1.8); 2.0457 (0.8); 1.9459 (16.0); 1.9398 (15.5); 1.5616 (12.1); 1.4383 (0.9); 1.4241 (1.0); 1.3742 (12.4); 1.3626 (14.1); 1.3438 (6.6); 1.3259 (6.5); 1.3084 (6.7); 1.2959 (5.2); 1.2861 (5.4); 1.2781 (10.8); 1.2683 (11.2); 1.2602 (6.4); 1.2505 (5.2); 1.2422 (0.5); 0.9047 (1.5); 0.8990 (0.7); 0.8819 (1.9); 0.8642 (0.8); 0.0079 (1.5); -0.0002 (51.7); -0.0085 (1.4) |
| I-011: ¹H-NMR(400.6 MHz, CDCl3): |
| δ= 7.3629 (1.6); 7.3616 (1.6); 7.3583 (3.2); 7.3571 (3.0); 7.3537 (1.7); 7.3525 (1.6); 7.2623 (27.3); 7.1121 (5.7); 7.1076 (5.7); 7.0854 (6.0); 7.0808 (5.5); 5.9896 (1.3); 5.9866 (1.3); 5.9799 (0.6); 5.9762 (0.6); 5.9722 (0.7); 5.9700 (0.6); 5.9661 (0.6); 5.9441 (0.5); 5.9344 (0.6); 5.9303 (0.7); 5.9283 (0.6); 5.9242 (0.6); 5.9010 (0.6); 5.8955 (1.2); 5.8898 (0.6); 5.8817 (0.9); 5.8361 (0.9); 5.8306 (2.1); 5.8249 |
| (0.9); 5.8226 (0.6); 5.8167 (0.9); 5.3007 (7.8); 4.1581 (1.0); 4.1504 (1.0); 4.1226 (1.3); 4.1148 (1.4); 3.9191 (1.3); 3.9034 (1.2); 3.8835 (0.9); 3.8679 (0.9); 3.7450 (15.4); 3.7381 (16.0); 3.5520 (0.6); 3.5463 (0.5); 3.5426 (0.5); 2.6029 (0.7); 2.5686 (0.8); 2.5416 (0.7); 2.5068 (0.7); 2.1347 (2.5); 2.0862 (1.1); 1.9579 (0.8); 1.9463 (0.8); 1.9401 (8.8); 1.9227 (8.8); 1.9118 (1.0); 1.8764 (0.6); 1.5792 (2.8); 1.4364 (1.2); 1.4223 (1.3); 1.3594 (8.8); 1.3514 (8.3); 1.2555 (0.6); 0.0079 (0.9); -0.0002 (30.8); -0.0029 (1.0); - 0.0036 (0.6); -0.0085 (0.8) |
| I-012: ¹H-NMR(400.6 MHz, CDCl3): |
| δ= 7.5056 (0.5); 7.4849 (0.5); 7.3598 (1.9); 7.3552 (3.8); 7.3520 (2.4); 7.3507 (2.4); 7.3476 (3.7); 7.3430 (2.0); 7.2618 (47.0); 7.1553 (6.7); 7.1507 (6.4); 7.0919 (8.3); 7.0873 (8.0); 6.2675 (1.9); 5.9680 (1.9); 5.3006 (2.2); 4.2495 (0.6); 4.2439 (0.7); 4.2331 (0.7); 4.2286 (1.0); 4.2218 (0.6); 4.2122 (0.8); 4.2074 (0.6); 4.1972 (1.2); 4.1804 (2.9); 4.1728 (1.6); 4.1625 (3.1); 4.1549 (4.8); 4.1446 (1.2); 4.1371 (4.9); 4.1190 (2.1); 4.1002 (1.6); 4.0813 (2.3); 4.0647 (2.2); 3.9202 (2.1); 3.9012 (2.0); 3.8848 (1.5); 3.8657 (1.4); 2.5424 (0.7); 2.5367 (3.9); 2.5210 (3.3); 2.5184 (2.6); 2.5042 (2.1); 2.4968 (2.0); 2.4814 (2.0); 2.1360 (0.9); 2.0887 (1.0); 1.9336 (16.0); 1.9274 (15.6); 1.5678 (13.2); 1.4235 (0.5); 1.3363 (14.3); 1.3332 (15.4); 1.3053 (5.5); 1.2874 (12.0); 1.2841 (9.0); 1.2824 (7.8); 1.2694 (7.1); 1.2672 (8.5); 1.2643 (12.8); 1.2556 (1.2); 1.2464 (5.7); 1.2384 (7.4); 1.2217 (7.3); 0.9047 (1.0); 0.0080 (1.4); -0.0002 (50.9); -0.0085 (1.5) |
| I-013: ¹H-NMR(400.6 MHz, CDCl3): |
| δ= 7.5209 (0.5); 7.3439 (1.6); 7.3426 (1.6); 7.3393 (3.2); 7.3380 (3.1); 7.3348 (1.8); 7.3334 (1.7); 7.2624 (23.0); 7.2016 (5.8); 7.1970 (5.4); 7.1854 (5.5); 7.1808 (5.2); 5.9713 (0.5); 5.9692 (0.5); 5.9615 (0.6); 5.9575 (0.6); 5.9554 (0.6); 5.9514 (0.6); 5.9295 (0.5); 5.9277 (0.5); 5.9200 (0.6); 5.9157 (0.7); 5.9139 (0.7); 5.9097 (0.6); 5.8677 (0.6); 5.8621 (1.2); 5.8565 (0.6); 5.8540 (0.5); 5.8483 (0.9); 5.8284 (0.6); 5.8227 (1.2); 5.8171 (0.6); 5.8146 (0.5); 5.8089 (0.9); 5.3007 (13.3); 4.1437 (1.3); 4.1342 (1.3); 4.1081 (1.9); 4.0986 (1.9); 3.9580 (1.7); 3.9480 (1.7); 3.9223 (1.2); 3.9125 (1.3); 3.7401 (15.9); 3.7316 (16.0); 3.5399 (0.5); 3.5340 (0.6); 3.5281 (0.5); 3.3752 (0.5); 2.5948 (0.8); 2.5816 (0.5); 2.5604 (1.2); 2.5393 (1.1); 2.5254 (0.5); 2.5043 (0.8); 2.0655 (0.6); 1.9227 (0.7); 1.9106 (0.6); 1.8983 (0.8); 1.8880 (0.9); 1.8633 (0.7); 1.7334 (0.5); 1.6133 (0.9); 1.5933 (0.5); 1.3418 (8.9); 1.3346 (9.0); 1.3212 (0.9); 1.3095 (1.2); 1.3017 (1.1); 1.2983 (0.7); 1.2901 (1.0); 1.2844 (0.7); 1.2822 (0.7); 1.2780 (0.6); 1.2597 (1.1); 1.2553 (1.2); 1.1275 (0.6); 1.1182 (0.8); 1.1052 (1.0); 1.0951 (0.7); 1.0883 (0.7); 1.0767 (0.7); 1.0654 (1.2); 1.0580 (1.4); 1.0560 (0.9); 1.0540 (1.1); 1.0465 (1.2); 0.9398 (1.2); 0.9324 (0.8); 0.9298 (0.7); 0.9199 (1.2); 0.9123 (0.8); 0.8819 (1.2); 0.8131 (0.8); 0.8066 (0.7); 0.7976 (1.4); 0.7935 (1.0); 0.7867 (0.9); 0.7824 (0.6); 0.7784 (1.4); 0.7761 (1.0); 0.7670 (0.5); 0.0080 (0.7); -0.0002 (25.8); -0.0085 (0.7) |
| I-014: ¹H-NMR(400.6 MHz, CDCl3): |
| δ= 7.3505 (2.0); 7.3460 (3.8); 7.3414 (2.1); 7.3327 (2.0); 7.3281 (4.1); 7.3235 (2.2); 7.2842 (0.6); 7.2617 (53.0); 7.2577 (0.9); 7.2569 (0.8); 7.2553 (0.6); 7.2544 (0.6); 7.2536 (0.6); 7.2497 (0.7); 7.2286 (0.6); 7.1853 (7.9); 7.1807 (7.4); 7.1669 (8.2); 7.1623 (7.8); 6.9371 (1.7); 6.9236 (1.7); 6.8979 (1.8); 6.8844 (1.9); 6.8786 (1.7); 6.8662 (1.7); 6.8393 (1.8); 6.8269 (1.8); 6.7591 (2.1); 6.2994 (1.7); 5.9437 (1.9); 5.9395 (1.9); 5.9044 (1.8); 5.9002 (1.8); 5.8737 (1.9); 5.8693 (1.8); 5.8344 (1.7); 5.8300 (1.7); 5.3005 (14.3); 4.6600 (0.5); 4.6561 (0.5); 4.6426 (0.5); 4.6386 (0.5); 4.2207 (1.5); 4.2100 (0.9); 4.2083 (1.0); 4.2028 (5.0); 4.1922 (2.8); 4.1906 (2.8); 4.1850 (5.2); 4.1743 (2.9); 4.1728 (2.7); 4.1672 (1.6); 4.1564 (1.0); 4.1228 (1.6); 4.1060 (1.7); 4.0870 (2.4); 4.0703 (2.4); 3.9641 (2.1); 3.9284 (1.4); 3.9002 (2.0); 3.8646 (1.5); 1.6409 (0.6); 1.6323 (0.6); 1.6209 (1.0); 1.6124 (0.5); 1.6094 (0.7); 1.6009 (0.8); 1.5894 (0.7); 1.5723 (0.6); 1.3573 (11.4); 1.3453 (11.7); 1.3399 (7.2); 1.3300 (1.2); 1.3224 (7.1); 1.3112 (7.2); 1.3077 (2.3); 1.3037 (1.8); 1.2936 (14.0); 1.2843 (1.6); 1.2756 (16.0); 1.2578 (8.1); 1.1292 (0.7); 1.1232 (1.2); 1.1181 (1.8); 1.1110 (2.2); 1.1068 (1.4); 1.1022 (1.4); 1.0998 (1.3); 1.0907 (0.6); 1.0859 (0.5); 1.0780 (0.9); 1.0746 (0.6); 1.0715 (0.7); 1.0692 (0.8); 1.0666 (1.3); 1.0594 (1.5); 1.0553 (1.0); 1.0478 (1.1); 0.9441 (1.1); 0.9367 (0.8); 0.9340 (0.7); 0.9242 (1.2); 0.9166 (0.8); 0.8381 (0.8); 0.8318 (0.8); 0.8235 (0.8); 0.8164 (2.4); 0.8095 (1.4); 0.8036 (1.1); 0.7962 (1.6); 0.7897 (1.1); 0.0079 (1.7); -0.0002 (57.7); -0.0052 (0.7); -0.0085 (1.6) |
| I-015: ¹H-NMR(400.6 MHz, CDCl3): |
| δ= 7.2633 (17.6); 6.8523 (0.8); 6.8466 (0.5); 6.8363 (0.8); 6.8305 (1.7); 6.8248 (1.0); 6.8088 (0.8); 6.8030 (0.5); 6.7818 (1.2); 6.7784 (0.6); 6.7760 (1.1); 6.7664 (0.6); 6.7632 (1.3); 6.7575 (1.0); 6.7534 (1.4); 6.7476 (1.2); 6.7382 (0.6); 6.7348 (1.4); 6.7292 (1.0); 6.0643 (0.8); 5.9632 (0.5); 5.9593 (0.6); 5.9570 (0.6); 5.9529 (0.6); 5.9374 (0.6); 5.9332 (0.6); 5.9313 (0.6); 5.9271 (0.6); 5.8764 (0.8); 5.8492 (0.6); 5.8436 (1.1); 5.8380 (0.6); 5.8354 (0.6); 5.8333 (0.7); 5.8297 (1.1); 5.8277 (1.2); 5.8242 (0.6); |
| 5.8220 (0.6); 5.8138 (0.9); 5.3010 (13.5); 4.1423 (1.4); 4.1310 (0.7); 4.1131 (0.9); 4.1067 (2.0); 3.9319 (1.3); 3.9206 (1.3); 3.8964 (0.9); 3.8851 (0.9); 3.7446 (14.7); 3.7392 (16.0); 3.3749 (1.4); 2.5737 (0.7); 2.5604 (0.5); 2.5394 (0.9); 2.5370 (0.8); 2.5232 (0.5); 2.5020 (0.8); 2.1326 (0.6); 2.0652 (1.4); 2.0456 (3.6); 1.9516 (9.5); 1.9446 (0.7); 1.9339 (9.9); 1.9285 (0.7); 1.9218 (0.6); 1.9177 (0.7); 1.8990 (0.6); 1.8828 (0.6); 1.3624 (8.7); 1.3543 (8.1); 1.2775 (1.1); 1.2597 (2.4); 1.2553 (0.5); 1.2420 (1.3); 1.2264 (0.7); 0.0079 (0.6); -0.0002 (19.4); -0.0027 (0.7); -0.0085 (0.6) |
| I-016: ¹H-NMR(400.6 MHz, CDCl3): |
| δ= 7.2629 (17.9); 6.9044 (0.7); 6.8988 (0.5); 6.8886 (0.7); 6.8829 (1.5); 6.8769 (1.8); 6.8713 (0.9); 6.8669 (0.5); 6.8613 (1.3); 6.8575 (2.0); 6.8443 (1.4); 6.8386 (1.2); 6.8262 (1.4); 6.8206 (0.9); 5.9669 (0.6); 5.9611 (0.9); 5.9570 (0.9); 5.9477 (0.5); 5.9437 (0.6); 5.9414 (0.6); 5.9375 (0.5); 5.8517 (0.6); 5.8461 (1.1); 5.8431 (0.6); 5.8404 (0.8); 5.8376 (1.3); 5.8321 (1.3); 5.8267 (0.6); 5.8237 (0.7); 5.4654 (0.6); 5.3009 (15.8); 5.2516 (0.6); 4.1977 (1.8); 4.1622 (2.4); 3.9638 (1.2); 3.9566 (1.4); 3.9282 (1.0); 3.9217 (2.2); 3.8932 (1.3); 3.8781 (2.0); 3.7441 (14.7); 3.7406 (16.0); 3.5406 (0.5); 3.5349 (0.5); 3.3748 (0.9); 2.5389 (0.9); 2.5179 (0.9); 2.5044 (1.0); 2.4832 (0.9); 2.1336 (0.6); 2.0823 (0.9); 2.0652 (1.0); 2.0456 (1.2); 1.9654 (0.6); 1.9310 (0.7); 1.9251 (0.6); 1.5898 (0.9); 1.3873 (8.8); 1.3766 (8.0); 1.2597 (1.0); 1.2551 (0.5); 1.2422 (0.6); 1.2264 (0.6); 0.0080 (0.6); -0.0002 (20.1); -0.0026 (0.7); -0.0085 (0.6) |
| I-017: ¹H-NMR(400.6 MHz, CDCl3): |
| δ= 7.4966 (0.5); 7.2624 (18.6); 6.8741 (0.8); 6.8684 (1.2); 6.8553 (1.4); 6.8517 (1.9); 6.8383 (1.4); 6.8326 (1.4); 6.8150 (1.2); 6.8093 (1.6); 6.8036 (0.6); 6.7934 (0.6); 6.7877 (0.7); 6.2778 (1.4); 6.1280 (1.4); 5.9477 (0.5); 5.9436 (0.6); 5.9415 (0.6); 5.9372 (0.6); 5.9176 (0.6); 5.9160 (0.6); 5.8255 (0.6); 5.8199 (1.1); 5.8164 (1.1); 5.8143 (0.7); 5.8112 (0.8); 5.8061 (0.9); 5.8026 (0.9); 5.3007 (16.0); 4.1333 (1.1); 4.1295 (1.1); 4.0976 (1.6); 4.0937 (1.6); 3.9633 (1.5); 3.9602 (1.4); 3.9277 (1.0); 3.9246 (1.0); 3.7400 (12.5); 3.7329 (13.4); 3.5376 (0.5); 3.3750 (0.8); 2.5657 (0.6); 2.5346 (0.8); 2.5316 (0.8); 2.4999 (0.7); 2.0654 (0.8); 2.0458 (0.6); 1.9046 (0.7); 1.8976 (0.7); 1.8699 (0.6); 1.8635 (0.6); 1.3640 (0.7); 1.3527 (1.1); 1.3442 (8.6); 1.3369 (7.5); 1.3245 (0.6); 1.2597 (0.7); 1.1147 (0.9); 1.1094 (0.6); 1.1029 (1.0); 1.0976 (0.8); 1.0929 (0.6); 1.0902 (0.7); 1.0858 (0.6); 1.0792 (0.6); 1.0679 (0.6); 1.0637 (0.5); 1.0615 (0.6); 0.8030 (0.7); 0.7964 (0.9); 0.7900 (1.3); 0.7828 (1.1); 0.7768 (0.8); 0.7699 (1.0); 0.7637 (0.5); 0.0079 (0.6); -0.0002 (19.4); -0.0085 (0.5) |
| I-018: ¹H-NMR(400.6 MHz, CDCl3): |
| δ= 7.2620 (33.2); 6.8795 (0.8); 6.8636 (0.8); 6.8578 (1.5); 6.8521 (0.9); 6.8361 (0.8); 6.8036 (1.2); 6.7979 (1.1); 6.7854 (1.4); 6.7798 (1.1); 6.7741 (1.3); 6.7683 (1.2); 6.7559 (1.3); 6.7502 (1.0); 5.9646 (0.5); 5.9607 (0.6); 5.9583 (0.6); 5.9542 (0.9); 5.9498 (0.5); 5.9479 (0.5); 5.9402 (0.6); 5.9360 (0.6); 5.9341 (0.6); 5.9299 (0.6); 5.8362 (0.8); 5.8341 (0.8); 5.8283 (1.5); 5.8226 (1.2); 5.8145 (1.0); 5.8088 (0.5); 5.3007 (12.9); 4.1746 (1.5); 4.1390 (2.0); 4.1034 (4.0); 3.9235 (1.7); 3.9167 (0.7); 3.8880 (1.7); 3.8789 (1.5); 3.8502 (1.1); 3.8337 (1.0); 3.8187 (0.8); 3.7400 (16.0); 3.7377 (15.4); 3.5435 (0.6); 3.5400 (0.5); 3.5379 (0.5); 3.5340 (0.5); 3.5281 (0.5); 3.3757 (0.7); 3.3523 (12.0); 3.3407 (10.3); 2.5563 (0.6); 2.5283 (0.6); 2.5220 (0.7); 2.4935 (0.6); 2.1357 (0.8); 2.0971 (1.0); 2.0658 (0.8); 1.9415 (0.6); 1.9224 (0.7); 1.9073 (0.6); 1.8877 (0.6); 1.3791 (8.2); 1.3695 (8.6); 1.2597 (0.6); 1.2549 (0.8); 0.0079 (1.0); - 0.0002 (37.2); -0.0028 (1.3); -0.0085 (1.0) |
| I-019: ¹H-NMR(400.6 MHz, CDCl3): |
| δ= 7.2617 (33.8); 6.8480 (0.8); 6.8423 (0.5); 6.8320 (0.8); 6.8262 (1.8); 6.8205 (1.0); 6.8044 (0.8); 6.7987 (0.5); 6.7681 (1.0); 6.7623 (1.0); 6.7495 (1.3); 6.7467 (1.7); 6.7438 (1.4); 6.7410 (1.3); 6.7282 (1.4); 6.7225 (1.1); 6.1616 (0.8); 5.9957 (0.8); 5.9588 (0.6); 5.9548 (0.6); 5.9527 (0.6); 5.9482 (0.7); 5.9334 (0.5); 5.9292 (0.6); 5.9273 (0.6); 5.9231 (0.5); 5.8463 (0.6); 5.8407 (1.1); 5.8338 (0.9); 5.8276 (1.4); 5.8216 (0.8); 5.8143 (0.8); 5.3006 (0.7); 4.1297 (1.1); 4.1273 (1.2); 4.0942 (1.5); 4.0918 (1.7); 3.9083 (1.4); 3.9015 (1.2); 3.8728 (1.0); 3.8661 (0.9); 3.7416 (13.9); 3.7376 (16.0); 3.5444 (0.5); 3.5385 (0.5); 3.5348 (0.5); 3.5289 (0.5); 2.5763 (0.7); 2.5627 (0.6); 2.5413 (1.1); 2.5270 (0.5); 2.5196 (0.6); 2.5058 (0.8); 2.1604 (0.9); 2.1416 (1.1); 2.1392 (1.1); 2.1357 (1.7); 2.1306 (1.2); 2.1207 (1.1); 2.1172 (1.3); 2.1122 (1.2); 2.0985 (0.6); 1.9302 (0.6); 1.9192 (1.2); 1.9081 (0.7); 1.8848 (1.0); 1.8737 (0.7); 1.5673 (0.6); 1.3611 (8.6); 1.3528 (7.5); 1.2550 (0.6); 1.1640 (0.6); 1.0925 (2.7); 1.0792 (3.3); 1.0741 (6.1); 1.0607 (6.8); 1.0555 (2.7); 1.0422 (2.9); 0.0080 (1.0); -0.0002 (37.6); -0.0085 (1.0) |
| I-020: ¹H-NMR(400.6 MHz, CDCl3): |
| δ= 7.2627 (16.8); 6.7461 (0.8); 6.7421 (1.7); 6.7386 (1.4); 6.7354 (0.7); 6.7316 (1.1); 6.7272 (2.5); 6.7211 (1.7); 6.7181 (1.2); 6.7140 (1.8); 6.7096 (1.6); 6.7030 (1.4); 6.6961 (1.0); 6.6913 (0.7); 5.9360 (0.6); 5.9325 (0.9); 5.9292 (0.9); 5.9263 (0.9); 5.9191 (0.6); 5.9149 (0.6); 5.9129 (0.6); 5.9088 (0.6); |
| 5.8330 (0.6); 5.8273 (1.1); 5.8216 (0.6); 5.8135 (0.9); 5.8077 (0.6); 5.8062 (0.6); 5.8005 (1.1); 5.7949 (0.6); 5.7867 (0.9); 5.3006 (4.7); 4.2000 (2.0); 4.1958 (1.5); 4.1822 (3.2); 4.1781 (1.6); 4.1649 (2.4); 4.1643 (2.3); 4.1604 (0.6); 4.1472 (0.7); 4.0369 (1.0); 4.0002 (4.0); 3.9792 (3.3); 3.9425 (0.8); 3.7398 (15.2); 3.7357 (16.0); 3.5324 (0.6); 3.5264 (0.5); 3.5229 (0.5); 2.5326 (0.7); 2.5196 (1.1); 2.5062 (0.5); 2.4984 (1.1); 2.4851 (0.8); 2.1328 (0.8); 2.0456 (0.6); 1.9044 (0.7); 1.8899 (0.7); 1.8792 (0.6); 1.8696 (0.7); 1.8555 (0.6); 1.5871 (2.0); 1.3546 (8.6); 1.3457 (8.3); 1.2595 (0.7); 1.2548 (0.5); 1.2331 (3.0); 1.2250 (3.1); 1.2154 (6.5); 1.2073 (6.4); 1.1976 (3.0); 1.1896 (3.0); 0.0080 (0.6); -0.0002 (20.4); -0.0085 (0.6) |
| I-021: ¹H-NMR(400.6 MHz, CDCl3): |
| δ= 7.2607 (39.7); 6.7451 (3.2); 6.7397 (0.8); 6.7281 (1.6); 6.7229 (1.8); 5.9314 (0.5); 5.9295 (0.5); 5.9218 (0.6); 5.9177 (0.7); 5.9157 (0.7); 5.9116 (0.6); 5.8328 (0.6); 5.8272 (1.2); 5.8215 (0.6); 5.8191 (0.5); 5.8134 (0.9); 5.3003 (3.2); 4.0381 (0.6); 4.0014 (2.2); 3.9788 (1.9); 3.9422 (0.5); 3.7439 (16.0); 3.7336 (14.6); 3.3510 (0.6); 2.5359 (0.7); 2.5015 (0.8); 1.8987 (0.7); 1.8644 (0.6); 1.5478 (11.0); 1.3507 (8.9); 0.0079 (1.4); -0.0002 (46.4); -0.0085 (1.2) |
| I-022: ¹H-NMR(400.6 MHz, CDCl3): |
| δ= 7.2609 (28.3); 6.7405 (0.5); 6.7279 (1.5); 6.7224 (1.8); 6.7183 (0.6); 6.7115 (0.9); 6.7043 (2.3); 5.9406 (0.6); 5.9366 (0.6); 5.9344 (0.6); 5.9304 (0.6); 5.8146 (0.6); 5.8090 (1.1); 5.8033 (0.6); 5.8009 (0.5); 5.7952 (0.9); 5.3003 (0.9); 4.0396 (0.6); 4.0029 (2.3); 3.9825 (2.0); 3.7438 (1.2); 3.7388 (16.0); 3.7337 (1.0); 3.7181 (14.0); 2.5181 (0.7); 2.5044 (0.5); 2.4833 (0.8); 1.9051 (0.7); 1.8703 (0.6); 1.5545 (3.0); 1.3592 (8.9); 1.3508 (0.6); 0.0079 (0.9); -0.0002 (31.1); -0.0027 (1.2); -0.0085 (0.9) |
| I-023: ¹H-NMR(300.1 MHz, d₆-DMSO): |
| δ= 7.5873 (2.9); 7.5764 (2.6); 7.5607 (3.0); 7.5497 (2.3); 7.1241 (2.2); 7.1170 (1.7); 7.0870 (7.5); 7.0731 (5.4); 7.0574 (6.9); 7.0445 (5.2); 6.0175 (3.1); 5.9819 (3.8); 5.9598 (4.2); 5.9242 (4.2); 5.8939 (2.5); 5.8823 (2.2); 5.8756 (4.0); 5.8690 (3.5); 5.8573 (1.6); 5.8507 (2.2); 5.7600 (1.2); 5.6809 (1.9); 5.6728 (3.7); 5.6648 (4.2); 5.6556 (4.0); 5.6468 (2.9); 5.6381 (1.1); 5.5718 (16.0); 5.3457 (3.3); 5.3404 (4.4); 5.2829 (3.8); 5.1168 (4.5); 5.0811 (4.2); 4.5558 (2.2); 4.5346 (1.6); 4.0551 (2.4); 4.0069 (8.7); 3.9793 (6.1); 3.9294 (1.7); 3.6373 (25.1); 3.6218 (30.3); 3.5819 (35.5); 3.5403 (2.2); 3.5303 (2.6); 3.5215 (2.6); 3.3344 (215.4); 2.7277 (0.7); 2.5077 (101.0); 2.5022 (127.1); 2.4968 (89.7); 2.4117 (1.5); 2.3828 (2.6); 2.3670 (1.9); 2.3545 (1.7); 2.3385 (2.9); 2.3109 (1.4); 2.2722 (0.8); 1.7680 (0.9); 1.7477 (1.4); 1.7287 (1.0); 1.7035 (1.4); 1.6859 (1.5); 1.6678 (1.9); 1.6427 (1.2); 1.6236 (1.8); 1.6051 (0.9); 1.2361 (0.4); - 0.0002 (28.6) |
| I-024: ¹H-NMR(400.6 MHz, CDCl3): |
| δ= 7.3423 (0.6); 7.3223 (0.6); 7.2626 (12.6); 6.7598 (0.8); 6.7547 (1.5); 6.7404 (1.7); 6.7354 (3.3); 6.7281 (1.0); 6.7232 (0.8); 6.7200 (1.7); 6.7180 (2.2); 6.7149 (1.8); 6.7115 (1.6); 6.7007 (0.7); 6.6988 (0.5); 6.6933 (0.6); 5.3005 (1.1); 4.2163 (0.7); 4.1989 (0.6); 4.1896 (0.6); 4.1845 (0.7); 4.1737 (1.3); 4.1717 (1.6); 4.1666 (1.5); 4.1559 (3.6); 4.1539 (1.8); 4.1488 (1.5); 4.1381 (3.5); 4.1310 (0.6); 4.1203 (1.1); 4.0986 (5.4); 4.0915 (2.4); 4.0822 (2.4); 3.7141 (16.0); 3.7112 (14.8); 2.5025 (1.7); 2.5003 (1.6); 2.4888 (3.0); 2.4861 (2.9); 2.4742 (1.4); 2.4711 (1.4); 1.5899 (0.9); 1.2966 (3.7); 1.2813 (4.6); 1.2788 (8.2); 1.2635 (9.0); 1.2610 (4.2); 1.2456 (4.1); 1.2356 (5.0); 1.2273 (4.9); 1.2186 (5.0); 1.2104 (4.7); 1.0937 (0.6); 1.0871 (0.6); 1.0733 (1.2); 1.0601 (0.6); 1.0531 (0.8); 0.5245 (0.7); 0.5222 (0.7); 0.5110 (0.8); 0.5084 (0.8); 0.4231 (0.6); 0.4192 (0.5); 0.4151 (0.9); 0.4069 (1.6); 0.4008 (1.3); 0.3924 (1.4); 0.3867 (1.2); 0.3787 (0.8); 0.2545 (0.6); 0.2478 (0.8); 0.2397 (0.6); 0.2329 (0.6); 0.2264 (0.5); 0.0080 (0.5); -0.0002 (16.9) |
| I-025: ¹H-NMR(400.6 MHz, CDCl3): |
| δ= 7.2627 (12.5); 6.8455 (0.5); 6.7720 (1.0); 6.7665 (1.5); 6.7524 (1.2); 6.7466 (1.8); 6.7410 (1.0); 6.7365 (0.9); 6.7295 (1.9); 6.7244 (1.7); 6.7231 (1.8); 6.7188 (2.4); 6.7128 (1.4); 6.7088 (0.8); 6.7055 (1.8); 6.7010 (0.9); 6.6969 (0.9); 5.3005 (2.1); 4.1280 (0.7); 4.0968 (4.7); 4.0913 (2.4); 4.0636 (2.1); 4.0268 (0.7); 3.9071 (0.5); 3.8909 (0.6); 3.7188 (15.2); 3.7143 (16.0); 3.6881 (13.7); 3.6613 (15.0); 2.3660 (0.6); 2.3499 (1.4); 2.3453 (1.5); 2.3290 (2.1); 2.3245 (1.0); 2.3137 (0.8); 2.3101 (1.1); 1.8503 (0.5); 1.8332 (0.7); 1.8303 (0.6); 1.8129 (0.8); 1.7968 (0.5); 1.7927 (0.7); 1.7758 (0.6); 1.5872 (1.4); 1.1642 (5.3); 1.1569 (5.1); 1.1476 (5.3); 1.1405 (5.0); 1.0977 (0.7); 1.0772 (0.5); 1.0604 (0.6); 0.5354 (0.7); 0.5234 (0.8); 0.5115 (0.8); 0.4292 (0.5); 0.4203 (0.7); 0.4071 (1.0); 0.4018 (1.2); 0.3976 (1.0); 0.3937 (0.6); 0.3850 (1.6); 0.3708 (0.8); 0.3602 (0.6); 0.2842 (0.6); 0.2713 (0.6); -0.0002 (17.0) |
| I-026: ¹H-NMR(400.6 MHz, CDCl3): |
| δ= 7.2625 (13.2); 6.9211 (1.2); 6.9077 (1.3); 6.8819 (1.4); 6.8792 (1.4); 6.8680 (1.9); 6.8399 (1.4); 6.8283 (1.5); 6.7349 (0.8); 6.7298 (2.2); 6.7247 (1.8); 6.7177 (0.6); 6.7132 (1.7); 6.7078 (5.1); 6.6916 (1.0); 6.6873 (2.8); 5.9045 (1.4); 5.9003 (1.4); 5.8828 (1.5); 5.8782 (1.4); 5.8653 (1.3); 5.8611 (1.3); 5.8435 (1.3); 5.8389 (1.3); 5.3003 (3.0); 4.2378 (1.1); 4.2200 (3.5); 4.2065 (0.9); 4.2021 (3.6); 4.1897 (2.3); 4.1888 (2.4); 4.1843 (1.2); 4.1718 (2.5); 4.1539 (0.8); 4.1137 (5.2); 4.0764 (4.8); 3.7312 (15.6); 3.7227 (16.0); 1.5794 (2.1); 1.3169 (4.9); 1.3092 (4.4); 1.3047 (5.0); 1.2993 (5.2); 1.2913 (11.4); 1.2873 (4.9); 1.2732 (11.4); 1.2552 (4.1); 1.1083 (0.6); 1.1013 (1.0); 1.0881 (0.8); 1.0675 (0.6); 0.5523 (0.7); 0.5432 (0.7); 0.5386 (0.8); 0.5338 (0.6); 0.5291 (0.6); 0.5190 (0.5); 0.4764 (0.8); 0.4718 (1.1); 0.4565 (1.3); 0.4537 (1.2); 0.4430 (0.8); 0.4400 (1.1); 0.4328 (0.6); 0.4291 (1.3); 0.4140 (1.0); 0.4107 (0.8); 0.3400 (0.6); 0.3191 (0.5); 0.0079 (0.5); 0.0022 (0.8); -0.0002 (18.2); -0.0027 (0.8); -0.0035 (0.5); - 0.0085 (0.5) |
| I-027: ¹H-NMR(400.6 MHz, CDCl3): |
| δ= 7.2619 (17.0); 6.7436 (0.6); 6.7418 (0.6); 6.7360 (2.4); 6.7296 (1.5); 6.7258 (1.5); 6.7211 (1.3); 6.7188 (1.5); 6.7167 (1.1); 6.7148 (1.0); 6.7119 (2.2); 6.7079 (0.8); 6.7028 (0.5); 5.3004 (3.4); 4.5824 (0.6); 4.5726 (0.8); 4.5597 (0.8); 4.5496 (0.7); 4.5466 (0.5); 4.0833 (6.4); 4.0496 (0.7); 4.0359 (0.8); 4.0262 (1.0); 4.0125 (1.0); 3.9980 (0.6); 3.8815 (0.5); 3.8759 (0.5); 3.8112 (14.6); 3.7964 (8.9); 3.7215 (16.0); 2.5747 (0.5); 2.5572 (0.6); 2.5406 (0.6); 2.5340 (0.5); 2.5231 (0.6); 2.0181 (0.6); 1.5644 (3.3); 1.0513 (0.6); 0.5159 (0.5); 0.4227 (0.8); 0.4170 (0.8); 0.4082 (1.1); 0.3982 (0.7); 0.3940 (0.6); 0.2524 (0.5); 0.0080 (0.6); -0.0002 (23.7); -0.0085 (0.6) |
| I-028: ¹H-NMR(400.6 MHz, CDCl3): |
| δ= 7.2623 (18.1); 6.7529 (1.2); 6.7492 (2.4); 6.7471 (2.1); 6.7339 (3.5); 6.7284 (3.9); 6.7240 (2.6); 6.7168 (2.3); 6.7128 (2.0); 6.7098 (2.3); 6.7061 (1.7); 6.7015 (1.3); 5.9540 (0.5); 5.9478 (0.8); 5.9438 (0.7); 5.9404 (0.8); 5.9364 (0.8); 5.9340 (1.0); 5.9300 (0.9); 5.9257 (0.6); 5.9215 (0.7); 5.9198 (0.7); 5.9155 (0.6); 5.9119 (0.7); 5.9076 (0.8); 5.9059 (0.8); 5.9018 (0.7); 5.8349 (0.7); 5.8294 (1.4); 5.8246 (1.5); 5.8192 (1.7); 5.8157 (1.5); 5.8109 (1.3); 5.8054 (1.1); 5.7997 (0.5); 5.3003 (0.6); 4.9406 (0.8); 4.9374 (0.8); 4.9279 (0.7); 4.9238 (0.7); 4.9201 (0.7); 4.1095 (3.0); 4.1019 (5.8); 4.0954 (3.1); 3.7637 (0.9); 3.7530 (1.0); 3.7398 (15.0); 3.7335 (16.0); 3.7206 (13.7); 3.7128 (5.7); 3.7108 (6.1); 3.7077 (14.6); 3.7018 (4.0); 3.6927 (3.6); 3.5379 (0.6); 3.5321 (0.8); 3.5284 (0.7); 3.5224 (0.8); 3.5165 (0.7); 3.5110 (0.6); 2.5429 (0.5); 2.5396 (0.8); 2.5260 (0.6); 2.5221 (0.9); 2.5187 (0.6); 2.5082 (0.7); 2.5052 (1.0); 2.5011 (0.6); 2.4872 (0.9); 2.4844 (0.7); 1.8987 (0.8); 1.8817 (0.5); 1.8717 (0.9); 1.8640 (0.9); 1.8526 (0.6); 1.8369 (0.8); 1.5798 (4.6); 1.1205 (0.7); 1.1140 (0.8); 1.1076 (0.7); 1.1004 (1.4); 1.0938 (0.8); 1.0870 (0.9); 1.0803 (0.9); 0.5326 (0.8); 0.5190 (1.3); 0.5102 (0.9); 0.5053 (1.0); 0.4968 (0.8); 0.4916 (0.6); 0.4536 (0.6); 0.4454 (0.8); 0.4402 (1.0); 0.4316 (1.2); 0.4249 (1.2); 0.4170 (2.0); 0.4027 (1.7); 0.3972 (1.0); 0.3930 (1.3); 0.3885 (1.1); 0.3801 (0.6); 0.2973 (0.5); 0.2842 (0.8); 0.2761 (1.2); 0.2673 (0.7); 0.2629 (0.7); 0.2537 (1.0); 0.0079 (0.6); -0.0002 (22.7); -0.0085 (0.8) |
| I-029: ¹H-NMR(400.6 MHz, CDCl3): |
| δ= 7.2627 (6.3); 6.7343 (3.2); 6.7287 (1.4); 6.7163 (3.0); 6.7114 (2.3); 6.6974 (0.7); 6.6924 (0.6); 5.3005 (3.6); 4.1829 (0.5); 4.0949 (4.3); 4.0909 (4.3); 3.7167 (16.0); 3.7124 (10.0); 3.7068 (8.7); 2.8849 (0.6); 2.8677 (0.6); 2.2337 (0.5); 2.2165 (0.6); 2.2120 (0.5); 1.9807 (1.0); 1.9722 (1.2); 1.9666 (1.4); 1.9632 (1.4); 1.9543 (1.4); 1.9488 (1.3); 1.9329 (1.1); 1.9223 (0.5); 1.7489 (0.5); 1.7324 (0.5); 1.5885 (2.8); 1.0899 (0.7); 1.0825 (0.7); 0.5206 (0.7); 0.5068 (0.9); 0.4291 (0.8); 0.4247 (0.9); 0.4087 (1.8); 0.3947 (1.3); 0.3875 (0.8); 0.2660 (0.7); 0.2450 (0.8); -0.0002 (7.9) |
| I-030: ¹H-NMR(400.6 MHz, d₆-DMSO): |
| δ= 7.4336 (1.0); 7.4290 (0.6); 7.3751 (3.0); 7.3704 (2.5); 5.7564 (1.6); 4.8615 (0.7); 4.8557 (0.7); 3.6073 (3.7); 3.5991 (3.8); 3.5833 (2.4); 3.5808 (2.3); 3.3176 (16.0); 2.5234 (0.8); 2.5187 (1.0); 2.5100 (13.3); 2.5054 (29.1); 2.5008 (40.6); 2.4962 (27.8); 2.4917 (12.1); 0.0080 (0.6); -0.0002 (18.6); -0.0085 (0.5) |
| I-031: ¹H-NMR(400.6 MHz, CDCl3): |
| δ= 7.2622 (17.3); 7.2583 (3.4); 7.2559 (3.1); 7.2538 (1.9); 7.2513 (1.6); 7.0836 (5.1); 7.0790 (4.8); 7.0673 (5.0); 7.0627 (4.8); 5.9645 (0.5); 5.9622 (0.5); 5.9584 (0.5); 5.9546 (0.7); 5.9508 (0.7); 5.9485 (0.7); 5.9446 (0.7); 5.9246 (0.5); 5.9204 (0.6); 5.9186 (0.6); 5.9144 (0.5); 5.9109 (0.7); 5.9066 (0.8); 5.9050 (0.8); 5.9007 (0.7); 5.8876 (0.7); 5.8821 (1.2); 5.8765 (0.7); 5.8740 (0.5); 5.8683 (0.9); 5.8276 (0.7); 5.8220 (1.2); 5.8163 (0.7); 5.8140 (0.6); 5.8082 (1.0); 5.8026 (0.5); 5.3003 (10.9); 4.9350 (0.6); 4.9293 (0.6); 4.9237 (0.6); 4.9193 (0.6); 4.9138 (0.6); 4.1002 (2.8); 4.0921 (4.6); 4.0859 (2.7); 3.7402 (15.6); 3.7333 (16.0); 3.7158 (13.4); 3.7030 (13.1); 3.5386 (0.6); 3.5351 (0.6); 3.5327 (0.6); 3.5291 (0.7); 2.5745 (0.8); 2.5402 (0.8); 2.5257 (0.8); 2.5121 (0.5); 2.4909 (0.8); 1.9234 (0.8); 1.8890 (0.7); |
| 1.8775 (0.8); 1.8674 (0.8); 1.8326 (0.7); 1.5810 (6.2); 1.1162 (0.6); 1.1096 (0.6); 1.1027 (0.5); 1.0959 (1.2); 1.0888 (0.5); 1.0824 (0.7); 1.0758 (0.7); 0.5324 (0.8); 0.5186 (1.2); 0.5101 (0.7); 0.5047 (0.9); 0.4970 (0.5); 0.4549 (0.6); 0.4468 (0.7); 0.4407 (0.7); 0.4330 (1.1); 0.4250 (1.0); 0.4182 (1.7); 0.4121 (1.1); 0.4044 (1.5); 0.3926 (0.9); 0.2884 (0.6); 0.2800 (0.9); 0.2670 (0.5); 0.2578 (0.7); 0.0080 (0.5); - 0.0002 (16.8) |
| I-032: ¹H-NMR(400.6 MHz, CDCl3): |
| δ= 7.5210 (0.7); 7.4994 (0.6); 7.2627 (10.9); 7.2554 (2.4); 7.2535 (2.1); 7.2489 (1.0); 7.0659 (5.0); 7.0612 (6.9); 7.0561 (3.4); 5.3006 (5.9); 4.5870 (0.8); 4.5771 (1.0); 4.5644 (1.4); 4.5542 (1.0); 4.5505 (0.8); 4.5412 (0.6); 4.4879 (0.5); 4.4805 (0.6); 4.4701 (0.8); 4.4628 (0.7); 4.0723 (5.3); 4.0491 (1.0); 4.0421 (0.9); 4.0354 (1.0); 4.0288 (1.3); 4.0259 (1.4); 4.0122 (1.0); 3.9779 (0.6); 3.9731 (0.6); 3.8762 (0.8); 3.8700 (0.9); 3.8530 (0.6); 3.8468 (0.6); 3.8103 (12.9); 3.7950 (8.2); 3.7517 (1.2); 3.7170 (16.0); 2.5755 (0.6); 2.5647 (0.6); 2.5578 (0.7); 2.5471 (0.6); 2.5416 (0.7); 2.5237 (0.7); 2.0812 (0.6); 2.0459 (0.8); 1.5828 (4.6); 1.0709 (0.8); 1.0640 (0.6); 1.0580 (0.6); 1.0502 (1.0); 1.0370 (0.5); 0.5291 (0.8); 0.5205 (0.9); 0.5151 (0.9); 0.5075 (0.8); 0.4987 (0.7); 0.4400 (0.5); 0.4311 (0.8); 0.4256 (1.2); 0.4185 (1.3); 0.4117 (1.7); 0.3989 (1.1); 0.3851 (0.6); 0.2802 (0.6); 0.2731 (0.5); 0.2653 (0.5); 0.2584 (0.7); 0.2521 (0.9); -0.0002 (10.7) |
| I-033: ¹H-NMR(400.6 MHz, CDCl3): |
| δ= 7.3395 (0.7); 7.2658 (6.6); 7.2567 (1.6); 7.2544 (1.7); 7.2521 (3.3); 7.2498 (3.1); 7.2475 (1.9); 7.2453 (1.6); 7.0922 (5.7); 7.0876 (5.5); 7.0673 (6.0); 7.0627 (5.8); 5.3007 (14.7); 4.2254 (0.5); 4.2089 (0.7); 4.2042 (0.6); 4.1949 (0.9); 4.1886 (0.9); 4.1770 (1.8); 4.1741 (1.6); 4.1709 (1.8); 4.1591 (1.9); 4.1563 (3.9); 4.1532 (1.8); 4.1384 (3.8); 4.1206 (1.2); 4.0883 (5.7); 4.0796 (2.7); 4.0741 (2.7); 3.7090 (16.0); 3.7060 (15.0); 2.5231 (1.6); 2.5157 (1.5); 2.5092 (1.6); 2.5032 (2.0); 2.5014 (1.8); 2.4894 (2.7); 2.4739 (1.4); 1.2981 (3.9); 1.2804 (9.2); 1.2705 (0.7); 1.2638 (10.2); 1.2580 (5.9); 1.2460 (4.6); 1.2411 (5.4); 1.2278 (5.0); 1.2198 (0.5); 1.2110 (5.0); 1.0894 (0.7); 1.0829 (0.6); 1.0689 (1.3); 1.0558 (0.7); 1.0489 (0.8); 0.5253 (0.7); 0.5220 (0.8); 0.5173 (0.5); 0.5119 (1.0); 0.5086 (0.9); 0.5001 (0.6); 0.4235 (0.7); 0.4174 (1.0); 0.4086 (1.8); 0.4033 (1.5); 0.3941 (1.6); 0.3878 (1.1); 0.3803 (0.9); 0.2746 (0.5); 0.2592 (0.7); 0.2525 (0.8); 0.2449 (0.6); 0.2376 (0.7); 0.2309 (0.6); -0.0002 (6.8) |
| I-034: ¹H-NMR(400.6 MHz, CDCl3): |
| δ= 7.2626 (15.8); 7.2585 (1.8); 7.2571 (2.0); 7.2538 (3.2); 7.2525 (3.6); 7.2492 (1.8); 7.2479 (2.0); 7.1034 (5.8); 7.0988 (5.6); 7.0595 (6.0); 7.0550 (5.8); 6.8458 (0.7); 5.3004 (1.2); 4.1151 (0.7); 4.0864 (5.8); 4.0784 (2.4); 4.0518 (2.3); 4.0150 (0.7); 3.9036 (0.5); 3.8913 (0.6); 3.8867 (0.7); 3.8695 (0.6); 3.7151 (14.8); 3.7094 (15.6); 3.6886 (14.6); 3.6618 (16.0); 2.3819 (0.6); 2.3687 (0.7); 2.3638 (0.8); 2.3612 (0.9); 2.3508 (1.5); 2.3443 (1.2); 2.3314 (1.8); 2.3241 (0.9); 2.3105 (1.0); 2.3079 (1.1); 1.8615 (0.6); 1.8471 (0.7); 1.8430 (0.7); 1.8354 (0.7); 1.8289 (0.8); 1.8267 (0.9); 1.8147 (1.0); 1.8084 (0.6); 1.7978 (0.8); 1.7142 (0.6); 1.5864 (2.2); 1.1852 (5.5); 1.1686 (5.4); 1.1559 (5.3); 1.1394 (5.2); 1.0930 (0.8); 1.0726 (0.8); 1.0523 (0.6); 0.5353 (0.8); 0.5251 (0.9); 0.5216 (0.8); 0.5117 (1.1); 0.4291 (0.6); 0.4264 (0.5); 0.4199 (0.8); 0.4068 (0.9); 0.4006 (1.5); 0.3952 (1.0); 0.3854 (1.5); 0.3817 (1.0); 0.3722 (0.8); 0.3679 (0.6); 0.2888 (0.6); 0.2807 (0.5); 0.2758 (0.7); -0.0002 (16.0) |
| I-035: ¹H-NMR(400.6 MHz, CDCl3): |
| δ= 7.2684 (1.7); 7.2614 (32.8); 7.2538 (1.8); 7.2492 (2.9); 7.2447 (1.6); 7.0845 (0.6); 7.0562 (5.1); 7.0516 (4.9); 7.0390 (6.0); 7.0345 (5.9); 7.0110 (0.5); 6.9563 (1.2); 6.9431 (1.2); 6.9171 (1.3); 6.9039 (1.3); 6.8913 (1.0); 6.8797 (1.0); 6.8520 (1.1); 6.8405 (1.1); 5.9336 (1.4); 5.9293 (1.3); 5.8943 (1.3); 5.8900 (1.4); 5.8872 (1.3); 5.8826 (1.2); 5.8478 (1.0); 5.8433 (1.1); 5.3003 (0.8); 4.1031 (5.4); 4.0615 (6.6); 3.7531 (16.0); 3.7289 (15.8); 3.7241 (14.7); 3.7184 (13.0); 1.7499 (0.7); 1.3408 (4.6); 1.3232 (4.6); 1.3059 (5.4); 1.2885 (5.3); 1.2548 (0.6); 1.0976 (1.1); 1.0907 (0.7); 1.0850 (0.6); 1.0777 (1.0); 1.0642 (0.7); 1.0574 (0.5); 0.5520 (0.7); 0.5457 (0.9); 0.5369 (0.9); 0.5320 (0.9); 0.5230 (0.7); 0.4724 (1.1); 0.4561 (1.5); 0.4418 (1.0); 0.4276 (1.3); 0.4110 (1.8); 0.3967 (0.6); 0.3473 (0.6); 0.3280 (0.5); 0.2652 (0.5); 0.0079 (1.3); -0.0002 (39.7); -0.0085 (1.4) |
| I-036: ¹H-NMR(400.6 MHz, CDCl3): |
| δ= 7.3085 (0.8); 7.2842 (0.9); 7.2620 (32.6); 7.2436 (2.3); 7.0320 (3.1); 7.0024 (3.1); 6.9527 (0.7); 6.9394 (0.7); 6.9129 (0.7); 6.9002 (0.8); 6.8653 (0.8); 6.8521 (0.8); 6.8255 (0.8); 6.8128 (0.7); 5.9434 (0.9); 5.9057 (1.7); 5.8680 (0.9); 5.3004 (2.5); 4.6773 (0.5); 4.6622 (0.6); 4.5676 (0.5); 4.5515 (0.5); 4.0602 (0.6); 4.0237 (1.4); 3.9970 (1.3); 3.9647 (1.6); 3.9275 (0.9); 3.9123 (1.6); 3.8758 (0.9); 3.7577 (11.2); 3.7361 (8.2); 3.7280 (16.0); 1.7305 (0.8); 1.7158 (0.9); 1.6991 (0.7); 1.6299 (0.6); 1.6125 (0.9); |
| 1.5949 (0.9); 1.5792 (0.7); 1.3332 (3.6); 1.3114 (6.0); 1.2936 (4.5); 1.2561 (1.3); 0.8799 (4.4); 0.0080 (0.7); -0.0002 (39.1); -0.0084 (2.0) |
| I-037: ¹H-NMR(400.6 MHz, CDCl3): |
| δ= 7.2624 (17.9); 6.7334 (0.7); 6.7281 (0.8); 6.7107 (3.2); 6.6893 (3.6); 5.3006 (2.1); 4.1632 (0.5); 4.0548 (0.8); 4.0472 (0.8); 4.0181 (1.6); 4.0105 (1.6); 3.9619 (2.0); 3.9587 (1.9); 3.9253 (0.9); 3.9219 (0.9); 3.7263 (16.0); 3.7243 (11.2); 3.7172 (9.6); 3.7086 (13.4); 2.8924 (0.6); 2.8707 (0.6); 2.2384 (0.6); 2.2207 (0.6); 2.2046 (0.7); 2.1868 (0.6); 1.9832 (1.1); 1.9736 (1.3); 1.9677 (1.4); 1.9558 (1.6); 1.9448 (1.2); 1.9340 (0.6); 1.9287 (1.2); 1.9180 (0.6); 1.9056 (0.6); 1.7962 (0.5); 1.7626 (0.5); 1.7497 (1.0); 1.7342 (1.0); 1.7162 (1.3); 1.6977 (1.0); 1.6267 (0.5); 1.6033 (0.7); 1.5892 (0.8); 1.5855 (0.8); 1.5714 (0.8); 1.5676 (0.7); 1.5522 (0.6); 0.8735 (1.7); 0.8664 (1.9); 0.8551 (3.7); 0.8480 (3.8); 0.8366 (1.7); 0.8296 (1.7); 0.0079 (0.6); -0.0002 (22.5); -0.0085 (0.7) |
| I-038: ¹H-NMR(400.6 MHz, CDCl3): |
| δ= 7.2641 (8.6); 6.7388 (3.4); 6.7351 (1.0); 6.7332 (1.0); 6.7214 (3.2); 6.7162 (3.2); 6.7049 (1.0); 6.6981 (2.4); 5.9485 (0.5); 5.9444 (0.6); 5.9423 (0.6); 5.9383 (0.5); 5.9214 (0.5); 5.9172 (0.6); 5.9154 (0.6); 5.9112 (0.5); 5.9077 (0.7); 5.9034 (0.8); 5.9017 (0.7); 5.8975 (0.6); 5.8126 (0.6); 5.8074 (1.4); 5.8021 (1.6); 5.7987 (0.7); 5.7966 (0.8); 5.7936 (1.2); 5.7884 (1.3); 5.7828 (0.5); 5.3007 (3.1); 4.0547 (1.8); 4.0181 (3.7); 3.9553 (2.4); 3.9186 (1.2); 3.7475 (16.0); 3.7350 (13.5); 3.7257 (14.8); 3.7116 (11.6); 3.5381 (0.6); 3.5321 (0.6); 3.5285 (0.6); 3.5263 (0.6); 3.5227 (0.6); 3.5205 (0.6); 3.5168 (0.6); 2.5283 (0.8); 2.5172 (0.7); 2.5153 (0.7); 2.4940 (0.9); 2.4828 (0.7); 1.8992 (0.8); 1.8736 (0.7); 1.8646 (0.9); 1.8388 (0.6); 1.7540 (0.7); 1.7513 (0.5); 1.7357 (1.1); 1.7201 (0.9); 1.7174 (0.8); 1.7016 (0.8); 1.6236 (2.2); 1.5904 (1.0); 1.5721 (1.2); 1.5566 (0.8); 1.5383 (0.8); 0.8943 (1.9); 0.8758 (4.8); 0.8549 (5.6); 0.8363 (2.1); -0.0002 (10.7) |
| I-039: ¹H-NMR(400.6 MHz, CDCl3): |
| δ= 7.6326 (0.8); 7.2648 (9.0); 6.7438 (0.5); 6.7383 (0.8); 6.7315 (0.8); 6.7184 (3.6); 6.7135 (3.3); 6.7020 (2.0); 6.6978 (3.5); 6.6950 (3.6); 6.6894 (0.7); 5.3011 (7.2); 4.5794 (0.9); 4.5736 (1.0); 4.5694 (1.0); 4.5648 (1.0); 4.5564 (1.0); 4.5499 (1.1); 4.5465 (1.0); 4.5411 (0.9); 4.4555 (0.5); 4.4511 (0.6); 4.4484 (0.6); 4.4446 (0.5); 4.4380 (0.6); 4.0386 (1.0); 4.0339 (1.6); 4.0256 (1.7); 4.0217 (1.0); 4.0153 (1.3); 4.0115 (1.4); 4.0016 (1.4); 3.9970 (3.0); 3.9894 (2.4); 3.9454 (2.1); 3.9369 (2.2); 3.9294 (0.9); 3.9226 (0.8); 3.9087 (1.1); 3.9001 (1.5); 3.8734 (0.7); 3.8664 (0.7); 3.8502 (0.6); 3.8431 (0.6); 3.8260 (15.1); 3.7943 (16.0); 3.7564 (1.6); 3.7543 (1.4); 3.7273 (15.8); 3.7241 (14.9); 2.5462 (0.5); 2.5409 (0.8); 2.5346 (0.5); 2.5292 (0.6); 2.5230 (0.9); 2.5186 (0.6); 2.5120 (0.6); 2.5055 (0.6); 2.5006 (0.5); 2.4884 (0.5); 2.0451 (0.5); 2.0328 (0.6); 2.0306 (0.7); 2.0219 (0.8); 1.9989 (0.6); 1.9964 (0.6); 1.9894 (0.7); 1.9874 (0.7); 1.7448 (0.6); 1.7267 (0.9); 1.7109 (1.1); 1.6929 (1.4); 1.6747 (0.9); 1.6243 (0.5); 1.5933 (0.8); 1.5780 (1.0); 1.5751 (0.9); 1.5596 (1.3); 1.5442 (0.7); 1.5413 (0.8); 1.5260 (0.6); 0.9150 (0.5); 0.8676 (2.4); 0.8491 (5.4); 0.8428 (2.8); 0.8306 (2.5); 0.8243 (5.6); 0.8058 (2.3); -0.0002 (11.0) |
| I-040: ¹H-NMR(400.6 MHz, CDCl3): |
| δ= 7.4758 (1.2); 7.4544 (1.3); 7.2618 (30.6); 6.7148 (6.5); 4.2170 (1.8); 4.1684 (3.4); 4.1485 (4.9); 4.1301 (4.1); 4.1128 (1.5); 4.0329 (1.0); 3.9961 (2.4); 3.9759 (2.6); 3.9553 (4.6); 3.9189 (1.6); 3.7171 (16.0); 2.5357 (0.7); 2.5117 (1.9); 2.4915 (2.7); 2.4785 (4.4); 2.4648 (3.4); 2.4400 (1.1); 1.7080 (1.6); 1.6906 (1.4); 1.5608 (1.5); 1.3010 (3.1); 1.2826 (7.4); 1.2603 (9.9); 1.2421 (5.9); 1.2255 (9.5); 1.2128 (9.6); 0.8583 (6.5); 0.8422 (6.8); -0.0002 (37.4) |
| I-041: ¹H-NMR(400.6 MHz, CDCl3): |
| δ= 7.5200 (0.6); 7.5115 (0.6); 7.4927 (0.6); 7.2616 (21.9); 6.7546 (3.3); 6.7498 (1.4); 6.7391 (2.4); 6.7333 (3.1); 6.7272 (1.3); 6.7234 (1.2); 6.7157 (1.7); 6.7096 (0.9); 5.3004 (1.1); 4.4945 (3.6); 4.4838 (2.1); 4.3768 (3.6); 4.3666 (2.3); 4.2526 (1.2); 4.2249 (0.6); 4.2155 (2.0); 4.2066 (2.0); 4.1978 (1.0); 4.1900 (1.0); 4.1799 (2.0); 4.1723 (2.4); 4.1699 (3.2); 4.1620 (1.9); 4.1527 (3.4); 4.1481 (0.6); 4.1443 (0.8); 4.1348 (3.2); 4.1170 (1.0); 4.0527 (1.2); 4.0501 (1.2); 4.0376 (1.0); 4.0157 (0.7); 4.0130 (0.8); 4.0005 (0.7); 3.7335 (13.6); 3.7311 (16.0); 2.4897 (2.0); 2.4770 (2.8); 2.4733 (1.9); 2.4687 (1.8); 2.4637 (1.8); 2.4544 (1.6); 1.5664 (5.3); 1.3025 (4.1); 1.2846 (8.6); 1.2795 (3.8); 1.2668 (4.4); 1.2617 (7.5); 1.2511 (1.6); 1.2438 (3.6); 1.2341 (1.3); 1.2270 (5.7); 1.2140 (4.9); 1.2102 (6.1); 1.1972 (4.3); 0.0080 (0.8); -0.0002 (28.6); -0.0085 (1.0) |
| I-042: ¹H-NMR(400.6 MHz, CDCl3): |
| δ= 7.2630 (14.3); 7.0039 (0.7); 6.9829 (0.7); 6.7555 (1.0); 6.7509 (1.9); 6.7435 (0.8); 6.7365 (1.8); 6.7310 (2.2); 6.7216 (2.9); 6.7161 (2.5); 6.7085 (0.9); 6.7050 (1.1); 6.7002 (1.2); 6.6976 (2.0); 6.6952 (1.5); 6.6920 (0.7); 6.6888 (0.5); 5.3006 (1.8); 4.0217 (0.9); 3.9926 (0.6); 3.9850 (2.3); 3.9559 (2.9); |
| 3.9492 (2.3); 3.9425 (2.8); 3.9125 (0.9); 3.9060 (0.8); 3.8860 (0.6); 3.7212 (16.0); 3.7171 (14.7); 3.6925 (14.8); 3.6640 (14.2); 3.6597 (3.1); 2.3640 (0.7); 2.3491 (1.6); 2.3434 (1.2); 2.3322 (3.0); 2.3124 (2.0); 1.8544 (0.6); 1.8340 (0.9); 1.8199 (0.8); 1.8143 (0.7); 1.8005 (0.9); 1.7910 (0.5); 1.7866 (0.7); 1.7826 (0.7); 1.7799 (0.9); 1.7700 (0.9); 1.7654 (0.6); 1.7622 (0.8); 1.7589 (0.5); 1.7513 (0.9); 1.7327 (0.8); 1.7284 (0.7); 1.7174 (0.9); 1.7141 (0.7); 1.7100 (0.7); 1.6988 (1.0); 1.6950 (0.9); 1.6764 (0.9); 1.6126 (0.5); 1.6039 (1.1); 1.5943 (1.0); 1.5856 (1.1); 1.5758 (1.0); 1.5704 (0.8); 1.5604 (0.7); 1.5520 (0.6); 1.5421 (0.6); 1.2555 (0.6); 1.1646 (1.6); 1.1611 (6.0); 1.1479 (6.7); 1.1448 (6.6); 1.1314 (5.0); 0.9427 (0.6); 0.9240 (1.3); 0.9053 (0.6); 0.8883 (2.4); 0.8741 (3.0); 0.8699 (5.4); 0.8558 (5.9); 0.8515 (2.6); 0.8373 (2.3); 0.0080 (0.5); -0.0002 (18.4); -0.0085 (0.6) |
| I-043: ¹H-NMR(400.6 MHz, CDCl3): |
| δ= 7.4544 (0.6); 7.4333 (0.7); 7.4136 (0.6); 7.2617 (43.7); 6.9297 (0.7); 6.9161 (0.7); 6.8910 (0.9); 6.8776 (1.5); 6.8648 (1.1); 6.8377 (1.8); 6.8259 (2.0); 6.8111 (2.7); 6.7985 (3.3); 6.7798 (2.2); 5.9592 (1.2); 5.9205 (1.2); 5.8817 (1.2); 5.8419 (1.2); 5.3005 (1.4); 5.1026 (0.8); 4.6614 (0.6); 4.6448 (0.6); 4.5544 (0.5); 4.4285 (0.5); 4.4106 (0.5); 4.1374 (1.0); 4.1195 (1.1); 4.1017 (1.0); 4.0640 (1.1); 4.0508 (1.0); 3.9954 (0.8); 3.8783 (0.8); 3.8400 (0.6); 3.7384 (16.0); 3.7346 (15.2); 2.0543 (4.0); 1.4191 (0.9); 1.4012 (1.9); 1.3734 (9.0); 1.3557 (4.8); 1.3392 (8.2); 1.3224 (5.0); 1.2807 (1.8); 1.2628 (3.8); 1.2565 (2.1); 1.2450 (1.6); 0.8816 (0.6); 0.0079 (1.9); -0.0002 (55.6); -0.0085 (2.2) |
| I-044: ¹H-NMR(400.6 MHz, CDCl3): |
| δ= 7.6889 (1.0); 7.5193 (0.9); 7.2609 (150.4); 7.2509 (3.5); 7.2466 (3.2); 7.0370 (6.3); 7.0327 (5.2); 6.9972 (0.9); 5.3003 (3.3); 4.5769 (1.5); 4.5638 (1.1); 4.5552 (1.7); 4.4292 (1.0); 4.0689 (0.7); 4.0451 (2.3); 4.0337 (2.3); 4.0208 (1.0); 4.0082 (2.6); 3.9983 (2.6); 3.9785 (1.4); 3.9594 (0.8); 3.9321 (1.8); 3.9194 (2.2); 3.8956 (0.9); 3.8827 (1.3); 3.8690 (1.0); 3.8260 (12.6); 3.7920 (16.0); 3.7252 (13.9); 2.5607 (0.8); 2.5453 (0.9); 2.5270 (0.9); 2.5111 (0.6); 2.0709 (1.2); 2.0192 (1.4); 1.9857 (1.2); 1.7458 (0.6); 1.7278 (1.0); 1.7117 (1.3); 1.6945 (1.3); 1.6779 (0.8); 1.5869 (0.8); 1.5681 (1.3); 1.5506 (1.2); 1.5334 (1.0); 1.5166 (0.7); 1.2547 (3.0); 0.8801 (1.0); 0.8705 (2.1); 0.8520 (4.2); 0.8405 (3.1); 0.8221 (4.9); 0.8032 (2.2); 0.1458 (0.6); 0.0080 (4.6); -0.0002 (186.8); -0.0085 (5.7); -0.0339 (0.5); -0.1495 (0.6) |
| I-045: ¹H-NMR(400.6 MHz, CDCl3): |
| δ= 7.2626 (16.4); 7.2581 (1.5); 7.2536 (3.5); 7.2490 (3.6); 7.2445 (1.4); 7.1413 (0.5); 7.1228 (0.9); 7.1041 (0.6); 7.0744 (3.8); 7.0699 (3.5); 7.0383 (4.4); 7.0339 (4.1); 4.0381 (0.9); 4.0015 (2.6); 3.9648 (2.4); 3.9598 (2.5); 3.9472 (2.1); 3.9230 (1.0); 3.9105 (0.8); 3.8914 (0.6); 3.8767 (0.7); 3.8614 (0.6); 3.7219 (11.2); 3.7173 (12.0); 3.6994 (11.8); 3.6667 (16.0); 2.3875 (0.6); 2.3693 (1.0); 2.3528 (1.8); 2.3485 (1.5); 2.3323 (2.1); 2.3135 (1.2); 1.8711 (0.5); 1.8522 (0.8); 1.8365 (1.0); 1.8216 (1.2); 1.8173 (1.1); 1.8122 (0.7); 1.8039 (1.1); 1.7915 (1.0); 1.7840 (0.7); 1.7742 (0.9); 1.7709 (0.8); 1.7534 (0.6); 1.7318 (0.7); 1.7170 (0.8); 1.6979 (0.9); 1.6770 (0.6); 1.6068 (0.6); 1.5970 (0.6); 1.5885 (0.7); 1.5785 (0.8); 1.5734 (0.6); 1.5627 (0.6); 1.1734 (5.6); 1.1631 (5.2); 1.1569 (5.9); 1.1468 (4.7); 0.8906 (2.2); 0.8724 (5.1); 0.8573 (4.6); 0.8390 (1.7); 0.0080 (0.6); -0.0002 (19.6); -0.0085 (0.6) |
| I-046: ¹H-NMR(400.6 MHz, CDCl3): |
| δ= 7.6160 (0.5); 7.5968 (0.6); 7.5410 (0.8); 7.5200 (1.0); 7.2615 (52.1); 7.2484 (4.1); 7.0688 (3.8); 7.0646 (3.7); 7.0438 (5.1); 7.0399 (4.9); 5.3004 (1.7); 4.2232 (0.7); 4.2042 (1.4); 4.1962 (1.3); 4.1861 (2.4); 4.1788 (2.1); 4.1684 (3.2); 4.1611 (1.9); 4.1506 (4.6); 4.1328 (4.4); 4.1147 (1.7); 4.0450 (1.1); 4.0250 (0.7); 4.0084 (2.4); 3.9881 (1.9); 3.9541 (4.2); 3.9173 (1.7); 3.7158 (16.0); 2.5513 (0.6); 2.5381 (0.6); 2.5146 (3.5); 2.5004 (3.8); 2.4890 (2.0); 2.4741 (1.9); 2.4502 (0.6); 2.4353 (0.5); 2.0487 (2.1); 1.7428 (0.7); 1.7250 (1.0); 1.7091 (1.2); 1.6905 (1.1); 1.5923 (0.6); 1.5783 (1.0); 1.5596 (1.1); 1.5439 (0.8); 1.5264 (0.6); 1.3065 (3.3); 1.2887 (6.9); 1.2793 (5.9); 1.2708 (4.0); 1.2613 (14.8); 1.2431 (10.4); 1.2271 (5.1); 1.2103 (4.9); 0.8792 (2.0); 0.8613 (5.7); 0.8443 (6.3); 0.8262 (2.4); 0.0080 (1.6); -0.0002 (63.1); -0.0085 (2.0) |
| I-047: ¹H-NMR(400.2 MHz, d₆-DMSO): |
| δ= 7.7194 (2.8); 7.7092 (3.0); 7.7000 (3.2); 7.6900 (2.7); 7.3764 (0.4); 7.3538 (0.4); 7.1484 (1.2); 7.1331 (7.7); 7.1295 (6.8); 7.1166 (6.9); 7.1119 (9.0); 7.0989 (4.0); 7.0944 (4.6); 7.0896 (3.5); 7.0766 (1.3); 7.0714 (2.0); 7.0661 (1.8); 5.8912 (16.0); 4.8536 (0.7); 4.7373 (0.7); 4.5827 (1.6); 4.5761 (1.6); 4.5589 (2.2); 4.5524 (2.1); 4.4658 (1.7); 4.4592 (1.7); 4.4419 (2.6); 4.4347 (4.5); 4.4098 (2.2); 4.3130 (2.9); 4.2897 (2.2); 4.1498 (0.4); 4.1039 (0.7); 4.0909 (0.8); 4.0698 (0.3); 4.0250 (3.0); 4.0144 (2.8); 3.9879 (4.2); 3.9774 (4.0); 3.9186 (0.5); 3.9006 (2.0); 3.8824 (3.6); 3.8643 (3.7); 3.8477 (4.3); 3.8373 (3.0); 3.8123 (2.0); 3.7997 (2.0); 3.7858 (0.4); 3.7765 (0.4); 3.6306 (0.6); 3.6046 (36.6); 3.5954 (52.9); 3.5899 |
| (32.2); 3.5722 (0.9); 3.5620 (0.4); 3.4183 (0.3); 3.4087 (0.5); 3.4032 (0.4); 3.3732 (0.6); 3.3608 (1.4); 3.3342 (169.4); 3.3052 (0.4); 3.3019 (0.5); 3.1766 (2.1); 3.1635 (2.1); 2.8301 (0.8); 2.8096 (2.9); 2.7895 (4.4); 2.7690 (3.2); 2.7482 (1.0); 2.5073 (22.2); 2.5029 (29.9); 2.4984 (21.9); 2.0735 (1.7); 2.0631 (0.9); 2.0437 (1.3); 2.0306 (1.2); 2.0244 (1.1); 2.0109 (1.6); 1.9913 (1.2); 1.9857 (1.5); 1.9720 (1.3); 1.9658 (1.1); 1.9530 (1.6); 1.9334 (1.0); 1.8586 (0.6); 1.8384 (1.2); 1.8264 (1.9); 1.8211 (1.4); 1.8066 (4.1); 1.7886 (5.6); 1.7745 (4.7); 1.7690 (4.4); 1.7556 (2.8); 1.7437 (2.2); 1.7262 (1.5); 1.7112 (1.3); 1.6942 (1.1); 1.6804 (1.1); 1.6639 (1.4); 1.6458 (1.2); 1.6228 (1.4); 1.6087 (1.6); 1.6031 (2.1); 1.5895 (2.8); 1.5762 (1.5); 1.5699 (2.5); 1.5566 (1.8); 1.5366 (1.0); 1.5005 (0.5); 1.4823 (1.5); 1.4668 (2.2); 1.4500 (2.0); 1.4383 (1.7); 1.4187 (1.1); 1.4017 (0.4); -0.0002 (4.8) |
| I-048: ¹H-NMR(400.6 MHz, CDCl3): |
| δ= 7.2625 (24.3); 6.8800 (0.6); 6.8554 (0.8); 6.8500 (1.2); 6.8455 (0.9); 6.8367 (1.3); 6.8311 (1.6); 6.8256 (2.9); 6.8212 (1.2); 6.8147 (1.0); 6.8090 (2.2); 6.8044 (2.0); 6.7969 (1.0); 6.7888 (0.9); 6.7832 (0.8); 5.9695 (0.6); 5.9655 (0.6); 5.9632 (0.6); 5.9591 (0.6); 5.9558 (0.7); 5.9518 (0.7); 5.9358 (0.7); 5.9317 (0.5); 5.9282 (0.6); 5.9221 (0.8); 5.9179 (0.6); 5.8369 (0.7); 5.8313 (1.2); 5.8261 (1.1); 5.8218 (1.4); 5.8174 (1.3); 5.8125 (0.9); 5.8078 (0.9); 5.3007 (4.9); 4.7009 (1.1); 4.6919 (1.1); 4.0676 (0.8); 4.0631 (0.8); 4.0555 (0.8); 4.0476 (0.8); 4.0192 (1.3); 4.0112 (1.5); 3.9973 (1.4); 3.9608 (0.7); 3.9341 (1.3); 3.9138 (1.2); 3.8978 (0.8); 3.8776 (0.8); 3.7427 (16.0); 3.7369 (12.0); 3.5469 (0.6); 3.5415 (0.7); 3.5356 (0.7); 3.5298 (0.7); 3.5258 (0.6); 3.5200 (0.5); 2.5763 (0.6); 2.5633 (0.7); 2.5422 (1.2); 2.5212 (0.7); 2.5076 (0.7); 1.9307 (0.8); 1.9191 (0.9); 1.9085 (1.0); 1.8967 (1.1); 1.8847 (0.9); 1.8738 (0.7); 1.8625 (0.5); 1.3785 (3.1); 1.3662 (3.4); 1.3447 (7.2); 1.3394 (6.9); 1.2546 (0.5); 0.0080 (0.8); -0.0002 (29.3); -0.0085 (1.0) |
| I-049: ¹H-NMR(400.6 MHz, CDCl3): |
| δ= 7.5161 (0.6); 7.4974 (0.6); 7.2637 (9.3); 7.2481 (2.5); 7.2436 (4.6); 7.2390 (2.6); 7.0483 (7.5); 7.0438 (7.1); 5.3008 (1.0); 4.1580 (0.5); 4.1434 (0.8); 4.1268 (0.7); 4.0513 (1.4); 4.0417 (1.2); 4.0147 (2.6); 4.0051 (2.3); 3.9338 (4.1); 3.8972 (2.3); 3.7249 (16.0); 3.7181 (14.5); 3.7120 (12.7); 3.7059 (14.3); 2.8926 (0.8); 2.8865 (0.7); 2.8766 (0.7); 2.8698 (0.9); 2.8534 (0.5); 2.2528 (0.7); 2.2425 (0.6); 2.2362 (0.9); 2.2302 (0.5); 2.2246 (0.6); 2.2194 (0.8); 2.2027 (0.5); 2.0009 (0.8); 1.9914 (1.4); 1.9839 (2.0); 1.9762 (1.5); 1.9666 (1.4); 1.9545 (1.0); 1.9427 (1.5); 1.9265 (1.7); 1.9158 (0.9); 1.9035 (1.0); 1.8875 (0.7); 1.8160 (0.8); 1.7992 (0.7); 1.7823 (0.7); 1.7668 (0.6); 1.7490 (0.8); 1.7310 (1.1); 1.7138 (1.3); 1.7031 (0.9); 1.6969 (1.2); 1.6865 (0.8); 1.6784 (0.5); 1.6704 (0.6); 1.6229 (1.0); 1.6089 (1.0); 1.5941 (0.6); 1.5872 (0.5); 1.5753 (1.0); 1.5693 (1.0); 1.5568 (1.0); 1.5510 (1.1); 1.5412 (0.7); 1.5355 (0.9); 1.5231 (0.7); 1.5172 (0.8); 0.8728 (2.3); 0.8673 (3.0); 0.8545 (5.1); 0.8489 (6.4); 0.8360 (2.6); 0.8304 (3.0); -0.0002 (10.8); -0.0082 (0.8) |
| I-050: ¹H-NMR(400.6 MHz, CDCl3): |
| δ= 7.2633 (4.8); 6.8790 (0.7); 6.8771 (0.5); 6.8662 (0.7); 6.8398 (0.7); 6.8269 (0.7); 6.7223 (0.6); 6.7141 (1.0); 6.7084 (1.0); 6.6994 (0.6); 6.6942 (1.1); 6.6900 (0.5); 6.6825 (0.6); 6.6630 (0.6); 5.9133 (0.5); 5.9105 (0.8); 5.9062 (0.7); 5.8712 (0.7); 5.8669 (0.7); 5.3005 (2.2); 4.0464 (0.5); 4.0097 (1.2); 3.9935 (0.7); 3.9658 (1.1); 3.9179 (0.7); 3.7549 (5.4); 3.7366 (5.4); 3.7257 (16.0); 1.5963 (0.6); 1.3049 (4.5); 1.2875 (4.4); 0.9039 (1.3); 0.8966 (0.9); 0.8854 (2.9); 0.8782 (2.0); 0.8669 (1.2); 0.8597 (0.8); -0.0002 (6.1) |
| I-051: ¹H-NMR(400.6 MHz, CDCl3): |
| δ= 7.4685 (0.6); 7.4570 (0.6); 7.2622 (17.9); 7.2565 (2.2); 7.2521 (4.0); 7.2476 (2.1); 7.0682 (4.3); 7.0636 (4.1); 7.0502 (4.7); 7.0457 (4.5); 5.9857 (0.5); 5.9818 (0.6); 5.9796 (0.6); 5.9756 (0.6); 5.9719 (0.7); 5.9680 (0.7); 5.9659 (0.7); 5.9620 (0.6); 5.9214 (0.5); 5.9195 (0.5); 5.9153 (0.5); 5.9117 (0.6); 5.9076 (0.7); 5.9057 (0.6); 5.9017 (0.5); 5.8691 (0.6); 5.8636 (1.1); 5.8579 (0.7); 5.8498 (0.9); 5.8121 (0.6); 5.8065 (1.0); 5.8008 (0.6); 5.7986 (0.5); 5.7927 (0.8); 5.3005 (3.5); 4.9048 (0.6); 4.8860 (0.6); 4.0606 (1.4); 4.0241 (2.6); 3.9497 (1.8); 3.9439 (1.6); 3.9132 (1.0); 3.9072 (1.0); 3.7498 (13.5); 3.7356 (16.0); 3.7234 (10.7); 3.7092 (12.0); 3.5430 (0.6); 3.5369 (0.6); 3.5337 (0.7); 2.5608 (0.6); 2.5397 (0.7); 2.5263 (0.7); 2.5186 (0.7); 2.5051 (0.8); 2.4839 (0.7); 1.9366 (0.6); 1.9022 (0.6); 1.8727 (0.7); 1.8380 (0.7); 1.7526 (0.7); 1.7361 (0.9); 1.7187 (1.0); 1.7009 (0.6); 1.5849 (1.0); 1.5666 (1.2); 1.5510 (0.9); 1.5327 (0.8); 0.8966 (2.1); 0.8782 (4.8); 0.8733 (2.4); 0.8596 (2.4); 0.8548 (4.3); 0.8363 (1.7); 0.0080 (0.8); -0.0002 (22.7); -0.0085 (0.6) |
| I-052: ¹H-NMR(300.1 MHz, d₆-DMSO): |
| δ= 13.2253 (0.5); 7.6132 (3.9); 7.5999 (4.7); 7.5887 (4.6); 7.5748 (4.4); 7.2515 (1.0); 7.1127 (3.2); 7.1051 (2.8); 7.0772 (10.3); 7.0678 (13.7); 7.0616 (11.5); 7.0384 (16.0); 6.9107 (1.1); 6.0094 (6.4); |
| 5.9738 (7.4); 5.9518 (8.4); 5.9161 (8.3); 5.6050 (0.6); 5.5751 (0.6); 5.5440 (0.7); 5.4335 (0.7); 5.4128 (0.8); 5.3967 (0.6); 5.3901 (0.6); 5.3351 (9.0); 5.2780 (7.6); 5.1061 (8.8); 5.0704 (8.2); 4.0497 (3.6); 4.0418 (2.8); 4.0009 (9.4); 3.9932 (9.3); 3.9646 (9.0); 3.9555 (8.8); 3.9162 (2.7); 3.9066 (3.6); 3.8751 (3.2); 3.8505 (6.1); 3.8263 (5.8); 3.8033 (3.1); 3.7797 (1.3); 3.7605 (0.7); 3.6909 (1.3); 3.6065 (61.9); 3.5918 (65.4); 3.5794 (72.4); 3.5128 (67.6); 3.3308 (1.4); 3.2362 (0.6); 2.8822 (0.5); 2.8621 (0.5); 2.8370 (0.8); 2.8138 (3.3); 2.7883 (5.1); 2.7594 (3.8); 2.7333 (2.6); 2.5405 (1.2); 2.5132 (118.8); 2.5073 (231.8); 2.5014 (305.6); 2.4956 (208.3); 2.4899 (96.0); 2.2717 (2.0); 2.0480 (1.6); 2.0252 (3.3); 2.0044 (3.4); 1.9835 (3.9); 1.9586 (2.0); 1.9391 (0.6); 1.8442 (1.6); 1.8168 (4.6); 1.8060 (4.5); 1.7892 (7.2); 1.7642 (7.6); 1.7563 (5.0); 1.7507 (4.8); 1.7238 (3.3); 1.6978 (3.4); 1.6744 (2.6); 1.6510 (2.6); 1.6239 (3.0); 1.5975 (2.9); 1.5802 (3.2); 1.5738 (3.3); 1.5551 (2.6); 1.5305 (2.4); 1.4942 (2.5); 1.4711 (2.7); 1.4456 (2.9); 1.4214 (2.8); 1.4062 (1.8); 1.3860 (1.2); 1.3527 (0.6); 1.2352 (0.8); 1.0786 (1.0); 1.0552 (1.5); 1.0319 (0.8); 0.0108 (2.8); -0.0002 (81.9); -0.0112 (2.8) |
| I-053: ¹H-NMR(300.0 MHz, d₆-DMSO): |
| δ= 7.4467 (1.1); 7.4273 (16.0); 7.4230 (5.8); 7.4069 (8.9); 7.4009 (5.8); 7.3874 (1.7); 7.3579 (1.4); 5.3728 (5.3); 5.3308 (3.6); 4.0255 (1.0); 3.9917 (1.4); 3.9766 (1.4); 3.9431 (2.0); 3.8104 (1.9); 3.7628 (1.8); 3.7177 (0.9); 3.6299 (0.8); 3.5811 (19.1); 3.5740 (15.4); 3.5581 (18.8); 3.5194 (0.5); 3.3260 (56.0); 3.1779 (0.5); 3.1604 (0.5); 2.5136 (7.8); 2.5079 (14.3); 2.5020 (18.6); 2.4963 (13.4); 2.2483 (1.2); 2.2352 (1.9); 2.2246 (2.1); 2.2197 (2.1); 2.2107 (3.1); 2.1972 (1.8); 2.1844 (2.0); 1.6519 (1.3); 1.6273 (2.6); 1.6030 (2.5); 1.5807 (1.0); 1.2355 (0.9); 1.1961 (11.1); 1.1872 (8.2); 0.9945 (4.0); 0.9706 (8.2); 0.9477 (5.7); 0.0107 (0.4); -0.0001 (7.2); -0.0111 (0.4) |
| I-054: ¹H-NMR(300.1 MHz, d₆-DMSO): |
| δ= 7.8989 (1.3); 7.8711 (1.4); 7.8482 (1.8); 7.8210 (1.8); 7.1188 (0.8); 7.1114 (0.9); 7.0919 (3.8); 7.0855 (4.6); 7.0581 (7.8); 7.0307 (4.2); 7.0085 (0.4); 6.8653 (1.4); 6.8469 (1.4); 6.8171 (2.1); 6.8130 (1.9); 6.7997 (2.1); 6.7944 (1.8); 6.7647 (2.0); 6.7473 (2.0); 6.0305 (1.4); 6.0134 (1.0); 5.9949 (1.6); 5.9775 (1.5); 5.9729 (2.0); 5.9560 (1.4); 5.9372 (1.8); 5.9206 (1.3); 5.8834 (1.8); 5.8782 (1.8); 5.8309 (1.5); 5.8256 (1.6); 5.7732 (2.3); 5.7679 (2.3); 5.7207 (2.1); 5.7154 (2.1); 5.6609 (7.4); 5.6113 (5.3); 5.5772 (0.3); 5.3664 (3.2); 5.3089 (2.6); 5.1426 (2.0); 5.1375 (2.0); 5.1296 (1.8); 5.1241 (1.6); 5.1068 (1.9); 5.1018 (2.0); 5.0941 (1.5); 5.0885 (1.4); 4.3996 (0.4); 4.3811 (0.6); 4.3543 (0.7); 4.3295 (0.7); 4.3061 (0.8); 4.2802 (0.8); 4.2621 (0.5); 4.1150 (1.4); 4.0668 (2.8); 4.0214 (3.2); 3.9684 (3.1); 3.9455 (2.2); 3.9196 (1.4); 3.8966 (1.4); 3.6615 (16.0); 3.6478 (1.2); 3.6295 (20.3); 3.5822 (20.0); 3.5762 (17.2); 3.3204 (79.3); 2.5131 (18.0); 2.5073 (35.9); 2.5014 (48.0); 2.4956 (34.1); 1.1646 (7.1); 1.1410 (7.6); 1.1323 (6.3); 1.1088 (5.5); 1.0777 (0.4); 0.0106 (0.7); -0.0002 (22.9); -0.0112 (0.9) |
| I-055: ¹H-NMR(300.0 MHz, d₆-DMSO): |
| δ= 7.5888 (1.0); 7.5618 (1.9); 7.5352 (1.1); 7.4281 (3.3); 7.4231 (3.8); 7.4190 (3.0); 7.3989 (9.3); 7.3933 (6.6); 5.3600 (7.2); 3.9770 (1.1); 3.9685 (1.1); 3.9283 (1.9); 3.9199 (2.0); 3.8462 (1.0); 3.8289 (2.2); 3.8201 (2.7); 3.7986 (1.6); 3.7809 (1.4); 3.7701 (1.4); 3.7516 (0.3); 3.6061 (14.3); 3.5959 (14.9); 3.5815 (13.9); 3.5780 (14.0); 3.3280 (105.2); 2.8177 (1.1); 2.7912 (1.8); 2.7640 (1.4); 2.7363 (0.5); 2.5077 (17.3); 2.5020 (22.5); 2.4964 (16.6); 2.0689 (0.4); 2.0461 (0.7); 2.0247 (1.0); 2.0029 (1.0); 1.9979 (1.0); 1.9764 (0.9); 1.9534 (0.5); 1.8470 (0.5); 1.8361 (0.8); 1.8201 (1.4); 1.8097 (1.7); 1.7874 (2.0); 1.7766 (1.8); 1.7631 (1.9); 1.7563 (1.7); 1.7400 (1.0); 1.7151 (1.0); 1.6919 (0.9); 1.6689 (0.6); 1.6208 (0.8); 1.5953 (1.5); 1.5774 (0.9); 1.5695 (1.0); 1.5521 (1.2); 1.5260 (0.8); 1.5122 (0.4); 1.4888 (0.8); 1.4727 (0.9); 1.4661 (0.9); 1.4507 (1.1); 1.4261 (0.7); 1.3088 (0.4); 1.2855 (0.4); 1.2360 (0.7); 1.1789 (16.0); - 0.0002 (3.9) |
| I-056: ¹H-NMR(300.1 MHz, d₆-DMSO): |
| δ= 7.5871 (1.0); 7.5614 (1.7); 7.5372 (1.0); 7.1189 (0.4); 7.0976 (5.5); 7.0700 (4.4); 7.0459 (1.0); 5.3561 (2.2); 3.9821 (1.1); 3.9717 (1.0); 3.9336 (1.8); 3.9232 (1.9); 3.8633 (0.8); 3.8379 (3.0); 3.8228 (2.2); 3.7895 (1.5); 3.7743 (1.1); 3.6045 (15.9); 3.5948 (16.9); 3.5840 (15.0); 3.5795 (14.2); 3.3368 (113.8); 3.2853 (0.4); 2.8165 (1.0); 2.7899 (1.6); 2.7629 (1.2); 2.7345 (0.6); 2.5141 (18.8); 2.5082 (37.1); 2.5023 (49.4); 2.4963 (33.7); 2.4906 (15.2); 2.0627 (0.3); 2.0373 (0.4); 2.0195 (0.7); 2.0100 (0.4); 1.9943 (1.0); 1.9774 (0.5); 1.9685 (0.7); 1.9529 (0.6); 1.9268 (0.4); 1.8329 (0.5); 1.8126 (1.2); 1.7989 (1.3); 1.7895 (1.4); 1.7765 (2.1); 1.7609 (1.4); 1.7384 (0.7); 1.7210 (0.7); 1.6954 (0.6); 1.6821 (0.6); 1.6603 (0.6); 1.6336 (0.8); 1.6075 (0.9); 1.5887 (1.1); 1.5719 (0.5); 1.5627 (1.0); 1.5456 (0.6); 1.5184 (0.4); 1.4947 (0.3); 1.4754 (0.8); 1.4541 (0.9); 1.4323 (0.7); 1.4080 (0.4); 1.1767 (16.0); 0.0107 (0.3); -0.0002 (9.2) |
| I-057: ¹H-NMR(400.6 MHz, CDCl3): |
| δ= 7.2607 (26.6); 7.2268 (0.8); 6.9461 (1.2); 6.9332 (1.3); 6.9068 (1.4); 6.8939 (1.3); 6.7913 (1.1); 6.7685 (1.1); 6.7180 (2.4); 6.6579 (1.1); 6.6338 (1.1); 5.9227 (1.7); 5.8833 (1.5); 4.6554 (0.7); 4.6411 (0.7); 3.9601 (6.5); 3.7485 (16.0); 3.7241 (15.5); 3.4423 (0.6); 2.3198 (11.8); 1.6172 (0.6); 1.3577 (12.8); 1.3332 (0.6); 1.3165 (6.7); 1.2990 (6.6); 1.2847 (0.6); 1.2560 (1.5); 1.1538 (0.8); 1.1356 (0.8); 1.1290 (0.8); 1.1238 (0.8); 1.1131 (0.7); 0.9323 (0.5); 0.9141 (0.9); 0.8962 (0.5); -0.0002 (38.9) |
| I-058: ¹H-NMR(400.6 MHz, CDCl3): |
| δ= 7.2621 (6.8); 7.2188 (0.8); 7.1983 (0.8); 6.8973 (1.3); 6.8848 (1.3); 6.8581 (1.4); 6.8455 (1.4); 6.8048 (1.1); 6.7817 (1.1); 6.7441 (2.4); 6.6862 (1.1); 6.6627 (1.2); 5.8900 (1.7); 5.8859 (1.5); 5.8507 (1.5); 5.8466 (1.4); 5.3001 (3.5); 4.5859 (0.6); 4.5691 (0.7); 3.9918 (7.5); 3.7278 (16.0); 3.7129 (15.6); 2.3374 (12.1); 2.0455 (0.6); 1.3662 (13.0); 1.3240 (6.6); 1.3065 (6.6); 1.2594 (0.8); -0.0002 (9.7) |
| I-059: ¹H-NMR(400.6 MHz, CDCl3): |
| δ= 7.4196 (0.6); 7.3689 (0.8); 7.3464 (3.4); 7.3223 (2.3); 7.2656 (7.1); 7.2635 (6.7); 7.1578 (7.9); 7.1304 (0.9); 6.9595 (0.6); 6.9470 (0.6); 6.9206 (0.7); 6.9077 (0.7); 6.8960 (0.9); 6.8839 (1.0); 6.8568 (1.0); 6.8447 (1.1); 5.9791 (0.9); 5.9395 (0.9); 5.8879 (1.6); 5.8486 (1.4); 5.3030 (7.5); 5.3010 (7.3); 4.7355 (1.4); 4.7036 (1.8); 4.6670 (0.6); 4.5751 (0.6); 4.5592 (0.7); 4.0637 (1.0); 4.0459 (1.0); 4.0272 (1.1); 4.0097 (1.6); 3.9297 (1.6); 3.8939 (1.0); 3.7895 (1.1); 3.7590 (0.8); 3.7533 (1.1); 3.7281 (13.3); 3.7234 (9.6); 1.3891 (1.8); 1.3550 (16.0); 1.3457 (6.7); 1.3293 (4.0); 1.3119 (3.5); 1.2565 (0.8); -0.0002 (10.7) |
| I-060: ¹H-NMR(400.6 MHz, CDCl3): |
| δ= 7.5765 (1.0); 7.5554 (1.7); 7.5315 (0.9); 7.3358 (5.2); 7.2649 (9.5); 7.2634 (9.3); 7.1832 (5.6); 7.1806 (5.4); 7.1599 (6.1); 7.1572 (5.9); 5.9777 (1.2); 5.9640 (1.5); 5.9338 (1.1); 5.9202 (1.4); 5.8800 (1.6); 5.8736 (1.3); 5.8666 (1.2); 5.8228 (1.4); 5.8163 (1.2); 5.8096 (1.2); 5.3024 (9.7); 5.3010 (9.5); 4.9248 (1.1); 4.9076 (1.1); 4.6693 (3.7); 4.0570 (1.4); 4.0464 (1.5); 4.0207 (2.2); 4.0102 (2.6); 3.9177 (2.6); 3.8956 (2.2); 3.8816 (1.6); 3.8594 (1.4); 3.7428 (14.8); 3.7372 (16.0); 3.5381 (1.3); 2.6103 (1.0); 2.5977 (0.6); 2.5899 (0.6); 2.5761 (1.1); 2.5688 (0.7); 2.5547 (0.6); 2.5474 (1.1); 2.5336 (0.7); 2.5261 (0.6); 2.5124 (1.2); 2.4911 (0.6); 1.9611 (0.6); 1.9497 (1.1); 1.9376 (0.6); 1.9276 (0.6); 1.9146 (1.4); 1.9023 (1.6); 1.8908 (0.7); 1.8781 (0.6); 1.8669 (1.1); 1.8563 (0.6); 1.3404 (14.6); 1.3360 (13.8); 1.2562 (0.5); 0.0013 (13.7); -0.0002 (14.0) |
| I-061: ¹H-NMR(400.1 MHz, d₆-DMSO): |
| δ= 7.4383 (1.4); 7.4169 (1.4); 7.3763 (1.8); 7.3544 (1.8); 7.1327 (0.5); 7.1226 (2.1); 7.1171 (2.9); 7.1003 (4.2); 7.0956 (6.3); 7.0843 (2.0); 7.0786 (3.2); 7.0731 (3.9); 7.0674 (3.3); 7.0614 (2.5); 7.0556 (0.8); 7.0489 (0.9); 7.0435 (1.3); 7.0379 (1.0); 5.3654 (6.2); 5.3226 (4.6); 4.0169 (1.9); 3.9807 (3.2); 3.9471 (3.1); 3.8174 (2.8); 3.7838 (2.9); 3.7479 (1.7); 3.6474 (0.4); 3.6292 (0.7); 3.6113 (1.2); 3.5876 (21.1); 3.5832 (24.2); 3.5736 (22.8); 3.5569 (26.9); 3.5138 (0.3); 3.3707 (0.4); 3.3309 (109.7); 2.5111 (13.0); 2.5068 (26.6); 2.5023 (35.8); 2.4978 (25.2); 2.4934 (11.6); 2.2424 (1.2); 2.2361 (1.4); 2.2297 (2.8); 2.2242 (2.4); 2.2113 (4.1); 2.1975 (1.8); 2.1914 (2.8); 1.6440 (1.7); 1.6251 (4.0); 1.6175 (1.9); 1.6071 (4.0); 1.5875 (1.4); 1.2354 (0.3); 1.1926 (16.0); 1.1817 (13.0); 0.9969 (7.0); 0.9803 (7.0); 0.9556 (8.6); 0.9390 (8.4); 0.0080 (0.4); -0.0001 (13.0); -0.0084 (0.4) |
| I-062: ¹H-NMR(300.0 MHz, d₆-DMSO): |
| δ= 7.5657 (0.9); 7.5418 (1.6); 7.5184 (0.9); 6.9507 (1.4); 6.9305 (3.8); 6.8803 (1.4); 6.8476 (1.4); 5.7543 (0.4); 5.3127 (6.7); 3.9665 (1.1); 3.9565 (1.1); 3.9183 (1.7); 3.9084 (1.7); 3.8705 (0.7); 3.8466 (1.5); 3.8226 (1.3); 3.7989 (0.7); 3.7750 (1.7); 3.7653 (1.6); 3.7266 (1.0); 3.7171 (1.1); 3.6042 (15.0); 3.5955 (15.3); 3.5816 (0.9); 3.5514 (12.4); 3.5465 (12.0); 3.4284 (0.4); 3.4139 (0.4); 3.4085 (0.4); 3.3806 (0.9); 3.3573 (1.6); 3.3323 (329.6); 3.2899 (0.3); 3.2743 (0.4); 3.2645 (0.6); 2.8161 (1.0); 2.7890 (1.6); 2.7620 (1.2); 2.7341 (0.6); 2.5069 (35.6); 2.5018 (46.5); 2.3211 (0.4); 2.2749 (16.0); 2.0730 (0.4); 2.0669 (0.4); 2.0422 (0.4); 2.0246 (0.6); 2.0150 (0.4); 1.9996 (0.9); 1.9847 (0.5); 1.9739 (0.6); 1.9591 (0.6); 1.9332 (0.4); 1.8421 (0.4); 1.8348 (0.5); 1.8283 (0.6); 1.8149 (1.1); 1.8073 (1.4); 1.8013 (1.3); 1.7922 (1.4); 1.7803 (2.0); 1.7760 (1.8); 1.7564 (1.5); 1.7386 (0.9); 1.7154 (0.8); 1.6953 (0.7); 1.6739 (0.5); 1.6503 (0.4); 1.6256 (0.6); 1.6095 (0.5); 1.5985 (0.9); 1.5825 (1.1); 1.5741 (0.6); 1.5666 (0.5); 1.5564 (1.0); 1.5401 (0.6); 1.5307 (0.4); 1.5122 (0.4); 1.4793 (0.8); 1.4557 (1.0); 1.4421 (0.6); 1.4172 (0.5); 1.1698 (15.9); -0.0001 (5.1) |
| I-063: ¹H-NMR(300.0 MHz, d₆-DMSO): |
| δ= 7.5081 (1.5); 7.4985 (1.5); 7.4817 (1.6); 7.4704 (1.4); 6.9691 (1.1); 6.9423 (3.7); 6.9341 (3.9); 6.9153 (1.3); 6.8851 (2.1); 6.8526 (2.1); 5.8849 (1.0); 5.8779 (1.6); 5.8717 (1.6); 5.8663 (2.3); 5.8595 (2.4); 5.8534 (1.8); 5.8475 (1.6); 5.8407 (1.0); 5.7154 (0.9); 5.7080 (1.6); 5.7002 (1.1); 5.6896 (1.4); 5.6819 (0.8); 5.6553 (0.9); 5.6478 (1.6); 5.6399 (1.1); 5.6294 (1.4); 5.6219 (0.8); 5.3542 (6.0); 5.3458 (5.7); |
| 4.5960 (0.6); 4.5712 (0.9); 4.5632 (0.8); 4.5547 (0.9); 4.5350 (0.7); 4.5285 (0.6); 3.9761 (1.5); 3.9585 (1.4); 3.9281 (2.4); 3.9103 (2.3); 3.8057 (2.1); 3.7848 (2.1); 3.7573 (1.3); 3.7366 (1.4); 3.6336 (19.3); 3.6232 (20.8); 3.5539 (22.3); 3.5302 (1.4); 3.5244 (1.7); 3.5176 (1.7); 3.5106 (1.3); 3.5031 (1.1); 3.4966 (1.0); 3.3190 (104.0); 3.1767 (0.3); 2.7270 (0.4); 2.5060 (50.9); 2.5009 (66.1); 2.4605 (0.3); 2.4261 (0.7); 2.3983 (1.1); 2.3819 (1.2); 2.3700 (0.8); 2.3541 (2.0); 2.3382 (0.8); 2.3242 (1.8); 2.3103 (1.2); 2.2810 (12.6); 2.2732 (13.1); 1.7266 (0.6); 1.7173 (0.8); 1.7084 (1.2); 1.6984 (1.3); 1.6899 (0.8); 1.6807 (1.1); 1.6730 (0.8); 1.6638 (1.1); 1.6539 (1.1); 1.6451 (0.7); 1.6350 (0.6); 1.6077 (0.9); 1.1837 (16.0); 0.0108 (1.4); -0.0001 (38.0); -0.0111 (1.3) |
| I-064: ¹H-NMR(400.6 MHz, CDCl3): |
| δ= 7.2620 (10.5); 6.8557 (1.1); 6.8504 (1.7); 6.8371 (1.4); 6.8314 (1.8); 6.8255 (0.6); 6.8094 (0.8); 6.8037 (1.0); 6.7821 (0.5); 5.9407 (0.6); 5.9352 (0.7); 5.9304 (0.6); 5.9269 (0.7); 5.9214 (0.9); 5.9166 (0.7); 5.8273 (0.7); 5.8216 (1.3); 5.8158 (0.8); 5.8078 (1.0); 5.8021 (0.6); 5.3006 (1.3); 4.6646 (1.5); 4.0547 (1.5); 4.0185 (2.4); 3.9109 (2.2); 3.8747 (1.4); 3.7424 (16.0); 2.5787 (0.9); 2.5656 (0.6); 2.5577 (0.5); 2.5446 (1.0); 2.5235 (0.5); 1.9408 (0.5); 1.9287 (0.9); 1.9167 (0.5); 1.8945 (0.8); 1.3391 (10.8); - 0.0002 (15.2) |
| I-065: ¹H-NMR(400.6 MHz, CDCl3): |
| δ= 7.5586 (0.7); 7.5363 (0.8); 7.5070 (0.6); 7.3365 (1.8); 7.3320 (4.0); 7.3287 (3.6); 7.3244 (1.7); 7.2637 (12.4); 7.2114 (6.4); 7.2068 (5.9); 7.1636 (7.2); 7.1590 (6.7); 5.3010 (1.3); 4.6667 (2.4); 4.2461 (0.5); 4.2302 (1.0); 4.2137 (1.1); 4.2099 (1.2); 4.2015 (0.9); 4.1979 (1.3); 4.1931 (1.2); 4.1839 (2.1); 4.1800 (2.3); 4.1663 (2.7); 4.1623 (2.1); 4.1498 (4.7); 4.1447 (0.8); 4.1320 (4.5); 4.1141 (1.4); 4.0278 (1.7); 4.0075 (1.4); 3.9915 (2.9); 3.9712 (2.7); 3.9101 (5.3); 3.8739 (2.9); 2.5561 (0.6); 2.5422 (0.7); 2.5361 (1.9); 2.5218 (3.5); 2.5179 (2.3); 2.5071 (2.0); 2.5041 (2.1); 2.4865 (1.8); 2.4708 (1.9); 2.4483 (0.6); 2.4326 (0.6); 1.3222 (12.0); 1.3142 (13.6); 1.3055 (4.9); 1.2876 (9.2); 1.2793 (12.2); 1.2698 (5.0); 1.2617 (16.0); 1.2435 (5.8); 1.2404 (7.0); 1.2236 (6.4); -0.0002 (17.8); -0.0085 (0.6) |
| I-066: ¹H-NMR(400.6 MHz, CDCl3): |
| δ= 7.3573 (0.6); 7.3364 (0.6); 7.2662 (3.1); 6.9366 (1.3); 6.9232 (1.3); 6.8974 (1.4); 6.8840 (1.4); 6.8289 (1.3); 6.8234 (2.0); 6.8095 (1.9); 6.8040 (2.1); 6.7978 (0.5); 6.7872 (1.0); 6.7815 (1.2); 6.7600 (0.6); 5.9596 (1.4); 5.9555 (1.4); 5.9204 (1.3); 5.9163 (1.3); 5.3016 (2.0); 4.7805 (1.4); 4.6709 (0.5); 4.6671 (0.5); 4.6536 (0.5); 4.6497 (0.5); 4.0662 (1.8); 4.0300 (2.3); 3.8056 (2.2); 3.7695 (1.7); 3.7248 (0.7); 3.7183 (16.0); 1.3538 (11.4); 1.3277 (6.0); 1.3102 (5.9); -0.0002 (4.5) |
| I-067: ¹H-NMR(400.6 MHz, CDCl3): |
| δ= 7.2620 (7.2); 6.7224 (1.0); 6.7176 (2.2); 6.7131 (1.5); 6.7009 (1.2); 6.6967 (3.2); 5.9838 (1.0); 5.9576 (1.2); 5.9412 (1.4); 5.9151 (1.4); 5.6053 (1.4); 5.6015 (1.5); 5.5627 (1.2); 5.5589 (1.1); 5.3004 (2.0); 5.2581 (1.4); 5.2543 (1.3); 5.2320 (1.3); 5.2282 (1.3); 4.2020 (0.7); 4.1979 (0.5); 4.1928 (0.7); 4.1872 (0.8); 4.1839 (0.6); 4.1812 (0.5); 4.1748 (1.8); 4.1694 (1.9); 4.1569 (1.9); 4.1516 (1.8); 4.1391 (0.6); 4.1338 (0.6); 4.1040 (1.4); 4.0672 (2.6); 3.9844 (2.4); 3.9475 (1.4); 3.7150 (16.0); 2.5029 (3.6); 2.4884 (3.4); 1.3023 (4.2); 1.2845 (8.7); 1.2666 (4.1); 1.2057 (6.1); 1.1889 (6.1); -0.0002 (10.4) |
| I-068: ¹H-NMR(400.6 MHz, CDCl3): |
| δ= 7.4647 (0.5); 7.4439 (0.5); 7.2627 (5.0); 6.7455 (0.6); 6.7238 (1.2); 6.7182 (1.0); 6.7027 (2.2); 6.6971 (1.9); 6.6833 (2.0); 6.6782 (1.2); 5.9781 (1.1); 5.9519 (1.2); 5.9355 (1.4); 5.9094 (1.4); 5.5966 (1.6); 5.5929 (1.6); 5.5540 (1.2); 5.5503 (1.2); 5.3005 (1.5); 5.2470 (1.5); 5.2433 (1.4); 5.2208 (1.4); 5.2171 (1.4); 4.1506 (1.3); 4.1327 (3.9); 4.1149 (4.0); 4.1103 (1.6); 4.0971 (1.3); 4.0734 (2.8); 4.0070 (2.7); 3.9701 (1.4); 3.7171 (16.0); 2.5276 (0.5); 2.5141 (0.6); 2.4890 (1.7); 2.4755 (1.7); 2.4577 (1.6); 2.4423 (1.6); 2.4191 (0.5); 2.4037 (0.5); 1.2638 (4.3); 1.2457 (11.9); 1.2277 (9.7); -0.0002 (7.5) |
| I-069: ¹H-NMR(400.6 MHz, CDCl3): |
| δ= 7.2630 (5.1); 6.9268 (1.2); 6.9133 (1.2); 6.8876 (1.3); 6.8741 (1.3); 6.7367 (0.5); 6.7149 (1.1); 6.7092 (0.9); 6.6909 (1.8); 6.6875 (1.3); 6.6854 (1.5); 6.6713 (1.8); 6.6660 (1.1); 5.9080 (1.4); 5.9039 (1.4); 5.8688 (1.3); 5.8646 (1.3); 5.3006 (4.1); 4.0140 (1.0); 3.9774 (2.8); 3.9431 (2.5); 3.9065 (0.9); 3.7506 (16.0); 3.7430 (15.7); 3.7312 (0.5); 1.3604 (10.6); 1.3159 (5.7); 1.2985 (5.6); -0.0002 (7.3) |
| I-070: ¹H-NMR(400.1 MHz, d₆-DMSO): |
| δ= 7.5813 (3.6); 7.5612 (3.7); 7.1173 (0.8); 7.1115 (1.6); 7.1060 (1.2); 7.0942 (1.5); 7.0885 (3.4); 7.0830 (3.1); 7.0689 (6.6); 7.0639 (6.2); 7.0471 (6.6); 7.0421 (4.0); 7.0323 (1.0); 6.0086 (3.1); 5.9819 (3.6); 5.9654 (3.8); 5.9387 (3.7); 5.8974 (2.2); 5.8923 (3.6); 5.8870 (2.5); 5.8836 (2.8); 5.8784 (4.1); 5.8733 (2.5); 5.6799 (2.4); 5.6743 (4.1); 5.6683 (2.9); 5.6667 (2.8); 5.6604 (3.7); 5.6548 (2.1); 5.5705 (16.0); 5.3418 (4.2); 5.3381 (4.3); 5.2985 (3.8); 5.2948 (3.7); 5.1182 (4.4); 5.1145 (4.1); 5.0914 (3.9); 5.0877 |
| (4.0); 4.5761 (0.6); 4.5714 (0.7); 4.5565 (1.8); 4.5516 (1.6); 4.5469 (1.3); 4.5419 (1.6); 4.5370 (1.8); 4.5219 (0.7); 4.5174 (0.6); 4.0501 (2.9); 4.0135 (7.2); 3.9784 (6.7); 3.9418 (2.7); 3.6231 (48.9); 3.5825 (35.4); 3.5419 (1.3); 3.5365 (1.4); 3.5267 (2.0); 3.5211 (2.6); 3.5156 (2.0); 3.5102 (1.3); 3.5057 (1.4); 3.5004 (1.3); 3.3302 (20.0); 2.5111 (2.7); 2.5070 (5.5); 2.5026 (7.4); 2.4982 (5.3); 2.4019 (1.4); 2.3809 (2.7); 2.3684 (1.9); 2.3599 (1.7); 2.3474 (2.9); 2.3264 (1.4); 1.6808 (1.6); 1.6663 (2.8); 1.6517 (1.8); 1.6474 (1.7); 1.6329 (2.6); 1.6185 (1.4); -0.0002 (1.5) |
| I-071: ¹H-NMR(400.1 MHz, d₆-DMSO): |
| δ= 7.8625 (1.4); 7.8417 (1.4); 7.7985 (1.6); 7.7779 (1.6); 6.9789 (1.3); 6.9739 (1.0); 6.9492 (5.6); 6.8829 (1.2); 6.8623 (1.9); 6.8541 (2.3); 6.8414 (3.4); 6.8289 (1.6); 6.8146 (1.5); 6.8020 (2.5); 6.7895 (1.7); 5.8544 (1.7); 5.8506 (1.7); 5.8149 (1.7); 5.8104 (2.2); 5.8085 (2.3); 5.8043 (1.9); 5.7688 (1.7); 5.7649 (1.7); 5.4347 (0.5); 4.4054 (0.4); 4.3905 (0.7); 4.3733 (0.7); 4.3564 (0.4); 4.3170 (0.4); 4.3031 (0.7); 4.2836 (0.7); 4.2693 (0.4); 4.0018 (1.7); 3.9759 (1.7); 3.9655 (2.2); 3.9398 (2.2); 3.7764 (1.9); 3.7520 (2.0); 3.7403 (1.6); 3.7158 (1.5); 3.6609 (16.0); 3.6404 (16.9); 3.5549 (15.0); 3.5501 (15.4); 3.3636 (0.7); 2.5066 (4.1); 2.5023 (5.4); 2.4980 (3.8); 2.2808 (12.3); 2.2682 (11.3); 1.2026 (15.4); 1.1508 (6.0); 1.1359 (8.8); 1.1191 (6.2); -0.0001 (1.0) |
| I-072: ¹H-NMR(300.1 MHz, d₆-DMSO): |
| δ= 7.4783 (1.6); 7.4492 (2.1); 7.4379 (3.4); 7.4085 (3.2); 7.3704 (0.4); 7.1186 (2.2); 7.1117 (1.7); 7.0830 (6.3); 7.0737 (8.6); 7.0444 (9.4); 6.0137 (2.5); 6.0022 (1.4); 5.9782 (3.0); 5.9668 (1.7); 5.9562 (3.4); 5.9447 (1.8); 5.9206 (3.3); 5.9093 (1.7); 5.7604 (0.8); 5.5487 (12.4); 5.5128 (6.0); 5.3423 (3.9); 5.3371 (4.4); 5.3280 (2.2); 5.2847 (3.3); 5.2794 (3.6); 5.1132 (3.8); 5.1084 (3.7); 5.1009 (2.3); 5.0957 (2.0); 5.0776 (3.6); 5.0728 (3.6); 5.0654 (2.0); 5.0602 (1.8); 4.1126 (2.1); 4.0952 (0.4); 4.0644 (5.4); 4.0162 (5.9); 3.9496 (5.7); 3.9141 (2.8); 3.9008 (3.0); 3.8652 (1.8); 3.8178 (0.4); 3.6623 (0.5); 3.6394 (1.5); 3.6152 (2.5); 3.5760 (66.5); 3.5453 (41.0); 3.5168 (1.1); 3.3329 (276.6); 3.1758 (1.1); 3.1584 (1.0); 2.7278 (1.4); 2.5074 (175.9); 2.5019 (222.2); 2.4966 (157.5); 2.2726 (2.3); 2.2597 (1.7); 2.2479 (2.0); 2.2333 (2.4); 2.2227 (1.5); 2.2071 (1.5); 2.1931 (3.7); 2.1682 (6.1); 2.1419 (4.4); 2.0760 (1.2); 1.6927 (0.3); 1.6666 (1.0); 1.6437 (2.5); 1.6323 (3.0); 1.6193 (3.2); 1.6075 (5.4); 1.5828 (4.9); 1.5572 (1.6); 1.2371 (0.9); 1.0912 (0.5); 1.0701 (0.6); 1.0537 (0.4); 0.9886 (14.1); 0.9669 (16.0); 0.9498 (7.3); 0.0102 (2.9); -0.0002 (58.5); -0.0107 (2.5) |
| I-073: ¹H-NMR(400.6 MHz, CDCl3): |
| δ= 7.3322 (0.5); 7.3125 (0.5); 7.2616 (14.1); 6.8998 (1.3); 6.8873 (1.3); 6.8604 (1.4); 6.8479 (1.5); 6.8347 (0.6); 6.8309 (0.9); 6.8274 (2.1); 6.8233 (0.9); 6.8179 (1.2); 6.8134 (1.8); 6.8104 (1.8); 6.8043 (1.8); 6.7962 (0.8); 6.7916 (0.5); 5.8933 (1.4); 5.8890 (1.4); 5.8540 (1.3); 5.8497 (1.3); 5.3005 (1.0); 4.6512 (1.6); 4.0361 (1.4); 3.9998 (2.8); 3.9397 (2.6); 3.9034 (1.3); 3.7268 (16.0); 1.3552 (11.4); 1.3414 (6.0); 1.3239 (5.9); 1.2548 (0.5); -0.0002 (18.7); -0.0085 (0.6) |
| I-074: ¹H-NMR(400.6 MHz, CDCl3): |
| δ= 7.2619 (12.6); 7.1385 (0.6); 7.1172 (0.6); 6.9010 (0.5); 6.8859 (1.7); 6.8795 (1.0); 6.8737 (1.8); 6.8581 (0.5); 6.8467 (1.4); 6.8343 (1.4); 6.8008 (1.6); 6.7953 (1.5); 6.7827 (1.6); 6.7772 (1.4); 5.9339 (0.5); 5.3005 (2.7); 4.0894 (0.8); 4.0526 (2.0); 4.0153 (1.9); 3.9786 (0.7); 3.7311 (16.0); 1.3955 (9.1); 1.3315 (5.5); 1.3140 (5.5); 1.2551 (0.6); -0.0002 (16.6); -0.0084 (0.5) |
| I-075: ¹H-NMR(400.6 MHz, CDCl3): |
| δ= 7.2616 (8.8); 6.8904 (1.1); 6.8779 (1.1); 6.8511 (1.2); 6.8385 (1.2); 6.7325 (0.6); 6.7292 (0.9); 6.7219 (1.5); 6.7164 (1.2); 6.7056 (1.3); 6.7017 (2.0); 6.6977 (0.9); 5.8866 (1.2); 5.8822 (1.2); 5.8473 (1.2); 5.8429 (1.2); 3.9984 (2.9); 3.9920 (2.7); 3.7316 (15.8); 3.7295 (16.0); 1.5648 (2.4); 1.4321 (1.8); 1.3688 (9.8); 1.3153 (4.9); 1.2978 (4.9); -0.0002 (12.4) |
| I-076: ¹H-NMR(400.6 MHz, CDCl3): |
| δ= 7.5083 (0.6); 7.4878 (0.6); 7.2612 (27.5); 6.8826 (2.0); 6.8769 (2.4); 6.8637 (2.2); 6.8581 (2.1); 6.8248 (0.6); 6.8190 (0.8); 6.8031 (1.1); 6.7974 (1.6); 6.7917 (0.7); 6.7814 (0.6); 6.7757 (0.8); 5.3004 (4.1); 4.6152 (2.4); 4.2464 (0.7); 4.2298 (0.8); 4.2256 (0.8); 4.2091 (0.7); 4.1979 (1.0); 4.1947 (1.2); 4.1800 (2.8); 4.1770 (2.7); 4.1621 (2.8); 4.1592 (2.6); 4.1442 (1.0); 4.1415 (0.9); 4.0107 (1.8); 3.9744 (3.9); 3.9211 (3.6); 3.8849 (1.6); 2.6168 (0.7); 2.5183 (2.5); 2.5036 (4.6); 2.4890 (2.4); 1.5628 (0.5); 1.3261 (16.0); 1.3053 (6.0); 1.2875 (12.2); 1.2696 (5.9); 1.2622 (0.9); 1.2549 (0.7); 1.2411 (8.6); 1.2243 (8.5); 0.0079 (1.4); -0.0002 (39.8); -0.0085 (1.1) |
| I-077: ¹H-NMR(400.6 MHz, CDCl3): |
| δ= 7.5597 (0.5); 7.5388 (0.5); 7.2609 (32.3); 6.8343 (1.2); 6.8305 (2.7); 6.8271 (1.8); 6.8165 (1.8); 6.8110 (1.9); 6.8067 (0.9); 6.8032 (1.3); 6.7966 (0.7); 6.7855 (0.5); 6.7798 (0.6); 5.3004 (2.2); 4.6134 |
| (1.9); 4.2297 (0.5); 4.1705 (1.4); 4.1526 (4.4); 4.1348 (4.4); 4.1170 (1.4); 4.0296 (1.7); 3.9933 (3.2); 3.9239 (3.0); 3.8877 (1.6); 2.6163 (1.3); 2.5589 (0.6); 2.5450 (0.6); 2.5205 (1.9); 2.5067 (1.9); 2.4877 (1.8); 2.4720 (1.8); 2.4494 (0.6); 2.4337 (0.6); 1.5596 (0.7); 1.3258 (1.4); 1.3195 (13.5); 1.2876 (0.7); 1.2803 (5.0); 1.2623 (16.0); 1.2447 (11.9); 1.2244 (0.5); 0.0080 (1.4); -0.0002 (47.3); -0.0085 (1.3) |
| I-078: ¹H-NMR(400.6 MHz, CDCl3): |
| δ= 7.2628 (9.2); 6.8255 (2.8); 6.8051 (2.1); 5.9620 (0.7); 5.9495 (0.8); 5.8312 (0.9); 5.8251 (0.7); 5.8175 (0.8); 5.3008 (2.5); 4.6784 (0.9); 4.0097 (0.9); 3.9337 (0.9); 3.8978 (0.5); 3.7361 (16.0); 2.5444 (0.7); 2.5097 (0.8); 1.9064 (0.8); 1.8718 (0.7); 1.3434 (8.1); -0.0002 (11.9) |
| I-079: ¹H-NMR(600.3 MHz, CDCl3): |
| δ= 7.9698 (1.8); 7.4655 (2.4); 7.4511 (2.4); 7.4341 (0.3); 7.2624 (24.4); 6.8899 (1.1); 6.8863 (1.9); 6.8827 (1.4); 6.8756 (2.2); 6.8720 (3.7); 6.8684 (2.5); 6.8613 (1.5); 6.8577 (2.2); 6.8541 (1.5); 6.8089 (6.1); 6.8053 (6.2); 6.7969 (6.5); 6.7934 (5.6); 6.1655 (0.3); 6.1524 (0.4); 6.1491 (0.3); 5.9648 (3.0); 5.9622 (2.9); 5.9598 (3.1); 5.9572 (3.3); 5.9558 (3.3); 5.9534 (2.8); 5.8309 (2.4); 5.8273 (4.4); 5.8230 (3.2); 5.8181 (4.0); 5.8145 (2.3); 5.2989 (0.7); 5.1656 (1.8); 5.0042 (11.8); 4.8857 (1.2); 4.8718 (2.3); 4.8591 (1.2); 4.1273 (0.3); 4.1153 (0.3); 4.0896 (4.7); 4.0652 (7.6); 3.9899 (7.5); 3.9655 (4.9); 3.7871 (1.2); 3.7382 (50.0); 3.7245 (2.6); 3.5350 (2.1); 3.5311 (2.2); 3.5254 (2.1); 3.5210 (2.3); 3.5171 (2.3); 3.1802 (0.5); 2.6148 (0.9); 2.4927 (1.6); 2.4786 (3.1); 2.4693 (2.0); 2.4646 (1.8); 2.4553 (3.3); 2.4413 (1.6); 2.2873 (0.3); 2.0431 (1.4); 2.0222 (0.8); 2.0162 (9.4); 2.0045 (35.7); 1.9958 (0.6); 1.8938 (1.6); 1.8879 (3.0); 1.8818 (1.7); 1.8705 (1.6); 1.8645 (2.9); 1.8586 (1.6); 1.6151 (11.8); 1.4526 (2.0); 1.3763 (37.1); 1.3133 (0.4); 1.3015 (0.7); 1.2903 (0.9); 1.2805 (0.9); 1.2656 (2.1); 1.2584 (2.8); 1.2469 (1.2); 0.8936 (1.6); 0.8821 (3.7); 0.8702 (1.9); 0.0052 (1.0); -0.0001 (23.4) |
| I-080: ¹H-NMR(400.6 MHz, CDCl3): |
| δ= 7.2625 (11.4); 7.2012 (0.6); 7.1801 (0.6); 6.9343 (1.2); 6.9205 (1.3); 6.8952 (1.9); 6.8812 (1.6); 6.8740 (1.1); 6.8683 (0.6); 6.8525 (0.6); 6.7875 (1.8); 6.7818 (1.7); 6.7693 (1.8); 6.7637 (1.6); 6.7207 (0.7); 5.9184 (1.5); 5.9143 (1.4); 5.8792 (1.3); 5.8752 (1.4); 5.3007 (3.6); 4.6317 (0.5); 4.6282 (0.5); 4.6143 (0.5); 4.6107 (0.5); 4.0913 (1.5); 4.0548 (2.4); 3.9534 (2.3); 3.9170 (1.5); 3.7435 (16.0); 3.7315 (0.7); 3.7197 (0.8); 2.1835 (2.0); 2.0392 (1.0); 1.3736 (10.8); 1.3569 (0.8); 1.3298 (0.7); 1.3206 (5.9); 1.3116 (0.7); 1.3032 (5.9); -0.0002 (15.3) |
| I-081: ¹H-NMR(400.6 MHz, CDCl3): |
| δ= 7.4413 (0.5); 7.4198 (0.5); 7.2616 (14.8); 6.8791 (1.0); 6.8735 (0.7); 6.8575 (0.6); 6.8520 (0.5); 6.8431 (1.5); 6.8375 (1.5); 6.8251 (1.8); 6.8196 (1.2); 5.9384 (0.6); 5.9346 (0.6); 5.9323 (0.6); 5.9284 (0.6); 5.9247 (0.7); 5.9208 (0.8); 5.9186 (0.8); 5.9148 (0.7); 5.8262 (0.7); 5.8207 (1.2); 5.8149 (0.8); 5.8069 (1.0); 5.8013 (0.5); 5.3004 (4.1); 4.0979 (1.2); 4.0614 (2.2); 3.9956 (2.1); 3.9591 (1.1); 3.7427 (16.0); 2.5132 (0.9); 2.4997 (0.6); 2.4787 (1.0); 2.0451 (2.4); 1.9380 (0.5); 1.9283 (0.9); 1.9183 (0.5); 1.8937 (0.8); 1.3658 (10.1); 1.2555 (0.6); 0.0079 (0.6); -0.0002 (18.6) |
| I-082: ¹H-NMR(300.0 MHz, d₆-DMSO): |
| δ= 7.5276 (0.9); 7.5049 (1.4); 7.4825 (0.9); 6.9105 (4.6); 6.8859 (2.8); 6.8582 (1.6); 4.8509 (2.4); 4.0776 (1.0); 4.0731 (1.0); 4.0288 (2.5); 4.0250 (2.5); 3.9762 (3.5); 3.9277 (1.5); 3.8497 (0.5); 3.8417 (0.5); 3.8254 (0.9); 3.8169 (0.9); 3.8013 (1.0); 3.7932 (0.8); 3.7781 (0.5); 3.7700 (0.4); 3.6234 (0.3); 3.6075 (15.5); 3.5999 (15.7); 3.5866 (0.5); 3.5580 (13.7); 3.5547 (13.5); 3.3214 (30.6); 2.8096 (0.8); 2.7827 (1.2); 2.7616 (0.8); 2.7554 (0.8); 2.7335 (0.4); 2.7274 (0.5); 2.5063 (29.1); 2.5011 (37.8); 2.2722 (16.0); 2.0742 (6.8); 2.0451 (0.4); 2.0397 (0.4); 2.0166 (0.8); 2.0022 (0.6); 1.9954 (0.8); 1.9881 (0.6); 1.9738 (0.9); 1.9505 (0.4); 1.9450 (0.4); 1.9080 (0.3); 1.8414 (0.4); 1.8325 (0.6); 1.8133 (1.4); 1.8078 (1.4); 1.7872 (2.2); 1.7629 (1.9); 1.7520 (1.0); 1.7383 (0.8); 1.7303 (0.7); 1.7225 (0.6); 1.7127 (0.8); 1.7060 (0.7); 1.6914 (0.7); 1.6850 (0.8); 1.6630 (0.6); 1.6315 (0.5); 1.6043 (0.7); 1.5877 (0.4); 1.5777 (0.4); 1.5635 (0.8); 1.5385 (0.8); 1.5224 (0.4); 1.5127 (0.5); 1.4961 (0.6); 1.4835 (0.6); 1.4689 (0.6); 1.4608 (0.7); 1.4379 (0.7); 1.4169 (0.7); 1.3995 (0.4); 1.3916 (0.4); 1.0854 (0.7); 1.0660 (1.0); 1.0630 (1.0); 1.0401 (0.8); 0.3941 (0.4); 0.3740 (1.0); 0.3553 (1.2); 0.3354 (0.7); 0.2830 (0.7); 0.2730 (1.7); 0.2685 (1.5); 0.2468 (2.2); 0.2362 (1.0); 0.2271 (1.5); 0.2086 (0.4); 0.2004 (0.4); 0.1690 (0.7); 0.1484 (1.1); 0.1435 (1.1); 0.1207 (0.8); 0.1025 (0.4); 0.0108 (0.6); -0.0002 (16.0); -0.0112 (0.5) |
| I-083: ¹H-NMR(300.0 MHz, d₆-DMSO): |
| δ= 7.6730 (0.6); 7.6674 (0.8); 7.6637 (0.7); 7.6290 (1.8); 7.6109 (2.2); 7.6041 (2.2); 7.5863 (1.8); 7.4517 (3.6); 7.4460 (7.2); 7.4403 (4.6); 7.3989 (0.4); 7.3931 (0.7); 7.3749 (16.0); 7.3694 (14.2); 6.0038 (2.5); 5.9682 (3.0); 5.9462 (3.3); 5.9106 (3.3); 5.5375 (11.1); 5.4871 (0.4); 5.4069 (0.3); 5.3386 (3.7); 5.2817 (3.0); 5.1182 (3.6); 5.0809 (3.2); 4.1900 (0.3); 4.1026 (0.4); 4.0851 (0.4); 4.0417 (1.5); 4.0367 (1.3); |
| 3.9925 (4.3); 3.9882 (4.4); 3.9583 (3.6); 3.9503 (3.6); 3.9098 (1.1); 3.9013 (1.3); 3.8754 (0.3); 3.8505 (1.3); 3.8268 (2.4); 3.8028 (2.3); 3.7793 (1.1); 3.6063 (25.6); 3.5907 (26.3); 3.5755 (24.8); 3.3220 (82.5); 3.1762 (1.6); 3.1587 (1.5); 2.8387 (0.4); 2.8118 (1.4); 2.7852 (2.1); 2.7633 (1.4); 2.7584 (1.5); 2.7336 (1.0); 2.7278 (1.0); 2.7214 (0.6); 2.5062 (79.5); 2.5013 (102.2); 2.2832 (0.3); 2.2773 (0.6); 2.2711 (0.8); 2.2652 (0.6); 2.2592 (0.3); 2.0759 (0.6); 2.0490 (1.4); 2.0325 (1.0); 2.0242 (1.5); 2.0057 (1.8); 1.9974 (0.9); 1.9812 (1.5); 1.9539 (0.7); 1.8472 (0.8); 1.8395 (1.1); 1.8195 (2.4); 1.8116 (1.8); 1.7932 (3.5); 1.7860 (2.2); 1.7708 (3.4); 1.7606 (2.7); 1.7432 (2.2); 1.7197 (2.0); 1.6975 (1.5); 1.6716 (0.8); 1.6574 (0.9); 1.6305 (1.3); 1.6141 (0.8); 1.6037 (0.9); 1.5872 (1.2); 1.5795 (1.0); 1.5530 (1.4); 1.5360 (0.8); 1.5264 (1.0); 1.5086 (1.8); 1.4840 (1.6); 1.4675 (0.7); 1.4600 (0.8); 1.4355 (1.0); 1.4125 (1.1); 1.3979 (0.6); 1.3887 (0.6); 1.3745 (0.6); 0.0107 (1.3); -0.0001 (37.1); -0.0112 (1.4) |
| I-084: ¹H-NMR(300.0 MHz, d₆-DMSO): |
| δ= 7.6695 (1.2); 7.5877 (5.2); 7.5609 (5.0); 7.4528 (11.3); 7.4469 (8.9); 7.3939 (13.5); 7.3878 (14.0); 7.3810 (16.0); 7.3749 (13.6); 6.1675 (0.4); 6.0130 (3.8); 5.9778 (4.4); 5.9557 (4.8); 5.9200 (5.0); 5.8979 (3.4); 5.8785 (4.3); 5.8493 (3.5); 5.6981 (3.6); 5.6899 (3.2); 5.6792 (3.5); 5.6694 (3.2); 5.6607 (3.6); 5.6425 (3.2); 5.5805 (14.7); 5.3416 (5.1); 5.2840 (4.2); 5.1269 (5.3); 5.0914 (4.9); 4.5417 (3.0); 4.0508 (2.2); 3.9957 (8.1); 3.9758 (9.0); 3.9253 (2.6); 3.8809 (1.1); 3.6377 (29.1); 3.6206 (31.2); 3.5782 (39.8); 3.5326 (5.8); 3.3235 (137.0); 3.1760 (2.1); 3.1588 (2.0); 2.7273 (4.6); 2.5012 (295.9); 2.4036 (11.4); 2.3836 (10.7); 2.3666 (9.5); 2.3583 (9.9); 2.3384 (8.7); 2.3102 (6.8); 2.2714 (6.9); 1.7758 (2.4); 1.7565 (3.1); 1.7325 (2.6); 1.7116 (2.9); 1.6936 (2.2); 1.6708 (2.2); 1.6520 (2.9); 1.6272 (2.4); 1.6072 (2.7); 1.5878 (2.0); 1.2320 (0.7); 0.1951 (0.7); -0.0002 (93.1); -0.1989 (1.0); -0.5123 (0.4) |
| I-085: ¹H-NMR(300.0 MHz, d₆-DMSO): |
| δ= 7.9158 (1.1); 7.8884 (1.2); 7.8690 (1.6); 7.8417 (1.6); 7.6790 (0.5); 7.6724 (0.8); 7.6658 (0.5); 7.4599 (2.0); 7.4537 (4.2); 7.4474 (3.0); 7.4437 (1.9); 7.4373 (3.4); 7.4311 (2.2); 7.4070 (0.6); 7.3966 (10.2); 7.3904 (8.5); 7.3741 (7.8); 7.3678 (6.6); 7.3528 (0.4); 6.8705 (1.5); 6.8524 (1.5); 6.8179 (1.7); 6.8111 (2.0); 6.7998 (1.8); 6.7937 (2.0); 6.7585 (2.0); 6.7411 (2.1); 6.0236 (1.3); 6.0124 (1.0); 5.9880 (1.6); 5.9769 (1.2); 5.9659 (1.8); 5.9551 (1.3); 5.9303 (1.7); 5.9196 (1.2); 5.8951 (1.8); 5.8897 (1.9); 5.8425 (1.6); 5.8371 (1.6); 5.7632 (2.3); 5.7577 (2.3); 5.7107 (2.0); 5.7051 (2.1); 5.6837 (5.9); 5.6370 (4.4); 5.6010 (0.4); 5.3748 (2.0); 5.3698 (3.1); 5.3646 (1.7); 5.3172 (1.7); 5.3122 (2.6); 5.3071 (1.4); 5.1547 (1.9); 5.1494 (1.8); 5.1409 (1.6); 5.1353 (1.4); 5.1191 (1.7); 5.1138 (1.8); 5.1054 (1.3); 5.0998 (1.3); 4.3709 (0.7); 4.3519 (0.5); 4.3479 (0.6); 4.3296 (0.4); 4.3241 (0.4); 4.3135 (0.4); 4.3088 (0.4); 4.2900 (0.7); 4.2640 (0.7); 4.2457 (0.4); 4.2405 (0.4); 4.1217 (1.3); 4.1065 (0.3); 4.0890 (0.4); 4.0724 (2.3); 4.0193 (2.6); 3.9583 (2.4); 3.9219 (1.8); 3.9096 (1.4); 3.8729 (1.2); 3.6589 (16.0); 3.6470 (1.4); 3.6432 (1.7); 3.6284 (19.8); 3.5779 (16.0); 3.5714 (14.3); 3.3255 (54.4); 3.1759 (1.2); 3.1585 (1.2); 2.7338 (0.6); 2.7277 (0.9); 2.7216 (0.6); 2.5068 (83.2); 2.5015 (110.3); 2.4964 (73.2); 2.2776 (0.6); 2.2714 (0.9); 2.2653 (0.6); 1.2585 (0.4); 1.2411 (0.5); 1.2354 (0.5); 1.2178 (0.4); 1.1765 (6.3); 1.1530 (6.2); 1.1250 (5.0); 1.1016 (5.0); 0.0108 (2.0); -0.0001 (65.9); -0.0112 (2.1) |
| I-086: ¹H-NMR(400.1 MHz, d₆-DMSO): |
| δ= 7.9102 (1.3); 7.8894 (1.3); 7.8453 (1.4); 7.8249 (1.4); 6.9401 (1.3); 6.9252 (3.0); 6.9087 (4.3); 6.8805 (2.3); 6.8758 (2.2); 6.8669 (3.1); 6.8537 (1.9); 6.8276 (1.6); 6.8141 (1.6); 6.8048 (1.5); 6.7918 (1.5); 6.7655 (1.6); 6.7524 (1.6); 6.0183 (1.2); 6.0027 (1.2); 5.9916 (1.4); 5.9755 (2.5); 5.9597 (1.4); 5.9484 (1.5); 5.9331 (1.4); 5.8800 (1.8); 5.8762 (1.8); 5.8406 (1.7); 5.8368 (1.7); 5.7610 (1.8); 5.7572 (1.8); 5.7217 (1.6); 5.7178 (1.6); 5.6367 (4.9); 5.6145 (3.0); 5.3750 (2.9); 5.3319 (2.5); 5.1287 (1.8); 5.1249 (1.8); 5.1199 (1.8); 5.1158 (1.6); 5.1020 (1.6); 5.0981 (1.8); 5.0933 (1.6); 5.0892 (1.5); 4.3952 (0.4); 4.3807 (0.8); 4.3640 (0.8); 4.3430 (0.4); 4.3202 (0.4); 4.3022 (0.7); 4.2831 (0.7); 4.2688 (0.4); 4.0806 (1.7); 4.0435 (3.0); 4.0053 (2.4); 3.9099 (2.3); 3.8832 (2.3); 3.8739 (1.6); 3.8469 (1.7); 3.6599 (16.0); 3.6466 (1.2); 3.6405 (1.2); 3.6288 (16.1); 3.5498 (14.4); 3.5458 (15.5); 3.3333 (17.9); 3.1749 (0.4); 3.1619 (0.4); 2.5064 (14.8); 2.5022 (19.4); 2.4979 (14.1); 2.3221 (0.8); 2.2813 (11.8); 2.2676 (11.5); 1.2386 (0.4); 1.1654 (5.9); 1.1478 (5.9); 1.1204 (5.8); 1.1029 (5.7); -0.0001 (2.9) |
| I-087: ¹H-NMR(400.1 MHz, d₆-DMSO): |
| δ= 7.4722 (1.3); 7.4508 (1.4); 7.4319 (1.4); 7.4100 (1.3); 6.9144 (7.3); 6.8915 (4.4); 6.8703 (2.3); 6.0031 (1.4); 5.9916 (1.3); 5.9764 (1.6); 5.9649 (1.6); 5.9600 (1.9); 5.9485 (1.7); 5.9333 (1.7); 5.9219 (1.6); 5.5134 (6.1); 5.5034 (3.5); 5.3438 (3.4); 5.3400 (2.1); 5.3007 (3.0); 5.1003 (2.0); 5.0962 (1.9); 5.0910 (2.0); 5.0868 (1.8); 5.0736 (1.8); 5.0695 (1.9); 5.0644 (1.8); 5.0601 (1.8); 4.0701 (1.9); 4.0334 (3.1); 3.9942 (2.8); 3.8925 (2.7); 3.8586 (3.2); 3.8228 (2.0); 3.6428 (0.7); 3.6243 (1.0); 3.6078 (1.0); 3.5963 (1.0); 3.5742 (22.5); 3.5452 (39.4); 3.3292 (34.3); 2.5243 (0.7); 2.5105 (17.5); 2.5062 (36.1); 2.5017 |
| (48.7); 2.4973 (34.7); 2.4930 (16.3); 2.3284 (0.3); 2.2809 (15.9); 2.2780 (16.0); 2.2650 (1.5); 2.2527 (1.2); 2.2448 (1.7); 2.2331 (2.2); 2.2265 (1.3); 2.2138 (1.2); 2.1849 (1.9); 2.1660 (2.7); 2.1462 (2.1); 1.6587 (0.8); 1.6415 (2.0); 1.6222 (2.8); 1.6021 (3.1); 1.5837 (2.2); 1.5661 (0.7); 0.9945 (7.1); 0.9779 (7.2); 0.9635 (7.2); 0.9470 (7.0); 0.0080 (0.6); -0.0002 (18.4); -0.0084 (0.6) |
| I-088: ¹H-NMR(400.1 MHz, d₆-DMSO): |
| δ= 7.5803 (2.1); 7.5748 (2.0); 7.5606 (2.2); 7.5542 (1.9); 6.9267 (4.7); 6.9149 (5.7); 6.8972 (3.7); 6.8723 (2.7); 6.0066 (3.2); 5.9799 (3.7); 5.9634 (4.0); 5.9367 (3.8); 5.8915 (1.3); 5.8863 (2.0); 5.8810 (1.4); 5.8777 (1.5); 5.8724 (2.3); 5.8675 (2.3); 5.8629 (1.8); 5.8575 (1.2); 5.8541 (1.4); 5.8489 (2.0); 5.8438 (1.2); 5.6829 (1.3); 5.6772 (2.2); 5.6713 (1.5); 5.6634 (2.0); 5.6575 (1.4); 5.6553 (1.5); 5.6494 (2.1); 5.6436 (1.4); 5.6414 (1.3); 5.6356 (1.8); 5.6299 (1.0); 5.5331 (5.2); 5.3483 (2.4); 5.3439 (3.3); 5.3386 (2.1); 5.3051 (2.1); 5.3005 (2.8); 5.2955 (1.8); 5.1079 (2.5); 5.1033 (3.6); 5.0987 (2.1); 5.0812 (2.3); 5.0768 (3.4); 5.0720 (2.0); 4.6017 (0.3); 4.5861 (0.8); 4.5811 (0.8); 4.5716 (1.1); 4.5668 (1.2); 4.5575 (1.0); 4.5524 (1.2); 4.5472 (1.1); 4.5379 (0.9); 4.5329 (1.0); 4.5176 (0.4); 4.5130 (0.3); 4.0221 (2.1); 4.0165 (1.7); 3.9858 (4.0); 3.9803 (3.7); 3.9322 (3.3); 3.9253 (3.5); 3.8960 (1.6); 3.8890 (1.8); 3.6370 (25.0); 3.6302 (2.5); 3.6218 (27.1); 3.6059 (0.4); 3.5915 (0.4); 3.5506 (20.3); 3.5482 (20.5); 3.5322 (2.3); 3.5235 (1.8); 3.5173 (2.1); 3.5116 (1.7); 3.5020 (0.9); 3.4968 (0.7); 3.3324 (9.0); 3.1681 (0.5); 2.5106 (17.3); 2.5063 (34.7); 2.5018 (46.3); 2.4973 (32.5); 2.4929 (14.9); 2.4018 (0.7); 2.3939 (0.8); 2.3807 (1.4); 2.3728 (1.7); 2.3684 (1.2); 2.3600 (1.8); 2.3516 (1.3); 2.3473 (1.6); 2.3394 (1.7); 2.3201 (1.8); 2.2825 (14.7); 2.2752 (16.0); 1.7587 (0.8); 1.7441 (1.3); 1.7293 (0.8); 1.7254 (0.8); 1.7106 (1.2); 1.6961 (0.6); 1.6650 (0.8); 1.6502 (1.4); 1.6352 (1.0); 1.6317 (0.9); 1.6167 (1.4); 1.6020 (0.8); 0.0080 (0.5); -0.0002 (11.0); -0.0085 (0.4) |
| I-089: ¹H-NMR(400.2 MHz, d₆-DMSO): |
| δ= 7.6145 (1.0); 7.6018 (1.2); 7.5957 (1.2); 7.5832 (1.0); 6.9099 (5.2); 6.8892 (2.8); 6.8673 (1.5); 5.9980 (1.7); 5.9713 (2.0); 5.9548 (2.1); 5.9281 (2.0); 5.4866 (0.7); 5.3391 (1.9); 5.2972 (1.7); 5.0945 (2.0); 5.0670 (1.9); 4.0117 (1.2); 4.0057 (1.1); 3.9755 (2.1); 3.9695 (2.1); 3.9084 (2.0); 3.9031 (2.0); 3.8721 (1.2); 3.8665 (1.8); 3.8476 (1.5); 3.8293 (1.5); 3.8113 (0.7); 3.6060 (14.0); 3.5918 (14.1); 3.5790 (0.5); 3.5475 (10.5); 3.5439 (10.3); 3.4197 (0.4); 3.3768 (1.9); 2.8096 (0.6); 2.8027 (0.7); 2.7900 (0.9); 2.7825 (1.1); 2.7695 (0.7); 2.7620 (0.8); 2.5235 (0.6); 2.5103 (9.7); 2.5059 (20.1); 2.5014 (27.3); 2.4969 (19.8); 2.4925 (9.2); 2.2765 (16.0); 2.0468 (0.3); 2.0300 (0.6); 2.0140 (0.9); 1.9941 (0.9); 1.9777 (0.7); 1.9608 (0.4); 1.8343 (0.4); 1.8254 (0.5); 1.8144 (1.2); 1.8086 (0.9); 1.8049 (0.9); 1.7950 (1.6); 1.7897 (1.3); 1.7823 (1.0); 1.7765 (1.4); 1.7624 (0.9); 1.7570 (1.0); 1.7437 (0.9); 1.7321 (0.6); 1.7237 (0.8); 1.7146 (0.6); 1.7075 (0.7); 1.6921 (0.7); 1.6799 (0.4); 1.6380 (0.4); 1.6177 (0.7); 1.6058 (0.4); 1.5974 (0.4); 1.5855 (0.6); 1.5772 (0.5); 1.5651 (0.4); 1.5574 (0.7); 1.5448 (0.4); 1.5374 (0.4); 1.5249 (0.6); 1.5007 (0.6); 1.4822 (0.5); 1.4710 (0.4); 1.4511 (0.4); 1.4356 (0.6); 1.4175 (0.5); 1.4071 (0.4); 0.0080 (0.4); - 0.0002 (9.6); -0.0084 (0.3) |
| I-090: ¹H-NMR(400.2 MHz, d₆-DMSO): |
| δ= 7.4371 (1.6); 7.4155 (1.7); 7.3766 (1.6); 7.3547 (1.7); 6.9784 (1.5); 6.9733 (1.3); 6.9562 (5.7); 6.9371 (4.6); 6.8789 (2.6); 6.8546 (2.6); 5.3388 (7.3); 5.3138 (6.7); 3.9958 (2.2); 3.9695 (2.1); 3.9596 (2.8); 3.9334 (2.6); 3.7479 (2.3); 3.7378 (0.4); 3.7201 (2.5); 3.7118 (2.0); 3.6839 (1.9); 3.6565 (0.5); 3.6396 (0.9); 3.6201 (1.2); 3.6028 (1.4); 3.5890 (1.6); 3.5697 (25.3); 3.5563 (30.0); 3.5529 (22.8); 3.5487 (19.4); 3.3326 (26.5); 2.5108 (7.5); 2.5064 (15.1); 2.5019 (20.2); 2.4974 (14.4); 2.4931 (6.6); 2.2810 (16.0); 2.2754 (15.6); 2.2333 (2.2); 2.2287 (2.8); 2.2170 (3.6); 2.2113 (5.0); 2.1951 (2.7); 2.1904 (2.8); 2.0759 (0.5); 1.6412 (2.0); 1.6225 (4.7); 1.6046 (4.5); 1.5860 (1.6); 1.1848 (14.7); 1.1745 (14.2); 0.9927 (8.0); 0.9762 (8.0); 0.9620 (8.0); 0.9454 (7.7); -0.0002 (4.2) |
| I-091: ¹H-NMR(300.0 MHz, d₆-DMSO): |
| δ= 7.8398 (1.5); 7.8120 (1.6); 7.7216 (1.3); 7.6949 (1.3); 6.9268 (3.6); 6.9060 (6.6); 6.8751 (4.3); 6.8577 (3.7); 6.8217 (2.6); 6.8047 (2.7); 6.7681 (1.3); 6.7513 (1.3); 5.8861 (1.9); 5.8814 (1.9); 5.8335 (1.8); 5.8292 (1.8); 5.8028 (1.7); 5.7982 (1.6); 5.7502 (1.5); 5.7456 (1.5); 4.9843 (2.4); 4.9537 (3.1); 4.3886 (0.5); 4.3636 (0.9); 4.3416 (0.9); 4.3204 (0.6); 4.2925 (0.6); 4.2705 (0.8); 4.2484 (0.8); 4.2288 (0.5); 4.1151 (1.5); 4.1050 (1.3); 4.0663 (3.0); 4.0566 (2.8); 3.9912 (2.8); 3.9814 (3.0); 3.9430 (1.4); 3.9326 (1.6); 3.6585 (16.0); 3.6416 (13.9); 3.5592 (19.1); 3.3277 (13.5); 2.5019 (27.7); 2.2771 (11.5); 2.2598 (13.3); 2.0751 (0.6); 1.2347 (0.8); 1.1568 (5.4); 1.1331 (6.1); 1.1116 (7.5); 1.0884 (7.7); 1.0265 (0.5); 0.4197 (1.7); 0.4094 (1.7); 0.4002 (1.9); 0.3822 (1.2); 0.3454 (1.0); 0.3352 (1.1); 0.2910 (2.4); 0.2711 (3.0); 0.2528 (2.4); 0.1698 (1.4); 0.1566 (1.9); 0.1295 (1.2); -0.0001 (7.1) |
| I-092: ¹H-NMR(300.0 MHz, d₆-DMSO): |
| δ= 7.4255 (1.1); 7.3970 (1.1); 7.3440 (1.1); 7.3153 (1.1); 6.9225 (6.4); 6.8936 (4.1); 6.8620 (2.0); 4.8877 (2.0); 4.8768 (2.8); 4.0847 (1.3); 4.0345 (3.5); 3.9894 (2.5); 3.9779 (2.4); 3.9409 (1.0); 3.9290 (1.0); 3.6582 (0.4); 3.6348 (0.8); 3.6089 (1.4); 3.5728 (17.2); 3.5591 (34.3); 3.3283 (23.1); 2.5018 (20.7); 2.2768 (16.0); 2.2560 (1.4); 2.2400 (2.2); 2.2292 (2.6); 2.2056 (2.8); 2.1814 (1.6); 1.6569 (0.9); 1.6325 (2.8); 1.6075 (3.7); 1.5820 (2.0); 1.5617 (0.6); 1.2352 (1.0); 1.0738 (1.3); 1.0619 (1.3); 1.0517 (1.0); 1.0159 (0.4); 0.9801 (5.4); 0.9573 (7.1); 0.9537 (6.9); 0.9308 (5.1); 0.4334 (0.4); 0.4145 (1.1); 0.3947 (1.5); 0.3746 (1.3); 0.3541 (0.6); 0.2823 (1.6); 0.2617 (3.1); 0.2405 (3.1); 0.2195 (1.5); 0.1658 (0.6); 0.1429 (1.2); 0.1331 (1.0); 0.1250 (1.2); 0.1165 (1.0); 0.0988 (1.0); 0.0806 (0.4); -0.0001 (3.2) |
| I-093: ¹H-NMR(300.1 MHz, d₆-DMSO): |
| δ= 7.4692 (1.4); 7.4418 (8.4); 7.3996 (2.2); 7.3727 (16.0); 6.0103 (1.6); 6.0045 (1.3); 5.9748 (1.9); 5.9687 (1.5); 5.9528 (2.1); 5.9467 (1.6); 5.9172 (2.2); 5.5462 (6.4); 5.5082 (4.5); 5.3416 (3.0); 5.2840 (2.5); 5.1233 (2.5); 5.1190 (2.4); 5.1120 (2.0); 5.0876 (2.3); 5.0834 (2.4); 5.0765 (1.8); 4.1178 (1.6); 4.0683 (3.9); 4.0189 (3.6); 3.9451 (3.4); 3.8982 (3.5); 3.8504 (1.5); 3.6572 (0.5); 3.6349 (1.2); 3.6110 (2.0); 3.5752 (38.4); 3.5477 (23.5); 3.5230 (0.5); 3.3117 (11.9); 2.7267 (0.3); 2.5008 (48.7); 2.2737 (1.3); 2.2654 (1.3); 2.2492 (1.9); 2.2384 (2.1); 2.2220 (1.3); 2.2139 (1.3); 2.1925 (2.4); 2.1672 (4.1); 2.1412 (2.8); 2.0728 (2.4); 1.6753 (0.9); 1.6510 (2.2); 1.6285 (3.0); 1.6058 (3.7); 1.5822 (3.0); 1.5581 (1.0); 1.0011 (8.7); 0.9789 (9.0); 0.9672 (6.8); 0.9451 (6.1); -0.0002 (10.3) |
| I-094: ¹H-NMR(300.0 MHz, d₆-DMSO): |
| δ= 7.4847 (1.9); 7.4579 (2.0); 6.9223 (5.5); 6.8945 (3.3); 6.8656 (1.6); 5.8966 (1.2); 5.8897 (2.0); 5.8827 (1.4); 5.8782 (1.5); 5.8712 (2.3); 5.8644 (1.4); 5.6617 (1.3); 5.6542 (2.2); 5.6463 (1.6); 5.6357 (2.0); 5.6282 (1.2); 4.8759 (4.2); 4.5534 (0.3); 4.5472 (0.4); 4.5275 (1.0); 4.5210 (0.9); 4.5145 (0.7); 4.5078 (0.9); 4.5013 (1.0); 4.4813 (0.4); 4.4753 (0.4); 4.0927 (1.6); 4.0441 (3.6); 3.9876 (3.4); 3.9392 (1.6); 3.6456 (0.8); 3.6278 (27.3); 3.5572 (19.9); 3.5361 (1.0); 3.5297 (0.8); 3.5223 (1.2); 3.5149 (1.5); 3.5076 (1.2); 3.5008 (0.8); 3.4940 (0.8); 3.4870 (0.7); 3.3251 (7.9); 2.5062 (11.4); 2.5014 (14.5); 2.3907 (0.8); 2.3628 (1.4); 2.3462 (1.1); 2.3348 (1.0); 2.3185 (1.8); 2.2898 (1.3); 2.2744 (16.0); 1.6582 (0.9); 1.6386 (1.5); 1.6186 (1.0); 1.6142 (1.0); 1.5941 (1.3); 1.5746 (0.8); 1.1043 (0.7); 1.0954 (0.8); 1.0774 (1.4); 1.0670 (0.6); 1.0592 (0.8); 1.0503 (0.8); 1.0319 (0.4); 0.4063 (0.4); 0.3971 (0.6); 0.3880 (1.1); 0.3693 (1.3); 0.3502 (0.7); 0.3425 (0.6); 0.3150 (0.4); 0.2982 (1.1); 0.2891 (1.3); 0.2795 (1.4); 0.2700 (1.6); 0.2495 (1.9); 0.2312 (1.1); 0.2216 (0.7); 0.2140 (0.5); 0.2044 (0.5); 0.1918 (0.6); 0.1854 (0.8); 0.1657 (1.2); 0.1572 (1.2); 0.1380 (0.8); 0.1277 (0.5); 0.1213 (0.4); -0.0001 (6.4) |
| I-095: ¹H-NMR(300.1 MHz, d₆-DMSO): |
| δ= 7.7049 (2.4); 7.6778 (2.3); 7.1258 (1.0); 7.1213 (1.0); 7.0947 (1.9); 7.0901 (2.0); 7.0829 (1.2); 7.0712 (0.7); 7.0634 (1.0); 7.0588 (1.1); 6.9952 (3.8); 6.9725 (3.7); 6.9684 (3.9); 6.3954 (3.2); 6.3410 (3.6); 6.3177 (2.3); 6.3044 (2.2); 5.8992 (0.9); 5.8924 (1.5); 5.8854 (1.8); 5.8795 (2.2); 5.8734 (2.3); 5.8670 (2.1); 5.8603 (1.8); 5.8535 (1.2); 5.7324 (1.0); 5.7249 (1.7); 5.7172 (1.2); 5.7065 (1.5); 5.6981 (1.6); 5.6897 (1.8); 5.6819 (1.2); 5.6712 (1.5); 5.6637 (0.9); 4.6119 (0.7); 4.6049 (0.7); 4.5928 (0.9); 4.5866 (1.0); 4.5768 (0.9); 4.5672 (1.0); 4.5609 (0.9); 4.5495 (0.8); 4.5426 (0.7); 4.5225 (0.4); 3.8998 (1.5); 3.8947 (1.5); 3.8720 (0.4); 3.8500 (3.1); 3.8455 (3.1); 3.7721 (4.5); 3.7228 (2.4); 3.6391 (19.4); 3.6291 (19.4); 3.6092 (0.8); 3.6007 (0.9); 3.5630 (0.7); 3.5562 (0.9); 3.5506 (1.1); 3.5435 (1.4); 3.5360 (1.6); 3.5296 (1.5); 3.5232 (1.5); 3.5156 (1.4); 3.5086 (1.1); 3.3208 (3.1); 2.5655 (9.0); 2.5616 (9.6); 2.5510 (9.7); 2.5471 (9.2); 2.5128 (10.6); 2.5070 (19.5); 2.5011 (25.1); 2.4952 (17.4); 2.4897 (8.6); 2.4365 (0.9); 2.4078 (1.5); 2.3917 (1.0); 2.3791 (1.5); 2.3629 (1.7); 2.3500 (0.8); 2.3342 (1.6); 2.3056 (0.6); 1.7991 (0.6); 1.7799 (1.1); 1.7552 (1.2); 1.7361 (1.9); 1.7170 (1.2); 1.7116 (0.8); 1.6922 (1.0); 1.6734 (0.6); 1.1607 (16.0); 1.1587 (15.8); -0.0002 (6.7) |
| I-096: ¹H-NMR(300.0 MHz, d₆-DMSO): |
| δ= 7.5236 (1.9); 7.4963 (1.9); 6.9281 (4.2); 6.9112 (1.3); 6.9031 (2.8); 6.8666 (1.5); 5.8709 (1.2); 5.8641 (2.1); 5.8570 (1.4); 5.8525 (1.5); 5.8455 (2.4); 5.8387 (1.4); 5.6310 (1.3); 5.6235 (2.3); 5.6156 (1.6); 5.6132 (1.5); 5.6050 (2.0); 5.5975 (1.2); 4.8808 (3.5); 4.5891 (0.3); 4.5828 (0.4); 4.5692 (0.7); 4.5626 (1.0); 4.5560 (0.9); 4.5493 (0.7); 4.5424 (0.9); 4.5358 (1.0); 4.5293 (0.7); 4.5155 (0.4); 4.5091 (0.4); 4.0802 (1.3); 4.0316 (3.8); 3.9965 (3.6); 3.9479 (1.3); 3.6358 (27.8); 3.5605 (21.0); 3.5366 (1.3); 3.5292 (1.6); 3.5218 (1.2); 3.5149 (0.8); 3.5081 (0.9); 3.5010 (0.7); 3.3247 (9.8); 2.5065 (13.7); 2.5014 (17.5); 2.4001 (0.8); 2.3721 (1.4); 2.3556 (1.1); 2.3441 (0.9); 2.3276 (1.7); 2.2995 (1.0); 2.2796 (16.0); 1.7368 (0.8); 1.7166 (1.5); 1.6955 (1.1); 1.6721 (1.3); 1.6519 (0.7); 1.1081 (0.7); 1.0964 (0.6); 1.0874 (1.4); 1.0776 (0.6); 1.0646 (0.8); 1.0421 (0.4); 0.3919 (0.4); 0.3722 (1.2); 0.3538 (1.3); 0.3342 (0.8); 0.2932 |
| (0.5); 0.2711 (1.9); 0.2500 (2.7); 0.2290 (1.8); 0.2085 (0.5); 0.2023 (0.6); 0.1815 (0.8); 0.1599 (1.3); 0.1404 (0.6); 0.1323 (0.9); 0.1140 (0.4); -0.0001 (6.2) |
| I-097: ¹H-NMR(400.1 MHz, d₆-DMSO): |
| δ= 7.5694 (3.6); 7.5488 (3.6); 7.1187 (0.8); 7.1129 (1.6); 7.1073 (1.3); 7.0882 (4.6); 7.0829 (7.6); 7.0779 (5.2); 7.0602 (8.0); 6.0057 (3.1); 5.9790 (3.6); 5.9625 (3.8); 5.9358 (3.7); 5.8719 (2.2); 5.8668 (3.7); 5.8615 (2.6); 5.8581 (2.8); 5.8529 (4.1); 5.8478 (2.5); 5.6689 (2.5); 5.6633 (4.3); 5.6573 (3.0); 5.6494 (3.8); 5.6438 (2.2); 5.5676 (16.0); 5.3329 (4.2); 5.3291 (4.3); 5.2896 (3.8); 5.2859 (3.7); 5.1111 (4.4); 5.1074 (4.0); 5.0844 (4.0); 5.0806 (4.0); 4.6028 (0.6); 4.5977 (0.7); 4.5828 (1.8); 4.5779 (1.6); 4.5729 (1.3); 4.5681 (1.6); 4.5633 (1.8); 4.5479 (0.7); 4.5434 (0.6); 4.0465 (2.6); 4.0100 (7.5); 3.9825 (7.0); 3.9459 (2.4); 3.6379 (50.0); 3.6226 (0.6); 3.5845 (36.6); 3.5532 (1.4); 3.5479 (1.4); 3.5433 (1.3); 3.5381 (2.0); 3.5325 (2.6); 3.5270 (2.0); 3.5214 (1.3); 3.5169 (1.4); 3.5118 (1.3); 3.3290 (57.4); 3.1749 (0.6); 3.1617 (0.6); 2.5108 (5.8); 2.5065 (11.8); 2.5021 (15.9); 2.4977 (11.4); 2.4935 (5.3); 2.3979 (1.4); 2.3769 (2.7); 2.3643 (1.9); 2.3557 (1.7); 2.3433 (2.9); 2.3223 (1.4); 2.0750 (0.4); 1.7584 (1.6); 1.7440 (2.8); 1.7294 (1.8); 1.7251 (1.7); 1.7104 (2.5); 1.6960 (1.4); -0.0001 (1.0) |
| I-098: ¹H-NMR(300.0 MHz, d₆-DMSO): |
| δ= 7.7308 (3.0); 7.7034 (3.0); 7.4648 (2.4); 7.4588 (6.0); 7.4528 (6.3); 7.4469 (2.6); 7.2887 (9.7); 7.2839 (13.1); 7.2785 (9.3); 7.2623 (0.5); 7.2538 (0.4); 6.3742 (6.3); 6.3369 (1.0); 6.3210 (8.1); 6.3092 (2.9); 6.2953 (0.9); 5.9124 (0.9); 5.9055 (1.5); 5.8986 (1.1); 5.8939 (1.3); 5.8872 (2.7); 5.8803 (2.6); 5.8736 (1.2); 5.8688 (1.3); 5.8620 (1.9); 5.8552 (1.2); 5.7292 (1.7); 5.7224 (3.0); 5.7137 (2.3); 5.7043 (2.5); 5.6978 (1.4); 4.5952 (0.7); 4.5893 (0.7); 4.5759 (0.9); 4.5701 (1.0); 4.5605 (0.9); 4.5512 (1.0); 4.5330 (0.7); 4.5257 (0.8); 3.8997 (1.6); 3.8934 (1.6); 3.8500 (3.0); 3.8438 (3.0); 3.7554 (4.7); 3.7059 (2.8); 3.6389 (22.2); 3.6281 (21.6); 3.5682 (0.6); 3.5615 (0.8); 3.5477 (1.3); 3.5414 (1.5); 3.5336 (1.6); 3.5260 (1.5); 3.5196 (1.3); 3.5064 (0.9); 3.4991 (0.6); 3.3279 (69.2); 3.0623 (0.5); 2.7277 (0.4); 2.5563 (9.8); 2.5526 (10.5); 2.5419 (10.5); 2.5382 (9.9); 2.5069 (42.1); 2.5016 (53.7); 2.4419 (0.7); 2.4364 (0.7); 2.4141 (1.3); 2.4083 (1.3); 2.3977 (1.0); 2.3915 (1.1); 2.3864 (1.0); 2.3802 (0.9); 2.3694 (1.5); 2.3637 (1.4); 2.3414 (0.7); 2.3356 (0.7); 2.2713 (0.4); 1.8194 (0.7); 1.8004 (1.2); 1.7812 (0.7); 1.7751 (0.7); 1.7556 (1.1); 1.7476 (0.8); 1.7368 (0.8); 1.7285 (1.3); 1.7093 (0.8); 1.7028 (0.7); 1.6838 (1.0); 1.6649 (0.6); 1.3453 (0.4); 1.2337 (0.3); 1.1611 (15.9); 1.1572 (16.0); 1.1260 (0.6); 0.0108 (0.7); -0.0002 (19.9); -0.0112 (0.7) |
| I-099: ¹H-NMR(400.2 MHz, d₆-DMSO): |
| δ= 7.6833 (1.0); 7.6577 (6.9); 7.6369 (6.7); 7.1689 (1.4); 7.1625 (2.5); 7.1525 (9.2); 7.1470 (12.3); 7.1306 (13.2); 7.1250 (12.7); 7.1159 (4.3); 7.1057 (6.6); 7.0999 (6.8); 7.0943 (2.9); 7.0824 (2.8); 7.0768 (3.3); 7.0712 (1.4); 5.9395 (3.0); 5.9266 (16.0); 5.8808 (5.1); 5.8756 (7.6); 5.8703 (5.6); 5.8670 (5.8); 5.8618 (7.9); 5.8567 (4.8); 5.6924 (5.1); 5.6867 (8.5); 5.6809 (5.7); 5.6789 (5.2); 5.6729 (7.3); 5.6672 (4.1); 5.6268 (0.6); 5.6211 (0.9); 5.6152 (0.6); 5.6073 (0.8); 5.6016 (0.4); 4.6354 (1.5); 4.6307 (1.7); 4.6153 (3.7); 4.6103 (3.4); 4.5961 (7.5); 4.5851 (2.2); 4.5802 (2.5); 4.5723 (6.4); 4.4792 (4.3); 4.4555 (5.8); 4.4428 (5.3); 4.4189 (4.3); 4.3225 (5.4); 4.2988 (4.3); 4.0321 (1.2); 4.0175 (7.0); 3.9949 (1.8); 3.9804 (10.8); 3.8744 (7.0); 3.8508 (1.3); 3.8372 (4.5); 3.8158 (1.2); 3.7798 (0.5); 3.6658 (0.9); 3.6344 (97.0); 3.6251 (12.8); 3.5982 (73.7); 3.5709 (0.7); 3.5476 (2.8); 3.5425 (3.0); 3.5326 (4.3); 3.5271 (5.3); 3.5216 (4.1); 3.5116 (3.0); 3.5065 (2.6); 3.4479 (0.6); 3.4116 (0.6); 3.3332 (35.0); 2.5111 (17.0); 2.5067 (33.2); 2.5022 (43.8); 2.4976 (30.9); 2.4932 (14.0); 2.4297 (0.3); 2.4088 (0.7); 2.3963 (0.5); 2.3879 (0.5); 2.3754 (0.8); 2.3627 (2.8); 2.3544 (0.8); 2.3416 (5.3); 2.3291 (4.0); 2.3205 (3.4); 2.3081 (5.5); 2.2871 (2.6); 1.7548 (0.6); 1.7275 (3.2); 1.7131 (5.5); 1.6986 (3.6); 1.6940 (3.6); 1.6796 (5.0); 1.6653 (2.8); -0.0002 (1.4) |
| I-100: ¹H-NMR(300.1 MHz, d₆-DMSO): |
| δ= 7.6762 (4.9); 7.6494 (5.1); 7.1340 (16.0); 7.1024 (12.9); 7.0770 (2.6); 7.0697 (2.4); 5.8918 (3.7); 5.8851 (5.8); 5.8779 (4.4); 5.8736 (4.6); 5.8667 (6.3); 5.8600 (3.9); 5.6305 (3.5); 5.6231 (5.8); 5.6150 (4.4); 5.6047 (5.1); 5.5972 (3.1); 4.6170 (3.0); 4.5855 (6.6); 4.5591 (2.9); 4.4631 (5.7); 4.4313 (5.9); 4.3041 (3.8); 4.2726 (2.8); 4.0388 (5.2); 3.9894 (8.4); 3.8570 (6.1); 3.8074 (3.8); 3.6247 (65.6); 3.5949 (54.2); 3.5514 (2.6); 3.5440 (2.9); 3.5314 (4.2); 3.5243 (5.2); 3.5170 (4.6); 3.5033 (4.5); 3.4963 (4.6); 3.4893 (4.2); 3.4482 (7.2); 3.3778 (1.2); 3.3473 (0.8); 2.5068 (28.7); 2.5010 (36.9); 2.4954 (26.0); 2.4427 (1.9); 2.4148 (3.8); 2.3983 (2.7); 2.3867 (2.3); 2.3704 (4.0); 2.3423 (1.9); 1.7545 (0.5); 1.7322 (2.3); 1.7128 (4.0); 1.6932 (2.7); 1.6877 (2.4); 1.6680 (3.5); 1.6489 (1.9); -0.0002 (0.8) |
| I-101: ¹H-NMR(400.2 MHz, d₆-DMSO): |
| δ= 7.7170 (1.2); 7.7082 (1.3); 7.6976 (1.3); 7.6891 (1.2); 7.0007 (1.6); 6.9736 (5.2); 6.9027 (1.5); 6.8787 (1.5); 5.8688 (4.5); 5.8645 (4.5); 4.5750 (0.6); 4.5702 (0.6); 4.5515 (0.8); 4.5465 (0.8); 4.4581 (0.7); 4.4531 (0.7); 4.4345 (0.9); 4.4296 (0.9); 4.4150 (1.0); 4.3917 (0.8); 4.2940 (1.0); 4.2714 (0.8); 4.0092 (1.1); 3.9985 (1.1); 3.9724 (1.4); 3.9618 (1.4); 3.9031 (0.8); 3.8850 (1.4); 3.8671 (1.4); 3.8492 (0.7); 3.7796 (1.2); 3.7425 (0.9); 3.7372 (0.9); 3.6028 (14.2); 3.5952 (14.0); 3.5813 (0.8); 3.5599 (10.1); 3.5554 (9.7); 3.3439 (7.8); 2.8282 (0.3); 2.8078 (1.2); 2.7875 (1.8); 2.7670 (1.3); 2.7463 (0.4); 2.5073 (2.1); 2.5029 (2.7); 2.4985 (2.0); 2.2813 (16.0); 2.0621 (0.4); 2.0429 (0.5); 2.0297 (0.5); 2.0234 (0.5); 2.0104 (0.6); 1.9986 (0.4); 1.9911 (0.5); 1.9791 (0.6); 1.9662 (0.5); 1.9601 (0.5); 1.9473 (0.6); 1.9277 (0.4); 1.8358 (0.4); 1.8239 (0.7); 1.8183 (0.6); 1.8039 (1.7); 1.7867 (2.2); 1.7720 (1.8); 1.7653 (1.6); 1.7538 (1.4); 1.7367 (0.8); 1.7201 (0.7); 1.7165 (0.7); 1.6988 (0.7); 1.6831 (0.6); 1.6668 (0.6); 1.6486 (0.5); 1.6158 (0.5); 1.5967 (0.9); 1.5798 (1.0); 1.5638 (0.9); 1.5471 (0.8); 1.5274 (0.4); 1.4942 (0.4); 1.4815 (0.7); 1.4776 (0.7); 1.4641 (0.9); 1.4571 (0.8); 1.4469 (0.8); 1.4397 (0.6); 1.4265 (0.4) |
| I-102: ¹H-NMR(300.0 MHz, d₆-DMSO): |
| δ= 7.6611 (1.7); 7.6359 (2.9); 7.6102 (1.8); 7.0146 (1.5); 7.0076 (1.8); 7.0013 (1.7); 6.9837 (5.1); 6.9763 (5.7); 6.9113 (2.5); 6.8789 (2.6); 5.8849 (2.8); 5.8785 (3.7); 5.8742 (3.5); 5.8669 (3.1); 5.8597 (3.3); 5.8555 (3.1); 5.8489 (1.8); 5.6911 (1.3); 5.6836 (2.2); 5.6758 (1.4); 5.6731 (1.3); 5.6651 (1.8); 5.6576 (1.1); 5.6252 (1.2); 5.6177 (2.0); 5.6098 (1.4); 5.5991 (1.8); 5.5916 (1.0); 4.6451 (0.4); 4.6086 (2.5); 4.5994 (1.6); 4.5938 (1.6); 4.5771 (3.3); 4.5468 (0.4); 4.5407 (0.4); 4.4526 (2.1); 4.4433 (2.5); 4.4212 (2.6); 4.4119 (2.0); 4.2827 (2.3); 4.2512 (1.8); 4.0194 (1.8); 4.0057 (1.8); 3.9704 (2.7); 3.9567 (2.7); 3.8192 (1.8); 3.7955 (1.7); 3.7702 (1.2); 3.7454 (1.1); 3.6329 (26.2); 3.6241 (25.6); 3.5642 (23.7); 3.5618 (23.0); 3.5440 (1.7); 3.5301 (2.0); 3.5227 (2.5); 3.5155 (1.9); 3.5082 (1.3); 3.5022 (1.4); 3.4952 (1.2); 3.3754 (2.1); 3.3167 (0.4); 2.5067 (16.7); 2.5013 (21.4); 2.4963 (14.2); 2.4416 (0.7); 2.4137 (1.3); 2.3971 (1.0); 2.3857 (0.9); 2.3694 (1.6); 2.3449 (1.6); 2.3285 (1.0); 2.3172 (0.9); 2.3000 (2.3); 2.2865 (16.0); 2.2797 (15.6); 2.0736 (0.8); 1.7260 (1.3); 1.7066 (2.2); 1.6829 (1.5); 1.6621 (2.0); 1.6424 (1.0); - 0.0001 (6.4) |
| I-103: ¹H-NMR(300.0 MHz, d₆-DMSO): |
| δ= 7.5733 (1.0); 7.5447 (1.1); 7.5292 (1.4); 7.4997 (1.3); 7.0279 (1.0); 7.0204 (1.2); 7.0130 (1.2); 7.0055 (1.0); 6.9952 (2.9); 6.9907 (3.2); 6.9800 (3.5); 6.9755 (3.8); 6.9062 (1.8); 6.8745 (1.8); 5.8837 (6.2); 5.8482 (5.2); 4.6265 (0.6); 4.6064 (0.7); 4.5952 (0.9); 4.5750 (1.0); 4.4707 (0.6); 4.4502 (0.9); 4.4394 (2.1); 4.4193 (1.1); 4.4083 (1.0); 4.2784 (1.3); 4.2472 (1.0); 4.0273 (1.4); 4.0134 (1.5); 3.9780 (1.8); 3.9644 (2.1); 3.7565 (1.3); 3.7490 (1.1); 3.7415 (1.0); 3.7065 (1.1); 3.6993 (1.2); 3.6758 (0.9); 3.6521 (1.2); 3.6288 (0.9); 3.6051 (0.5); 3.5638 (23.6); 3.5599 (26.1); 3.5524 (23.4); 3.3267 (13.3); 2.5129 (4.6); 2.5070 (8.4); 2.5015 (11.0); 2.4962 (7.3); 2.2835 (16.0); 2.2317 (1.2); 2.2246 (1.8); 2.2015 (2.7); 2.1958 (2.8); 2.1801 (1.4); 2.1733 (2.1); 2.0734 (0.4); 1.6455 (1.4); 1.6191 (3.0); 1.5984 (2.4); 1.5938 (2.3); 1.5731 (1.0); 0.9991 (5.1); 0.9770 (5.3); 0.9640 (6.4); 0.9418 (6.1); -0.0001 (4.2) |
| I-104: ¹H-NMR(400.2 MHz, d₆-DMSO): |
| δ= 8.0067 (1.2); 7.9859 (1.2); 7.9248 (1.2); 7.9042 (1.2); 7.0218 (1.0); 7.0169 (0.8); 7.0029 (1.3); 6.9974 (1.6); 6.9845 (2.6); 6.9794 (3.5); 6.9757 (3.3); 6.9088 (0.9); 6.8844 (1.6); 6.8571 (0.9); 6.8318 (1.5); 6.8267 (1.4); 6.8178 (1.7); 6.8144 (1.4); 6.7923 (1.6); 6.7873 (1.5); 6.7783 (1.8); 6.7751 (1.6); 5.9917 (2.4); 5.9209 (1.9); 5.8405 (1.7); 5.8365 (1.7); 5.8162 (1.7); 5.8118 (1.7); 5.8010 (1.6); 5.7970 (1.6); 5.7768 (1.5); 5.7725 (1.5); 4.6160 (0.6); 4.6027 (0.6); 4.5924 (0.8); 4.5791 (0.8); 4.4992 (0.7); 4.4860 (0.7); 4.4756 (0.9); 4.4626 (0.9); 4.4428 (1.1); 4.4313 (1.0); 4.4254 (0.9); 4.4191 (1.1); 4.4079 (1.2); 4.3910 (0.4); 4.3872 (0.4); 4.3630 (0.3); 4.3588 (0.3); 4.3456 (0.6); 4.3423 (0.5); 4.3221 (1.1); 4.3115 (1.1); 4.2986 (0.7); 4.2877 (0.7); 4.0142 (1.5); 4.0076 (1.4); 3.9772 (2.0); 3.9708 (1.8); 3.7818 (1.5); 3.7449 (1.2); 3.6580 (16.0); 3.6479 (1.1); 3.6339 (15.1); 3.5652 (10.6); 3.5537 (13.3); 3.3272 (3.0); 2.5103 (4.9); 2.5058 (10.3); 2.5012 (14.2); 2.4967 (10.4); 2.4922 (5.1); 2.2871 (9.2); 2.2733 (9.5); 2.0728 (1.5); 1.1511 (5.2); 1.1330 (9.2); 1.1147 (5.0); -0.0002 (7.3) |
| I-105: ¹H-NMR(400.1 MHz, d₆-DMSO): |
| δ= 7.6011 (2.4); 7.5796 (2.5); 7.5539 (3.5); 7.5317 (3.5); 7.1832 (0.4); 7.1772 (0.9); 7.1667 (3.9); 7.1611 (4.8); 7.1432 (9.2); 7.1266 (6.6); 7.1212 (7.1); 7.1064 (3.6); 7.1006 (3.8); 7.0975 (2.9); 7.0915 (2.8); 7.0841 (1.9); 7.0776 (1.9); 7.0739 (1.5); 7.0684 (1.3); 7.0625 (0.6); 5.9255 (13.3); 5.8780 (10.2); 4.6155 (1.4); 4.5913 (3.5); 4.5670 (2.6); 4.4987 (1.5); 4.4745 (3.6); 4.4503 (2.8); 4.4379 (3.8); 4.4142 (3.1); 4.3173 (3.9); 4.2938 (3.1); 4.0426 (3.2); 4.0232 (4.1); 4.0052 (4.1); 3.9861 (5.4); 3.8180 (3.2); 3.7952 (2.2); 3.7905 (2.3); 3.7815 (2.6); 3.7583 (1.6); 3.7531 (1.6); 3.7330 (0.4); 3.6790 (1.0); 3.6600 (2.0); 3.6442 (2.5); 3.5923 (53.9); 3.5785 (2.6); 3.5676 (35.5); 3.5521 (48.9); 3.5222 (0.9); 3.3662 (0.3); |
| 3.3322 (39.9); 3.3087 (0.3); 3.1751 (0.9); 3.1620 (0.8); 2.5114 (16.9); 2.5071 (33.6); 2.5027 (44.4); 2.4982 (31.1); 2.4939 (14.0); 2.2693 (0.6); 2.2510 (1.0); 2.2282 (3.9); 2.2116 (7.2); 2.1914 (7.4); 2.1729 (2.5); 2.1502 (0.8); 2.1319 (0.4); 1.6667 (0.5); 1.6523 (1.2); 1.6357 (4.2); 1.6169 (7.6); 1.5983 (5.1); 1.5835 (2.0); 1.5779 (1.5); 1.0990 (0.6); 1.0825 (0.5); 0.9999 (11.5); 0.9833 (11.4); 0.9578 (16.0); 0.9412 (15.6); 0.0080 (0.9); -0.0002 (20.9); -0.0085 (0.6) |
| I-106: ¹H-NMR(400.1 MHz, d₆-DMSO): |
| δ= 8.0433 (1.3); 8.0224 (1.3); 7.9705 (1.4); 7.9500 (1.4); 7.1745 (0.4); 7.1640 (1.9); 7.1586 (2.7); 7.1419 (4.3); 7.1257 (2.4); 7.1199 (2.4); 7.1108 (1.7); 7.0939 (0.9); 7.0889 (1.1); 7.0718 (0.9); 7.0662 (1.2); 7.0604 (0.6); 7.0489 (0.5); 7.0429 (0.6); 6.8285 (2.5); 6.8151 (2.6); 6.7891 (2.8); 6.7757 (2.9); 6.0386 (4.1); 5.9590 (4.1); 5.8415 (1.8); 5.8377 (1.7); 5.8176 (1.8); 5.8133 (1.8); 5.8021 (1.6); 5.7983 (1.6); 5.7782 (1.6); 5.7741 (1.6); 4.6271 (0.7); 4.6104 (0.8); 4.6035 (1.0); 4.5864 (1.0); 4.5103 (0.8); 4.4935 (0.9); 4.4869 (1.0); 4.4697 (1.3); 4.4647 (1.2); 4.4550 (1.0); 4.4407 (1.2); 4.4312 (1.0); 4.4227 (0.7); 4.4052 (0.6); 4.3884 (0.4); 4.3438 (1.4); 4.3349 (1.5); 4.3205 (1.4); 4.3111 (1.0); 4.0291 (1.6); 4.0209 (1.6); 3.9917 (2.2); 3.9840 (2.2); 3.8402 (1.7); 3.8049 (1.1); 3.6587 (16.0); 3.6483 (1.0); 3.6336 (16.7); 3.5959 (14.4); 3.5822 (14.5); 3.5719 (0.9); 3.5078 (0.7); 3.3291 (23.3); 3.3046 (0.4); 3.1738 (0.6); 3.1611 (0.6); 2.6709 (0.4); 2.6663 (0.4); 2.5106 (27.1); 2.5064 (55.1); 2.5020 (73.7); 2.4976 (52.4); 2.4934 (24.4); 2.3292 (0.5); 2.0757 (0.4); 1.2622 (0.6); 1.2441 (0.7); 1.1528 (5.6); 1.1346 (10.3); 1.1161 (6.0); 1.0989 (0.4); 0.0080 (1.6); -0.0002 (45.9); -0.0084 (1.6) |
| I-107: ¹H-NMR(300.1 MHz, d₆-DMSO): |
| δ= 8.0862 (1.0); 8.0572 (1.0); 8.0046 (1.0); 7.9770 (1.0); 7.1161 (0.7); 7.1081 (0.8); 7.0846 (1.4); 7.0767 (1.6); 7.0536 (0.8); 7.0453 (0.8); 7.0030 (1.6); 6.9972 (1.6); 6.9748 (1.8); 6.9532 (0.8); 6.9375 (1.7); 6.9319 (1.8); 6.9109 (2.4); 6.8939 (1.4); 6.8608 (1.7); 6.8417 (1.5); 6.8101 (1.0); 6.7921 (1.0); 6.6347 (3.4); 6.3813 (3.2); 6.3153 (1.0); 6.3007 (1.0); 6.2854 (0.7); 6.2714 (1.0); 6.2565 (1.0); 5.8418 (1.3); 5.8025 (1.3); 5.7938 (1.2); 5.7895 (1.3); 5.7543 (1.0); 5.7503 (1.1); 4.4595 (0.4); 4.4392 (0.6); 4.4139 (0.6); 4.3892 (0.4); 4.3502 (0.3); 4.3304 (0.6); 4.3089 (0.6); 4.2825 (0.3); 3.9070 (1.8); 3.8576 (3.1); 3.7757 (1.6); 3.7218 (2.0); 3.6710 (1.6); 3.6574 (10.6); 3.6414 (9.8); 3.3134 (41.6); 2.5648 (7.6); 2.5509 (7.8); 2.5006 (22.5); 2.0748 (0.4); 1.1682 (16.0); 1.1532 (6.4); 1.1449 (5.6); -0.0002 (0.4) |
| I-108: ¹H-NMR(300.0 MHz, d₆-DMSO): |
| δ= 7.5791 (0.9); 7.5595 (1.2); 7.5525 (1.2); 7.5325 (1.1); 6.3409 (2.3); 6.3342 (2.7); 6.3038 (2.9); 6.2984 (2.6); 6.2205 (0.7); 6.2131 (1.1); 6.2058 (0.7); 6.1890 (1.3); 6.1816 (2.1); 6.1742 (1.2); 6.1574 (0.7); 6.1500 (1.1); 6.1426 (0.6); 5.8740 (0.9); 5.8669 (1.7); 5.8592 (1.7); 5.8485 (2.0); 5.8409 (1.8); 5.8335 (0.8); 5.6921 (0.7); 5.6843 (1.1); 5.6766 (0.8); 5.6740 (0.8); 5.6659 (1.0); 5.6581 (1.3); 5.6501 (1.2); 5.6424 (0.8); 5.6396 (0.8); 5.6317 (1.0); 5.6240 (0.7); 4.8165 (0.4); 4.8112 (0.4); 4.7906 (1.0); 4.7846 (1.0); 4.7635 (1.1); 4.7436 (0.6); 4.3098 (0.7); 4.2610 (0.6); 3.6374 (2.8); 3.6225 (26.9); 3.5744 (0.7); 3.5590 (1.2); 3.5510 (1.0); 3.5270 (2.2); 3.5149 (1.7); 3.5078 (1.4); 3.4994 (1.0); 3.4124 (1.4); 3.3618 (0.8); 2.7753 (6.5); 2.7554 (7.0); 2.5129 (10.4); 2.5070 (19.6); 2.5015 (26.0); 2.4962 (17.3); 2.4269 (0.5); 2.4104 (0.5); 2.3996 (0.8); 2.3831 (1.2); 2.3719 (0.6); 2.3668 (0.7); 2.3557 (1.3); 2.3392 (0.9); 2.3284 (0.5); 2.3117 (0.5); 2.0739 (2.8); 1.7626 (0.6); 1.7542 (1.6); 1.7400 (0.9); 1.7287 (0.9); 1.7181 (0.9); 1.7065 (0.8); 1.6963 (0.9); 1.6851 (0.8); 1.6735 (0.5); 1.6617 (0.4); 1.5276 (0.5); 1.5035 (1.6); 1.4857 (16.0); -0.0002 (0.8) |
| I-109: ¹H-NMR(300.1 MHz, d₆-DMSO): |
| δ= 8.0180 (0.3); 7.9771 (2.0); 7.9501 (1.8); 7.4697 (16.0); 7.4598 (2.0); 7.4315 (0.8); 6.8329 (2.2); 6.8167 (2.1); 6.7804 (2.3); 6.7644 (2.4); 6.6679 (4.6); 6.6621 (5.0); 6.5707 (1.6); 6.5654 (2.4); 6.5596 (1.2); 6.1341 (0.5); 6.1160 (0.8); 6.0929 (0.5); 6.0679 (4.3); 6.0313 (4.7); 5.9567 (1.1); 5.8515 (0.3); 5.8291 (1.4); 5.8228 (2.6); 5.8164 (1.6); 5.7949 (0.4); 5.7770 (1.3); 5.7700 (2.3); 5.7639 (1.6); 4.7160 (0.5); 4.6850 (0.8); 4.6399 (0.6); 4.6083 (0.9); 4.5594 (0.8); 4.5405 (1.2); 4.5295 (1.4); 4.5098 (1.2); 4.5000 (1.3); 4.4847 (1.1); 4.4669 (0.9); 4.4535 (1.0); 4.3807 (0.8); 4.3507 (1.0); 4.3380 (1.4); 4.3092 (1.1); 4.2930 (0.5); 4.0271 (1.3); 3.9783 (2.0); 3.8353 (1.2); 3.7860 (0.8); 3.6585 (2.8); 3.6488 (1.4); 3.6338 (14.2); 3.6201 (12.6); 3.5930 (12.3); 3.5795 (2.9); 3.5698 (1.2); 3.4237 (0.5); 3.3997 (0.5); 3.3786 (0.8); 3.3546 (0.8); 3.3226 (35.7); 3.1848 (0.7); 3.1678 (0.8); 3.1394 (0.6); 3.1236 (0.5); 2.7279 (0.5); 2.5072 (59.5); 2.5014 (78.0); 2.4956 (55.7); 2.2706 (0.5); 1.2040 (4.4); 1.1806 (4.4); 1.1468 (5.3); 1.1232 (5.4); 1.0987 (0.7); 1.0757 (0.3); -0.0001 (50.3); -0.0110 (2.0) |
| I-110: ¹H-NMR(300.1 MHz, d₆-DMSO): |
| δ= 7.5987 (0.7); 7.5699 (0.8); 7.5527 (1.1); 7.5232 (1.1); 7.4772 (3.0); 7.4712 (4.6); 7.4549 (16.0); 5.9148 (1.2); 5.8691 (0.6); 4.6445 (0.4); 4.6177 (0.7); 4.6137 (0.7); 4.5871 (0.8); 4.4884 (0.5); 4.4727 |
| (1.3); 4.4626 (0.9); 4.4409 (1.0); 4.4318 (0.9); 4.3122 (1.2); 4.2808 (0.9); 4.0498 (0.8); 4.0305 (1.1); 3.9999 (1.2); 3.9809 (1.6); 3.8095 (1.0); 3.7726 (0.7); 3.7614 (0.8); 3.7287 (0.5); 3.7229 (0.5); 3.6867 (0.3); 3.6636 (0.7); 3.6405 (1.0); 3.6159 (0.9); 3.5883 (17.8); 3.5674 (9.9); 3.5528 (14.0); 3.3853 (1.3); 2.5068 (24.9); 2.5009 (32.7); 2.4951 (23.4); 2.2312 (1.5); 2.2198 (1.0); 2.2066 (2.6); 2.1929 (1.7); 2.1795 (1.8); 2.1687 (0.9); 1.6433 (1.0); 1.6312 (1.2); 1.6178 (2.1); 1.6081 (1.3); 1.5927 (1.6); 1.5720 (0.6); 0.9970 (3.2); 0.9747 (3.8); 0.9664 (5.1); 0.9442 (4.6); 0.0105 (0.3); -0.0002 (8.0); -0.0109 (0.4) |
| I-111: ¹H-NMR(300.1 MHz, d₆-DMSO): |
| δ= 7.6985 (1.0); 7.6736 (2.0); 7.6475 (1.1); 7.4636 (16.0); 7.4527 (7.6); 7.4475 (3.7); 5.9295 (1.1); 5.8958 (0.8); 5.8887 (1.3); 5.8777 (1.8); 5.8707 (1.9); 5.8633 (1.4); 5.8601 (1.5); 5.8529 (0.9); 5.6964 (0.7); 5.6890 (1.2); 5.6812 (0.9); 5.6706 (1.1); 5.6631 (0.7); 5.6502 (0.7); 5.6427 (1.2); 5.6348 (0.8); 5.6242 (1.0); 5.6167 (0.6); 4.6208 (1.1); 4.6090 (0.7); 4.5895 (2.0); 4.5739 (0.9); 4.5549 (0.7); 4.5461 (0.6); 4.4754 (1.4); 4.4656 (1.2); 4.4435 (1.2); 4.4339 (1.5); 4.3155 (1.3); 4.2837 (1.0); 4.0363 (1.0); 4.0145 (0.9); 3.9868 (1.5); 3.9649 (1.6); 3.8688 (1.1); 3.8451 (1.1); 3.8201 (0.7); 3.7955 (0.7); 3.6353 (13.8); 3.6234 (13.9); 3.5915 (16.5); 3.5544 (1.0); 3.5477 (1.0); 3.5339 (1.3); 3.5266 (1.6); 3.5203 (1.3); 3.5066 (1.0); 3.3924 (1.5); 2.7276 (0.3); 2.5131 (19.9); 2.5075 (40.4); 2.5016 (54.4); 2.4957 (40.1); 2.4436 (1.1); 2.4154 (1.0); 2.3985 (0.8); 2.3884 (0.8); 2.3706 (1.0); 2.3623 (0.9); 2.3447 (0.8); 2.3342 (0.6); 2.3175 (0.8); 2.2894 (0.5); 2.2774 (0.4); 2.2714 (0.4); 2.2655 (0.3); 1.7398 (0.4); 1.7205 (1.2); 1.7010 (1.2); 1.6760 (1.2); 1.6566 (1.1); 1.6367 (0.5); 1.3696 (0.9); 1.2348 (3.2); 0.8535 (0.4); 0.0107 (0.9); -0.0002 (26.6); -0.0111 (1.4) |
| I-112: ¹H-NMR(300.1 MHz, d₆-DMSO): |
| δ= 7.7369 (1.2); 7.7249 (1.1); 7.7118 (1.2); 7.6999 (1.1); 7.4496 (16.0); 5.8946 (4.5); 5.8902 (4.2); 4.6065 (0.6); 4.5745 (0.9); 4.4657 (1.2); 4.4511 (0.8); 4.4342 (1.0); 4.4188 (1.0); 4.3059 (1.1); 4.2743 (0.8); 4.0264 (1.1); 4.0194 (1.0); 3.9770 (1.7); 3.9694 (1.6); 3.8902 (0.7); 3.8650 (1.4); 3.8404 (2.3); 3.7915 (0.8); 3.7797 (0.8); 3.6041 (14.2); 3.5943 (15.4); 3.5880 (14.4); 3.3230 (32.7); 2.8162 (1.0); 2.7895 (1.7); 2.7625 (1.2); 2.7339 (0.6); 2.5071 (44.8); 2.5013 (58.8); 2.4955 (41.9); 2.2710 (0.3); 2.0761 (0.4); 2.0513 (0.4); 2.0315 (0.7); 2.0059 (1.0); 1.9822 (0.7); 1.9598 (0.6); 1.9354 (0.4); 1.8357 (0.5); 1.8089 (1.5); 1.7970 (1.4); 1.7823 (1.9); 1.7723 (1.8); 1.7584 (1.6); 1.7202 (0.9); 1.7002 (0.6); 1.6806 (0.6); 1.6572 (0.4); 1.6189 (0.8); 1.5921 (1.3); 1.5753 (0.8); 1.5666 (0.8); 1.5496 (1.1); 1.5228 (0.6); 1.5075 (0.3); 1.4843 (0.9); 1.4601 (1.1); 1.4458 (0.7); 1.4221 (0.5); 0.0106 (1.3); -0.0002 (32.4); - 0.0111 (1.3) |
| I-113: ¹H-NMR(600.3 MHz, CDCl3): |
| δ= 7.2603 (26.6); 6.9552 (0.8); 6.9410 (0.8); 6.7416 (0.4); 6.7378 (0.8); 6.7340 (0.7); 6.7259 (1.0); 6.7218 (2.1); 6.7171 (3.2); 6.7136 (2.7); 6.7058 (2.8); 6.7037 (4.0); 5.9556 (2.0); 5.9382 (2.2); 5.9273 (2.4); 5.9098 (2.2); 5.5870 (2.4); 5.5845 (2.5); 5.5586 (2.1); 5.5562 (2.1); 5.2492 (2.4); 5.2468 (2.4); 5.2318 (2.2); 5.2293 (2.4); 4.0886 (2.7); 4.0640 (4.0); 3.9750 (3.6); 3.9503 (2.4); 3.8985 (0.3); 3.8843 (0.7); 3.8736 (0.9); 3.8640 (0.7); 3.8498 (0.4); 3.7188 (26.3); 3.7074 (0.4); 3.6983 (27.0); 3.5945 (0.3); 3.4650 (0.4); 2.3947 (0.3); 2.3829 (0.4); 2.3811 (0.4); 2.3678 (1.4); 2.3540 (1.8); 2.3490 (1.3); 2.3421 (1.4); 2.3380 (1.7); 2.3354 (1.6); 2.3243 (1.4); 2.3109 (0.4); 2.3083 (0.4); 2.2973 (0.4); 2.0792 (1.6); 1.8662 (0.4); 1.8636 (0.4); 1.8618 (0.4); 1.8521 (0.9); 1.8427 (0.7); 1.8404 (1.0); 1.8384 (0.9); 1.8311 (0.7); 1.8293 (0.8); 1.8266 (0.6); 1.8175 (0.5); 1.8074 (0.6); 1.7965 (0.8); 1.7934 (1.2); 1.7825 (1.3); 1.7795 (0.7); 1.7730 (0.4); 1.7688 (0.8); 1.7591 (0.6); 1.7562 (0.4); 1.5708 (0.4); 1.5552 (50.0); 1.5354 (0.4); 1.2538 (0.8); 1.1282 (10.7); 1.1172 (10.7); 0.9303 (0.4); 0.9180 (0.7); 0.9057 (0.3); 0.0053 (1.0); - 0.0001 (29.7); -0.0057 (1.1) |
| I-114: ¹H-NMR(600.3 MHz, CDCl3): |
| δ= 7.2600 (22.7); 7.1567 (0.5); 7.1427 (0.5); 6.9148 (1.2); 6.9058 (1.2); 6.8886 (1.2); 6.8796 (1.2); 6.7327 (0.5); 6.7289 (0.3); 6.7219 (0.6); 6.7181 (1.1); 6.7143 (0.7); 6.7035 (0.5); 6.6997 (0.3); 6.6652 (1.6); 6.6617 (1.5); 6.6526 (1.7); 6.6491 (1.4); 5.9675 (0.9); 5.9500 (1.0); 5.9391 (1.1); 5.9216 (1.1); 5.9097 (1.2); 5.9070 (1.3); 5.8835 (1.2); 5.8809 (1.2); 5.6201 (1.3); 5.6179 (1.4); 5.5917 (1.1); 5.5895 (1.1); 5.2776 (1.3); 5.2753 (1.3); 5.2602 (1.2); 5.2579 (1.3); 4.6111 (0.5); 4.6088 (0.5); 4.5996 (0.5); 4.5972 (0.5); 4.2357 (0.3); 4.2277 (0.4); 4.2196 (0.3); 4.0708 (1.3); 4.0462 (2.3); 3.9843 (2.1); 3.9597 (1.2); 3.7543 (14.2); 3.7333 (13.6); 3.7234 (0.4); 3.7156 (0.4); 3.7110 (0.4); 3.7074 (0.4); 3.6995 (0.4); 3.6484 (0.4); 3.6394 (0.4); 3.5948 (0.4); 3.5860 (0.4); 3.4762 (0.3); 3.4650 (0.6); 3.4538 (0.3); 2.0794 (2.3); 1.5693 (0.4); 1.5546 (0.5); 1.5476 (50.0); 1.2815 (5.6); 1.2698 (5.6); 1.2538 (0.7); 0.9304 (0.5); 0.9180 (1.0); 0.9057 (0.5); 0.0053 (0.7); -0.0001 (23.4) |
| I-115: ¹H-NMR(400.6 MHz, CDCl3): |
| δ= 7.2614 (15.2); 6.9510 (0.8); 6.9300 (0.8); 6.7455 (0.8); 6.7399 (0.6); 6.7236 (1.4); 6.7181 (1.1); 6.7017 (1.2); 6.6935 (2.5); 6.6883 (2.1); 6.6742 (2.5); 6.6690 (1.7); 5.9837 (1.1); 5.9575 (1.2); 5.9412 (1.4); 5.9150 (1.4); 5.5976 (1.8); 5.5943 (1.7); 5.5551 (1.4); 5.5521 (1.4); 5.3003 (4.7); 5.2572 (1.7); 5.2543 (1.6); 5.2311 (1.6); 5.2280 (1.5); 4.1050 (1.5); 4.0682 (3.0); 4.0040 (2.9); 3.9671 (1.5); 3.8902 (0.6); 3.8757 (0.7); 3.8605 (0.6); 3.7159 (15.7); 3.6983 (0.8); 3.6431 (16.0); 3.4654 (0.6); 2.2937 (1.0); 2.2760 (1.6); 2.2625 (1.5); 2.2580 (1.5); 2.2421 (1.2); 2.0813 (1.7); 1.8162 (0.7); 1.7984 (0.8); 1.7825 (0.7); 1.7619 (0.6); 1.7445 (0.6); 1.7392 (0.9); 1.7232 (1.0); 1.7034 (0.6); 1.5612 (13.8); 1.1757 (6.8); 1.1591 (6.8); 0.9178 (0.9); -0.0002 (17.4) |
| I-116: ¹H-NMR(400.6 MHz, CDCl3): |
| δ= 7.2615 (22.7); 7.1331 (0.6); 7.1124 (0.6); 6.8535 (1.3); 6.8413 (1.3); 6.8142 (1.4); 6.8020 (1.4); 6.7562 (0.6); 6.7401 (0.5); 6.7343 (1.1); 6.7287 (0.8); 6.7123 (0.6); 6.6934 (1.6); 6.6879 (1.6); 6.6741 (1.8); 6.6686 (1.4); 5.9705 (1.0); 5.9444 (1.1); 5.9280 (1.4); 5.9019 (1.4); 5.8236 (1.4); 5.8192 (1.5); 5.7843 (1.4); 5.7799 (1.5); 5.6369 (1.4); 5.6334 (1.6); 5.5944 (1.2); 5.5909 (1.2); 5.2986 (1.4); 5.2951 (1.4); 5.2725 (1.2); 5.2691 (1.3); 4.1289 (1.4); 4.0920 (2.7); 4.0234 (2.6); 3.9866 (1.4); 3.7242 (15.5); 3.7120 (16.0); 1.5728 (14.0); 1.3250 (5.8); 1.3075 (5.8); 0.0080 (0.8); -0.0002 (28.2); -0.0084 (1.2) |
| I-117: ¹H-NMR(300.0 MHz, d₆-DMSO): |
| δ= 7.8284 (1.4); 7.8005 (1.6); 7.0313 (1.2); 7.0153 (1.2); 7.0010 (0.6); 6.9913 (0.8); 6.9756 (0.8); 6.8645 (1.1); 6.8487 (1.2); 6.8309 (1.2); 6.8157 (1.4); 6.8123 (1.6); 6.7962 (1.3); 6.7783 (1.3); 6.7636 (1.3); 6.6972 (0.5); 6.6295 (8.2); 6.6251 (6.7); 6.5657 (1.6); 6.5602 (2.3); 6.5543 (1.5); 6.5485 (2.1); 6.5429 (2.9); 6.2598 (0.6); 6.2373 (1.2); 6.2154 (0.8); 6.1865 (0.8); 6.1659 (0.4); 5.8857 (1.0); 5.8801 (1.0); 5.8342 (2.1); 5.8288 (2.1); 5.7823 (1.3); 5.7765 (1.3); 4.5720 (0.4); 4.5295 (0.9); 3.7094 (0.5); 3.6887 (0.6); 3.6485 (11.7); 3.6379 (19.6); 3.6136 (0.8); 3.5921 (0.6); 3.5604 (1.0); 3.5375 (1.5); 3.5146 (1.1); 3.4892 (0.8); 3.4677 (0.3); 3.3197 (19.5); 2.6071 (6.1); 2.5984 (5.8); 2.5919 (7.1); 2.5836 (5.0); 2.5072 (30.6); 2.5015 (40.4); 2.4960 (28.3); 1.5115 (16.0); 1.4801 (0.4); 1.2835 (0.4); 1.1794 (5.2); 1.1559 (5.4); 1.1354 (0.8); 1.1137 (3.3); 1.0903 (3.0); 0.0108 (0.8); -0.0002 (20.0); -0.0112 (0.6) |
| I-118: ¹H-NMR(400.1 MHz, d₆-DMSO): |
| δ= 6.4786 (1.4); 6.4623 (2.7); 6.4460 (1.5); 6.3497 (1.9); 6.3448 (2.2); 6.3222 (2.4); 6.3170 (2.5); 6.3098 (2.7); 6.3046 (2.8); 6.2821 (2.5); 6.2772 (2.4); 6.2454 (0.5); 6.2403 (0.7); 6.2165 (1.9); 6.2112 (1.6); 6.1926 (1.7); 6.1873 (2.0); 6.1687 (0.6); 6.1635 (0.8); 3.5969 (16.6); 3.5802 (0.7); 3.5195 (18.9); 3.4632 (0.8); 3.4444 (0.8); 3.4249 (1.7); 3.4063 (1.8); 3.3859 (1.4); 3.3677 (1.4); 3.3258 (24.3); 3.2977 (1.4); 3.2823 (1.5); 3.2702 (1.2); 3.2561 (1.5); 3.2449 (1.0); 3.2329 (0.9); 3.2180 (0.8); 2.8952 (7.0); 2.7909 (4.8); 2.7759 (3.8); 2.5062 (19.9); 2.5022 (26.3); 2.4984 (20.5); 2.4337 (1.2); 2.4137 (2.8); 2.3952 (2.6); 2.3804 (1.3); 2.3666 (0.9); 2.3525 (1.2); 2.3391 (1.0); 2.3295 (0.7); 2.3140 (0.6); 2.2948 (0.6); 2.2548 (0.6); 2.2418 (0.8); 2.2281 (0.8); 2.2144 (1.1); 2.2000 (0.5); 2.1863 (0.6); 2.1733 (0.5); 2.0822 (0.6); 2.0753 (2.5); 2.0666 (0.8); 2.0465 (0.8); 2.0293 (0.8); 2.0095 (0.7); 1.9967 (0.5); 1.9746 (0.8); 1.9624 (0.6); 1.9486 (0.6); 1.9355 (0.4); 1.7911 (0.5); 1.7784 (0.6); 1.7637 (0.7); 1.7525 (0.8); 1.7444 (0.6); 1.7369 (0.7); 1.7287 (0.6); 1.7169 (0.4); 1.6081 (12.1); 1.3791 (16.0); 1.3537 (8.3); 1.3386 (13.8); - 0.0002 (7.6) |
| I-119: ¹H-NMR(300.0 MHz, d₆-DMSO): |
| δ= 7.5736 (1.1); 7.5488 (1.8); 7.5241 (1.2); 6.6683 (9.0); 6.5767 (3.0); 6.5714 (4.8); 6.3192 (1.0); 6.2972 (2.1); 6.2749 (1.0); 5.8731 (0.7); 5.8658 (1.3); 5.8556 (1.9); 5.8480 (2.0); 5.8379 (1.6); 5.8309 (0.9); 5.6952 (0.8); 5.6877 (1.2); 5.6795 (1.0); 5.6680 (1.5); 5.6604 (1.6); 5.6514 (1.0); 5.6411 (1.0); 5.6338 (0.7); 4.8081 (0.4); 4.7870 (1.2); 4.7606 (1.2); 4.7396 (0.5); 3.6226 (23.5); 3.5838 (0.9); 3.5575 (1.7); 3.5340 (2.6); 3.5150 (1.6); 3.5000 (0.9); 3.4451 (0.9); 3.4321 (1.1); 3.4239 (1.2); 3.4114 (1.0); 3.3965 (0.7); 3.3835 (0.9); 3.3750 (1.0); 3.3619 (1.1); 3.3257 (105.6); 2.7660 (10.1); 2.7283 (0.7); 2.5068 (29.9); 2.5020 (38.2); 2.4236 (0.6); 2.4094 (0.6); 2.3961 (0.9); 2.3810 (1.2); 2.3668 (1.0); 2.3531 (1.2); 2.3384 (1.0); 2.3249 (0.5); 2.3110 (0.5); 1.7608 (0.5); 1.7497 (0.6); 1.7383 (0.9); 1.7266 (1.0); 1.7165 (1.0); 1.7043 (0.9); 1.6948 (0.9); 1.6830 (0.8); 1.6724 (0.5); 1.6599 (0.4); 1.4859 (16.0); -0.0001 (3.1) |
| I-120: ¹H-NMR(400.6 MHz, CDCl3): |
| δ= 7.2614 (19.0); 7.2559 (2.5); 7.2513 (1.3); 7.0072 (4.7); 7.0026 (4.6); 6.9459 (1.0); 6.9327 (1.0); 6.9066 (1.1); 6.8934 (1.1); 5.9719 (0.7); 5.9458 (0.8); 5.9315 (1.3); 5.9277 (1.5); 5.9033 (1.0); 5.8924 (1.1); 5.8882 (1.1); 5.6303 (1.1); 5.6266 (1.2); 5.5878 (0.9); 5.5841 (0.9); 5.2908 (1.1); 5.2871 (1.0); 5.2646 (1.0); 5.2610 (1.0); 4.0674 (0.9); 4.0306 (1.9); 3.9737 (1.8); 3.9368 (0.9); 3.7548 (13.2); 3.7315 (12.6); 1.5606 (16.0); 1.2847 (4.6); 1.2672 (4.7); 0.9007 (0.6); 0.8820 (0.5); 0.0079 (0.6); -0.0002 (20.9); -0.0085 (0.6) |
| I-121: ¹H-NMR(400.6 MHz, CDCl3): |
| δ= 7.2617 (17.4); 7.2551 (1.5); 7.0555 (5.2); 7.0509 (5.0); 5.9617 (0.8); 5.9356 (1.0); 5.9192 (1.2); 5.8931 (1.2); 5.5973 (1.2); 5.5935 (1.2); 5.5548 (1.0); 5.5510 (0.9); 5.2630 (1.2); 5.2592 (1.1); 5.2369 (1.1); 5.2331 (1.1); 4.0902 (1.1); 4.0534 (2.0); 3.9635 (1.8); 3.9267 (1.1); 3.7166 (13.5); 3.7009 (13.7); 2.3849 (0.6); 2.3708 (0.7); 2.3645 (0.9); 2.3539 (0.9); 2.3514 (0.8); 2.3465 (0.7); 2.3341 (0.7); 1.8458 (0.5); 1.8152 (0.7); 1.7984 (0.7); 1.5660 (16.0); 1.1301 (5.0); 1.1136 (5.0); 0.9007 (1.5); 0.0079 (0.5); - 0.0002 (19.0); -0.0085 (0.6) |
| I-122: ¹H-NMR(400.6 MHz, CDCl3): |
| δ= 7.2647 (2.0); 7.2619 (12.4); 7.2557 (1.5); 7.0522 (4.9); 7.0476 (4.6); 6.0032 (0.9); 5.9770 (1.0); 5.9606 (1.2); 5.9344 (1.2); 5.9220 (0.5); 5.9178 (0.6); 5.9160 (0.6); 5.9118 (0.6); 5.9082 (0.7); 5.9040 (0.8); 5.9023 (0.8); 5.8980 (0.6); 5.8000 (0.7); 5.7944 (1.2); 5.7887 (0.7); 5.7864 (0.6); 5.7806 (1.0); 5.7750 (0.5); 5.6059 (1.3); 5.6023 (1.3); 5.5634 (1.0); 5.5597 (1.0); 5.2649 (1.3); 5.2612 (1.2); 5.2387 (1.1); 5.2350 (1.1); 4.0964 (1.0); 4.0598 (2.2); 4.0014 (2.1); 3.9647 (1.0); 3.7464 (16.0); 3.7217 (13.5); 3.5345 (0.5); 2.5718 (0.8); 2.5583 (0.5); 2.5373 (0.8); 1.9405 (0.8); 1.9061 (0.7); 1.5759 (6.2); 0.8820 (0.5); -0.0002 (13.5) |
| I-123: ¹H-NMR(400.6 MHz, CDCl3): |
| δ= 7.2720 (1.6); 7.2674 (3.2); 7.2618 (17.3); 7.0286 (5.7); 7.0240 (5.4); 6.8520 (1.3); 6.8398 (1.3); 6.8126 (1.4); 6.8005 (1.4); 5.9690 (1.0); 5.9428 (1.1); 5.9265 (1.3); 5.9004 (1.3); 5.8206 (1.4); 5.8161 (1.4); 5.7813 (1.3); 5.7768 (1.3); 5.6382 (1.4); 5.6347 (1.4); 5.5957 (1.1); 5.5922 (1.0); 5.3050 (1.4); 5.3015 (1.3); 5.2789 (1.2); 5.2754 (1.2); 4.1166 (1.2); 4.0798 (2.3); 4.0099 (2.2); 3.9730 (1.2); 3.7210 (15.0); 3.7115 (16.0); 2.0459 (0.6); 1.5685 (11.7); 1.3403 (5.4); 1.3228 (5.5); 1.2645 (0.9); 0.9007 (2.0); 0.8820 (1.5); 0.8643 (0.6); 0.0079 (0.6); -0.0002 (18.8); -0.0085 (0.5) |
| I-124: ¹H-NMR(400.6 MHz, CDCl3): |
| δ= 7.2611 (14.0); 7.0269 (0.8); 7.0223 (0.8); 3.7125 (2.2); 3.6438 (2.3); 1.5547 (16.0); 1.1906 (0.8); 1.1740 (0.8); 0.0079 (0.5); -0.0002 (15.8) |
| I-125: ¹H-NMR(400.6 MHz, CDCl3): |
| δ= 7.2655 (1.6); 7.2628 (7.4); 7.2565 (1.4); 7.0381 (4.7); 7.0336 (4.5); 6.0014 (0.8); 5.9752 (1.0); 5.9666 (0.5); 5.9588 (1.5); 5.9528 (0.7); 5.9489 (0.8); 5.9468 (0.8); 5.9429 (0.7); 5.9326 (1.1); 5.8826 (0.6); 5.8771 (1.2); 5.8715 (0.7); 5.8691 (0.5); 5.8633 (0.9); 5.6188 (1.1); 5.6151 (1.2); 5.5762 (0.9); 5.5726 (0.9); 5.2716 (1.2); 5.2679 (1.1); 5.2454 (1.1); 5.2417 (1.1); 4.1036 (1.0); 4.0669 (2.0); 4.0072 (2.0); 3.9705 (1.0); 3.7320 (16.0); 3.7087 (12.9); 2.4957 (0.7); 2.4609 (0.8); 1.8638 (0.8); 1.8290 (0.7); 1.5963 (4.9); 0.9007 (1.5); 0.8819 (0.8); -0.0002 (7.8) |
| I-126: ¹H-NMR(400.6 MHz, CDCl3): |
| δ= 7.4604 (0.5); 7.4387 (0.5); 7.2712 (1.8); 7.2666 (3.6); 7.2613 (58.6); 7.0501 (5.9); 7.0455 (5.8); 5.9840 (1.1); 5.9712 (0.6); 5.9655 (0.9); 5.9577 (2.0); 5.9519 (1.0); 5.9474 (0.8); 5.9414 (1.5); 5.9152 (1.5); 5.8330 (0.8); 5.8274 (1.5); 5.8216 (0.8); 5.8136 (1.2); 5.8078 (0.6); 5.5943 (1.4); 5.5908 (1.5); 5.5518 (1.2); 5.5483 (1.2); 5.3005 (2.5); 5.2622 (1.5); 5.2587 (1.4); 5.2361 (1.4); 5.2325 (1.4); 4.0953 (1.4); 4.0586 (2.7); 3.9928 (2.7); 3.9561 (1.4); 3.7187 (16.0); 3.5964 (0.5); 3.5903 (0.5); 2.6294 (0.5); 2.6076 (1.0); 2.5942 (0.6); 2.5863 (0.6); 2.5726 (1.0); 2.5513 (0.5); 2.1046 (8.2); 2.0461 (1.8); 2.0020 (1.6); 1.9908 (1.8); 1.9797 (1.3); 1.9670 (1.1); 1.9559 (1.4); 1.9446 (1.0); 0.0080 (1.8); -0.0002 (61.8); - 0.0085 (1.8) |
| I-127: ¹H-NMR(400.6 MHz, CDCl3): |
| δ= 7.9082 (3.7); 7.5199 (0.6); 7.4657 (0.6); 7.4464 (0.6); 7.2745 (2.1); 7.2699 (4.2); 7.2614 (106.3); 7.0386 (6.2); 7.0340 (5.9); 6.9979 (0.5); 6.4883 (3.6); 5.9961 (0.8); 5.9875 (1.8); 5.9779 (0.8); 5.9610 (1.4); 5.9447 (1.6); 5.9184 (1.6); 5.8812 (0.8); 5.8757 (1.5); 5.8698 (0.9); 5.8618 (1.2); 5.8563 (0.6); 5.6096 (1.5); 5.6063 (1.5); 5.5671 (1.2); 5.5637 (1.2); 5.3006 (5.1); 5.2737 (1.6); 5.2703 (1.5); 5.2475 (1.4); 5.2440 (1.4); 4.1057 (1.5); 4.0690 (2.9); 4.0069 (2.9); 3.9702 (1.5); 3.7096 (16.0); 3.5617 (0.5); 2.5663 (0.5); 2.5441 (1.1); 2.5306 (0.7); 2.5223 (0.6); 2.5088 (1.2); 2.4872 (0.6); 2.1017 (13.7); 1.9238 (0.7); 1.9132 (1.2); 1.9028 (0.6); 1.8879 (0.6); 1.8776 (1.0); 1.8673 (0.6); 0.0079 (4.3); -0.0002 (124.4); -0.0085 (3.4) |
| I-128: ¹H-NMR(400.6 MHz, CDCl3): |
| δ= 7.2612 (9.8); 6.9416 (1.2); 6.9286 (1.2); 6.9024 (1.3); 6.8893 (1.3); 6.7927 (0.6); 6.7696 (0.6); 6.6952 (1.4); 6.6260 (0.7); 6.6024 (0.7); 5.9909 (0.9); 5.9647 (1.1); 5.9484 (1.3); 5.9274 (1.4); 5.9228 (2.4); 5.8881 (1.3); 5.8838 (1.3); 5.6242 (1.3); 5.6203 (1.3); 5.5817 (1.1); 5.5778 (1.0); 5.2739 (1.3); 5.2699 |
| (1.2); 5.2478 (1.2); 5.2438 (1.2); 4.0911 (1.2); 4.0544 (2.3); 3.9849 (2.0); 3.9482 (1.0); 3.7528 (16.0); 3.7115 (15.0); 2.3189 (7.9); 1.5660 (1.0); 1.2846 (5.4); 1.2672 (5.5); -0.0002 (15.0) |
| I-129: ¹H-NMR(400.6 MHz, CDCl3): |
| δ= 7.2602 (48.7); 6.7271 (0.9); 5.9808 (0.5); 5.9547 (0.6); 5.9382 (0.7); 5.9122 (0.7); 5.5927 (0.8); 5.5886 (0.8); 5.5503 (0.6); 5.5462 (0.6); 5.2469 (0.7); 5.2431 (0.7); 5.2209 (0.7); 5.2169 (0.7); 4.1046 (0.8); 4.0679 (1.3); 3.9758 (1.2); 3.9390 (0.7); 3.6966 (16.0); 2.3763 (0.5); 2.3563 (0.7); 2.3483 (0.8); 2.3343 (5.0); 1.8056 (0.6); 1.5422 (9.9); 1.1328 (3.4); 1.1162 (3.3); 0.0079 (2.5); -0.0002 (74.4); -0.0085 (2.9) |
| I-130: ¹H-NMR(400.6 MHz, CDCl3): |
| δ= 7.2610 (11.7); 6.7989 (0.6); 6.7760 (0.6); 6.7296 (1.4); 6.6715 (0.7); 6.6481 (0.7); 6.0167 (1.0); 5.9906 (1.1); 5.9742 (1.3); 5.9480 (1.3); 5.9198 (0.6); 5.9141 (0.8); 5.9095 (0.7); 5.9060 (0.8); 5.9003 (1.0); 5.8958 (0.8); 5.8013 (0.7); 5.7956 (1.3); 5.7899 (0.7); 5.7878 (0.7); 5.7819 (1.0); 5.7762 (0.6); 5.6043 (1.2); 5.6004 (1.2); 5.5617 (1.0); 5.5578 (1.0); 5.2498 (1.2); 5.2459 (1.2); 5.2237 (1.1); 5.2197 (1.1); 4.1139 (1.2); 4.0774 (2.3); 4.0075 (2.0); 3.9710 (1.1); 3.7464 (16.0); 3.7018 (13.4); 2.5586 (0.8); 2.5453 (0.5); 2.5243 (0.9); 2.3350 (8.1); 2.0457 (1.0); 1.9321 (0.9); 1.8977 (0.8); 1.5696 (1.0); 0.0080 (0.5); -0.0002 (17.7); -0.0085 (0.5) |
| I-131: ¹H-NMR(400.6 MHz, CDCl3): |
| δ= 7.2614 (8.0); 7.1674 (0.9); 6.8588 (1.3); 6.8465 (1.0); 6.8195 (1.7); 6.8073 (2.0); 6.7853 (0.9); 6.7171 (1.8); 6.6486 (1.1); 6.6311 (0.8); 5.9815 (1.1); 5.9557 (1.2); 5.9390 (1.1); 5.9129 (1.2); 5.8268 (1.5); 5.7874 (1.3); 5.7830 (1.0); 5.6357 (1.6); 5.5931 (1.2); 5.3000 (3.0); 5.2863 (1.5); 5.2601 (1.3); 4.5722 (0.8); 4.1321 (1.2); 4.0956 (2.1); 4.0181 (2.0); 3.9812 (1.1); 3.7099 (16.0); 3.7024 (11.7); 2.3339 (8.5); 1.5708 (2.2); 1.3297 (6.1); 1.3122 (4.8); 0.9009 (0.6); -0.0002 (12.3) |
| I-132: ¹H-NMR(400.6 MHz, CDCl3): |
| δ= 7.2613 (13.6); 6.9839 (1.1); 6.7968 (1.6); 6.7754 (1.3); 6.7171 (2.6); 6.6507 (1.4); 6.6316 (1.1); 5.9932 (1.5); 5.9670 (1.9); 5.9506 (1.8); 5.9247 (1.6); 5.5970 (2.0); 5.5540 (1.6); 5.3001 (1.7); 5.2467 (2.0); 5.2204 (1.6); 4.1088 (1.6); 4.0724 (2.9); 4.0005 (2.9); 3.9644 (1.6); 3.8790 (1.3); 3.6947 (15.7); 3.6413 (16.0); 2.3277 (11.5); 2.2939 (1.7); 2.2757 (2.5); 2.2655 (2.1); 1.8000 (1.1); 1.7831 (0.9); 1.7383 (1.2); 1.7220 (1.2); 1.5657 (4.0); 1.1830 (8.6); 1.1665 (6.6); 0.9009 (0.9); -0.0002 (22.4) |
| I-133: ¹H-NMR(400.6 MHz, CDCl3): |
| δ= 7.4568 (1.2); 7.2614 (9.6); 6.8011 (1.6); 6.7801 (1.2); 6.7249 (2.4); 6.6579 (1.4); 6.6333 (1.0); 6.0130 (1.2); 5.9866 (1.4); 5.9704 (2.0); 5.9448 (2.7); 5.8497 (2.2); 5.8361 (1.3); 5.6170 (1.7); 5.5746 (1.4); 5.3001 (4.1); 5.2552 (1.9); 5.2292 (1.5); 4.9102 (1.1); 4.1167 (1.4); 4.0802 (2.8); 4.0147 (2.5); 3.9786 (1.2); 3.7523 (4.0); 3.7315 (16.0); 3.7119 (3.8); 3.6912 (13.4); 3.5110 (1.4); 2.5047 (1.2); 2.4919 (1.1); 2.4704 (1.3); 2.4501 (0.5); 2.3348 (10.6); 1.8659 (1.3); 1.8310 (1.1); 1.5731 (2.8); 0.9010 (0.7); 0.0208 (3.0); -0.0002 (15.2) |
| I-134: ¹H-NMR(400.6 MHz, CDCl3): |
| δ= 7.4808 (0.6); 7.4602 (0.5); 7.2608 (27.0); 6.8043 (0.8); 6.7813 (0.8); 6.7293 (1.9); 6.6749 (0.6); 6.6701 (0.9); 6.6465 (0.9); 6.0003 (1.2); 5.9741 (1.5); 5.9633 (0.9); 5.9577 (2.6); 5.9531 (0.9); 5.9495 (1.0); 5.9442 (1.3); 5.9393 (1.0); 5.9315 (1.6); 5.8298 (0.9); 5.8241 (1.5); 5.8183 (0.9); 5.8103 (1.2); 5.8046 (0.7); 5.5963 (1.5); 5.5926 (1.5); 5.5538 (1.3); 5.5501 (1.2); 5.3002 (1.2); 5.2502 (1.6); 5.2465 (1.5); 5.2240 (1.4); 5.2202 (1.4); 4.1152 (1.5); 4.0786 (2.7); 3.9993 (2.5); 3.9626 (1.4); 3.6998 (16.0); 3.5919 (0.6); 3.5861 (0.6); 2.6029 (0.9); 2.5896 (0.6); 2.5814 (0.6); 2.5680 (1.0); 2.3371 (10.4); 2.1080 (6.4); 1.9942 (0.6); 1.9824 (1.0); 1.9705 (0.5); 1.9474 (0.9); 0.0079 (1.2); -0.0002 (41.3); -0.0085 (1.2) |
| I-135: ¹H-NMR(400.6 MHz, CDCl3): |
| δ= 7.2608 (27.7); 6.8056 (0.7); 6.7826 (0.7); 6.7256 (1.5); 6.6580 (0.7); 6.6345 (0.7); 5.9978 (1.0); 5.9836 (0.6); 5.9780 (0.8); 5.9716 (1.6); 5.9650 (0.9); 5.9596 (0.7); 5.9552 (1.4); 5.9291 (1.3); 5.8510 (0.7); 5.8453 (1.3); 5.8396 (0.8); 5.8316 (1.1); 5.8261 (0.6); 5.6079 (1.2); 5.6043 (1.3); 5.5654 (1.0); 5.5617 (1.0); 5.3002 (0.8); 5.2560 (1.3); 5.2523 (1.3); 5.2298 (1.2); 5.2261 (1.2); 4.1255 (1.3); 4.0889 (2.3); 4.0143 (2.1); 3.9777 (1.2); 3.6925 (13.9); 2.5513 (0.8); 2.5377 (0.5); 2.5160 (0.9); 2.3375 (8.4); 2.1053 (16.0); 1.9028 (0.8); 1.8677 (0.7); 0.0080 (1.2); -0.0002 (43.4); -0.0085 (1.2) |
| I-136: ¹H-NMR(400.6 MHz, CDCl3): |
| δ= 8.4090 (3.7); 8.3849 (3.8); 7.2617 (26.2); 6.8355 (1.1); 6.8302 (1.6); 6.8161 (1.5); 6.8106 (1.7); 6.8024 (1.2); 6.7955 (1.9); 6.7881 (2.0); 6.7844 (2.0); 6.7813 (2.0); 6.7758 (1.5); 6.7718 (1.9); 6.7668 (1.0); 6.7600 (0.9); 5.9450 (0.5); 5.9411 (0.6); 5.9313 (1.2); 5.9273 (1.4); 5.9251 (1.4); 5.9212 (1.3); 5.9178 (0.8); 5.9138 (0.8); 5.9113 (0.8); 5.9076 (0.7); 5.8304 (0.6); 5.8248 (1.2); 5.8189 (0.8); 5.8110 |
| (1.0); 5.8053 (0.5); 5.7057 (0.6); 5.7001 (1.1); 5.6943 (0.7); 5.6863 (1.0); 5.6807 (0.5); 5.3006 (2.8); 5.2637 (3.3); 5.0504 (3.4); 4.8615 (0.7); 4.1961 (1.1); 4.1912 (1.0); 4.1602 (2.3); 4.1553 (2.3); 4.1021 (3.8); 4.0661 (1.8); 3.7389 (16.0); 3.7356 (15.4); 3.5180 (0.8); 3.5111 (0.8); 3.5019 (0.8); 2.4893 (0.8); 2.4755 (0.6); 2.4699 (0.7); 2.4544 (1.0); 2.4492 (1.0); 2.4353 (0.7); 2.4281 (0.6); 2.4146 (0.9); 1.9125 (0.8); 1.8776 (0.7); 1.8210 (0.8); 1.7864 (0.8); 1.5680 (11.4); 1.3880 (10.4); 1.3826 (10.7); 1.2544 (0.8); 0.0080 (0.8); -0.0002 (30.1); -0.0085 (1.3) |
| I-137: ¹H-NMR(400.6 MHz, CDCl3): |
| δ= 8.3978 (4.4); 8.3900 (4.3); 7.6231 (0.8); 7.6051 (0.8); 7.2614 (39.3); 6.8216 (3.0); 6.8188 (2.8); 6.8139 (1.6); 6.8043 (3.2); 6.7916 (1.6); 6.7877 (2.0); 6.7824 (1.7); 6.7709 (0.6); 6.7657 (0.8); 6.7611 (0.8); 5.3399 (3.5); 5.3005 (3.6); 5.2579 (3.7); 4.5716 (0.9); 4.5635 (1.5); 4.5559 (1.1); 4.5484 (1.1); 4.5404 (1.6); 4.5323 (1.0); 4.4510 (0.5); 4.4404 (0.5); 4.4337 (0.6); 4.4155 (0.6); 4.4102 (0.6); 4.3976 (0.6); 4.1636 (0.9); 4.1579 (1.0); 4.1275 (2.8); 4.1218 (2.8); 4.0946 (2.7); 4.0868 (2.6); 4.0585 (1.0); 4.0508 (1.0); 4.0338 (0.9); 4.0204 (0.9); 4.0102 (1.4); 3.9970 (1.2); 3.9701 (1.0); 3.9572 (1.0); 3.9467 (1.4); 3.9339 (1.2); 3.9135 (0.9); 3.9070 (0.9); 3.8886 (0.6); 3.8836 (0.6); 3.8211 (15.0); 3.8016 (16.0); 3.7737 (0.9); 3.7694 (0.9); 2.5267 (0.5); 2.5210 (0.9); 2.5096 (0.7); 2.5035 (1.0); 2.4982 (0.6); 2.4923 (0.7); 2.4867 (1.1); 2.4809 (0.6); 2.4692 (1.0); 2.4642 (0.7); 2.4465 (0.6); 2.2268 (0.5); 2.0805 (0.7); 2.0460 (0.6); 1.9863 (0.7); 1.9523 (0.6); 1.5583 (10.9); 1.3643 (11.8); 1.3596 (12.5); 1.2839 (0.7); 1.2552 (3.1); 0.8804 (0.6); 0.0080 (1.3); -0.0002 (45.0); -0.0084 (2.0) |
| I-138: ¹H-NMR(400.6 MHz, CDCl3): |
| δ= 8.4053 (5.7); 7.2618 (28.2); 7.1656 (0.5); 7.1438 (1.0); 7.1232 (0.6); 6.8650 (1.2); 6.8524 (1.2); 6.8415 (1.3); 6.8278 (1.6); 6.8258 (1.6); 6.8130 (2.1); 6.8071 (2.2); 6.8024 (2.6); 6.7996 (2.1); 6.7949 (2.5); 6.7910 (5.3); 6.7838 (3.2); 6.7737 (3.2); 6.7684 (2.5); 6.7544 (0.8); 6.7493 (0.6); 5.8704 (1.4); 5.8660 (1.4); 5.8513 (1.4); 5.8472 (1.6); 5.8412 (3.5); 5.8311 (1.4); 5.8267 (1.4); 5.8121 (1.3); 5.8080 (1.4); 5.4186 (3.5); 5.3006 (1.3); 4.5942 (0.5); 4.5907 (0.6); 4.5734 (0.8); 4.5694 (0.6); 4.5562 (0.7); 4.5393 (0.6); 4.5354 (0.5); 4.2171 (1.2); 4.2134 (1.0); 4.1993 (4.0); 4.1951 (3.7); 4.1815 (4.1); 4.1778 (3.4); 4.1681 (1.3); 4.1637 (1.7); 4.1578 (2.8); 4.1321 (2.5); 4.1114 (2.3); 4.0935 (2.4); 4.0753 (1.0); 4.0576 (0.9); 1.5619 (11.3); 1.4018 (11.1); 1.3965 (11.4); 1.3082 (5.7); 1.2971 (8.1); 1.2908 (6.3); 1.2793 (16.0); 1.2612 (10.7); 1.2423 (6.0); 0.0080 (0.9); -0.0002 (32.6); -0.0084 (1.5) |
| I-139: ¹H-NMR(400.6 MHz, CDCl3): |
| δ= 8.3579 (4.3); 8.3348 (4.4); 7.3269 (1.3); 7.3227 (2.9); 7.3198 (2.9); 7.3155 (1.5); 7.2642 (14.7); 7.1605 (6.9); 7.1560 (6.7); 7.1210 (6.9); 7.1165 (6.6); 5.9490 (0.6); 5.9423 (1.1); 5.9385 (1.1); 5.9355 (1.2); 5.9317 (1.2); 5.9286 (1.3); 5.9248 (1.2); 5.9182 (0.7); 5.8280 (0.6); 5.8224 (1.2); 5.8166 (0.7); 5.8086 (1.0); 5.8030 (0.5); 5.7223 (0.5); 5.7167 (1.0); 5.7097 (0.6); 5.7030 (0.8); 5.3013 (7.8); 5.1861 (2.0); 4.9866 (2.1); 4.8644 (0.6); 4.8427 (0.6); 4.1998 (1.2); 4.1953 (1.2); 4.1639 (2.4); 4.1595 (2.4); 4.0923 (4.0); 4.0565 (2.1); 3.7408 (16.0); 3.7359 (15.9); 3.5245 (0.7); 3.5183 (0.8); 3.5150 (0.7); 3.5121 (0.7); 3.5091 (0.7); 2.4853 (1.3); 2.4716 (0.6); 2.4641 (1.3); 2.4505 (1.5); 2.4294 (1.4); 2.2268 (1.4); 1.9069 (0.8); 1.8719 (0.7); 1.8352 (0.8); 1.8007 (0.7); 1.6205 (2.8); 1.3886 (10.8); 1.3814 (10.7); -0.0002 (16.7); -0.0085 (0.5) |
| I-140: ¹H-NMR(400.6 MHz, CDCl3): |
| δ= 8.3418 (6.6); 8.3273 (6.4); 7.4439 (0.6); 7.4224 (0.6); 7.3732 (0.6); 7.3532 (0.6); 7.3227 (2.1); 7.3180 (4.8); 7.3128 (5.2); 7.3082 (2.4); 7.2631 (28.1); 7.1890 (9.7); 7.1845 (9.1); 7.1361 (9.4); 7.1317 (9.0); 5.5383 (3.1); 5.3010 (6.8); 5.1834 (3.2); 4.2078 (0.8); 4.1909 (1.3); 4.1847 (1.5); 4.1748 (3.4); 4.1706 (3.0); 4.1668 (3.0); 4.1571 (3.2); 4.1528 (6.5); 4.1491 (4.0); 4.1390 (6.1); 4.1351 (6.1); 4.1309 (1.5); 4.1215 (1.1); 4.1142 (3.8); 4.0936 (3.6); 4.0740 (3.6); 4.0576 (1.5); 4.0382 (1.4); 2.4907 (3.6); 2.4769 (3.2); 2.4751 (3.2); 2.4445 (3.2); 2.4423 (3.1); 2.4298 (3.2); 2.4280 (3.2); 2.2268 (1.8); 1.5974 (8.3); 1.3707 (15.8); 1.3621 (16.0); 1.2961 (6.3); 1.2784 (14.6); 1.2621 (14.0); 1.2443 (6.3); 1.2239 (8.5); 1.2070 (8.5); 1.1790 (7.9); 1.1621 (7.8); 0.0079 (1.0); -0.0002 (37.2); -0.0085 (1.1) |
| 1-141: ¹H-NMR(400.6 MHz, CDCl3): |
| δ= 8.3472 (5.1); 8.3423 (4.7); 7.6273 (0.9); 7.6078 (0.9); 7.3298 (1.4); 7.3254 (3.8); 7.3211 (4.0); 7.3168 (1.6); 7.2646 (15.0); 7.1512 (7.1); 7.1467 (7.5); 7.1448 (8.4); 7.1402 (7.4); 5.3015 (7.7); 5.2494 (2.5); 5.1851 (2.8); 4.5753 (0.9); 4.5662 (1.8); 4.5575 (1.1); 4.5521 (1.0); 4.5429 (1.9); 4.5341 (1.0); 4.4226 (0.5); 4.4162 (0.6); 4.4095 (0.5); 4.4029 (0.6); 4.3969 (0.6); 4.1655 (1.4); 4.1295 (3.4); 4.0812 (2.6); 4.0739 (2.4); 4.0453 (1.2); 4.0380 (1.1); 4.0287 (0.9); 4.0154 (0.8); 4.0051 (1.2); 3.9918 (2.0); 3.9789 (0.9); 3.9684 (1.2); 3.9555 (1.1); 3.9030 (0.9); 3.8963 (0.9); 3.8796 (0.6); 3.8731 (0.6); 3.8303 (1.0); 3.8207 (15.2); 3.8065 (0.9); 3.7988 (16.0); 2.5259 (0.6); 2.5197 (0.6); 2.5158 (0.6); 2.5092 (1.0); 2.5023 |
| (0.6); 2.4992 (0.7); 2.4919 (1.0); 2.4852 (0.7); 2.4818 (0.6); 2.4759 (0.6); 2.4679 (0.6); 2.4585 (0.5); 2.2268 (1.5); 2.0737 (0.7); 2.0392 (0.6); 1.9998 (0.6); 1.9657 (0.6); 1.6212 (3.5); 1.3639 (11.1); 1.3566 (11.8); -0.0002 (16.8); -0.0085 (0.5) |
| I-142: ¹H-NMR(400.6 MHz, CDCl3): |
| δ= 8.3572 (9.5); 7.3369 (1.8); 7.3325 (3.6); 7.3280 (2.0); 7.3183 (1.9); 7.3139 (3.8); 7.3094 (2.0); 7.2627 (32.3); 7.1764 (0.6); 7.1552 (0.6); 7.1293 (8.7); 7.1248 (8.8); 7.1213 (9.5); 7.1168 (9.3); 6.8689 (1.7); 6.8624 (1.7); 6.8554 (1.8); 6.8499 (1.7); 6.8297 (1.8); 6.8231 (1.8); 6.8163 (1.9); 6.8105 (1.9); 5.8736 (1.9); 5.8694 (2.0); 5.8667 (1.9); 5.8623 (1.8); 5.8329 (4.0); 5.8305 (3.5); 5.8274 (2.1); 5.8230 (1.8); 5.3247 (3.5); 5.3009 (7.8); 4.6033 (0.6); 4.5994 (0.6); 4.5859 (0.6); 4.5821 (0.6); 4.5389 (0.5); 4.5348 (0.5); 4.5179 (0.5); 4.2181 (1.5); 4.2122 (1.2); 4.2003 (4.9); 4.1946 (3.8); 4.1825 (5.0); 4.1768 (3.7); 4.1646 (2.0); 4.1600 (3.6); 4.1228 (2.9); 4.0991 (2.6); 4.0776 (2.8); 4.0632 (1.3); 4.0417 (1.2); 2.5529 (0.5); 2.2267 (1.5); 1.5841 (9.1); 1.4015 (13.0); 1.3904 (12.8); 1.3076 (7.2); 1.2971 (8.1); 1.2901 (7.4); 1.2793 (16.0); 1.2672 (7.4); 1.2617 (8.7); 1.2550 (2.5); 1.2497 (7.2); 0.0079 (1.1); -0.0002 (44.7); - 0.0085 (1.4) |

The present invention furthermore provides the use of one or more compounds of the general formula (I), salts or N-oxides thereof, as defined above, preferably in one of the embodiments identified as preferably, more preferably, particularly preferably or most preferably, in particular one or more compounds of the formulae (I-001) to (I-142), salts or N-oxides thereof, in each case as defined above, as herbicide and/or plant growth regulator, preferably in crops of useful plants and/or ornamental plants.

The present invention furthermore provides a method for controlling harmful plants and/or for regulating the growth of plants, characterized in that an effective amount
- of one or more compounds of the general formula (I), salts or N-oxides thereof, as defined above, preferably in one of the embodiments identified as preferred or particularly preferred, in particular one or more compounds of the formulae (I-001) to (I-142), salts or N-oxides thereof, in each case as defined above, or
- of a composition according to the invention, as defined below,
is applied to the (harmful) plants, seeds of (harmful) plants, the soil in which or on which the (harmful) plants grow or the area under cultivation.

The present invention also provides a method for controlling unwanted plants, preferably in crops of useful plants, characterized in that an effective amount
- of one or more compounds of the general formula (I), salts or N-oxides thereof, as defined above, preferably in one of the embodiments identified as preferred or particularly preferred, in particular one or more compounds of the formulae (I-001) to (I-142), salts or N-oxides thereof, in each case as defined above, or
- of a composition according to the invention, as defined below,
is applied to unwanted plants (for example harmful plants such as mono- or dicotyledonous weeds or unwanted crop plants), the seed of the unwanted plants (i.e. plant seeds, for example grains, seeds or vegetative propagation organs such as tubers or shoot parts with buds), the soil in which or on which the unwanted plants grow (for example the soil of crop land or non-crop land) or the area under cultivation (i.e. the area on which the unwanted plants will grow).

The present invention furthermore also provides methods for regulating the growth of plants, preferably of useful plants, characterized in that an effective amount
- of one or more compounds of the general formula (I), salts or N-oxides thereof, as defined above, preferably in one of the embodiments identified as preferred or particularly preferred, in particular one or more compounds of the formulae (I-001) to (I-142), salts or N-oxides thereof, in each case as defined above, or
- of a composition according to the invention, as defined below,
is applied to the plant, the seed of the plant (i.e. plant seed, for example grains, seeds or vegetative propagation organs such as tubers or shoot parts with buds), the soil in which or on which the plants grow (for example the soil of crop land or non-crop land) or the area under cultivation (i.e. the area on which the plants will grow).

In this context, the compounds according to the invention or the compositions according to the invention can be applied for example by pre-sowing (if appropriate also by incorporation into the soil), pre-emergence and/or post-emergence processes. Specific examples of some representatives of the monocotyledonous and dicotyledonous weed flora which can be controlled by the compounds according to the invention are as follows, though there is no intention to restrict the enumeration to particular species.

In a method according to the invention for controlling harmful plants or for regulating the growth of plants, one or more compounds of the general formula (I), salts or N-oxides thereof are preferably employed for controlling harmful plants or for regulating growth in crops of useful plants or ornamental plants, where in a preferred embodiment the useful plants or ornamental plants are transgenic plants.

The compounds of the general formula (I) according to the invention and/or their salts and N-oxides are suitable for controlling the following genera of monocotyledonous and dicotyledonous harmful plants:
Monocotyledonous harmful plants of the genera: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Dicotyledonous harmful plants of the genera: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

When the compounds according to the invention are applied to the soil surface before germination of the harmful plants (weed grasses and/or broad-leaved weeds) (pre-emergence method), either the seedlings of the weed grasses or broad-leaved weeds are prevented completely from emerging or they grow until they have reached the cotyledon stage, but then stop growing and eventually, after three to four weeks have elapsed, die completely.

If the active compounds are applied post-emergence to the green parts of the plants, growth stops after the treatment, and the harmful plants remain at the growth stage at the time of application, or they die completely after a certain time, so that in this manner competition by the weeds, which is harmful to the crop plants, is eliminated very early and in a sustained manner.

Although the compounds according to the invention display an outstanding herbicidal activity against monocotyledonous and dicotyledonous weeds, crop plants of economically important crops, for example dicotyledonous crops of the genera Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, or monocotyledonous crops of the genera Allium, Ananas, Asparagus, Avena, Hordeum, Oryza, Panicum, Saccharum, Secale, Sorghum, triticale, triticum, Zea, are damaged only to an insignificant extent, or not at all, depending on the structure of the respective compound according to the invention and its application rate. For these reasons, the present compounds are very suitable for selective control of unwanted plant growth in plant crops such as agriculturally useful plants or ornamental plants.

In addition, the compounds of the invention (depending on their particular structure and the application rate deployed) have outstanding growth-regulating properties in crop plants. They intervene in the plants' own metabolism with regulatory effect and can thus be used for the controlled influencing of plant constituents and to facilitate harvesting, for example by triggering desiccation and stunted growth. Furthermore, they are also suitable for the general control and inhibition of unwanted vegetative growth without killing the plants in the process. Inhibition of vegetative growth plays a major role for many mono- and dicotyledonous crops since, for example, this can reduce or completely prevent lodging.

By virtue of their herbicidal and plant growth regulatory properties, the active compounds can also be used to control harmful plants in crops of genetically modified plants or plants modified by conventional mutagenesis. In general, the transgenic plants are characterized by particular advantageous properties, for example by resistances to certain pesticides, in particular certain herbicides, resistances to plant diseases or pathogens of plant diseases, such as certain insects or microorganisms such as fungi, bacteria or viruses. Other specific characteristics relate, for example, to the harvested material with regard to quantity, quality, storability, composition and specific constituents. For instance, there are known transgenic plants with an elevated starch content or altered starch quality, or those with a different fatty acid composition in the harvested material.

It is preferred with a view to transgenic crops to use the compounds according to the invention and/or their salts and N-oxides in economically important transgenic crops of useful plants and ornamentals, for example of cereals such as wheat, barley, rye, oats, millet, rice and corn or else crops of sugar beet, cotton, soybean, oilseed rape, potato, tomato, peas and other vegetables.

It is preferred to employ the compounds according to the invention as herbicides in crops of useful plants which are resistant, or have been made resistant by recombinant means, to the phytotoxic effects of the herbicides.

By virtue of their herbicidal and plant growth regulatory properties, the active compounds can also be used to control harmful plants in crops of genetically modified plants which are known or are yet to be developed. In general, the transgenic plants are characterized by particular advantageous properties, for example by resistances to certain pesticides, in particular certain herbicides, resistances to plant diseases or pathogens of plant diseases, such as certain insects or microorganisms such as fungi, bacteria or viruses. Other specific characteristics relate, for example, to the harvested material with regard to quantity, quality, storability, composition and specific constituents. For instance, there are known transgenic plants with an elevated starch content or altered starch quality, or those with a different fatty acid composition in the harvested material. Further special properties may be tolerance or resistance to abiotic stressors, for example heat, cold, drought, salinity and ultraviolet radiation.

Preference is given to the use of the compounds of the general formula (I) according to the invention and/or their salts and N-oxides in economically important transgenic crops of useful plants and ornamental plants, for example of cereals such as wheat, barley, rye, oats, triticale, millet, rice, cassava and corn, or else crops of sugar beet, cotton, soybean, oilseed rape, potatoes, tomatoes, peas and other vegetables.

The compounds of the general formula (I) can preferably be used as herbicides in crops of useful plants which are resistant, or have been made resistant by recombinant means, to the phytotoxic effects of the herbicides.

Conventional ways of producing novel plants which have modified properties in comparison to existing plants consist, for example, in traditional cultivation methods and the generation of mutants. Alternatively, novel plants with altered properties can be generated with the aid of recombinant methods.

A large number of molecular-biological techniques by means of which novel transgenic plants with modified properties can be generated are known to the person skilled in the art. For such recombinant manipulations, nucleic acid molecules which allow mutagenesis or sequence alteration by recombination of DNA sequences can be introduced into plasmids. With the aid of standard methods, it is possible, for example, to undertake base exchanges, remove parts of sequences or add natural or synthetic sequences. To connect the DNA fragments to each other, adapters or linkers may be added to the fragments.

For example, the generation of plant cells with a reduced activity of a gene product can be achieved by expressing at least one corresponding antisense RNA, a sense RNA for achieving a cosuppression effect, or by expressing at least one suitably constructed ribozyme which specifically cleaves transcripts of the abovementioned gene product.

To this end, it is firstly possible to use DNA molecules which encompass the entire coding sequence of a gene product inclusive of any flanking sequences which may be present, and also DNA molecules which only encompass portions of the coding sequence, in which case it is necessary for these portions to be long enough to have an antisense effect in the cells. It is also possible to use DNA sequences which have a high degree of homology to the coding sequences of a gene product, but are not completely identical to them.

When expressing nucleic acid molecules in plants, the protein synthesized may be localized in any desired compartment of the plant cell. However, to achieve localization in a particular compartment, it is possible, for example, to join the coding region to DNA sequences which ensure localization in a particular compartment. Such sequences are known to those skilled in the art (see, for example, Braun et al., EMBO J. 11 (1992), 3219-3227). The nucleic acid molecules can also be expressed in the organelles of the plant cells.

The transgenic plant cells can be regenerated by known techniques to give rise to entire plants. In principle, the transgenic plants may be plants of any desired plant species, i.e. not only monocotyledonous but also dicotyledonous plants.

Thus, transgenic plants can be obtained whose properties are altered by overexpression, suppression or inhibition of homologous (= natural) genes or gene sequences or expression of heterologous (= foreign) genes or gene sequences.

It is preferred to employ the compounds (I) according to the invention in transgenic crops which are resistant to growth regulators such as, for example, dicamba, or to herbicides which inhibit essential plant enzymes, for example acetolactate synthases (ALS), EPSP synthases, glutamine synthases (GS) or hydroxyphenylpyruvate dioxygenases (HPPD), or to herbicides from the group of the sulfonylureas, glyphosate, glufosinate or benzoylisoxazoles and analogous active compounds.

When the active compounds of the invention are employed in transgenic crops, not only do the effects toward harmful plants observed in other crops occur, but frequently also effects which are specific to application in the particular transgenic crop, for example an altered or specifically widened spectrum of weeds which can be controlled, altered application rates which can be used for the application, preferably good combinability with the herbicides to which the transgenic crop is resistant, and influencing of growth and yield of the transgenic crop plants.

The invention therefore also relates to the use of the compounds of the general formula (I) according to the invention and/or their salts and N-oxides as herbicides for controlling harmful plants in crops of useful plants or ornamentals, optionally in transgenic crop plants.

Preference is given to the use in cereals, here preferably corn, wheat, barley, rye, oats, millet or rice, by the pre- or post-emergence method.

Preference is also given to the use in soybeans by the pre- or post-emergence method.

The use according to the invention for the control of harmful plants or for growth regulation of plants also includes the case in which the active compound of the general formula (I) or its salt is not formed from a precursor substance ("prodrug") until after application on the plant, in the plant or in the soil.

The invention also provides for the use of one or more compounds of the general formula (I), salts or N-oxides thereof or of a composition according to the invention (as defined below) (in a method) for controlling harmful plants or for regulating the growth of plants which comprises applying an effective amount of one or more compounds of the general formula (I), salts or N-oxides thereof onto the plants (harmful plants, if appropriate together with the useful plants), plant seeds, the soil in which or on which the plants grow or the area under cultivation.

The invention also provides a herbicidal and/or plant growth-regulating composition, characterized in that the composition comprises
(a) one or more compounds of the general formula (I), salts or N-oxides thereof, as defined above, preferably in one of the embodiments identified as preferred or particularly preferred, in particular one or more compounds of the formulae (I-001) to (I-142), salts or N-oxides thereof, in each case as defined above,and
(b) one or more further substances selected from groups (i) and/or (ii):
   (i) one or more further agrochemically active substances, preferably selected from the group consisting of insecticides, acaricides, nematicides, further herbicides (i.e. those not corresponding to the general formula (I) defined above), fungicides, safeners, fertilizers and/or further growth regulators,
   (ii) one or more formulation auxiliaries customary in crop protection.

Here, the further agrochemically active substances of component (i) of a composition according to the invention are preferably selected from the group of substances mentioned in "The Pesticide Manual", 16th edition, The British Crop Protection Council and the Royal Soc. of Chemistry, 2012.

A herbicidal or plant growth-regulating composition according to the invention comprises preferably one, two, three or more formulation auxiliaries (ii) customary in crop protection selected from the group consisting of surfactants, emulsifiers, dispersants, film-formers, thickeners, inorganic salts, dusting agents, carriers solid at 25 °C and 1013 mbar, preferably adsorbent granulated inert materials, wetting agents, antioxidants, stabilizers, buffer substances, antifoam agents, water, organic solvents, preferably organic solvents miscible with water in any ratio at 25 °C and 1013 mbar.

The compounds of general formula (I) according to the invention can be used in the form of wettable powders, emulsifiable concentrates, sprayable solutions, dusting products or granules in the customary formulations. The invention therefore also provides herbicidal and plant growth-regulating compositions which comprise compounds of the general formula (I), salts or N-oxides thereof.

The compounds of the general formula (I), salts or N-oxides thereof can be formulated in various ways according to which biological and/or physicochemical parameters are required. Possible formulations include, for example: wettable powders (WP), water-soluble powders (SP), water-soluble concentrates, emulsifiable concentrates (EC), emulsions (EW), such as oil-in-water and water-in-oil emulsions, sprayable solutions, suspension concentrates (SC), dispersions based on oil or water, oil-miscible solutions, capsule suspensions (CS), dusting products (DP), dressings, granules for scattering and soil application, granules (GR) in the form of microgranules, spray granules, absorption and adsorption granules, water-dispersible granules (WG), water-soluble granules (SG), ULV formulations, microcapsules and waxes.

These individual formulation types and the formulation assistants, such as inert materials, surfactants, solvents and further additives, are known to the person skilled in the art and are described, for example, in: Watkins, "Handbook of Insecticide Dust Diluents and Carriers", 2nd Ed., Darland Books, Caldwell N.J.; H.v. Olphen, "Introduction to Clay Colloid Chemistry", 2nd ed., J. Wiley & Sons, N.Y.; C. Marsden, "Solvents Guide", 2nd ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Athylenoxidaddukte" [Interface-active Ethylene Oxide Adducts], Wiss. Verlagsgesellschaft, Stuttgart 1976; Winnacker-Küchler, "Chemische Technologie" [Chemical Technology], volume 7, C. Hanser Verlag Munich, 4th Ed. 1986.

Wettable powders are preparations which can be dispersed uniformly in water and, in addition to the active compound, apart from a diluent or inert substance, also comprise surfactants of the ionic and/or nonionic type (wetting agents, dispersants), for example polyoxyethylated alkylphenols, polyoxyethylated fatty alcohols, polyoxyethylated fatty amines, fatty alcohol polyglycol ether sulfates, alkanesulfonates, alkylbenzenesulfonates, sodium lignosulfonate, sodium 2,2'-dinaphthylmethane-6,6'-disulfonate, sodium dibutylnaphthalenesulfonate or else sodium oleoylmethyltaurate. To produce the wettable powders, the herbicidally active compounds are finely ground, for example in customary apparatuses such as hammer mills, blower mills and air-jet mills, and simultaneously or subsequently mixed with the formulation auxiliaries.

Emulsifiable concentrates are produced by dissolving the active compound in an organic solvent, for example butanol, cyclohexanone, dimethylformamide, xylene, or else relatively high-boiling aromatics or hydrocarbons or mixtures of the organic solvents, with addition of one or more ionic and/or nonionic surfactants (emulsifiers). Examples of emulsifiers which may be used are: calcium alkylarylsulfonates such as calcium dodecylbenzenesulfonate, or nonionic emulsifiers such as fatty acid polyglycol esters, alkylaryl polyglycol ethers, fatty alcohol polyglycol ethers, propylene oxide-ethylene oxide condensation products, alkyl polyethers, sorbitan esters, for example sorbitan fatty acid esters, or polyoxyethylene sorbitan esters, for example polyoxyethylene sorbitan fatty acid esters.

Dusting products are obtained by grinding the active compound with finely distributed solids, for example talc, natural clays, such as kaolin, bentonite and pyrophyllite, or diatomaceous earth.

Suspension concentrates may be water- or oil-based. They may be prepared, for example, by wet-grinding by means of commercial bead mills and optional addition of surfactants as have, for example, already been listed above for the other formulation types.

Emulsions, for example oil-in-water emulsions (EW), can be produced, for example, by means of stirrers, colloid mills and/or static mixers using aqueous organic solvents and optionally surfactants as already listed above, for example, for the other formulation types.

Granules can be produced either by spraying the active compound onto adsorptive granular inert material or by applying active compound concentrates to the surface of carriers, such as sand, kaolinites or granular inert material, by means of adhesives, for example polyvinyl alcohol, sodium polyacrylate or else mineral oils. Suitable active compounds can also be granulated in the manner customary for the production of fertilizer granules - if desired as a mixture with fertilizers.

Water-dispersible granules are produced generally by the customary processes such as spray-drying, fluidized-bed granulation, pan granulation, mixing with high-speed mixers and extrusion without solid inert material.

For the production of pan, fluidized-bed, extruder and spray granules, see e.g. processes in "Spray Drying Handbook" 3rd Ed. 1979, G. Goodwin Ltd., London, J.E. Browning, "Agglomeration", Chemical and Engineering 1967, pages 147 ff; "Perry's Chemical Engineer's Handbook", 5th Ed., McGraw Hill, New York 1973, p. 8-57.

For further details regarding the formulation of crop protection compositions, see, for example, G.C. Klingman, "Weed Control as a Science", John Wiley and Sons, Inc., New York, 1961, pages 81-96 and J.D. Freyer, S.A. Evans, "Weed Control Handbook", 5th Ed., Blackwell Scientific Publications, Oxford, 1968, pages 101-103.

The agrochemical preparations, preferably herbicidal or plant growth-regulating compositions, of the present invention preferably comprise a total amount of from 0.1 to 99% by weight, preferably 0.5 to 95% by weight, particularly preferably 1 to 90% by weight, especially preferably 2 to 80% by weight, of active compounds of the general formula (I), salts or N-oxides thereof.

In wettable powders, the active compound concentration is, for example, about 10 to 90% by weight, the remainder to 100% by weight consisting of customary formulation constituents. In emulsifiable concentrates, the active compound concentration may be about 1% to 90% and preferably 5% to 80% by weight. Formulations in the form of dusts comprise 1% to 30% by weight of active compound, preferably usually 5% to 20% by weight of active compound; sprayable solutions contain about 0.05% to 80% by weight, preferably 2% to 50% by weight of active compound. In the case of water-dispersible granules, the active compound content depends partially on whether the active compound is in liquid or solid form and on which granulation auxiliaries, fillers, etc., are used. In the water-dispersible granules, the content of active compound is, for example, between 1% and 95% by weight, preferably between 10% and 80% by weight.

In addition, the active compound formulations mentioned optionally comprise the respective customary stickers, wetters, dispersants, emulsifiers, penetrants, preservatives, antifreeze agents and solvents, fillers, carriers and dyes, defoamers, evaporation inhibitors and agents which influence the pH and the viscosity. Examples of formulation auxiliaries are described inter alia in "Chemistry and Technology of Agrochemical Formulations", ed. D.A. Knowles, Kluwer Academic Publishers (1998).

The compounds of the general formula (I), salts or N-oxides thereof can be used as such or in the form of their preparations (formulations) in a combination with other pesticidally active substances, for example insecticides, acaricides, nematicides, herbicides, fungicides, safeners, fertilizers and/or growth regulators, for example in the form of a finished formulation or of a tank mix. The combination formulations can be prepared on the basis of the abovementioned formulations, while taking account of the physical properties and stabilities of the active compounds to be combined.

Active compounds which can be employed in combination with the compounds of general formula (I) according to the invention in mixture formulations or in a tank mix are, for example, known active compounds based on inhibition of, for example, acetolactate synthase, acetyl-CoA carboxylase, cellulose synthase, enolpyruvylshikimate-3-phosphate synthase, glutamine synthetase, p-hydroxyphenylpyruvate dioxygenase, phytoendesaturase, photosystem I, photosystem II, protoporphyrinogen oxidase, as described, for example, in Weed Research 26 (1986) 441-445 or "The Pesticide Manual", 16th edition, The British Crop Protection Council and the Royal Soc. of Chemistry, 2012 and literature cited therein.

Of particular interest is the selective control of harmful plants in crops of useful plants and ornamentals. Although the compounds of genarl formula (I) according to the invention have already demonstrated very good to adequate selectivity in a large number of crops, in principle, in some crops and in particular also in the case of mixtures with other, less selective herbicides, phytotoxicities on the crop plants may occur. In this connection, combinations of compounds of general formula (I) according to the invention are of particular interest which comprise the compounds of general formula (I) or their combinations with other herbicides or pesticides and safeners. The safeners, which are used in an antidotically effective amount, reduce the phytotoxic side effects of the herbicides/pesticides employed, for example in economically important crops, such as cereals (wheat, barley, rye, corn, rice, millet), sugarbeet, sugarcane, oilseed rape, cotton and soybeans, preferably cereals.

The weight ratios of herbicide (mixture) to safener depend generally on the herbicide application rate and the efficacy of the safener in question and may vary within wide limits, for example in the range from 200:1 to 1:200, preferably 100:1 to 1:100, in particular 20:1 to 1:20. Analogously to the compounds (I) or mixtures thereof, the safeners can be formulated with further herbicides/pesticides and be provided and employed as a finished formulation or tank mix with the herbicides.

For application, the herbicide or herbicide/safener formulations present in commercial form are, if appropriate, diluted in a customary manner, for example in the case of wettable powders, emulsifiable concentrates, dispersions and water-dispersible granules with water. Dust-type preparations, granules for soil application or granules for scattering and sprayable solutions are not normally diluted further with other inert substances prior to application.

The application rate of the compounds of the general formula (I), salts or N-oxides thereof is affected to a certain extent by external conditions such as temperature, humidity, etc. Here, the application rate may vary within wide limits. For the application as a herbicide for controlling harmful plants, the total amount of compounds of the general formula (I), salts or N-oxides thereof are preferably in the range from 0.001 to 10.0 kg/ha, with preference in the range from 0.005 to 5 kg/ha, more preferably in the range from 0.01 to 1.5 kg/ha, in particular preferably in the range from 0.05 to 1 kg/ha. This applies both to the pre-emergence and the post-emergence application.

When the compounds of the general formula (I), salts or N-oxides thereof are used as plant growth regulators, for example as culm stabilizer for crop plants like those mentioned above, preferably cereal plants, such as wheat, barley, rye, triticale, millet, rice or corn, the total application rate is preferably in the range of from 0.001 to 2 kg/ha, preferably in the range of from 0.005 to 1 kg/ha, in particular in the range of from 10 to 500 g/ha, very particularly in the range from 20 to 250 g/ha. This applies both to the pre-emergence and the post-emergence application.

The application as culm stabilizer may take place at various stages of the growth of the plants. Preferred is, for example, the application after the tilling phase, at the beginning of the longitudinal growth.

As an alternative, application as plant growth regulator is also possible by treating the seed, which includes various techniques for dressing and coating seed. Here, the application rate depends on the particular techniques and can be determined in preliminary tests.

Active compounds which can be employed in combination with the compounds of the general formula (I) according to the invention in compositions according to the invention (for example in mixed formulations or in the tank mix) are, for example, known active compounds which are based on the inhibition of, for example, acetolactate synthase, acetyl-CoA carboxylase, cellulose synthase, enolpyruvylshikimate-3-phosphate synthase, glutamine synthetase, p-hydroxyphenylpyruvate dioxygenase, phytoene desaturase, photosystem I, photosystem II, protoporphyrinogen oxidase, as are described in, for example, Weed Research 26 (1986) 441-445 or "The Pesticide Manual", 16th edition, The British Crop Protection Council and the Royal Soc. of Chemistry, 2012 and the literature cited therein. Known herbicides or plant growth regulators which can be combined with the compounds according to the invention are, for example, the following active compounds, where the compounds are designated either with the "common name" in accordance with the International Organization for Standardization (ISO) or with the chemical name or with the code number. They always encompass all of the application forms such as, for example, acids, salts, esters and also all isomeric forms such as stereoisomers and optical isomers, even if not explicitly mentioned.

Acetochlor, Acifluorfen, Acifluorfen-methyl, Acifluorfen-Sodium, Aclonifen, Alachlor, Allidochlor, Alloxydim, Alloxydim-Sodium, Ametryn, Amicarbazone, Amidochlor, Amidosulfuron, 4-Amino-3-chloro-6-(4-chloro-2-fluoro-3-methylphenyl)-5-fluoropyridin-2-carboxylic acid, Aminocyclopyrachlor, Aminocyclopyrachlor-potassium, Aminocyclopyrachlor-methyl, Aminopyralid, Aminopyralid-dimethylammonium, Aminopyralid-tripromine, Amitrol, Ammoniumsulfamate, Anilofos, Asulam, Asulam-potassium, Asulam-sodium, Atrazin, Azafenidin, Azimsulfuron, Beflubutamide, (S)-(-)-Beflubutamide, Beflubutamide-M, Benazolin, Benazolin-ethyl, Benazolin-dimethylammonium, Benazolin-Potassium, Benfluralin, Benfuresate, Bensulfuron, Bensulfuron-methyl, Bensulid, Bentazone, Bentazone-Sodium, Benzobicyclon, Benzofenap, Bicyclopyrone, Bifenox, Bilanafos, Bilanafos-Sodium, Bipyrazone, Bispyribac, Bispyribac-Natium, Bixlozone, Bromacil, Bromacil-lithium, Bromacil-Sodium, Bromobutid, Bromofenoxim, Bromoxynil, Bromoxynilbutyrate, Bromoxynil-Potassium, Bromoxynil-heptanoate and Bromoxynil-octanoate, Busoxinone, Butachlor, Butafenacil, Butamifos, Butenachlor, Butralin, Butroxydim, Butylate, Cafenstrole, Cambendichlor, Carbetamide, Carfentrazone, Carfentrazone-Ethyl, Chloramben, Chloramben-ammonium, Chloramben-diolamin, Chlroamben-methyl, Chloramben-methylammonium, Chloramben-Sodium, Chlorbromuron, Chlorfenac, Chlorfenac-ammonium, Chlorfenac-Sodium, Chlorfenprop, Chlorfenprop-methyl, Chlorflurenol, Chlorflurenol-methyl, Chloridazon, Chlorimuron, Chlorimuron-ethyl, Chlorophthalim, Chlorotolurone, Chlorsulfuron, Chlorthal, Chlorthal-dimethyl, Chlorthal-monomethyl, Cinflubrolin, (1S,2R,4R)- 2-[(2-Bromo-6-fluorophenyl)methoxy]-1-methyl-4-(1-methylethyl)-7-oxabicyclo[2.2.1]heptan, (1R,2S,4S)- 2-[(2-Bromo-6-fluorophenyl)methoxy]-1-methyl-4-(1-methylethyl)-7-oxabicyclo[2.2.1]heptane, Cinidon, Cinidon-ethyl, Cinmethylin, exo-(+)-Cinmethylin, i.e. (1R,2S,4S)-4-isopropyl-1-methyl-2-[(2-methylbenzyl)oxy]-7-oxabicyclo[2.2.1]heptan, exo-(-)-Cinmethylin, i.e. (1R,2S,4S)-4-isopropyl-1-methyl-2-[(2-methylbenzyl)oxy]-7-oxabicyclo[2.2.1]heptan, Cinosulfuron, Clacyfos, Clethodim, Clodinafop, Clodinafop-ethyl, Clodinafop-propargyl, Clomazone, Clomeprop, Clopyralid, Clopyralid-methyl, Clopyralid-olamin, Clopyralid-Potassium, Clopyralid-tripomin, Cloransulam, Cloransulam-methyl, Cumylurone, Cyanamide, Cyanazine, Cycloate, Cyclopyranil, Cyclopyrimorat, Cyclosulfamuron, Cycloxydim, Cyhalofop, Cyhalofop-butyl, Cyprazin, 2,4-D (including the Ammonium, Butotyl, Butyl, Choline, Diethylammonium, Dimethylammonium, Diolamin, Doboxyl, Dodecylammonium, Etexyl, Ethyl, 2-Ethylhexyl, Heptylammonium, Isobutyl, Isooctyl, Isopropyl, Isopropylammonium, Lithium, Meptyl, Methyl, Potassium, Sodium, Tetradecylammonium, Triethylammonium, Triisopropanol-ammonium, Tripromin and Trolamin salts thereof), 2,4-DB, 2,4-DB-butyl, 2,4-DB-Dimethylammonium, 2,4-DB-isooctyl, 2,4-DB-Potassium and 2,4-DB-Sodium, Daimurone (Dymron), Dalapon, Dalapon-Calcium, Dalapon-Magnesium, Dalapon-Natium, Dazomet, Dazomet-Sodium, n-Decanol, 7-Deoxy-D-sedoheptulose, Desmedipham, Detosyl-pyrazolat (DTP), Dicamba and its salts (e.g. Dicamba-biproamine, Dicamba-N,N-Bis(3-aminopropyl)methylamine, Dicamba-butotyl, Dicamba-choline, Dicamba-Diglycolamine, Dicamba-Dimethylammonium, Dicamba-Diethanolaminemmonium, Dicamba-Diethylammonium, Dicamba-isopropylammonium, Dicamba-methyl, Dicamba-monoethanolamine, Dicamba-olamine, Dicamba-Potassium, Dicamba-Sodium, Dicamba-Triethanolamin), Dichlobenil, 2-(2,4-Dichlorobenzyl)-4,4-dimethyl-1,2-oxazolidin-3-one, 2-(2,5-Dichlorobenzyl)-4,4-dimethyl-1,2-oxazolidin-3-one, Dichlorprop, Dichlorprop-butotyl, Dichlorprop-Dimethylammonium, Dichhlorpropetexyl, Dichlorprop-ethylammonium, Dichlorprop-isoctyl, Dichlorprop-methyl, Dichlorprop-Potassium, Dichlorprop-Sodium, Dichlorprop-P, Dichlorprop-P-Dimethylammonium, Dichlorprop-P-etexyl, Dichlorprop-P-Potassium, Dichlorprop-Sodium, Diclofop, Diclofop-methyl, Diclofop-P, Diclofop-P-methyl, Diclosulam, Difenzoquat, Difenzoquat-metilsulfate, Diflufenican, Diflufenzopyr, Diflufenzopyr-Sodium, Dimefuron, Dimepiperate, Dimesulfazet, Dimethachlor, Dimethametryn, Dimethenamid, Dimethenamid-P, Dimetrasulfuron, Dinitramine, Dinoterb, Dinoterb-Acetate, Diphenamid, Diquat, Diquat-Dibromide, Diquat-Dichloride, Dithiopyr, Diuron, DNOC, DNOC-Ammonium, DNOC-Potassium, DNOC-Sodium, Endothal, Endothal-Diammonium, Endothal-Dipotassium, Endothal-Disodium, Epyrifenacil, EPTC, Esprocarb, Ethalfluralin, Ethametsulfuron, Ethametsulfuron-Methyl, Ethiozin, Ethofumesate, Ethoxyfen, Ethoxyfen-Ethyl, Ethoxysulfuron, Etobenzanid, F-5231, d.h. N-[2-Chloro-4-fluoro-5-[4-(3-fluoropropyl)-4,5-dihydro-5-oxo-1H-tetrazol-1-yl]-phenyl]-ethansulfonamide, F-7967, i.e. 3-[7-Chloro-5-fluoro-2-(trifluoromethyl)-1H-benzimidazol-4-yl]-1-methyl-6-(trifluoromethyl)pyrimidin-2,4(1H,3H)-dione, Fenoxaprop, Fenoxaprop-P, Fenoxaprop-Ethyl, Fenoxaprop-P-Ethyl, Fenoxasulfone, Fenpyrazone, Fenquinotrione, Fentrazamide, Feproxydim, Flamprop, Flamprop-Isoproyl, Flamprop-Methyl, Flamprop-M-Isopropyl, Flamprop-M-Methyl, Flazasulfuron, Florasulam, Florpyrauxifen, Florpyrauxifen-benzyl, Fluazifop, Fluazifop-Butyl, Fluazifop-Methyl, Fluazifop-P, Fluazifop-P-Butyl, Flucarbazone, Flucarbazone-Sodium, Flucetosulfuron, Fluchloralin, Fluchloraminopyr, Fluchloraminopyr-tefuryl, Flufenacet, Flufenpyr, Flufenpyr-Ethyl, Flumetsulam, Flumiclorac, Flumiclorac-Pentyl, Flumioxazin, Fluometuron, Flurenol, Flurenol-Butyl, -Dimethylammonium and -Methyl, Fluoroglycofen, Fluoroglycofen-Ethyl, Flupropanat, Flupropanat-Sodium, Flupyrsulfuron, Flupyrsulfuron-Methyl, Flupyrsulfuron-Methyl-Sodium, Fluridon, Flurochloridon, Fluroxypyr, Fluroxypyr-Butometyl, Fluroxypyr-Meptyl, Flurtamon, Fluthiacet, Fluthiacet-Methyl, Fomesafen, Fomesafen-Sodium, Foramsulfuron, Foramsulfuron-Sodium, Fosamine, Fosamine-Ammonium, Glufosinate, Glufosinate-Ammonium, Glufosinate-Sodium, L-Glufosinate-Ammonium, L-Glufosinate-Sodium, Glufosinate-P-Sodium, Glufosinate-P-Ammonium, Glyphosate, Glyphosate-Ammonium, Glyphosate-Isopropylammonium, Glyphosate-Diammonium, Glyphosate-Dimethylammonium, Glyphosate-Potassium, Glyphosate-Sodium, Glyphosate-Sesquisodium and Glyphosate-Trimesium, H-9201, i.e. O-(2,4-Dimethyl-6-nitrophenyl)-O-ethyl-isopropylphosphoramidothioate, Halauxifen, Halauxifen-methyl, Halosafen, Halosulfuron, Halosulfuron-Methyl, Haloxyfop, Haloxyfop-P, Haloxyfop-Ethoxyethyl, Haloxyfop-P-Ethoxyethyl, Haloxyfop-Methyl, Haloxyfop-P-Methyl, Haloxifop-Sodium, Hexazinon, HNPC-A8169, i.e. Prop-2-yn-1-yl (2S)-2-{3-[(5-tert-butylpyridin-2-yl)oxy]phenoxy}propanoate, HW-02, i.e. 1-(Dimethoxyphosphoryl)-ethyl-(2,4-dichlorophenoxy)acetate, Hydantocidine, Icafolin, Icafolin-Methyl, Imazamethabenz, Imazamethabenz-Methyl, Imazamox, Imazamox-Ammonium, Imazapic, Imazapic-Ammonium, Imazapyr, Imazapyr-Isopropylammonium, Imazaquin, Imazaquin-Ammonium, Imazaquin-Methyl, Imazethapyr, Imazethapyr-Ammonium, Imazosulfuron, Indanofan, Indaziflam, Indolauxipyr, Iodosulfuron, Iodosulfuron-Methyl, Iodosulfuron-Methyl-Sodium, Ioxynil, Ioxynil-Lithium, -Octanoate, -Potassium and Sodium, Ipfencarbazone, Iptriazopyrid, i.e. 3-[(Isopropylsulfonyl)methyl]-N-(5-methyl-1,3,4-oxadiazol-2-yl)-5-(trifluoromethyl)[1,2,4]triazolo-[4,3-a]pyridin-8-carboxamide, Isoproturon, Isouron, Isoxaben, Isoxaflutole, Karbutilate, KUH-043, i.e. 3-({[5-(Difluoromethyl)-1-methyl-3-(trifluoromethyl)-1H-pyrazol-4-yl]methyl } sulfonyl)-5,5-dimethyl-4,5-dihydro-1,2-oxazole, Ketospiradox, Ketospiradox-Potassium, Lactofen, Lenacil, Linuron, MCPA, MCPA-Butotyl, -Butyl, - Dimethylammonium, -Diolamin, -2-Ethylhexyl, -Ethyl, -Isobutyl, Isoctyl, -Isopropyl, - Isopropylammonium, -Methyl, Olamin, -Potassium, -Sodium and -Trolamin, MCPB, MCPB-Methyl, -Ethyl and -Sodium, Mecoprop, Mecoprop-Butotyl, Mecoprop- dimethylammonium, Mecoprop-Diolamin, Mecoprop-Etexyl, Mecoprop-Ethadyl, Mecoprop-Isoctyl, Mecoprop-Methyl, Mecoprop-Potassium, Mecoprop-Sodium, and Mecoprop-Trolamin, Mecoprop-P, Mecoprop-P-Butotyl, - Dimethylammonium, -2-Ethylhexyl and -Potassium, Mefenacet, Mefluidid, Mefluidid-Diolamine, Mefluidid-Potassium, Mesosulfuron, Mesosulfuron-Methyl, Mesosulfuron-Sodium, Mesotrion, Methabenzthiazuron, Metam, Metamifop, Metamitron, Metazachlor, Metazosulfuron, Methabenzthiazuron, Methiopyrsulfuron, Methiozoline, Methyl isothiocyanate, Metobromuron, Metolachlor, S-Metolachlor, Metosulam, Metoxuron, Metproxybicyclone, Metribuzine, Metsulfuron, Metsulfuron-Methyl, Molinat, Monolinuron, Monosulfuron, Monosulfuron-Methyl, MT-5950, i.e. N-[3-Chloro-4-(1-methylethyl)-phenyl]-2-methylpentanamide, NGGC-011, Napropamid, NC-310, i.e. 4-(2,4-Dichlorobenzoyl)-1-methyl-5-benzyloxypyrazole, Neburone, Nicosulfuron, Nonanoic acid (Pelargonic acid), Norflurazone, Orbencarb, Orthosulfamuron, Oryzalin, Oxadiargyl, Oxadiazone, Oxasulfuron, Oxaziclomefone, Oxyfluorfen, Paraquat, Paraquat-dichloride, Paraquat-Dimethylsulfate, Pebulat, Pendimethaline, Penoxsulam, Pentachlorphenol, Pentoxazone, Pethoxamid, Petroleum oil, Phenmedipham, Phenmedipham-Ethyl, Picloram, Picloram-dimethylammonium, Picloram-Etexyl, Picloram-Isoctyl, Picloram-Methyl, Picloram-Olamin, Picloram-Potassium, Picloram-Triethylammonium, Picloram-Tripromin, Picloram-Trolamin, Picolinafen, Pinoxaden, Piperophos, Pretilachlor, Primisulfuron, Primisulfuron-Methyl, Prodiamine, Profoxydim, Prometon, Prometryn, Propachlor, Propanil, Propaquizafop, Propazine, Propham, Propisochlor, Propoxycarbazone, Propoxycarbazone-Sodium, Propyrisulfuron, Propyzamid, Prosulfocarb, Prosulfuron, Pyraclonil, Pyraflufen, Pyraflufen-Ethyl, Pyraquinate, Pyrasulfotol, Pyrazolynat (Pyrazolat), Pyrazosulfuron, Pyrazosulfuron-Ethyl, Pyrazoxyfen, Pyribambenz, Pyribambenz-Isopropyl, Pyribambenz-Propyl, Pyribenzoxim, Pyributicarb, Pyridafol, Pyridat, Pyriftalid, Pyriminobac, Pyriminobac-Methyl, Pyrimisulfan, Pyrithiobac, Pyrithiobac-Sodium, Pyroxasulfon, Pyroxsulam, Quinclorac, Quinclorac-Dimethylammonium, Quinclorac-Methyl, Quinmerac, Quinoclamin, Quizalofop, Quizalofop-Ethyl, Quizalofop-P, Quizalofop-P-Ethyl, Quizalofop-P-Tefuryl, QYM201, i.e. 1-{2-Chloro-3-[(3-cyclopropyl-5-hydroxy-1-methyl-1H-pyrazol-4-yl)carbonyl]-6-(trifluoromethyl)phenyl}piperidin-2-on, Rimisoxafen, Rimsulfuron, Saflufenacil, Sethoxydim, Siduron, Simazine, Simetryn, SL-261, Sulcotrione, Sulfentrazone, Sulfometuron, Sulfometuron-Methyl, Sulfosulfuron, SYP-249, i.e. 1-Ethoxy-3-methyl-1-oxobut-3-en-2-yl-5-[2-chloro-4-(trifluoromethyl)phenoxy]-2-nitrobenzoate, SYP-300, i.e. 1-[7-Fluoro-3-oxo-4-(prop-2-yn-1-yl)-3,4-dihydro-2H-1,4-benzoxazin-6-yl]-3-propyl-2-thioxoimidazolidin-4,5-dione, 2,3,6-TBA, TCA (Trichloroacetic acid) and its salts, z.B. TCA-ammonium, TCA-Calcium, TCA-Ethyl, TCA-Magnesium, TCA-Sodium, Tebuthiuron, Tefuryltrione, Tembotrione, Tepraloxydim, Terbacil, Terbucarb, Terbumeton, Terbuthylazine, Terbutryn, Tetflupyrolimet, Thaxtomin, Thenylchlor, Thiazopyr, Thiencarbazone, Thiencarbazone-Methyl, Thifensulfuron, Thifensulfuron-Methyl, Thiobencarb, Tiafenacil, Tolpyralat, Topramezone, Tralkoxydim, Triafamon, Tri-allate, Triasulfuron, Triaziflam, Tribenuron, Tribenuron-Methyl, Triclopyr, Triclopyr-Butotyl, Triclopyr-Choline, Triclopyr-Ethyl, Triclopyr-Triethylammonium, Trietazine, Trifloxysulfuron, Trifloxysulfuron-Sodium, Trifludimoxazin, Trifluralin, Triflusulfuron, Triflusulfuron-Methyl, Tritosulfuron, Harnstoffsulfat, Vernolat, XDE-848, ZJ-0862, i.e. 3,4-Dichloro-N-{2-[(4,6-dimethoxypyrimidin-2-yl)oxy]benzyl}aniline, Methyl 3-(2-Chloro-4-fluoro-5-(3-methyl-2,6-dioxo-4-trifluoromethyl-3,6-dihydropyrimidin-1(2H)-yl)phenyl)-5-methyl-4,5-dihydroisoxazol-5-carboxylate, Ethyl 3-(2-Chloro-4-fluoro-5-(3-methyl-2,6-dioxo-4-trifluoromethyl-3,6-dihydropyrimidin-1(2H)-yl)phenyl)-5-methyl-4,5-dihydroisoxazol-5-carboxylate, 3-(2-Chloro-4-fluoro-5-(3-methyl-2,6-dioxo-4-trifluoromethyl-3,6-dihydropyrimidin-1(2H)-yl)phenyl)-5-methyl-4,5-dihydroisoxazol-5-carboxylic acid, Ethyl-[(3-{2-chloro-4-fluoro-5-[3-methyl-2,6-dioxo-4-(trifluoromethyl)-3,6-dihydropyrimidin-1(2H)-yl]phenoxy}pyridin-2-yl)oxy]acetate, 3-Chloro-2-[3-(difluoromethyl)isoxazolyl-5-yl]phenyl-5-chloropyrimidin-2-ylether, 2-(3,4-Dimethoxyphenyl)-4-[(2-hydroxy-6-oxocyclohex-1-en-1-yl)carbonyl]-6-methylpyridazin-3(2H)-one, 2-({2-[(2-Methoxyethoxy)methyl]-6-methylpyridin-3-yl}carbonyl)cyclohexane-1,3-dione, (5-Hydroxy-1-methyl-1H-pyrazol-4-yl)(3,3,4-trimethyl-1,1-dioxido-2,3-dihydro-1-benzothiophen-5-yl)methanone, 1-Methyl-4-[(3,3,4-trimethyl-1,1-dioxido-2,3-dihydro-1-benzothiophen-5-yl)carbonyl]-1H-pyrazol-5-yl propan-1-sulfonate, 4-{2-Chloro-3-[(3,5-dimethyl-1H-pyrazol-1-yl)methyl]-4-(methylsulfonyl)benzoyl}-1-methyl-1H-pyrazol-5-yl-1,3-dimethyl-1H-pyrazol-4-carboxylate; Cyanomethyl-4-amino-3-chloro-5-fluoro-6-(7-fluoro-1H-indol-6-yl)pyridin-2-carboxylate, Prop-2-yn-1-yl 4-amino-3-chloro-5-fluoro-6-(7-fluoro-1H-indol-6-yl)pyridin-2-carboxylate, Methyl-4-amino-3-chloro-5-fluoro-6-(7-fluoro-1H-indol-6-yl)pyridin-2-carboxylate, Benzyl-4-amino-3-chloro-5-fluoro-6-(7-fluoro-1H-indol-6-yl)pyridin-2-carboxylate, Ethyl-4-amino-3-chloro-5-fluoro-6-(7-fluoro-1H-indol-6-yl)pyridin-2-carboxylate, Methyl-4-amino-3-chloro-5-fluoro-6-(7-fluoro-1-isobutyryl-1H-indol-6-yl)pyridin-2-carboxylate, Methyl 6-(1-acetyl-7-fluoro-1H-indol-6-yl)-4-amino-3-chloro-5-fluoropyridin-2-carboxylate, Methyl-4-amino-3-chloro-6-[1-(2,2-dimethylpropanoyl)-7-fluoro-1H-indol-6-yl]-5-fluoropyridin-2-carboxylate, Methyl-4-amino-3-chloro-5-fluoro-6-[7-fluoro-1-(methoxyacetyl)-1H-indol-6-yl]pyridin-2-carboxylate, Potassium 4-amino-3-chloro-5-fluoro-6-(7-fluoro-1H-indol-6-yl)pyridin-2-carboxylate, Sodium-4-amino-3-chloro-5-fluoro-6-(7-fluoro-1H-indol-6-yl)pyridin-2-carboxylate, Butyl-4-amino-3-chloro-5-fluoro-6-(7-fluoro-1H-indol-6-yl)pyridin-2-carboxylate, 4-Hydroxy-1-methyl-3-[4-(trifluoromethyl)pyridin-2-yl]imidazolidin-2-one, 3-(5-tert-butyl-1,2-oxazol-3-yl)-4-hydroxy-1-methylimidazolidin-2-one, 3-[5-Chloro-4-(trifluoromethyl)pyridin-2-yl]-4-hydroxy-1-methylimidazolidin-2-one, 4-Hydroxy-1-methoxy-5-methyl-3-[4-(trifluoromethyl)pyridin-2-yl]imidazolidin-2-one, 6-[(2-Hydroxy-6-oxocyclohex-1-en-1-yl)carbonyl]-1,5-dimethyl-3-(2-methylphenyl)quinazolin-2,4(1H,3H)-dione, 3-(2,6-Dimethylphenyl)-6-[(2-hydroxy-6-oxocyclohex-1-en-1-yl)carbonyl]-1-methylquinazolin-2,4(1H,3H)-dione, 2-[2-chloro-4-(methylsulfonyl)-3-(morpholin-4-ylmethyl)benzoyl]-3-hydroxycyclohex-2-en-1-one, 1-(2-carboxyethyl)-4-(pyrimidin-2-yl)pyridazin-1-ium salt (combined with suitable anions such as chloride, acetate or trifluoroacetate), 1-(2-Carboxyethyl)-4-(pyridazin-3-yl)pyridazin-1-ium salt (combined with suitable anions such as chloride, acetate or trifluoroacetate), 4-(Pyrimidin-2-yl)-1-(2-sulfoethyl)pyridazin-1-ium salt (combined with suitable anions such as chloride, acetate or trifluoroacetate), 4-(Pyridazin-3-yl)-1-(2-sulfoethyl)pyridazin-1-ium salt (combined with suitable anions such as chloride, acetate or trifluoroacetate), 1-(2-Carboxyethyl)-4-(1,3-thiazol-2-yl)pyridazin-1-ium salt (combined with suitable anions such as chloride, acetate or trifluoroacetate), 1-(2-Carboxyethyl)-4-(1,3,4-thiadiazol-2-yl)pyridazin-1-ium salt (combined with suitable anions such as chloride, acetate or trifluoroacetate), Methyl (2R)-2-{[(E)-({2-chloro-4-fluoro-5-[3-methyl-2,6-dioxo-4-(trifluoromethyl)-3,6-dihydropyrimidin-1(2H)-yl]phenyl}methyliden)amino]oxy}propanoate, Methyl (2S)-2-{[(E)-({2-chloro-4-fluoro-5-[3-methyl-2,6-dioxo-4-(trifluoromethyl)-3,6-dihydropyrimidin-1(2H)-yl]phenyl}methyliden)amino]oxy}propanoate, Methyl (2R/S)-2-{[(E)-({2-chloro-4-fluoro-5-[3-methyl-2,6-dioxo-4-(trifluoromethyl)-3,6-dihydropyrimidin-1(2H)-yl]phenyl}methyliden)amino]oxy}propanoate, (E)-2-(Trifluoromethyl)benzaldehyde-O-{2,6-bis[(4,6-dimethoxypyrimidin-2-yl)oxy]benzoyl}oxim, 2-Fluoro-N-(5-methyl-1,3,4-oxadiazol-2-yl)-3-[(R)-propylsulfinyl]-4-(trifluoromethyl)benzamide, (2R)-2-[(4-Amino-3,5-dichloro-6-fluoro-2-pyridyl)oxy]propane carboxylic acid, 2-Ethoxy-2-oxoethyl-1-{2-chloro-4-fluoro-5-[3-methyl-2,6-dioxo-4-(trifluoromethyl)-3,6-dihydropyrimidin-1(2H)-yl]phenoxy}cyclopropancarboxylate, 2-Methoxy-2-oxoethyl-1-{2-chloro-4-fluoro-5-[3-methyl-2,6-dioxo-4-(trifluoromethyl)-3,6-dihydropyrimidin-1(2H)-yl]phenoxy}cyclopropancarboxylate, {[(1-{2-Chloro-4-fluoro-5-[3-methyl-2,6-dioxo-4-(trifluoromethyl)-3,6-dihydropyrimidin-1(2H)-yl]phenoxy}cyclopropyl)carbonyl]oxy}acetic acid, 2-(2-Bromo-4-chlorobenzyl)-4,4-dimethyl-1,2-oxazolidin-3-one, Methyl 3-{2-chloro-4-fluoro-5-[3-methyl-2,6-dioxo-4-(trifluoromethyl)-3,6-dihydropyrimidin-1(2H)-yl]phenyl}-3a,4,5,6-tetrahydro-6aH-cyclopenta[d][1,2]oxazol-6a-carboxylate, Ethyl 3-{2-chloro-4-fluoro-5-[3-methyl-2,6-dioxo-4-(trifluoromethyl)-3,6-dihydropyrimidin-1(2H)-yl]phenyl}-3a,4,5,6-tetrahydro-6aH-cyclopenta[d][1,2]oxazol-6a-carboxylate, Methyl-3-{2-chloro-4-fluoro-5-[3-methyl-2,6-dioxo-4-(trifluoromethyl)-3,6-dihydropyrimidin-1(2H)-yl]phenyl}-6-methyl-3a,4,5,6-tetrahydro-6aH-cyclopenta[d][1,2]oxazol-6a-carboxylate, 3-{2-Chloro-4-fluoro-5-[3-methyl-2,6-dioxo-4-(trifluoromethyl)-3,6-dihydropyrimidin-1(2H)-yl]phenyl}-6-methyl-3a,4,5,6-tetrahydro-6aH-cyclopenta[d][1,2]oxazol-6a-carboxylic acid, 3-{2-Chloro-4-fluoro-5-[3-methyl-2,6-dioxo-4-(trifluoromethyl)-3,6-dihydropyrimidin-1(2H)-yl]phenyl}-3a,4,5,6-tetrahydro-6aH-cyclopenta[d][1,2]oxazol-6a-carboxylic acid,

Abscisic acid and related analogs [e.g. (2Z,4E)-5-[6-Ethynyl-1-hydroxy-2,6-dimethyl-4-oxocyclohex-2-en-1-yl]-3-methylpenta-2,4-dienoic acid, methyl-(2Z,4E)-5-[6-ethynyl-1-hydroxy-2,6-dimethyl-4-oxocyclohex-2-en-1-yl]-3-methylpenta-2,4-dienoate, (2Z,4E)-3-ethyl-5-(1-hydroxy-2,6,6-trimethyl-4-oxocyclohex-2-en-1-yl)penta-2,4-dienoic acid, (2E,4E)-5-(1-hydroxy-2,6,6-trimethyl-4-oxocyclohex-2-en-1-yl)-3-(trifluoromethyl)penta-2,4-dienoic acid, methyl (2E,4E)-5-(1-hydroxy-2,6,6-trimethyl-4-oxocyclohex-2-en-1-yl)-3-(trifluoromethyl)penta-2,4-dienoate, (2Z,4E)-5-(2-hydroxy-1,3-dimethyl-5-oxobicyclo[4.1.0]hept-3-en-2-yl)-3-methylpenta-2,4-dienoic acid], acibenzolar, acibenzolar-S-methyl, S-adenosylhomocysteine, allantoin, 2-Aminoethoxyvinylglycine (AVG), aminooxyacetic acid and related esters [e.g. (Isopropylidene)-aminooxyacetic acid-2-(methoxy)-2-oxoethylester, (Isopropylidene)-aminooxyacetic acid-2-(hexyloxy)-2-oxoethylester, (Cyclohexylidene)-aminooxyacetic acid-2-(isopropyloxy)-2-oxoethylester], 1-aminocycloprop-1-ylcarboxylic acid, N-Methyl-1-aminocyclopropyl-1-carboxylic acid, 1-aminocyclopropyl-1-carboxamide, 1-aminocycloprop-1-yl carboxylic acid and derivatives thereof, e.g. disclosed in DE3335514, EP30287, DE2906507 or US5123951, 1-aminocyclopropyl-1-hydroxamic acid, 5-aminolevulinic acid, ancymidol, 6-benzylaminopurine, bikinin, brassinolide, brassinolide-ethyl, L-canaline, catechin and catechines (e.g. (2S,3R)-2-(3,4-Dihydroxyphenyl)-3,4-dihydro-2H-chromen-3,5,7-triol), chitooligosaccharides (CO; COs differ from LCOs in that they lack the pendant fatty acid chain that is characteristic of LCOs. COs, sometimes referred to as N-acetylchitooligosaccharides, are also composed of GlcNAc residues but have side chain decorations that make them different from chitin molecules [(C₈H₁₃NO₅)ₙ, CAS No. 1398-61-4] and chitosan molecules [(C₅H₁₁NO₄)ₙ, CAS No. 9012-76-4]), chitinous compounds, chlormequat chloride, cloprop, cyclanilide, 3-(Cycloprop-1-enyl)propionic acid, 1-[2-(4-cyano-3,5-dicyclopropylphenyl)acetamido]cyclohexanecarboxylic acid, 1-[2-(4-cyano-3-cyclopropylphenyl)acetamido]cyclohexanecarboxylic acid, daminozide, dazomet, dazomet-sodium, n-decanol, dikegulac, dikegulac-sodium, endothal, endothal-dipotassium, -disodium, and mono(N,N-dimethylalkylammonium), ethephon, flumetralin, flurenol, flurenol-butyl, flurenol-methyl, flurprimidol, forchlorfenuron, gibberellic acid, inabenfide, indol-3-acetic acid (IAA), 4-indol-3-ylbutyric acid, isoprothiolane, probenazole, jasmonic acid, Jasmonic acid or derivatives thereof (e.g. jasmonic acid methyl ester, jasmonic acid ethyl ester), lipo-chitooligosaccharides (LCO, sometimes referred to as symbiotic nodulation (Nod) signals (or Nod factors) or as Myc factors, consist of an oligosaccharide backbone of β-l,4-linked *N*-acetyl-D-glucosamine ("GlcNAc") residues with an N-linked fatty acyl chain condensed at the non-reducing end. As understood in the art, LCOs differ in the number of GlcNAc residues in the backbone, in the length and degree of saturation of the fatty acyl chain and in the substitutions of reducing and non-reducing sugar residues), linoleic acid or derivatives thereof, linolenic acid or derivatives thereof, maleic hydrazide, mepiquat chloride, mepiquat pentaborate, 1-methylcyclopropene, 3-methylcyclopropene, 1-ethylcyclopropene, 1-n-propylcyclopropene, 1-cyclopropenylmethanol, methoxyvinylglycin (MVG), 3'-methyl abscisic acid, 1-(4-methylphenyl)-N-(2-oxo-1-propyl-1,2,3,4-tetrahydroquinolin-6-yl)methanesulfonamide and related substituted tetrahydroquinolin-6-yl)methanesulfonamides, (3E,3aR,8bS)-3-({[(2R)-4-Methyl-5-oxo-2,5-dihydrofuran-2-yl]oxy}methylen)-3,3a,4,8b-tetrahydro-2H-indeno[1,2-b]furan-2-one and related lactones as outlined in EP2248421, 2-(1-naphthyl)acetamide, 1-naphthylacetic acid, 2- naphthyloxyacetic acid, nitrophenolate-mixture, 4-Oxo-4[(2-phenylethyl)amino]butyric acid, paclobutrazol, 4-phenylbutyric acid and its related salts (e.g. sodium-4-phenylbutanoate, potassium-4-phenylbutanoate), phenylalanine, N-phenylphthalamic acid, prohexadione, prohexadione-calcium, putrescine, prohydrojasmon, rhizobitoxin, salicylic acid, salicylic acid methyl ester, sarcosine, sodium cycloprop-1-en-1-yl acetate, sodium cycloprop-2-en-1-yl acetate, sodium-3-(cycloprop-2-en-1-yl)propanoate, sodium-3-(cycloprop-1-en-1-yl) propanoate, sidefungin, spermidine, spermine, strigolactone, tecnazene, thidiazuron, triacontanol, trinexapac, trinexapac-ethyl, tryptophan, tsitodef, uniconazole, uniconazole-P, 2-fluoro-N-(3-methoxyphenyl)-9H-purin-6-amine, 2-chloro-N-(3-methoxyphenyl)-9H-purin-6-amine.

S1) Compounds from the group of heterocyclic carboxylic acid derivatives: 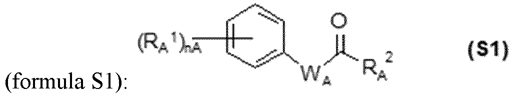 wherein symbols and indices are defined as follows:
n_{A} is an integer value in the range of 0 to 5, preferably 0 to 3;
R_{A}¹ is halogen, (C₁-C₄)-alkyl, (C₁-C₄)-alkoxy, nitro or (C₁-C₄)-haloalkyl; 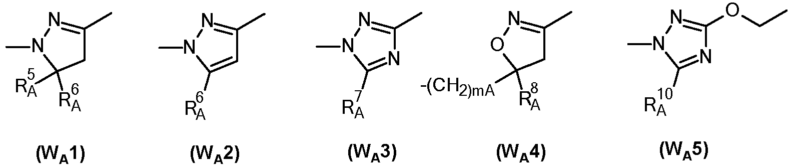
W_{A} is an unsubstituted or substituted divalent heterocyclic moiety selected from the group of partially unsaturated or aromatic five-membered heterocycles carrying 1 to 3 hetero ring atoms selected from the group of nitrogen (N) und oxygen (O), and carrying at least one N-atom and not more than one O-atom in the ring, preferably a five-membered heterocyclic moiety selected from the group (W_{A}¹) to (W_{A}⁴), m_{A} is 0 or 1;
R_{A}² is OR_{A}³, SR_{A}³ or NR_{A}³R_{A}⁴ or a saturated or unsaturated 3- to 7-membered heterocycle containing at least one N-atom and up to 3 heteroatoms, preferably combined with other heteroatoms from the group of O (oxygen) and S (sulfur), and which is linked to the carbonyl group in (S1) via a nitrogen atom, and which ist unsubstituted or substituted by moieties selected from the group of (C₁-C₄)-alkyl, (C₁C₄)-alkoxy or possibly substituted phenyl, preferably OR_{A}³, NHR_{A}⁴ or N(CH₃)₂, particularly OR_{A}³
R_{A}³ is hydrogen or an unsubstituted or substituted aliphatic hydrocarbon moiety, preferably containing 1 to 18 C-atoms;
R_{A}⁴ is hydrogen, (C₁-C₆)-alkyl, (C₁-C₆)-alkoxy or substituted or unsubstituted phenyl;
R_{A}⁵ is hydrogen, (C₁-C₈)-alkyl, (C₁-C₈)-haloalkyl, (C₁-C₄)-alkoxy-(C₁-C₈)-alkyl, cyano or COOR_{A}⁹, wherein R_{A}⁹ is hydrogen, (C₁-C₈)-alkyl, (C₁-C₈)-haloalkyl, (C₁-C₄)-alkoxy-(C₁C₄)-alkyl, (C₁-C₆)-hydroxyalkyl, (C₃-C₁₂)-cycloalkyl oder tris-(C₁-C₄)-alkylsilyl;
R_{A}⁶, R_{A}⁷, R_{A}⁸ are independently hydrogen, (C₁-C₈)-alkyl, (C₁-C₈)-haloalkyl, (C₃-C₁₂)-cycloalkyl or substituted or unsubstituted phenyl;
R_{A}¹⁰ is H, (C₃-C₁₂)-cycloalkyl, substituted or unsubstituted phenyl or substituted or unsubstituted heteroaryl;
preferably
S1^{a}) Compounds of the dichlorophenylpyrazoline-3-carboxylic acid type (S1^{a}), preferably compounds such as
   1-(2,4-dichlorophenyl)-5-(ethoxycarbonyl)-5-methyl-2-pyrazoline-3-carboxylic acid, ethyl 1-(2,4-dichlorophenyl)-5-(ethoxycarbonyl)-5-methyl-2-pyrazoline-3-carboxylate (S1-1) ("mefenpyr-diethyl"), and related compounds as described in WO-A-91/07874;
S1^{b}) Derivatives of dichlorophenylpyrazolecarboxylic acid (S1^{b}), preferably compounds such as ethyl 1-(2,4-dichlorophenyl)-5-methylpyrazole-3-carboxylate (S1-2), ethyl 1-(2,4-dichlorophenyl)-5-isopropylpyrazole-3-carboxylate (S1-3), ethyl 1-(2,4-dichlorophenyl)-5-(1,1-dimethylethyl)pyrazole-3-carboxylate (S1-4) and related compounds as described in EP-A-333131 131 and EP-A-269806;
S1^{c}) Derivatives of 1,5-diphenylpyrazole-3-carboxylic acid (S1^{c}), preferably compounds such as ethyl 1-(2,4-dichlorophenyl)-5-phenylpyrazole-3-carboxylate (S1-5), methyl 1-(2-chlorophenyl)-5-phenylpyrazole-3-carboxylate (S1-6) and related compounds as described, for example, in EP-A-268554;
S1^{d}) Compounds of the triazolecarboxylic acid type (S1^{d}), preferably compounds such as fenchlorazole (ethyl ester), i.e. ethyl 1-(2,4-dichlorophenyl)-5-trichloromethyl-1H-1,2,4-triazole-3-carboxylate (S1-7), and related compounds, as described in EP-A-174562 and EP-A-346620;
S1^{e}) Compounds of the 5-benzyl- or 5-phenyl-2-isoxazoline-3-carboxylic acid or of the 5,5-diphenyl-2-isoxazoline-3-carboxylic acid type (S1^{e}), preferably compounds such as ethyl 5-(2,4-dichlorobenzyl)-2-isoxazoline-3-carboxylate (S1-8) or ethyl 5-phenyl-2-isoxazoline-3-carboxylate (S1-9) and related compounds as described in WO-A-91/08202, or 5,5-diphenyl-2-isoxazolinecarboxylic acid (S1-10) or ethyl 5,5-diphenyl-2-isoxazoline-3-carboxylate (S1-11) ("isoxadifen-ethyl") or n-propyl 5,5-diphenyl-2-isoxazoline-3-carboxylate (S1-12) or ethyl 5-(4-fluorophenyl)-5-phenyl-2-isoxazoline-3-carboxylate (S1-13), as described in patent application WO-A-95/07897.
S1^{f}) compounds of the triazolyloxy acetic acid type (S1^{f}), preferably compounds such as methyl-{[1,5-bis(4-chloro-2-fluorophenyl)-1H-1,2,4-triazol-3-yl]oxy}acetate (S1-14) or {[1,5-bis(4-chloro-2-fluorophenyl)-1H-1,2,4-triazol-3-yl]oxy}acetic acid (S1-15) or methyl-{[5-(4-chloro-2-fluorophenyl)-1-(2,4-difluorophenyl)-1H-1,2,4-triazol-3-yl]oxy}acetate (S1-16) or {[5-(4-chloro-2-fluorophenyl)-1-(2,4-difluorophenyl)-1H-1,2,4-triazol-3-yl]oxy}acetic acid (S1-17) or methyl-{[1-(4-chloro-2-fluorophenyl)-5-(2,4-difluorophenyl)-1H-1,2,4-triazol-3-yl]oxy}acetate (S1-18) or {[1-(4-chloro-2-fluorophenyl)-5-(2,4-difluorophenyl)-1H-1,2,4-triazol-3-yl]oxy}acetic acid (S1-19), as described in patent application WO2021/105101.
S2) Compounds from the group of the 8-quinolinoxy derivatives (S2):
   wherein symbols and indices are defined as follows:
   - R_{B}¹: is halogen, (C₁-C₄)-alkyl, (C₁-C₄)-alkoxy, nitro or (C₁-C₄)-haloalkyl;
   - n_{B}: an integer from 0 to 5, preferably 0 to 3;
   - R_{B}²: is OR_{B}³, SR_{B}³ or NR_{B}³R_{B}⁴ or a saturated or unsaturated 3- to 7-membered heterocycle containing at least one nitrogen atom and up to 3 heteroatoms, preferably from the group consisting of O (oxygen) and S (sulfur), which is connected to the carbonyl group in (S2) via a nitrogen atom and which is unsubstituted or substituted by (C₁-C₄)-alkyl, (C₁-C₄)-alkoxy oder potentially substituted phenyl, preferably OR_{B}³, NHR_{B}⁴ oder N(CH₃)₂, in particular OR_{B}³;
   - R_{B}³: is hydrogen or an unsubstituted or substituted aliphatic hydrocarbon moiety preferably consisting of 1 to 18 carbon atoms;
   - R_{B}⁴: is hydrogen, (C₁-C₆)-alkyl, (C₁-C₆)-alkoxy or substituted or unsubstituted phenyl;
   - T_{B}: is a (C₁ or C₂)-alkandiyl chain, which is unsubstituted or substituted by one or two (C₁-C₄)-Alkyl moieties or by a [(C₁-C₃)-alkoxy]carbonyl moiety;
   preferably:
   S2^{a}) Compounds of the 8-quinolinoxyacetic acid type (S2^{a}), preferably 1-methylhexyl (5-chloro-8-quinolinoxy)acetate ("cloquintocet-mexyl") (S2-1), 1,3-dimethylbut-1-yl (5-chloro-8-quinolinoxy)acetate (S2-2), 4-allyloxybutyl (5-chloro-8-quinolinoxy)acetate (S2-3), 1-allyloxyprop-2-yl (5-chloro-8-quinolinoxy)acetate (S2-4), ethyl (5-chloro-8-quinolinoxy)acetate (S2-5), methyl 5-chloro-8-quinolinoxyacetate (S2-6), allyl (5-chloro-8-quinolinoxy)acetate (S2-7), 2-(2-propylideneiminoxy)-1-ethyl (5-chloro-8-quinolinoxy)acetate (S2-8), 2-oxoprop-1-yl (5-chloro-8-quinolinoxy)acetate (S2-9) and related compounds, as described in EP-A-86750, EP-A-94349 and EP-A-191736 or EP-A-0 492 366, and also (5-chloro-8-quinolinoxy)acetic acid (S2-10), hydrates and salts thereof, for example the lithium, sodium, potassium, calcium, magnesium, aluminum, iron, ammonium, quaternary ammonium, sulfonium or phosphonium salts thereof, as described in WO-A-2002/34048;
   S2^{b}) Compounds of the (5-chloro-8-quinolinoxy)malonic acid type (S2^{b}), preferably compounds such as diethyl (5-chloro-8-quinolinoxy)malonate, diallyl (5-chloro-8-quinolinoxy)malonate, methyl ethyl (5-chloro-8-quinolinoxy)malonate and related compounds, as described in EP-A-0 582 198.
   S3) Active compounds of the dichloroacetamide type (S3), which are frequently used as pre-emergence safeners (soil-acting safeners), for example
      "dichlormid" (N,N-diallyl-2,2-dichloroacetamide) (S3-1),
      "R-29148" (3-dichloroacetyl-2,2,5-trimethyl-1,3-oxazolidine) from Stauffer (S3-2),
      "R-28725" (3-dichloroacetyl-2,2-dimethyl-1,3-oxazolidine) from Stauffer (S3-3),
      "benoxacor" (4-dichloroacetyl-3,4-dihydro-3-methyl-2H-1,4-benzoxazine) (S3-4),
      "PPG-1292" (N-allyl-N-[(1,3-dioxolan-2-yl)methyl]dichloroacetamide) from PPG Industries (S3-5), "DKA-24" (N-allyl-N-[(allylaminocarbonyl)methyl]dichloroacetamide) from Sagro-Chem (S3-6), "AD-67" or "MON 4660" (3-dichloroacetyl-1-oxa-3-azaspiro[4.5]decane) from Nitrokemia or Monsanto (S3-7),
      "TI-35" (1-dichloroacetylazepane) from TRI-Chemical RT (S3-8),
      "Diclonon" (Dicyclonon) or "BAS145138" or "LAB145138" (S3-9)
      ((RS)-1-dichloroacetyl-3,3,8a-trimethylperhydropyrrolo[1,2-a]pyrimidin-6-one) from BASF, "furilazole" or "MON 13900" ((RS)-3-dichloroacetyl-5-(2-furyl)-2,2-dimethyloxazolidine) (S3-10), and the (R) isomer thereof (S3-11).
   S4) Compounds from the class of the acylsulfonamides (S4):
      S4^{a}) N-Acylsulfonamides of the formula (S4^{a}) and salts thereof, as described in WO-A-97/45016, 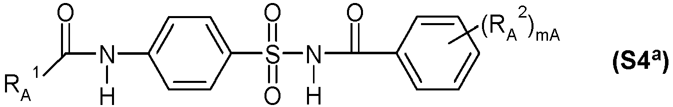 in which
         - R_{A}¹: is (C₁-C₆)-alkyl, (C₃-C₆)-cycloalkyl, where the 2 latter radicals are substituted by v_{A} substituents from the group of halogen, (C₁-C₄)-alkoxy, (C₁-C₆)-haloalkoxy and (C₁-C₄)-alkylthio and, in the case of cyclic radicals, also by (C₁-C₄)-alkyl and (C₁-C₄)-haloalkyl;
         - R_{A}²: is halogen, (C₁-C₄)-alkyl, (C₁-C₄)-alkoxy, CF₃;
         - m_{A}: is 1 or 2;
         - v_{A}: is 0, 1, 2 or 3;
         - S4^{b}): Compounds of the 4-(benzoylsulfamoyl)benzamide type of the formula (S4^{b}) and salts thereof, as described in WO-A-99/16744, 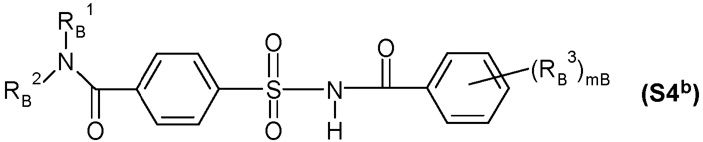
         in which
         - R_{B}¹, R_{B}²: are independently hydrogen, (C₁-C₆)-alkyl, (C₃-C₆)-cycloalkyl, (C₃-C₆)-alkenyl, (C₃-C₆)-alkynyl,
         - R_{B}³: is halogen, (C₁-C₄)-alkyl, (C₁-C₄)-haloalkyl or (C₁-C₄)-alkoxy and
         - m_{B}: is 1 or 2,
         for example those in which
         R_{B}¹ = cyclopropyl, R_{B}² = hydrogen and (R_{B}³) = 2-OMe ("cyprosulfamide", S4-1),
         R_{B}¹ = cyclopropyl, R_{B}² = hydrogen and (R_{B}³) = 5-Cl-2-OMe (S4-2),
         R_{B}¹ = ethyl, R_{B}² = hydrogen and (R_{B}³) = 2-OMe (S4-3),
         R_{B}¹ = isopropyl, R_{B}² = hydrogen and (R_{B}³) = 5-Cl-2-OMe (S4-4) and
         R_{B}¹ = isopropyl, R_{B}² = hydrogen and (R_{B}³) = 2-OMe (S4-5);
      S4^{c}) Compounds from the class of the benzoylsulfamoylphenylureas of the formula (S4^{c}), as described in EP-A-365484, 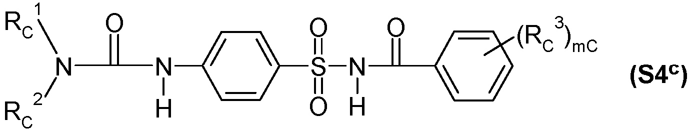 in which
         - R_{C}¹, R_{C}²: are independently hydrogen, (C₁-C₈)-alkyl, (C₃-C₈)-cycloalkyl, (C₃-C₆)-alkenyl, (C₃-C₆)-alkynyl,
         - R_{C}³: is halogen, (C₁-C₄)-alkyl, (C₁-C₄)-alkoxy, CF₃ and
         - m_{C}: is 1 or 2;
         for example
         1-[4-(N-2-methoxybenzoylsulfamoyl)phenyl]-3-methylurea,
         1-[4-(N-2-methoxybenzoylsulfamoyl)phenyl]-3,3-dimethylurea,
         1-[4-(N-4,5-dimethylbenzoylsulfamoyl)phenyl]-3-methylurea;
      S4^{d}) Compounds of the N-phenylsulfonylterephthalamide type of the formula (S4^{d}) and salts thereof, which are known, for example, from CN 101838227, 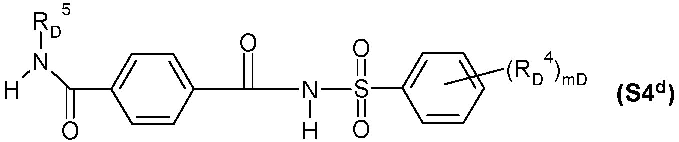 in which
         - R_{D}⁴: is halogen, (C₁-C₄)-alkyl, (C₁-C₄)-alkoxy, CF₃;
         - m_{D}: is 1 or 2;
         - R_{D}⁵: is hydrogen, (C₁-C₆)-alkyl, (C₃-C₆)-cycloalkyl, (C₂-C₆)-alkenyl, (C₂-C₆)-alkynyl or (C₅-C₆)-cycloalkenyl.
   S5) Active compounds from the class of the hydroxyaromatics and the aromatic-aliphatic carboxylic acid derivatives (S5), for example ethyl 3,4,5-triacetoxybenzoate, 3,5-dimethoxy-4-hydroxybenzoic acid, 3,5-dihydroxybenzoic acid, 4-hydroxysalicylic acid, 4-fluorosalicyclic acid,
      2-hydroxycinnamic acid, 2,4-dichlorocinnamic acid, as described in WO-A-2004/084631,
      WO-A-2005/015994, WO-A-2005/016001.
   S6) Active compounds from the class of the 1,2-dihydroquinoxalin-2-ones (S6), for example 1-methyl-3-(2-thienyl)-1,2-dihydroquinoxalin-2-one, 1-methyl-3-(2-thienyl)-1,2-dihydroquinoxaline-2-thione, 1-(2-aminoethyl)-3-(2-thienyl)-1,2-dihydroquinoxalin-2-one hydrochloride, 1-(2-methylsulfonylaminoethyl)-3-(2-thienyl)-1,2-dihydroquinoxalin-2-one, as described in WO-A-2005/112630.
   S7) Compounds from the class of the diphenylmethoxyacetic acid derivatives (S7), e.g. methyl diphenylmethoxyacetate (CAS Reg. No. 41858-19-9) (S7-1), ethyl diphenylmethoxyacetate or diphenylmethoxyacetic acid, as described in WO-A-98/38856.
   S8) Compounds of the formula (S8), as described in WO-A-98/27049, 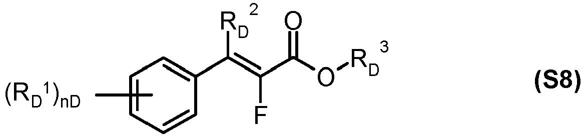 in which the symbols and indices are defined as follows:
      R_{D}¹ is halogen, (C₁-C₄)-alkyl, (C₁-C₄)-haloalkyl, (C₁-C₄)-alkoxy, (C₁-C₄)-haloalkoxy,
      R_{D}² is hydrogen or (C₁-C₄)-alkyl,
      R_{D}³ is hydrogen, (C₁-C₈)-alkyl, (C₂-C₄)-alkenyl, (C₂-C₄)-alkynyl or aryl, where each of the aforementioned carbon-containing radicals is unsubstituted or substituted by one or more, preferably up to three identical or different radicals from the group consisting of halogen and alkoxy; or salts thereof,
      n_{D} is an integer from 0 to 2.
   S9) active compounds from the class of the 3-(5-tetrazolylcarbonyl)-2-quinolones (S9), for example 1,2-dihydro-4-hydroxy-1-ethyl-3-(5-tetrazolylcarbonyl)-2-quinolone (CAS Reg. No. 219479-18-2), 1,2-dihydro-4-hydroxy-1-methyl-3-(5-tetrazolylcarbonyl)-2-quinolone (CAS Reg. No. 95855-00-8), as described in WO-A-199/000020;
   S10) Compounds of the formula (S10^{a}) or (S10^{b})
      as described in WO-A-2007/023719 and WO-A-2007/023764 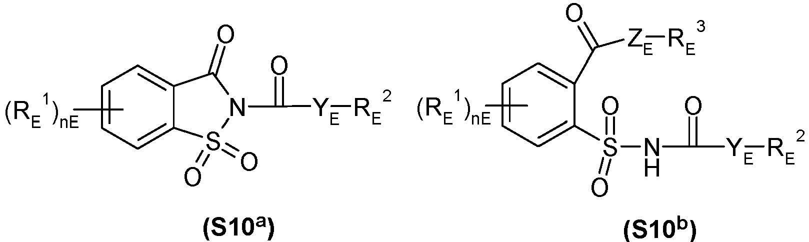 in which
      - R_{E}¹: is halogen, (C₁-C₄)-alkyl, methoxy, nitro, cyano, CF₃, OCF₃
      - Y_{E}, Z_{E}: are independently O or S,
      - n_{E}: is an integer from 0 to 4,
      - R_{E}²: is (C₁-C₁₆)-alkyl, (C₂-C₆)-alkenyl, (C₃-C₆)-cycloalkyl, aryl; benzyl, halobenzyl,
      - R_{E}³: is hydrogen or (C₁-C₆)-alkyl.
   S11) Active compounds of the oxyimino compound type (S11), which are known as seed-dressing agents, for example
      "oxabetrinil" ((Z)-1,3-dioxolan-2-ylmethoxyimino(phenyl)acetonitrile) (S11-1), which is known as a seed-dressing safener for millet/sorghum against metolachlor damage,
      "fluxofenim" (1-(4-chlorophenyl)-2,2,2-trifluoro-1-ethanone O-(1,3-dioxolan-2-ylmethyl)oxime) (S11-2), which is known as a seed-dressing safener for millet/sorghum against metolachlor damage, and "cyometrinil" or "CGA-43089" ((Z)-cyanomethoxyimino(phenyl)acetonitrile) (S11-3), which is known as a seed-dressing safener for millet/sorghum against metolachlor damage.
   S12) active compounds from the class of the isothiochromanones (S12), for example methyl [(3-oxo-1H-2-benzothiopyran-4(3H)-ylidene)methoxy]acetate (CAS Reg. No. 205121-04-6) (S12-1) and related compounds from WO-A-1998/13361.
   S13) One or more compounds from group (S13):
      "naphthalic anhydride" (1,8-naphthalenedicarboxylic anhydride) (S13-1), which is known as a seed-dressing safener for corn against thiocarbamate herbicide damage,
      "fenclorim" (4,6-dichloro-2-phenylpyrimidine) (S13-2), which is known as a safener for pretilachlor in sown rice,
      "flurazole" (benzyl 2-chloro-4-trifluoromethyl-1,3-thiazole-5-carboxylate) (S13-3), which is known as a seed-dressing safener for millet/sorghum against alachlor and metolachlor damage,
      "CL 304415" (CAS Reg. No. 31541-57-8)
      (4-carboxy-3,4-dihydro-2H-1-benzopyran-4-acetic acid) (S13-4) from American Cyanamid, which is known as a safener for corn against damage by imidazolinones,
      "MG 191" (CAS Reg. No. 96420-72-3) (2-dichloromethyl-2-methyl-1,3-dioxolane) (S13-5) from Nitrokemia, which is known as a safener for corn,
      "MG 838" (CAS Reg. No. 133993-74-5)
      (2-propenyl 1-oxa-4-azaspiro[4.5]decane-4-carbodithioate) (S13-6) from Nitrokemia "disulfoton" (O,O-diethyl S-2-ethylthioethyl phosphorodithioate) (S13-7),
      "dietholate" (O,O-diethyl O-phenyl phosphorothioate) (S13-8),
      "mephenate" (4-chlorophenyl methylcarbamate) (S13-9).
   S14) active compounds which, in addition to herbicidal action against weeds, also have safener action on crop plants such as rice, for example
      "dimepiperate" or "MY-93" (S-1-methyl 1-phenylethylpiperidine-1-carbothioate), which is known as a safener for rice against damage by the herbicide molinate,
      "daimuron" or "SK 23" (1-(1-methyl-1-phenylethyl)-3-p-tolylurea), which is known as safener for rice against imazosulfuron herbicide damage,
      "cumyluron" = "JC-940" (3-(2-chlorophenylmethyl)-1-(1-methyl-1-phenylethyl)urea, see JP-A-60087254), which is known as safener for rice against damage by some herbicides,
      "methoxyphenone" or "NK 049" (3,3'-dimethyl-4-methoxybenzophenone), which is known as a safener for rice against damage by some herbicides,
      "CSB" (1-bromo-4-(chloromethylsulfonyl)benzene) from Kumiai, (CAS Reg. No. 54091-06-4), which is known as a safener against damage by some herbicides in rice.
   S15) Compounds of the formula (S15) or tautomers thereof 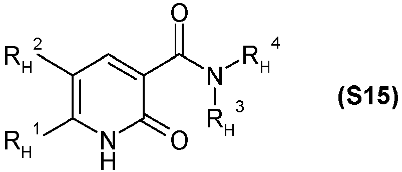 as described in WO-A-2008/131861 and WO-A-2008/131860
      in which
      R_{H}¹ is a (C₁-C₆)-haloalkyl radical and
      R_{H}² is hydrogen or halogen and
      R_{H}³, R_{H}⁴ are each independently hydrogen, (C₁-C₁₆)-alkyl, (C₂-C₁₆)-alkenyl or (C₂-C₁₆)-alkynyl, where each of the 3 latter radicals is unsubstituted or substituted by one or more radicals from the group of halogen, hydroxyl, cyano, (C₁-C₄)-alkoxy, (C₁-C₄)-haloalkoxy, (C₁-C₄)-alkylthio, (C₁-C₄)-alkylamino, di[(C₁-C₄)-alkyl]amino, [(C₁-C₄)-alkoxy]carbonyl, [(C₁-C₄)-haloalkoxy]carbonyl, (C₃-C₆)-cycloalkyl which is unsubstituted or substituted, phenyl which is unsubstituted or substituted, and heterocyclyl which is unsubstituted or substituted, or (C₃-C₆)-cycloalkyl, (C₄-C₆)-cycloalkenyl, (C₃-C₆)-cycloalkyl fused on one side of the ring to a 4 to 6-membered saturated or unsaturated carbocyclic ring, or (C₄-C₆)-cycloalkenyl fused on one side of the ring to a 4 to 6-membered saturated or unsaturated carbocyclic ring, where each of the 4 latter radicals is unsubstituted or substituted by one or more radicals from the group of halogen, hydroxyl, cyano, (C₁-C₄)alkyl, (C₁-C₄)haloalkyl, (C₁-C₄)alkoxy, (C₁-C₄)haloalkoxy, (C₁-C₄)alkylthio, (C₁-C₄)alkylamino, di[(C₁-C₄)alkyl]amino, [(C₁-C₄)alkoxy]carbonyl, [(C₁-C₄)haloalkoxy]carbonyl, (C₃-C₆)cycloalkyl which is unsubstituted or substituted, phenyl which is unsubstituted or substituted, and heterocyclyl which is unsubstituted or substituted,
         or
      R_{H}³ is (C₁-C₄)-alkoxy, (C₂-C₄)-alkenyloxy, (C₂-C₆)-alkynyloxy or (C₂-C₄)-haloalkoxy and
      R_{H}⁴ is hydrogen or (C₁-C₄)-alkyl or
      R_{H}³ and R_{H}⁴ together with the directly bonded nitrogen atom are a four- to eight-membered heterocyclic ring which, as well as the nitrogen atom, may also contain further ring heteroatoms, preferably up to two further ring heteroatoms from the group of N, O and S, and which is unsubstituted or substituted by one or more radicals from the group of halogen, cyano, nitro, (C₁-C₄)-alkyl, (C₁-C₄)-haloalkyl, (C₁-C₄)-alkoxy, (C₁-C₄)-haloalkoxy and (C₁-C₄)-alkylthio.
   S16) Active compounds which are used primarily as herbicides but also have safener action on crop plants, for example
      (2,4-dichlorophenoxy)acetic acid (2,4-D),
      (4-chlorophenoxy)acetic acid,
      (R,S)-2-(4-chloro-o-tolyloxy)propionic acid (mecoprop),
      4-(2,4-dichlorophenoxy)butyric acid (2,4-DB),
      (4-chloro-o-tolyloxy)acetic acid (MCPA),
      4-(4-chloro-o-tolyloxy)butyric acid,
      4-(4-chlorophenoxy)butyric acid,
      3,6-dichloro-2-methoxybenzoic acid (dicamba),
      1-(ethoxycarbonyl)ethyl 3,6-dichloro-2-methoxybenzoate (lactidichlor-ethyl).

Preferred safeners in combination with the compounds of the general formula (I) according to the invention and/or salts thereof, in particular with the compounds of the formulae (I-1) to (I-84) and and/or salts thereof, are: cloquintocet-mexyl, cyprosulfamide, fenchlorazole-ethyl, isoxadifen-ethyl, mefenpyr-diethyl, fenclorim, cumyluron, S1-14, S1-15, S1-16, S1-17, S1-18, S1-19, S4-1 and S4-5, and particularly preferred safeners are: cloquintocet-mexyl, cyprosulfamide, isoxadifen-ethyl and mefenpyr-diethyl.

### Biological examples:

The following abbreviations are used in the examples and tables below:

### Tested harmful plants:

- AGSTE:: Agrostis capillari
- DIPTE:: Diplotaxis tenuifolia
- LOLPE:: Lolium perenne
- MATCH:: Matricaria chamomilla
- POAAN:: Poa annua
- SETVI:: Setaria viridis
- STEME:: Stellaria media
- VERPE:: Veronica persica

### A. Herbicidal microtiter plate efficacy

Seeds from mono- and dicotyledonous plants were sown in quartz sand in 96-well microtiter plates and grown in a climate chamber under controlled growth conditions. Five to seven days after sowing, the test plants were treated at the leafless stage with the test compound, formulated in the form of an emulsifiable concentrate, at an application rate of approximately 2200 liters of water per hectare. The test plants were placed in a climate chamber under optimum growth conditions and then the effect of the compound was visually assessed in comparison to untreated controls (herbicidal effect in percent (%): 100% effect = plants have died, 0% effect = as control plants). nine to twelve days of growing.

Tables A1 to A8, below, show the effects of selected compounds of the general formula (I) according to Table 1 on various harmful plants and an application rate corresponding to 950 g/ha obtained by the experimental procedure mentioned above.

**Table A1**

| Example Number | Dosage [g/ha] | AGSTE |
|---|---|---|
| I-001 | 950 | 80 |
| I-002 | 950 | 80 |
| I-003 | 950 | 80 |
| I-004 | 950 | 80 |
| I-005 | 950 | 80 |
| I-006 | 950 | 80 |
| I-007 | 950 | 80 |
| I-008 | 950 | 80 |
| I-139 | 950 | 80 |
| I-142 | 950 | 80 |
| I-020 | 950 | 80 |
| I-021 | 950 | 80 |
| I-022 | 950 | 80 |
| I-023 | 950 | 80 |
| I-024 | 950 | 80 |
| I-025 | 950 | 80 |
| I-026 | 950 | 80 |
| I-027 | 950 | 80 |
| I-028 | 950 | 80 |
| I-029 | 950 | 80 |
| I-030 | 950 | 80 |
| I-031 | 950 | 80 |
| I-032 | 950 | 80 |
| I-033 | 950 | 80 |
| I-034 | 950 | 80 |
| I-035 | 950 | 80 |
| I-036 | 950 | 80 |
| I-037 | 950 | 80 |
| I-038 | 950 | 80 |
| I-039 | 950 | 80 |
| I-040 | 950 | 80 |
| I-041 | 950 | 80 |
| I-042 | 950 | 80 |
| I-043 | 950 | 80 |
| I-044 | 950 | 80 |
| I-045 | 950 | 80 |
| I-046 | 950 | 80 |
| I-047 | 950 | 80 |
| I-048 | 950 | 80 |
| I-049 | 950 | 80 |
| I-050 | 950 | 80 |
| I-051 | 950 | 100 |
| I-052 | 950 | 80 |
| I-053 | 950 | 80 |
| I-054 | 950 | 100 |
| I-055 | 950 | 100 |
| I-056 | 950 | 80 |
| I-057 | 950 | 80 |
| I-059 | 950 | 80 |
| I-060 | 950 | 80 |
| I-061 | 950 | 80 |
| I-062 | 950 | 80 |
| I-063 | 950 | 80 |
| I-064 | 950 | 80 |
| I-066 | 950 | 80 |
| I-067 | 950 | 80 |
| I-068 | 950 | 80 |
| I-069 | 950 | 80 |
| I-070 | 950 | 80 |
| I-071 | 950 | 80 |
| I-072 | 950 | 80 |
| I-073 | 950 | 80 |
| I-074 | 950 | 80 |
| I-078 | 950 | 80 |
| I-079 | 950 | 80 |
| I-080 | 950 | 80 |
| I-081 | 950 | 80 |
| I-083 | 950 | 80 |
| I-084 | 950 | 80 |
| I-085 | 950 | 80 |
| I-086 | 950 | 80 |
| I-087 | 950 | 80 |
| I-088 | 950 | 80 |
| I-090 | 950 | 80 |
| I-091 | 950 | 80 |
| I-092 | 950 | 80 |
| I-093 | 950 | 80 |
| I-095 | 950 | 80 |
| I-097 | 950 | 80 |
| I-098 | 950 | 80 |
| I-099 | 950 | 80 |
| I-101 | 950 | 80 |
| I-102 | 950 | 80 |
| I-103 | 950 | 80 |
| I-104 | 950 | 80 |
| I-105 | 950 | 80 |
| I-106 | 950 | 80 |
| I-107 | 950 | 80 |
| I-108 | 950 | 80 |
| I-109 | 950 | 80 |
| I-110 | 950 | 80 |
| I-111 | 950 | 80 |
| I-112 | 950 | 80 |
| I-113 | 950 | 80 |
| I-114 | 950 | 80 |
| I-115 | 950 | 80 |
| I-116 | 950 | 80 |
| I-117 | 950 | 80 |
| I-118 | 950 | 80 |
| I-119 | 950 | 80 |
| I-120 | 950 | 80 |
| I-121 | 950 | 80 |
| I-122 | 950 | 80 |
| I-126 | 950 | 80 |
| I-128 | 950 | 80 |
| I-129 | 950 | 80 |
| I-130 | 950 | 80 |

**Table A2**

| Example Number | Dosage [g/ha] | LOLPE |
|---|---|---|
| I-001 | 950 | 80 |
| I-002 | 950 | 80 |
| I-003 | 950 | 80 |
| I-004 | 950 | 80 |
| I-005 | 950 | 80 |
| I-006 | 950 | 80 |
| I-007 | 950 | 80 |
| I-008 | 950 | 80 |
| I-020 | 950 | 80 |
| I-021 | 950 | 80 |
| I-022 | 950 | 80 |
| I-023 | 950 | 80 |
| I-024 | 950 | 80 |
| I-025 | 950 | 80 |
| I-026 | 950 | 80 |
| I-027 | 950 | 80 |
| I-028 | 950 | 80 |
| I-029 | 950 | 80 |
| I-031 | 950 | 80 |
| I-032 | 950 | 80 |
| I-033 | 950 | 80 |
| I-034 | 950 | 80 |
| I-035 | 950 | 80 |
| I-036 | 950 | 80 |
| I-037 | 950 | 80 |
| I-038 | 950 | 80 |
| I-039 | 950 | 80 |
| I-040 | 950 | 80 |
| I-041 | 950 | 80 |
| I-042 | 950 | 80 |
| I-044 | 950 | 80 |
| I-045 | 950 | 100 |
| I-046 | 950 | 80 |
| I-047 | 950 | 80 |
| I-048 | 950 | 80 |
| I-049 | 950 | 100 |
| I-051 | 950 | 100 |
| I-052 | 950 | 100 |
| I-053 | 950 | 80 |
| I-054 | 950 | 100 |
| I-055 | 950 | 80 |
| I-056 | 950 | 80 |
| I-057 | 950 | 100 |
| I-061 | 950 | 80 |
| I-062 | 950 | 80 |
| I-063 | 950 | 80 |
| I-067 | 950 | 80 |
| I-069 | 950 | 80 |
| I-071 | 950 | 80 |
| I-072 | 950 | 80 |
| I-078 | 950 | 80 |
| I-080 | 950 | 80 |
| I-081 | 950 | 80 |
| I-083 | 950 | 100 |
| I-084 | 950 | 80 |
| I-085 | 950 | 100 |
| I-086 | 950 | 100 |
| I-087 | 950 | 100 |
| I-088 | 950 | 100 |
| I-089 | 950 | 100 |
| I-090 | 950 | 100 |
| I-091 | 950 | 80 |
| I-092 | 950 | 80 |
| I-093 | 950 | 80 |
| I-096 | 950 | 100 |
| I-097 | 950 | 100 |
| I-099 | 950 | 80 |
| I-101 | 950 | 80 |
| I-102 | 950 | 80 |
| I-104 | 950 | 80 |
| I-105 | 950 | 80 |
| I-106 | 950 | 80 |
| I-109 | 950 | 80 |
| I-110 | 950 | 80 |
| I-111 | 950 | 80 |
| I-112 | 950 | 80 |
| I-113 | 950 | 80 |
| I-114 | 950 | 80 |
| I-117 | 950 | 80 |
| I-119 | 950 | 80 |
| I-120 | 950 | 100 |
| I-121 | 950 | 80 |
| I-122 | 950 | 80 |
| I-126 | 950 | 80 |
| I-127 | 950 | 80 |
| I-128 | 950 | 80 |
| I-130 | 950 | 80 |
| I-134 | 950 | 80 |

**Table A3**

| Example Number | Dosage [g/ha] | POAAN |
|---|---|---|
| I-001 | 950 | 80 |
| I-002 | 950 | 100 |
| I-003 | 950 | 100 |
| I-004 | 950 | 80 |
| I-005 | 950 | 80 |
| I-006 | 950 | 80 |
| I-007 | 950 | 80 |
| I-008 | 950 | 80 |
| I-020 | 950 | 80 |
| I-021 | 950 | 80 |
| I-022 | 950 | 80 |
| I-023 | 950 | 80 |
| I-024 | 950 | 80 |
| I-025 | 950 | 80 |
| I-026 | 950 | 80 |
| I-027 | 950 | 80 |
| I-028 | 950 | 80 |
| I-029 | 950 | 80 |
| I-030 | 950 | 80 |
| I-031 | 950 | 80 |
| I-032 | 950 | 80 |
| I-033 | 950 | 80 |
| I-034 | 950 | 80 |
| I-035 | 950 | 100 |
| I-036 | 950 | 80 |
| I-037 | 950 | 100 |
| I-038 | 950 | 80 |
| I-039 | 950 | 80 |
| I-040 | 950 | 80 |
| I-041 | 950 | 80 |
| I-042 | 950 | 80 |
| I-043 | 950 | 80 |
| I-044 | 950 | 80 |
| I-045 | 950 | 100 |
| I-046 | 950 | 80 |
| I-047 | 950 | 80 |
| I-048 | 950 | 100 |
| I-049 | 950 | 100 |
| I-050 | 950 | 80 |
| I-051 | 950 | 100 |
| I-052 | 950 | 100 |
| I-053 | 950 | 80 |
| I-054 | 950 | 100 |
| I-055 | 950 | 100 |
| I-056 | 950 | 80 |
| I-057 | 950 | 80 |
| I-059 | 950 | 80 |
| I-060 | 950 | 80 |
| I-061 | 950 | 80 |
| I-062 | 950 | 80 |
| I-063 | 950 | 80 |
| I-064 | 950 | 80 |
| I-067 | 950 | 80 |
| I-069 | 950 | 100 |
| I-070 | 950 | 80 |
| I-071 | 950 | 80 |
| I-072 | 950 | 80 |
| I-073 | 950 | 80 |
| I-074 | 950 | 80 |
| I-075 | 950 | 80 |
| I-078 | 950 | 80 |
| I-079 | 950 | 80 |
| I-080 | 950 | 80 |
| I-081 | 950 | 80 |
| I-083 | 950 | 80 |
| I-084 | 950 | 80 |
| I-085 | 950 | 80 |
| I-086 | 950 | 80 |
| I-087 | 950 | 80 |
| I-088 | 950 | 80 |
| I-089 | 950 | 80 |
| I-090 | 950 | 80 |
| I-093 | 950 | 80 |
| I-094 | 950 | 80 |
| I-095 | 950 | 80 |
| I-096 | 950 | 80 |
| I-097 | 950 | 80 |
| I-099 | 950 | 80 |
| I-100 | 950 | 80 |
| I-101 | 950 | 80 |
| I-102 | 950 | 80 |
| I-103 | 950 | 80 |
| I-104 | 950 | 80 |
| I-105 | 950 | 80 |
| I-106 | 950 | 80 |
| I-107 | 950 | 80 |
| I-108 | 950 | 80 |
| I-109 | 950 | 80 |
| I-110 | 950 | 80 |
| I-111 | 950 | 80 |
| I-112 | 950 | 80 |
| I-113 | 950 | 80 |
| I-114 | 950 | 80 |
| I-115 | 950 | 80 |
| I-116 | 950 | 80 |
| I-117 | 950 | 80 |
| I-119 | 950 | 80 |
| I-120 | 950 | 80 |
| I-121 | 950 | 80 |
| I-122 | 950 | 80 |
| I-126 | 950 | 80 |
| I-128 | 950 | 80 |
| I-129 | 950 | 80 |
| I-130 | 950 | 80 |
| I-134 | 950 | 80 |

**Table A4**

| Example Number | Dosage [g/ha] | SETVI |
|---|---|---|
| I-001 | 950 | 80 |
| I-002 | 950 | 100 |
| I-003 | 950 | 100 |
| I-005 | 950 | 80 |
| I-006 | 950 | 80 |
| I-007 | 950 | 80 |
| I-008 | 950 | 80 |
| I-020 | 950 | 80 |
| I-023 | 950 | 80 |
| I-024 | 950 | 80 |
| I-025 | 950 | 80 |
| I-026 | 950 | 80 |
| I-027 | 950 | 80 |
| I-028 | 950 | 80 |
| I-029 | 950 | 80 |
| I-031 | 950 | 80 |
| I-032 | 950 | 80 |
| I-033 | 950 | 80 |
| I-034 | 950 | 80 |
| I-035 | 950 | 80 |
| I-036 | 950 | 80 |
| I-037 | 950 | 80 |
| I-038 | 950 | 80 |
| I-039 | 950 | 80 |
| I-040 | 950 | 80 |
| I-041 | 950 | 80 |
| I-042 | 950 | 80 |
| I-044 | 950 | 80 |
| I-045 | 950 | 100 |
| I-046 | 950 | 80 |
| I-047 | 950 | 80 |
| I-048 | 950 | 80 |
| I-049 | 950 | 80 |
| I-050 | 950 | 80 |
| I-051 | 950 | 100 |
| I-052 | 950 | 80 |
| I-053 | 950 | 80 |
| I-054 | 950 | 100 |
| I-055 | 950 | 100 |
| I-057 | 950 | 80 |
| I-061 | 950 | 80 |
| I-063 | 950 | 80 |
| I-067 | 950 | 80 |
| I-069 | 950 | 100 |
| I-071 | 950 | 80 |
| I-072 | 950 | 80 |
| I-078 | 950 | 80 |
| I-080 | 950 | 80 |
| I-081 | 950 | 80 |
| I-083 | 950 | 80 |
| I-084 | 950 | 80 |
| I-085 | 950 | 80 |
| I-087 | 950 | 80 |
| I-088 | 950 | 100 |
| I-093 | 950 | 100 |
| I-095 | 950 | 80 |
| I-097 | 950 | 100 |
| I-099 | 950 | 80 |
| I-102 | 950 | 80 |
| I-103 | 950 | 80 |
| I-104 | 950 | 80 |
| I-105 | 950 | 80 |
| I-106 | 950 | 80 |
| I-107 | 950 | 80 |
| I-109 | 950 | 80 |
| I-110 | 950 | 80 |
| I-111 | 950 | 80 |
| I-112 | 950 | 80 |
| I-113 | 950 | 80 |
| I-114 | 950 | 80 |
| I-119 | 950 | 80 |
| I-120 | 950 | 80 |
| I-121 | 950 | 80 |
| I-122 | 950 | 80 |
| I-126 | 950 | 80 |
| I-129 | 950 | 100 |
| I-130 | 950 | 80 |

**Table A5**

| Example Number | Dosage [g/ha] | DIPTE |
|---|---|---|
| I-005 | 950 | 80 |
| I-006 | 950 | 80 |
| I-007 | 950 | 80 |
| I-008 | 950 | 80 |
| I-018 | 950 | 80 |
| I-020 | 950 | 80 |
| I-021 | 950 | 80 |
| I-023 | 950 | 80 |
| I-024 | 950 | 80 |
| I-025 | 950 | 80 |
| I-026 | 950 | 80 |
| I-027 | 950 | 80 |
| I-028 | 950 | 80 |
| I-031 | 950 | 80 |
| I-032 | 950 | 80 |
| I-033 | 950 | 80 |
| I-034 | 950 | 80 |
| I-038 | 950 | 80 |
| I-039 | 950 | 80 |
| I-040 | 950 | 80 |
| I-041 | 950 | 80 |
| I-042 | 950 | 80 |
| I-044 | 950 | 80 |
| I-045 | 950 | 80 |
| I-046 | 950 | 80 |
| I-048 | 950 | 80 |
| I-049 | 950 | 80 |
| I-050 | 950 | 80 |
| I-053 | 950 | 80 |
| I-054 | 950 | 80 |
| I-055 | 950 | 80 |
| I-056 | 950 | 80 |
| I-059 | 950 | 80 |
| I-060 | 950 | 80 |
| I-061 | 950 | 80 |
| I-063 | 950 | 80 |
| I-067 | 950 | 80 |
| I-069 | 950 | 80 |
| I-071 | 950 | 80 |
| I-072 | 950 | 80 |
| I-078 | 950 | 80 |
| I-080 | 950 | 80 |
| I-081 | 950 | 80 |
| I-084 | 950 | 80 |
| I-085 | 950 | 80 |
| I-086 | 950 | 80 |
| I-087 | 950 | 80 |
| I-090 | 950 | 80 |
| I-093 | 950 | 80 |
| I-095 | 950 | 80 |
| I-097 | 950 | 80 |
| I-103 | 950 | 80 |
| I-104 | 950 | 80 |
| I-105 | 950 | 80 |
| I-106 | 950 | 80 |
| I-108 | 950 | 80 |
| I-109 | 950 | 80 |
| I-110 | 950 | 80 |
| I-111 | 950 | 80 |
| I-112 | 950 | 80 |
| I-113 | 950 | 80 |
| I-114 | 950 | 80 |
| I-119 | 950 | 80 |
| I-120 | 950 | 80 |
| I-121 | 950 | 80 |
| I-122 | 950 | 80 |
| I-126 | 950 | 80 |
| I-128 | 950 | 80 |
| I-129 | 950 | 80 |

**Table A6**

| Example Number | Dosage [g/ha] | MATCH |
|---|---|---|
| I-002 | 950 | 80 |
| I-003 | 950 | 80 |
| I-005 | 950 | 80 |
| I-006 | 950 | 80 |
| I-008 | 950 | 80 |
| I-021 | 950 | 80 |
| I-024 | 950 | 80 |
| I-026 | 950 | 80 |
| I-027 | 950 | 80 |
| I-028 | 950 | 80 |
| I-029 | 950 | 80 |
| I-031 | 950 | 80 |
| I-032 | 950 | 80 |
| I-033 | 950 | 80 |
| I-034 | 950 | 80 |
| I-035 | 950 | 80 |
| I-040 | 950 | 80 |
| I-041 | 950 | 80 |
| I-044 | 950 | 80 |
| I-045 | 950 | 80 |
| I-046 | 950 | 80 |
| I-047 | 950 | 80 |
| I-048 | 950 | 80 |
| I-050 | 950 | 80 |
| I-051 | 950 | 100 |
| I-052 | 950 | 80 |
| I-053 | 950 | 80 |
| I-054 | 950 | 100 |
| I-055 | 950 | 100 |
| I-056 | 950 | 80 |
| I-061 | 950 | 80 |
| I-063 | 950 | 80 |
| I-064 | 950 | 80 |
| I-065 | 950 | 80 |
| I-067 | 950 | 80 |
| I-069 | 950 | 80 |
| I-071 | 950 | 80 |
| I-072 | 950 | 80 |
| I-073 | 950 | 80 |
| I-074 | 950 | 80 |
| I-075 | 950 | 80 |
| I-078 | 950 | 80 |
| I-079 | 950 | 80 |
| I-080 | 950 | 80 |
| I-081 | 950 | 80 |
| I-083 | 950 | 80 |
| I-084 | 950 | 80 |
| I-085 | 950 | 80 |
| I-086 | 950 | 80 |
| I-087 | 950 | 80 |
| I-088 | 950 | 80 |
| I-091 | 950 | 80 |
| I-092 | 950 | 80 |
| I-093 | 950 | 80 |
| I-096 | 950 | 80 |
| I-097 | 950 | 80 |
| I-098 | 950 | 80 |
| I-099 | 950 | 80 |
| I-101 | 950 | 80 |
| I-102 | 950 | 80 |
| I-103 | 950 | 80 |
| I-104 | 950 | 80 |
| I-105 | 950 | 80 |
| I-106 | 950 | 80 |
| I-108 | 950 | 80 |
| I-109 | 950 | 80 |
| I-110 | 950 | 80 |
| I-111 | 950 | 80 |
| I-112 | 950 | 80 |
| I-113 | 950 | 80 |
| I-114 | 950 | 80 |
| I-115 | 950 | 80 |
| I-118 | 950 | 80 |
| I-119 | 950 | 80 |
| I-120 | 950 | 80 |
| I-121 | 950 | 80 |
| I-122 | 950 | 80 |
| I-126 | 950 | 80 |
| I-130 | 950 | 80 |

**Table A7**

| Example Number | Dosage [g/ha] | STEME |
|---|---|---|
| I-001 | 950 | 80 |
| I-002 | 950 | 80 |
| I-003 | 950 | 100 |
| I-004 | 950 | 80 |
| I-005 | 950 | 80 |
| I-006 | 950 | 80 |
| I-007 | 950 | 80 |
| I-008 | 950 | 80 |
| I-011 | 950 | 80 |
| I-020 | 950 | 80 |
| I-022 | 950 | 80 |
| I-023 | 950 | 80 |
| I-024 | 950 | 80 |
| I-025 | 950 | 80 |
| I-026 | 950 | 80 |
| I-028 | 950 | 80 |
| I-029 | 950 | 80 |
| I-031 | 950 | 80 |
| I-032 | 950 | 80 |
| I-034 | 950 | 80 |
| I-036 | 950 | 80 |
| I-037 | 950 | 80 |
| I-038 | 950 | 80 |
| I-039 | 950 | 80 |
| I-040 | 950 | 80 |
| I-041 | 950 | 80 |
| I-043 | 950 | 80 |
| I-044 | 950 | 80 |
| I-045 | 950 | 100 |
| I-046 | 950 | 80 |
| I-047 | 950 | 80 |
| I-048 | 950 | 80 |
| I-049 | 950 | 80 |
| I-052 | 950 | 80 |
| I-053 | 950 | 80 |
| I-054 | 950 | 80 |
| I-055 | 950 | 80 |
| I-056 | 950 | 80 |
| I-069 | 950 | 80 |
| I-071 | 950 | 80 |
| I-072 | 950 | 80 |
| I-074 | 950 | 80 |
| I-078 | 950 | 80 |
| I-080 | 950 | 80 |
| I-081 | 950 | 80 |
| I-083 | 950 | 80 |
| I-084 | 950 | 80 |
| I-086 | 950 | 80 |
| I-087 | 950 | 80 |
| I-088 | 950 | 80 |
| I-089 | 950 | 80 |
| I-090 | 950 | 80 |
| I-093 | 950 | 80 |
| I-095 | 950 | 80 |
| I-096 | 950 | 80 |
| I-097 | 950 | 80 |
| I-098 | 950 | 80 |
| I-100 | 950 | 80 |
| I-101 | 950 | 80 |
| I-102 | 950 | 80 |
| I-103 | 950 | 80 |
| I-104 | 950 | 80 |
| I-105 | 950 | 80 |
| I-106 | 950 | 80 |
| I-108 | 950 | 80 |
| I-109 | 950 | 80 |
| I-110 | 950 | 80 |
| I-111 | 950 | 80 |
| I-112 | 950 | 80 |
| I-113 | 950 | 80 |
| I-114 | 950 | 80 |
| I-115 | 950 | 80 |
| I-117 | 950 | 80 |
| I-118 | 950 | 80 |
| I-119 | 950 | 80 |
| I-120 | 950 | 80 |
| I-121 | 950 | 80 |
| I-122 | 950 | 80 |
| I-126 | 950 | 80 |
| I-127 | 950 | 80 |
| I-134 | 950 | 80 |

**Table A8**

| Example Number | Dosage [g/ha] | VERPE |
|---|---|---|
| I-137 | 950 | 80 |
| I-002 | 950 | 80 |
| I-003 | 950 | 80 |
| I-005 | 950 | 80 |
| I-006 | 950 | 80 |
| I-008 | 950 | 80 |
| I-024 | 950 | 80 |
| I-025 | 950 | 80 |
| I-026 | 950 | 80 |
| I-027 | 950 | 80 |
| I-028 | 950 | 80 |
| I-029 | 950 | 80 |
| I-030 | 950 | 80 |
| I-031 | 950 | 80 |
| I-032 | 950 | 80 |
| I-033 | 950 | 80 |
| I-034 | 950 | 80 |
| I-035 | 950 | 80 |
| I-040 | 950 | 80 |
| I-041 | 950 | 80 |
| I-044 | 950 | 80 |
| I-046 | 950 | 80 |
| I-047 | 950 | 80 |
| I-048 | 950 | 80 |
| I-049 | 950 | 80 |
| I-053 | 950 | 80 |
| I-056 | 950 | 80 |
| I-060 | 950 | 80 |
| I-061 | 950 | 80 |
| I-062 | 950 | 80 |
| I-063 | 950 | 80 |
| I-067 | 950 | 80 |
| I-069 | 950 | 80 |
| I-070 | 950 | 80 |
| I-071 | 950 | 80 |
| I-072 | 950 | 80 |
| I-074 | 950 | 80 |
| I-078 | 950 | 80 |
| I-079 | 950 | 80 |
| I-080 | 950 | 80 |
| I-082 | 950 | 80 |
| I-084 | 950 | 80 |
| I-086 | 950 | 80 |
| I-087 | 950 | 80 |
| I-088 | 950 | 80 |
| I-089 | 950 | 80 |
| I-090 | 950 | 80 |
| I-095 | 950 | 80 |
| I-102 | 950 | 80 |
| I-103 | 950 | 80 |
| I-104 | 950 | 80 |
| I-105 | 950 | 80 |
| I-106 | 950 | 80 |
| I-108 | 950 | 80 |
| I-109 | 950 | 80 |
| I-110 | 950 | 80 |
| I-111 | 950 | 80 |
| I-112 | 950 | 80 |
| I-113 | 950 | 80 |
| I-114 | 950 | 80 |
| I-115 | 950 | 80 |
| I-121 | 950 | 80 |
| I-126 | 950 | 80 |
| I-127 | 950 | 80 |

As the results show, various compounds of the general formula (I) according to the invention have very good herbicidal microtiter plate efficacy against a broad spectrum of harmful mono- and dicotyledonous plants such as Agrostis capillari (AGSTE), Diplotaxis tenuifolia (DIPTE), Lolium perenne (LOLPE), Matricaria chamomilla (MATCH), Poa annua (POAAN), Setaria viridis (SETVI), Stellaria media (STEME) and Veronica persica (VERPE) at an application rate of 950 g of active ingredient per hectare.

### B. Herbicidal microtiter plate efficacy

Seeds from mono- and dicotyledonous plants were sown in quartz sand in 96-well microtiter plates and grown in a climate chamber under controlled growth conditions. Five to seven days after sowing, the test plants were treated at the leafless stage with the test compound, formulated in the form of an emulsifiable concentrate, at an application rate of approximately 2200 liters of water per hectare. The test plants were placed in a climate chamber under optimum growth conditions and then the effect of the compound was visually assessed in comparison to untreated controls (herbicidal effect in percent (%): 100% effect = plants have died, 0% effect = as control plants). nine to twelve days of growing.

Tables B1 to B8, below, show the effects of selected compounds of the general formula (I) according to Table 1 on various harmful plants and an application rate corresponding to 238 g/ha obtained by the experimental procedure mentioned above.

**Table B1**

| Example Number | Dosage [g/ha] | AGSTE |
|---|---|---|
| I-001 | 238 | 80 |
| I-002 | 238 | 80 |
| I-003 | 238 | 80 |
| I-004 | 238 | 80 |
| I-005 | 238 | 80 |
| I-006 | 238 | 80 |
| I-007 | 238 | 80 |
| I-008 | 238 | 80 |
| I-020 | 238 | 80 |
| I-021 | 238 | 80 |
| I-022 | 238 | 80 |
| I-023 | 238 | 80 |
| I-024 | 238 | 80 |
| I-025 | 238 | 80 |
| I-026 | 238 | 80 |
| I-027 | 238 | 80 |
| I-028 | 238 | 80 |
| I-029 | 238 | 80 |
| I-030 | 238 | 80 |
| I-031 | 238 | 80 |
| I-032 | 238 | 80 |
| I-033 | 238 | 80 |
| I-034 | 238 | 80 |
| I-035 | 238 | 80 |
| I-036 | 238 | 80 |
| I-037 | 238 | 80 |
| I-038 | 238 | 80 |
| I-039 | 238 | 80 |
| I-040 | 238 | 80 |
| I-041 | 238 | 80 |
| I-042 | 238 | 80 |
| I-044 | 238 | 80 |
| I-045 | 238 | 100 |
| I-046 | 238 | 80 |
| I-047 | 238 | 80 |
| I-049 | 238 | 80 |
| I-050 | 238 | 80 |
| I-051 | 238 | 80 |
| I-052 | 238 | 80 |
| I-053 | 238 | 80 |
| I-054 | 238 | 100 |
| I-055 | 238 | 100 |
| I-056 | 238 | 80 |
| I-057 | 238 | 80 |
| I-059 | 238 | 80 |
| I-061 | 238 | 80 |
| I-066 | 238 | 80 |
| I-067 | 238 | 80 |
| I-069 | 238 | 80 |
| I-071 | 238 | 80 |
| I-072 | 238 | 80 |
| I-080 | 238 | 80 |
| I-081 | 238 | 80 |
| I-083 | 238 | 80 |
| I-084 | 238 | 80 |
| I-085 | 238 | 80 |
| I-086 | 238 | 80 |
| I-087 | 238 | 80 |
| I-090 | 238 | 80 |
| I-091 | 238 | 80 |
| I-093 | 238 | 80 |
| I-095 | 238 | 80 |
| I-097 | 238 | 80 |
| I-098 | 238 | 80 |
| I-099 | 238 | 100 |
| I-101 | 238 | 80 |
| I-102 | 238 | 80 |
| I-103 | 238 | 80 |
| I-104 | 238 | 80 |
| I-105 | 238 | 80 |
| I-106 | 238 | 80 |
| I-107 | 238 | 80 |
| I-109 | 238 | 80 |
| I-110 | 238 | 80 |
| I-111 | 238 | 80 |
| I-112 | 238 | 80 |
| I-113 | 238 | 80 |
| I-114 | 238 | 80 |
| I-115 | 238 | 80 |
| I-116 | 238 | 80 |
| I-117 | 238 | 80 |
| I-118 | 238 | 80 |
| I-119 | 238 | 80 |
| I-120 | 238 | 80 |
| I-121 | 238 | 80 |
| I-122 | 238 | 80 |
| I-126 | 238 | 80 |
| I-128 | 238 | 80 |
| I-129 | 238 | 80 |

**Table B2**

| Example Number | Dosage [g/ha] | LOLPE |
|---|---|---|
| I-002 | 238 | 80 |
| I-003 | 238 | 80 |
| I-004 | 238 | 80 |
| I-005 | 238 | 80 |
| I-006 | 238 | 80 |
| I-008 | 238 | 80 |
| I-020 | 238 | 80 |
| I-021 | 238 | 80 |
| I-023 | 238 | 80 |
| I-024 | 238 | 80 |
| I-025 | 238 | 80 |
| I-026 | 238 | 80 |
| I-027 | 238 | 80 |
| I-028 | 238 | 80 |
| I-029 | 238 | 80 |
| I-031 | 238 | 80 |
| I-033 | 238 | 80 |
| I-034 | 238 | 80 |
| I-036 | 238 | 80 |
| I-037 | 238 | 80 |
| I-038 | 238 | 80 |
| I-039 | 238 | 80 |
| I-040 | 238 | 80 |
| I-041 | 238 | 80 |
| I-042 | 238 | 80 |
| I-046 | 238 | 80 |
| I-047 | 238 | 80 |
| I-049 | 238 | 80 |
| I-050 | 238 | 80 |
| I-051 | 238 | 100 |
| I-052 | 238 | 80 |
| I-053 | 238 | 80 |
| I-054 | 238 | 100 |
| I-055 | 238 | 80 |
| I-056 | 238 | 80 |
| I-057 | 238 | 80 |
| I-061 | 238 | 80 |
| I-067 | 238 | 80 |
| I-069 | 238 | 80 |
| I-071 | 238 | 80 |
| I-072 | 238 | 80 |
| I-078 | 238 | 80 |
| I-081 | 238 | 80 |
| I-083 | 238 | 80 |
| I-084 | 238 | 100 |
| I-085 | 238 | 80 |
| I-086 | 238 | 100 |
| I-088 | 238 | 100 |
| I-090 | 238 | 100 |
| I-093 | 238 | 100 |
| I-096 | 238 | 100 |
| I-097 | 238 | 100 |
| I-099 | 238 | 80 |
| I-103 | 238 | 80 |
| I-104 | 238 | 80 |
| I-105 | 238 | 80 |
| I-106 | 238 | 80 |
| I-109 | 238 | 80 |
| I-110 | 238 | 80 |
| I-111 | 238 | 80 |
| I-112 | 238 | 80 |
| I-113 | 238 | 80 |
| I-114 | 238 | 80 |
| I-120 | 238 | 80 |
| I-121 | 238 | 100 |
| I-122 | 238 | 100 |
| I-126 | 238 | 80 |
| I-128 | 238 | 80 |
| I-129 | 238 | 80 |
| I-130 | 238 | 80 |

**Table B3**

| Example Number | Dosage [g/ha] | POAAN |
|---|---|---|
| I-136 | 238 | 80 |
| I-001 | 238 | 80 |
| I-002 | 238 | 100 |
| I-003 | 238 | 100 |
| I-004 | 238 | 80 |
| I-005 | 238 | 80 |
| I-006 | 238 | 80 |
| I-007 | 238 | 80 |
| I-008 | 238 | 80 |
| I-020 | 238 | 80 |
| I-021 | 238 | 80 |
| I-022 | 238 | 80 |
| I-023 | 238 | 100 |
| I-024 | 238 | 80 |
| I-025 | 238 | 80 |
| I-026 | 238 | 80 |
| I-027 | 238 | 80 |
| I-028 | 238 | 80 |
| I-029 | 238 | 80 |
| I-030 | 238 | 80 |
| I-031 | 238 | 80 |
| I-032 | 238 | 80 |
| I-033 | 238 | 80 |
| I-034 | 238 | 80 |
| I-035 | 238 | 100 |
| I-036 | 238 | 80 |
| I-037 | 238 | 80 |
| I-038 | 238 | 80 |
| I-039 | 238 | 80 |
| I-040 | 238 | 80 |
| I-041 | 238 | 80 |
| I-042 | 238 | 80 |
| I-044 | 238 | 80 |
| I-045 | 238 | 100 |
| I-046 | 238 | 80 |
| I-047 | 238 | 80 |
| I-048 | 238 | 100 |
| I-049 | 238 | 100 |
| I-050 | 238 | 80 |
| I-051 | 238 | 100 |
| I-052 | 238 | 100 |
| I-053 | 238 | 80 |
| I-054 | 238 | 100 |
| I-055 | 238 | 100 |
| I-056 | 238 | 80 |
| I-057 | 238 | 80 |
| I-059 | 238 | 80 |
| I-061 | 238 | 80 |
| I-062 | 238 | 80 |
| I-063 | 238 | 80 |
| I-067 | 238 | 80 |
| I-069 | 238 | 100 |
| I-071 | 238 | 80 |
| I-072 | 238 | 80 |
| I-080 | 238 | 80 |
| I-081 | 238 | 80 |
| I-083 | 238 | 80 |
| I-084 | 238 | 80 |
| I-085 | 238 | 80 |
| I-086 | 238 | 80 |
| I-087 | 238 | 80 |
| I-088 | 238 | 80 |
| I-089 | 238 | 80 |
| I-090 | 238 | 80 |
| I-093 | 238 | 80 |
| I-097 | 238 | 80 |
| I-099 | 238 | 80 |
| I-100 | 238 | 80 |
| I-101 | 238 | 80 |
| I-103 | 238 | 80 |
| I-104 | 238 | 80 |
| I-105 | 238 | 80 |
| I-106 | 238 | 80 |
| I-107 | 238 | 80 |
| I-108 | 238 | 80 |
| I-109 | 238 | 80 |
| I-110 | 238 | 80 |
| I-111 | 238 | 80 |
| I-112 | 238 | 80 |
| I-113 | 238 | 80 |
| I-114 | 238 | 80 |
| I-116 | 238 | 80 |
| I-117 | 238 | 80 |
| I-119 | 238 | 80 |
| I-120 | 238 | 80 |
| I-121 | 238 | 80 |
| I-122 | 238 | 80 |
| I-126 | 238 | 80 |
| I-128 | 238 | 80 |
| I-129 | 238 | 80 |
| I-134 | 238 | 80 |

**Table B4**

| Example Number | Dosage [g/ha] | SETVI |
|---|---|---|
| I-002 | 238 | 80 |
| I-003 | 238 | 80 |
| I-005 | 238 | 80 |
| I-006 | 238 | 80 |
| I-007 | 238 | 80 |
| I-008 | 238 | 80 |
| I-021 | 238 | 80 |
| I-023 | 238 | 80 |
| I-024 | 238 | 80 |
| I-025 | 238 | 80 |
| I-026 | 238 | 80 |
| I-027 | 238 | 80 |
| I-028 | 238 | 80 |
| I-029 | 238 | 80 |
| I-031 | 238 | 80 |
| I-032 | 238 | 80 |
| I-033 | 238 | 80 |
| I-036 | 238 | 80 |
| I-037 | 238 | 80 |
| I-038 | 238 | 80 |
| I-040 | 238 | 80 |
| I-041 | 238 | 80 |
| I-042 | 238 | 80 |
| I-045 | 238 | 100 |
| I-046 | 238 | 80 |
| I-047 | 238 | 80 |
| I-049 | 238 | 80 |
| I-050 | 238 | 80 |
| I-052 | 238 | 80 |
| I-053 | 238 | 80 |
| I-054 | 238 | 100 |
| I-055 | 238 | 100 |
| I-057 | 238 | 80 |
| I-061 | 238 | 80 |
| I-067 | 238 | 80 |
| I-069 | 238 | 100 |
| I-071 | 238 | 80 |
| I-072 | 238 | 80 |
| I-083 | 238 | 100 |
| I-084 | 238 | 100 |
| I-085 | 238 | 100 |
| I-087 | 238 | 80 |
| I-090 | 238 | 80 |
| I-093 | 238 | 100 |
| I-097 | 238 | 100 |
| I-098 | 238 | 80 |
| I-102 | 238 | 80 |
| I-104 | 238 | 80 |
| I-105 | 238 | 80 |
| I-106 | 238 | 80 |
| I-107 | 238 | 80 |
| I-109 | 238 | 80 |
| I-110 | 238 | 80 |
| I-111 | 238 | 80 |
| I-112 | 238 | 80 |
| I-113 | 238 | 80 |
| I-114 | 238 | 80 |
| I-120 | 238 | 80 |
| I-121 | 238 | 80 |
| I-122 | 238 | 80 |
| I-126 | 238 | 80 |
| I-129 | 238 | 80 |

**Table B5**

| Example Number | Dosage [g/ha] | DIPTE |
|---|---|---|
| I-004 | 238 | 80 |
| I-007 | 238 | 80 |
| I-008 | 238 | 80 |
| I-023 | 238 | 80 |
| I-024 | 238 | 80 |
| I-025 | 238 | 80 |
| I-026 | 238 | 80 |
| I-027 | 238 | 80 |
| I-028 | 238 | 80 |
| I-032 | 238 | 80 |
| I-033 | 238 | 80 |
| I-034 | 238 | 80 |
| I-038 | 238 | 80 |
| I-039 | 238 | 80 |
| I-041 | 238 | 80 |
| I-042 | 238 | 80 |
| I-044 | 238 | 80 |
| I-045 | 238 | 80 |
| I-046 | 238 | 80 |
| I-048 | 238 | 80 |
| I-050 | 238 | 80 |
| I-053 | 238 | 80 |
| I-054 | 238 | 80 |
| I-061 | 238 | 80 |
| I-063 | 238 | 80 |
| I-067 | 238 | 80 |
| I-069 | 238 | 80 |
| I-071 | 238 | 80 |
| I-072 | 238 | 80 |
| I-080 | 238 | 80 |
| I-081 | 238 | 80 |
| I-085 | 238 | 80 |
| I-086 | 238 | 80 |
| I-087 | 238 | 80 |
| I-093 | 238 | 80 |
| I-097 | 238 | 80 |
| I-104 | 238 | 80 |
| I-105 | 238 | 80 |
| I-106 | 238 | 80 |
| I-108 | 238 | 80 |
| I-109 | 238 | 80 |
| I-110 | 238 | 80 |
| I-111 | 238 | 80 |
| I-113 | 238 | 80 |
| I-114 | 238 | 80 |
| I-119 | 238 | 80 |
| I-120 | 238 | 80 |
| I-126 | 238 | 80 |
| I-128 | 238 | 80 |
| I-129 | 238 | 80 |

**Table B6**

| Example Number | Dosage [g/ha] | MATCH |
|---|---|---|
| I-002 | 238 | 80 |
| I-005 | 238 | 80 |
| I-006 | 238 | 80 |
| I-008 | 238 | 80 |
| I-024 | 238 | 80 |
| I-025 | 238 | 80 |
| I-028 | 238 | 80 |
| I-038 | 238 | 80 |
| I-039 | 238 | 80 |
| I-046 | 238 | 80 |
| I-048 | 238 | 80 |
| I-049 | 238 | 80 |
| I-051 | 238 | 80 |
| I-054 | 238 | 100 |
| I-067 | 238 | 80 |
| I-069 | 238 | 80 |
| I-072 | 238 | 80 |
| I-081 | 238 | 80 |
| I-084 | 238 | 80 |
| I-085 | 238 | 80 |
| I-086 | 238 | 80 |
| I-099 | 238 | 80 |
| I-102 | 238 | 80 |
| I-105 | 238 | 80 |
| I-106 | 238 | 80 |
| I-109 | 238 | 80 |
| I-110 | 238 | 80 |
| I-111 | 238 | 80 |
| I-113 | 238 | 80 |
| I-114 | 238 | 80 |
| I-119 | 238 | 80 |
| I-120 | 238 | 80 |
| I-121 | 238 | 80 |
| I-122 | 238 | 80 |
| I-126 | 238 | 80 |

**Table B7**

| Example Number | Dosage [g/ha] | STEME |
|---|---|---|
| I-001 | 238 | 80 |
| I-003 | 238 | 80 |
| I-005 | 238 | 80 |
| I-008 | 238 | 80 |
| I-020 | 238 | 80 |
| I-024 | 238 | 80 |
| I-025 | 238 | 80 |
| I-026 | 238 | 80 |
| I-028 | 238 | 80 |
| I-033 | 238 | 80 |
| I-038 | 238 | 80 |
| I-039 | 238 | 80 |
| I-041 | 238 | 80 |
| I-045 | 238 | 80 |
| I-046 | 238 | 80 |
| I-047 | 238 | 80 |
| I-048 | 238 | 80 |
| I-049 | 238 | 80 |
| I-050 | 238 | 80 |
| I-051 | 238 | 80 |
| I-053 | 238 | 80 |
| I-054 | 238 | 80 |
| I-055 | 238 | 80 |
| I-057 | 238 | 80 |
| I-069 | 238 | 80 |
| I-072 | 238 | 80 |
| I-080 | 238 | 80 |
| I-081 | 238 | 80 |
| I-083 | 238 | 80 |
| I-084 | 238 | 80 |
| I-085 | 238 | 80 |
| I-087 | 238 | 80 |
| I-088 | 238 | 80 |
| I-093 | 238 | 80 |
| I-097 | 238 | 80 |
| I-100 | 238 | 80 |
| I-102 | 238 | 80 |
| I-105 | 238 | 80 |
| I-106 | 238 | 80 |
| I-108 | 238 | 80 |
| I-109 | 238 | 80 |
| I-110 | 238 | 80 |
| I-111 | 238 | 80 |
| I-113 | 238 | 80 |
| I-114 | 238 | 80 |
| I-115 | 238 | 80 |
| I-118 | 238 | 80 |
| I-120 | 238 | 80 |
| I-121 | 238 | 80 |
| I-122 | 238 | 80 |

**Table B8**

| Example Number | Dosage [g/ha] | VERPE |
|---|---|---|
| I-137 | 238 | 80 |
| I-002 | 238 | 80 |
| I-003 | 238 | 80 |
| I-005 | 238 | 80 |
| I-007 | 238 | 80 |
| I-008 | 238 | 80 |
| I-024 | 238 | 80 |
| I-025 | 238 | 80 |
| I-026 | 238 | 80 |
| I-027 | 238 | 80 |
| I-028 | 238 | 80 |
| I-029 | 238 | 80 |
| I-031 | 238 | 80 |
| I-032 | 238 | 80 |
| I-033 | 238 | 80 |
| I-034 | 238 | 80 |
| I-035 | 238 | 80 |
| I-038 | 238 | 80 |
| I-039 | 238 | 80 |
| I-041 | 238 | 80 |
| I-044 | 238 | 80 |
| I-046 | 238 | 80 |
| I-047 | 238 | 80 |
| I-048 | 238 | 80 |
| I-049 | 238 | 80 |
| I-051 | 238 | 80 |
| I-052 | 238 | 80 |
| I-053 | 238 | 80 |
| I-055 | 238 | 80 |
| I-056 | 238 | 80 |
| I-061 | 238 | 80 |
| I-063 | 238 | 80 |
| I-067 | 238 | 80 |
| I-069 | 238 | 80 |
| I-071 | 238 | 80 |
| I-072 | 238 | 80 |
| I-080 | 238 | 80 |
| I-083 | 238 | 80 |
| I-087 | 238 | 80 |
| I-090 | 238 | 80 |
| I-095 | 238 | 80 |
| I-097 | 238 | 80 |
| I-101 | 238 | 80 |
| I-102 | 238 | 80 |
| I-103 | 238 | 80 |
| I-104 | 238 | 80 |
| I-105 | 238 | 80 |
| I-106 | 238 | 80 |
| I-108 | 238 | 80 |
| I-109 | 238 | 80 |
| I-110 | 238 | 80 |
| I-111 | 238 | 80 |
| I-112 | 238 | 80 |
| I-113 | 238 | 80 |
| I-114 | 238 | 80 |
| I-119 | 238 | 80 |
| I-121 | 238 | 80 |
| I-126 | 238 | 80 |

As the results show, various compounds of the general formula (I) according to the invention have very good herbicidal microtiter plate efficacy against a broad spectrum of harmful mono- and dicotyledonous plants such as Agrostis capillari (AGSTE), Diplotaxis tenuifolia (DIPTE), Lolium perenne (LOLPE), Matricaria chamomilla (MATCH), Poa annua (POAAN), Setaria viridis (SETVI), Stellaria media (STEME) and Veronica persica (VERPE) at an application rate of 238 g of active ingredient per hectare.

## Claims

1. Substituted formyl tertiary alcohol derivatives of the general formula (I), salts or N-oxides thereof, 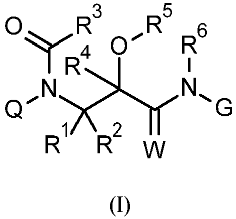 in which
Q represents an aryl or heteroaryl residue, wherein each of the previously mentioned two residues is unsubstituted or is independently substituted by one or more residues selected from the group R⁷,
R¹ and R² represent independently of one another hydrogen, halogen, cyano, (C₁-C₈)-alkyl, (C₁-C₈)-alkoxy or, together with the carbon atom to which they are attached form a (C₃-C₈)-cycloalkyl or (C₃-C₈)-cycloalkoxy ring system,
R³ represents hydrogen, amino, (C₁-C₈)-alkyl, (C₁-C₈)-haloalkyl, (C₂-C₈)-alkenyl, (C₂-C₈)-haloalkenyl, (C₂-C₈)-alkynyl, (C₂-C₈)-haloalkynyl, (C₁-C₈)-alkoxy, (C₁-C₈)-haloalkoxy, (C₁-C₈)-alkoxy-(C₁-C₈)-alkyl, (C₁-C₈)-haloalkoxy-(C₁-C₈)-alkyl, (C₁-C₈)-alkoxy-(C₃-C₈)-cycloalkyl, (C₁-C₈)-haloalkoxy-(C₃-C₈)-cycloalkyl, (C₃-C₈)-cycloalkyl, (C₃-C₈)-halocycloalkyl, (C₃-C₈)-cycloalkoxy, (C₃-C₈)-halocycloalkoxy, (C₁-C₈)-alkylamino, (C₂-C₁₂)-dialkylamino, amino-C(O)NR⁹R¹⁰, (C₁-C₈)-alkylamino-C(O)NR⁹R¹⁰, or represents phenoxy, unsubstituted or independently substituted by one to five residues selected from halogen, (C₁-C₈)-alkyl, (C₁-C₈)-haloalkyl, (C₁-C₈)-alkoxy, (C₁-C₈)-haloalkoxy or (C₃-C₈)-cycloalkyl,
R⁴ represents hydrogen, halogen, cyano, (C₁-C₈)-alkyl, (C₁-C₈)-haloalkyl, (C₂-C₈)-alkenyl, (C₂-C₈)-haloalkenyl, (C₂-C₈)-alkynyl, (C₂-C₈)-haloalkynyl, (C₁-C₈)-alkoxy, (C₁-C₈)-haloalkoxy, (C₃-C₈)-cycloalkyl or (C₃-C₈)-halocycloalkyl,
R⁵ represents hydrogen, (C₁-C₈)-alkyl, (C₁-C₈)-haloalkyl, (C₂-C₈)-alkenyl, (C₂-C₈)-haloalkenyl, (C₂-C₈)-alkynyl, (C₂-C₈)-haloalkynyl, (C₃-C₈)-cycloalkyl, (C₃-C₈)-halocycloalkyl, -C(O)R⁸, - C(O)OR⁸, -S(O)OR⁸, -CONR⁹R¹⁰ or -S(O)₂NR⁹R¹⁰, or represents phenyl-(C₁-C₈)-carbonyl, unsubstituted or independently substituted by one to five residues selected from halogen, cyano, (C₁-C₈)-alkyl, (C₁-C₈)-haloalkyl, (C₁-C₈)-alkoxy, (C₁-C₈)-haloalkoxy or (C₃-C₈)-cycloalkyl,
R⁶ represents hydrogen, (C₁-C₈)-alkyl, (C₁-C₈)-haloalkyl, (C₁-C₈)-hydroxyalkyl, (C₁-C₈)-hydroxyhaloalkyl, (C₃-C₈)-cycloalkyl, (C₃-C₈)-halocycloalkyl, (C₁-C₈)-cyanoalkyl, (C₃-C₈)-cycloalkyl-(C₁-C₈)-alkyl, (C₂-C₈)-alkenyl, (C₂-C₈)-haloalkenyl, (C₂-C₈)-alkynyl, (C₂-C₈)-halo alkynyl, (C₁-C₈)-alkoxy, (C₁-C₈)-haloalkoxy, (C₁-C₈)-alkylcarbonyl, arylcarbonyl, (C₁-C₈)-alkyloxycarbonyl or aryloxycarbonyl,
R⁷ represents halogen, nitro, cyano, (C₁-C₈)-alkyl, (C₁-C₈)-haloalkyl, (C₂-C₈)-alkenyl, (C₂-C₈)-haloalkenyl, (C₂-C₈)-alkynyl, (C₂-C₈)-haloalkynyl, (C₁-C₈)-alkoxy, (C₁-C₈)-haloalkoxy, (C₁-C₈)-alkylthio, (C₁-C₈)-haloalkylthio, (C₁-C₈)-alkylsulfinyl, (C₁-C₈)-haloalkylsulfinyl, (C₁-C₈)-alkylsulfonyl, (C₁-C₈)-haloalkylsulfonyl, (C₃-C₈)-cycloalkyl, (C₃-C₈)-halocycloalkyl, (C₃-C₈)-cycloalkoxy, (C₃-C₈)-halocycloalkoxy, (C₃-C₈)-cycloalkylthio, (C₃-C₈)-halocycloalkylthio, (C₃-C₈)-cycloalkylsulfinyl, (C₃-C₈)-halocycloalkylsulfinyl, (C₃-C₈)-cycloalkylsulfonyl, (C₃-C₈)-halocycloalkylsulfonyl, (C₃-C₈)-cycloalkyl-(C₁-C₈)-alkyl or (C₁-C₈)-alkyl-(C₃-C₈)-cycloalkyl,
W represents oxygen or sulfur,
G represents a residue selected from G1-G25 shown in the table below, wherein the selected residue is substituted by a residue selected from the group Y,
| | | | | |
|---|---|---|---|---|
| 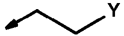 | 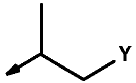 | 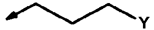 | 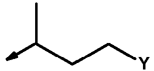 | 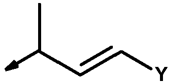 |
| G-1 | G-2 | G-3 | G-4 | G-5 |
| 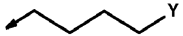 | 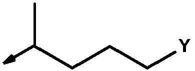 | 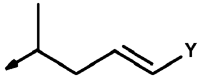 | 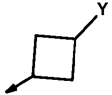 | 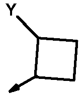 |
| G-6 | G-7 | G-8 | G-9 | G-10 |
| 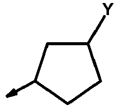 | 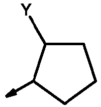 | 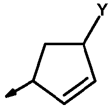 | 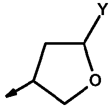 | 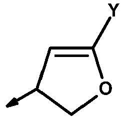 |
| G-11 | G-12 | G-13 | G-14 | G-15 |
| 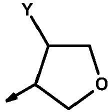 | 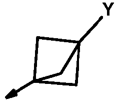 | 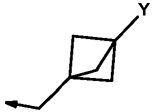 | 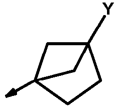 | 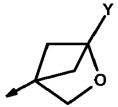 |
| G-16 | G-17 | G-18 | G-19 | G-20 |
| 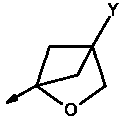 | 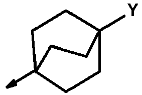 | 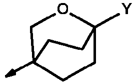 | 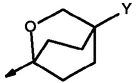 | 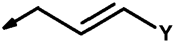 |
| G-21 | G-22 | G-23 | G-24 | G-25 |
where the arrow represents a bond to the nitrogen atom of formula (I)
Y represents -C(O)OR⁸, -C(O)NR⁹R¹⁰, -C(S)NR⁹R¹⁰, -C(O)N(R⁹)S(O)₂R¹¹ or - C(O)N(R⁹)S(O)₂NR⁹R¹⁰,
R⁸, R⁹, R¹⁰ and R¹¹ represent the same or different substituents selected independently from eachother from hydrogen, (C₁-C₈)-alkyl, (C₂-C₈)-alkenyl, (C₂-C₈)-alkynyl, (C₁-C₈)-cyanoalkyl, (C₁-C₈)-haloalkyl, (C₂-C₈)-haloalkenyl, (C₂-C₈)-haloalkynyl, (C₃-C₈)-cycloalkyl, (C₃-C₈)-halocycloalkyl, (C₄-C₈)-cycloalkenyl, (C₄-C₈)-halocycloalkenyl, (C₁-C₈)-alkoxy, (C₁-C₈)-haloalkoxy, (C₁-C₈)-alkoxy-(C₁-C₈)-alkyl, (C₁-C₈)-haloalkoxy-(C₁-C₈)-alkyl, (C₁-C₈)-alkylthio-(C₁-C₈)-alkyl, (C₁-C₈)-alkylS(O)ₓ-(C₁-C₈)-alkyl, (C₁-C₈)-haloalkylthio-(C₁-C₈)-alkyl, (C₁-C₈)-alkoxy-(C₁-C₈)-haloalkyl, aryl, aryl-(C₁-C₈)-alkyl, heteroaryl, heteroaryl-(C₁-C₈)-alkyl, (C₃-C₈)-cycloalkyl-(C₁-C₄)-alkyl, (C₄-C₈)-cycloalkenyl-(C₁-C₈)-alkyl, heterocyclyl, bis-[(C₁-C₈)-alkyl]aminocarbonyl-(C₁-C₈)-alkyl, (C₁-C₈)-alkyl-amino-carbonyl-(C₁-C₈)-alkyl, aryl-(C₁-C₈)-alkyl-aminocarbonyl-(C₁-C₈)-alkyl or heterocyclyl-(C₁-C₈)-alkyl,
R⁹ and R¹⁰ can represent, with the nitrogen atom to which they are attached, a fully saturated or partially saturated, optionally interrupted by heteroatoms and optionally further substituted 3- to 10-membered monocyclic or bicyclic ring,
and
x represents 0, 1 or 2.

2. Compounds of the general formula (I) as claimed in claim 1, salts or N-oxides thereof, in which
Q represents an aryl or heteroaryl residue, wherein each of the previously mentioned two residues is unsubstituted or is independently substituted by one or more residues selected from the group R⁷,
R¹ and R² represent independently of one another hydrogen, halogen, cyano, (C₁-C₄)-alkyl, (C₁-C₄)-alkoxy or, together with the carbon atom to which they are attached form a (C₃-C₆)-cycloalkyl or (C₃-C₆)-cycloalkoxy ring system,
R³ represents hydrogen, amino, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₁-C₄)-alkoxy, (C₁-C₄)-haloalkoxy, (C₁-C₄)-alkoxy-(C₁-C₄)-alkyl, (C₁-C₄)-haloalkoxy-(C₁-C₄)-alkyl, (C₁-C₄)-alkoxy-(C₃-C₆)-cycloalkyl, (C₁-C₄)-haloalkoxy-(C₃-C₆)-cycloalkyl, (C₃-C₆)-cycloalkyl, (C₃-C₆)-halocycloalkyl, (C₃-C₆)-cycloalkoxy, (C₃-C₆)-halocycloalkoxy, (C₁-C₄)-alkylamino, (C₂-C₈)-dialkylamino, amino-C(O)NR⁹R¹⁰, (C₁-C₄)-alkylamino-C(O)NR⁹R¹⁰, or represents phenoxy, unsubstituted or independently substituted by one to five residues selected from halogen, (C₁-C₄)-alkyl, (C₁-C₄)-haloalkyl, (C₁-C₄)-alkoxy, (C₁-C₄)-haloalkoxy or (C₃-C₆)-cycloalkyl,
R⁴ represents hydrogen, halogen, cyano, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₁-C₄)-alkoxy, (C₁-C₄)-haloalkoxy, (C₃-C₆)-cycloalkyl or (C₃-C₆)-halocycloalkyl,
R⁵ represents hydrogen, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₃-C₆)-cycloalkyl, (C₃-C₆)-halocycloalkyl, -C(O)R⁸, - C(O)OR⁸, -S(O)OR⁸, -CONR⁹R¹⁰ or -S(O)₂NR⁹R¹⁰, or represents phenyl-(C₁-C₄)-carbonyl, unsubstituted or independently substituted by one to five residues selected from halogen, cyano, (C₁-C₄)-alkyl, (C₁-C₄)-haloalkyl, (C₁-C₄)-alkoxy, (C₁-C₄)-haloalkoxy or (C₃-C₆)-cycloalkyl,
R⁶ represents hydrogen, (C₁-C₄)-alkyl, (C₁-C₄)-haloalkyl, (C₁-C₄)-hydroxyalkyl, (C₁-C₄)-hydroxyhaloalkyl, (C₃-C₆)-cycloalkyl, (C₃-C₆)-halocycloalkyl, (C₁-C₄)-cyanoalkyl, (C₃-C₆)-cycloalkyl-(C₁-C₄)-alkyl, (C₂-C₄)-alkenyl, (C₂-C₄)-haloalkenyl, (C₂-C₄)-alkynyl, (C₂-C₄)-halo alkynyl, (C₁-C₄)-alkoxy, (C₁-C₄)-haloalkoxy, (C₁-C₄)-alkylcarbonyl, arylcarbonyl, (C₁-C₄)-alkyloxycarbonyl or aryloxycarbonyl,
R⁷ represents halogen, nitro, cyano, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₁-C₄)-alkoxy, (C₁-C₄)-haloalkoxy, (C₁-C₄)-alkylthio, (C₁-C₄)-haloalkylthio, (C₁-C₄)-alkylsulfinyl, (C₁-C₄)-haloalkylsulfinyl, (C₁-C₄)-alkylsulfonyl, (C₁-C₄)-haloalkylsulfonyl, (C₃-C₆)-cycloalkyl, (C₃-C₆)-halocycloalkyl, (C₃-C₆)-cycloalkoxy, (C₃-C₆)-halocycloalkoxy, (C₃-C₆)-cycloalkylthio, (C₃-C₆)-halocycloalkylthio, (C₃-C₆)-cycloalkylsulfinyl, (C₃-C₆)-halocycloalkylsulfinyl, (C₃-C₆)-cycloalkylsulfonyl, (C₃-C₆)-halocycloalkylsulfonyl, (C₃-C₆)-cycloalkyl-(C₁-C₄)-alkyl or (C₁-C₄)-alkyl-(C₃-C₆)-cycloalkyl, W represents oxygen or sulfur,
G represents a residue selected from G1-G25 shown in the table below, wherein the selected residue is substituted by a residue selected from the group Y,
| | | | | |
|---|---|---|---|---|
| 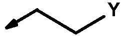 | 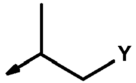 | 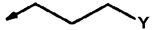 | 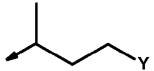 | 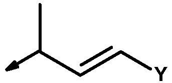 |
| G-1 | G-2 | G-3 | G-4 | G-5 |
| 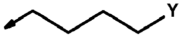 | 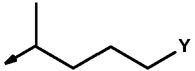 | 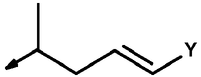 | 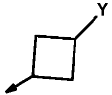 | 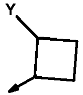 |
| G-6 | G-7 | G-8 | G-9 | G-10 |
| 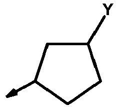 | 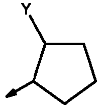 | 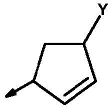 | 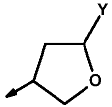 | 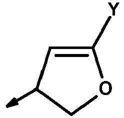 |
| G-11 | G-12 | G-13 | G-14 | G-15 |
| 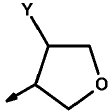 | 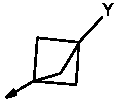 | 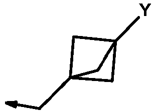 | 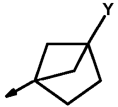 | 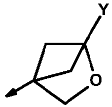 |
| G-16 | G-17 | G-18 | G-19 | G-20 |
| 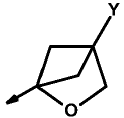 | 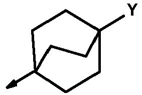 | 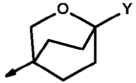 | 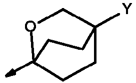 | 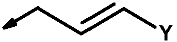 |
| G-21 | G-22 | G-23 | G-24 | G-25 |
where the arrow represents a bond to the nitrogen atom of formula (I)
Y represents -C(O)OR⁸, -C(O)NR⁹R¹⁰, -C(S)NR⁹R¹⁰, -C(O)N(R⁹)S(O)₂R¹¹ or - C(O)N(R⁹)S(O)₂NR⁹R¹⁰,
R⁸, R⁹, R¹⁰ and R¹¹ represent the same or different substituents selected independently from eachother from hydrogen, (C₁-C₄)-alkyl, (C₂-C₄)-alkenyl, (C₂-C₄)-alkynyl, (C₁-C₄)-cyanoalkyl, (C₁-C₄)-haloalkyl, (C₂-C₄)-haloalkenyl, (C₂-C₄)-haloalkynyl, (C₃-C₆)-cycloalkyl, (C₃-C₆)-halocycloalkyl, (C₄-C₆)-cycloalkenyl, (C₄-C₆)-halocycloalkenyl, (C₁-C₄)-alkoxy, (C₁-C₄)-haloalkoxy, (C₁-C₄)-alkoxy-(C₁-C₄)-alkyl, (C₁-C₄)-haloalkoxy-(C₁-C₄)-alkyl, (C₁-C₄)-alkylthio-(C₁-C₄)-alkyl, (C₁-C₄)-alkylS(O)ₓ-(C₁-C₄)-alkyl, (C₁-C₄)-haloalkylthio-(C₁-C₄)-alkyl, (C₁-C₄)-alkoxy-(C₁-C₄)-haloalkyl, aryl, aryl-(C₁-C₄)-alkyl, heteroaryl, heteroaryl-(C₁-C₄)-alkyl, (C₃-C₆)-cycloalkyl-(C₁-C₄)-alkyl, (C₄-C₆)-cycloalkenyl-(C₁-C₄)-alkyl, heterocyclyl, bis-[(C₁-C₄)-alkyl]aminocarbonyl-(C₁-C₄)-alkyl, (C₁-C₄)-alkyl-amino-carbonyl-(C₁-C₄)-alkyl, aryl-(C₁-C₄)-alkyl-aminocarbonyl-(C₁-C₄)-alkyl or heterocyclyl-(C₁-C₄)-alkyl,
R⁹ and R¹⁰ can represent, with the nitrogen atom to which they are attached, a fully saturated or partially saturated, optionally interrupted by heteroatoms and optionally further substituted 3- to 10-membered monocyclic or bicyclic ring,
and
x represents 0, 1 or 2.

3. Compound of the general formula (I) as claimed in claim 1 or 2 salts or N-oxides thereof, in which
Q represents a phenyl or heteroaryl residue, wherein each of the previously mentioned two residues is unsubstituted or is independently substituted by one or more residues selected from the group R⁷,
R¹ and R² represent independently of one another hydrogen, halogen, cyano, (C₁-C₄)-alkyl, (C₁-C₄)-alkoxy or, together with the carbon atom to which they are attached form a (C₃-C₆)-cycloalkyl or (C₃-C₆)-cycloalkoxy ring system,
R³ represents hydrogen, amino, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₁-C₄)-alkoxy, (C₁-C₄)-haloalkoxy, (C₁-C₄)-alkoxy-(C₁-C₄)-alkyl, (C₁-C₄)-haloalkoxy-(C₁-C₄)-alkyl, (C₁-C₄)-alkoxy-(C₃-C₆)-cycloalkyl, (C₃-C₆)-cycloalkyl, (C₃-C₆)-halocycloalkyl, (C₃-C₆)-cycloalkoxy, (C₃-C₆)-halocycloalkoxy, (C₁-C₄)-alkylamino, (C₂-C₈)-dialkylamino, amino-C(O)NR⁹R¹⁰, (C₁-C₄)-alkylamino-C(O)NR⁹R¹⁰,
R⁴ represents hydrogen, halogen, cyano, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₁-C₄)-alkoxy, (C₁-C₄)-haloalkoxy, (C₃-C₆)-cycloalkyl or (C₃-C₆)-halocycloalkyl,
R⁵ represents hydrogen, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₃-C₆)-cycloalkyl, (C₃-C₆)-halocycloalkyl,
R⁶ represents hydrogen, (C₁-C₄)-alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₃-C₆)-cycloalkyl, (C₃-C₆)-halocycloalkyl,
R⁷ represents nitro, cyano, (C₁-C₄)-alkyl, (C₁-C₄)-haloalkyl,
W represents oxygen or sulfur,
G represents a residue selected from G1-G25 shown in the table below, wherein the selected residue is substituted by a residue selected from the group Y,
| | | | | |
|---|---|---|---|---|
| 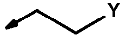 | 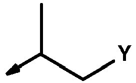 | 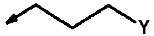 | 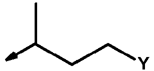 | 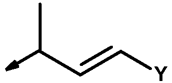 |
| G-1 | G-2 | G-3 | G-4 | G-5 |
| 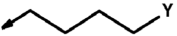 | 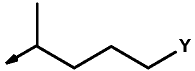 | 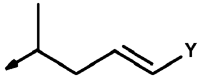 | 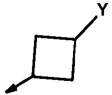 | 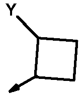 |
| G-6 | G-7 | G-8 | G-9 | G-10 |
| 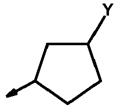 | 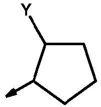 | 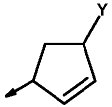 | 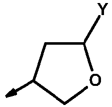 | 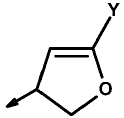 |
| G-11 | G-12 | G-13 | G-14 | G-15 |
| 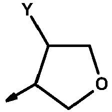 | 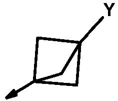 | 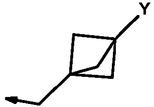 | 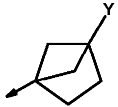 | 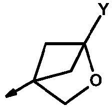 |
| G-16 | G-17 | G-18 | G-19 | G-20 |
| 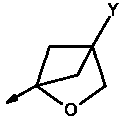 | 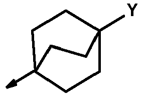 | 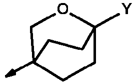 | 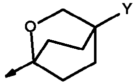 | 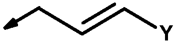 |
| G-21 | G-22 | G-23 | G-24 | G-25 |
where the arrow represents a bond to the nitrogen atom of formula (I)
Y represents -C(O)OR⁸, -C(O)NR⁹R¹⁰, -C(S)NR⁹R¹⁰,
R⁸, R⁹and R¹⁰ represent the same or different substituents selected independently from each other from hydrogen, (C₁-C₄)-alkyl, (C₂-C₄)-alkenyl, (C₂-C₄)-alkynyl, (C₃-C₆)-cycloalkyl,
and
R⁹ and R¹⁰ can represent, with the nitrogen atom to which they are attached, a fully saturated or partially saturated, optionally interrupted by heteroatoms and optionally further substituted 3- to 6-membered monocyclic or bicycle ring.

4. Compounds of the general formula (I) as claimed in any of claims 1 to 3, salts or N-oxides thereof, in which
Q represents phenyl which is unsubstituted or independently substituted by one or more residues selected from the group R⁷,
R¹ and R² represent independently of one another hydrogen, fluoro, chloro, cyano, methyl, ethyl, methoxy, ethoxy,
R³ represents hydrogen, amino, methyl, ethyl, propyl, chloromethyl, fluoromethyl, difluoromethyl, trifluoromethyl, cyclopropyl, methoxy, ethoxy, propoxy, methoxymethyl, ethoxymethyl, methoxyethyl, ethoxyethyl, methoxypropyl, methylamino, ethylamino, propylamino, dimethylamino, methylethylamino, vinyl, 1-propenyl, 2-propenyl, ethinyl or ureido,
R⁴ represents hydrogen, halogen fluoro, chloro, cyano, methyl, ethyl, propyl, chloromethyl, fluoromethyl, difluoromethyl, trifluoromethyl, vinyl, 1-propenyl, 2-propenyl, ethinyl, cyclopropyl, methoxy, ethoxy, propoxy,
R⁵ represents hydrogen, methyl, ethyl, cyclopropyl, vinyl, 1-propenyl, 2-propenyl, ethinyl,
R⁶ represents hydrogen, methyl, ethyl, cyclopropyl, vinyl, 1-propenyl, 2-propenyl, ethinyl,
R⁷ represents fluoro, chloro, nitro, cyano, methyl, ethyl,
W represents oxygen or sulfur,
G represents a residue selected from G1-G25 shown in the table below, wherein the selected residue is substituted by a residue selected from the group Y,
| | | | | |
|---|---|---|---|---|
| 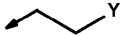 | 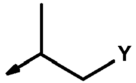 | 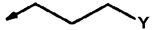 | 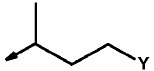 | 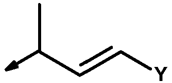 |
| G-1 | G-2 | G-3 | G-4 | G-5 |
| 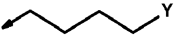 | 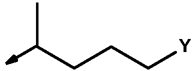 | 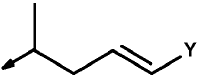 | | |
| G-6 | G-7 | G-8 | | |
| 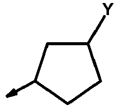 | 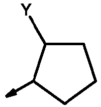 | 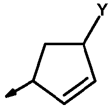 | 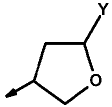 | 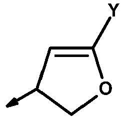 |
| G-11 | G-12 | G-13 | G-14 | G-15 |
| 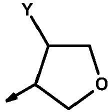 | | | | |
| G-16 | | | | |
| | | | | 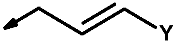 |
| | | | | G-25 |
where the arrow represents a bond to the nitrogen atom of formula (I)
Y represents -C(O)OR⁸, -C(O)NR⁹R¹⁰, -C(S)NR⁹R¹⁰, -C(O)N(R⁹)S(O)₂R¹¹ or - C(O)N(R⁹)S(O)₂NR⁹R¹⁰,
R⁸, R⁹, R¹⁰ and R¹¹ represent the same or different substituents selected independently from each other from hydrogen, methyl, ethyl, cyclopropyl, vinyl, 1-propenyl, 2-propenyl, ethinyl.

5. Compound of the general formula (I) as claimed in any of claims 1 to 4, salts or N-oxides thereof, in which
Q represents 3,5-difluorophenyl, 3,5-dichlorophenyl or 3-fluoro-5-methyl-phenyl,
R¹ represents hydrogen,
R² represents hydrogen,
R³ represents hydrogen, methyl, ethyl, cyclopropyl, chloromethyl, methoxy, ethoxy, methoxymethyl, amino, methylamino, dimethylamino or ureido,
R⁴ represents methyl, ethyl, fluoromethyl, cyclopropyl or vinyl,
R⁵ represents hydrogen,
R⁶ represents hydrogen,
W represents oxygen,
G rac-(3S,5R)-5-methoxycarbonyltetrahydrofuran-3-yl, (1S,4S)-4-methoxycarbonylcyclopent-2-en-1-yl, (E,1R)-4-ethoxy-1-methyl-4-oxo-but-2-enyl, (1R)-3-ethoxy-1-methyl-3-oxo-propyl, (1R)-4-methoxy-1-methyl-4-oxo-butyl, (1R,3S)-3-methoxycarbonylcyclopentyl, (E,1R)-4-methoxy-1-methyl-4-oxo-but-2-enyl or (1S,4S)-4-carboxycyclopent-2-en-1-yl.

6. A herbicidal composition or plant growth-regulating composition, **characterized in that** it comprises one or more compounds of the general formula (I), salts or N-oxide thereof as claimed in any of claims 1 to 5.

7. The herbicidal composition as claimed in claim 6, further comprising a formulation auxiliary.

8. The herbicidal composition as claimed in claim 6 or 7, comprising at least one further active compound from the group of insecticides, acaricides, herbicides, fungicides, safeners and/or growth regulators.

9. The herbicidal composition as claimed in claim 6, 7 or 8, comprising a safener.

10. The herbicidal composition as claimed in claim 9, in which the safener is selected from the group consisting of mefenpyr-diethyl, cyprosulfamide, isoxadifen-ethyl, cloquintocet-mexyl, benoxacor and dichlormid.

11. A method of controlling unwanted plants, **characterized in that** an effective amount of at least one compound of the formula (I) as claimed in any of claims 1 to 5 or of a herbicidal composition as claimed in any of claims 6 to 10 is applied to the plants or to the site of the unwanted vegetation.

12. The use of compounds of the formula (I) as claimed in any of claims 1 to 5 or of herbicidal compositions as claimed in any of claims 6 to 10 for controlling unwanted plants.

13. The use as claimed in claim 12, **characterized in that** the compounds of the formula (I) are used for controlling unwanted plants in crops of useful plants.

14. The use as claimed in claim 13, **characterized in that** the useful plants are transgenic useful plants.
